(19) Europäisches Patentamt / European Patent Office / Office européen des brevets

(11) **EP 2 230 310 B1**

(12) **EUROPEAN PATENT SPECIFICATION**

(45) Date of publication and mention
of the grant of the patent:
**16.01.2013   Bulletin 2013/03**

(51) Int Cl.:
**C07K 14/415** (2006.01)   **C12N 15/82** (2006.01)

(21) Application number: **09178578.2**

(22) Date of filing: **02.05.2008**

(54) **Plants having enhanced yield-related traits and a method for making the same**

Pflanzen mit verbesserten Eigenschaften bezüglich des Ertrags und Verfahren zu ihrer Herstellung

Plantes dotées de caractéristiques de rendement améliorées et procédé de fabrication de celles-ci

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **05.07.2007   US 948036 P**
**29.06.2007   US 937989 P**
**06.06.2007   US 942214 P**
**08.05.2007   US 916575 P**
**19.06.2007   EP 07110557**
**29.05.2007   EP 07109068**
**19.06.2007   EP 07110548**
**29.05.2007   EP 07109052**
**11.06.2007   EP 07109961**
**03.05.2007   EP 07107448**

(43) Date of publication of application:
**22.09.2010   Bulletin 2010/38**

(62) Document number(s) of the earlier application(s) in accordance with Art. 76 EPC:
**08767542.7 / 2 069 509**

(73) Proprietor: **BASF Plant Science GmbH**
**67056 Ludwigshafen (DE)**

(72) Inventors:
• **Hatzfeld, Yves**
**59000 Lille (FR)**
• **Sanz Molinero, Ana Isabel**
**9050 Gentbrugge (BE)**
• **Shirley, Amber**
**Durham, NC 27703 (US)**
• **Darnielle, Lalitree**
**Durham, NC 27707 (US)**
• **Frankard, Valerie**
**1410 Waterloo (BE)**
• **Vandenabeele, Steven**
**9700 Oudenaarde (BE)**
• **McKersie, Bryan**
**Raleigh, NC 27617 (US)**

(74) Representative: **Mistry, Meeta et al**
**Greaves Brewster LLP**
**Copa House**
**Station Road**
**Cheddar, BS27 3AH (GB)**

(56) References cited:
• YI KEKE ET AL: "OsPTF1, a novel transcription factor involved in tolerance to phosphate starvation in rice" PLANT PHYSIOLOGY, AMERICAN SOCIETY OF PLANT PHYSIOLOGISTS, ROCKVILLE, MD, US LNKD-DOI:10.1104/PP.105.063115, vol. 138, no. 4, 1 August 2005 (2005-08-01), pages 2087-2096, XP002400487 ISSN: 0032-0889
• KIRIBUCHI K ET AL: "RERJ1, a jasmonic acid-responsive gene from rice, encodes a basic helix-loop-helix protein" BIOCHEMICAL AND BIOPHYSICAL RESEARCH COMMUNICATIONS, ACADEMIC PRESS INC. ORLANDO, FL, US LNKD- DOI:10.1016/J.BBRC.2004.10.126, vol. 325, no. 3, 17 December 2004 (2004-12-17), pages 857-863, XP004635504 ISSN: 0006-291X
• YUAN YOU XI ET AL: "AtbHLH29 of Arabidopsis thaliana is a functional ortholog of tomato FER involved in controlling iron acquisition in strategy I plants" CELL RESEARCH, vol. 15, no. 8, August 2005 (2005-08), pages 613-621, XP002594934 ISSN: 1001-0602

**(Cont. next page)**

EP 2 230 310 B1

- BUCK MICHAEL J ET AL: "Phylogenetic analysis of plant basic helix-loop-helix proteins." JOURNAL OF MOLECULAR EVOLUTION, vol. 56, no. 6, June 2003 (2003-06), pages 742-750, XP002594935 ISSN: 0022-2844
- HEIM MARC A ET AL: "The basic helix-loop-helix transcription factor family in plants: a genome-wide study of protein structure and functional diversity" MOLECULAR BIOLOGY AND EVOLUTION, THE UNIVERSITY OF CHICAGO PRESS, US LNKD- DOI: 10.1093/MOLBEV/MSG088, vol. 20, no. 5, 1 May 2003 (2003-05-01), pages 735-747, XP002435312 ISSN: 0737-4038
- LI XIAOXING ET AL: "Genome-wide analysis of basic/helix-loop-helix transcription factor family in rice and Arabidopsis" PLANT PHYSIOLOGY (ROCKVILLE), vol. 141, no. 4, August 2006 (2006-08), pages 1167-1184, XP002594936 ISSN: 0032-0889

**Description**

The present invention relates generally to the field of molecular biology and concerns a method for increasing seed yield in plants by increasing expression in a plant of a nucleic acid encoding a bHLH11-like (basic Helix-Loop-Helix 11) protein under the control of a medium strength constitutive promoter. The present invention also concerns plants having increased expression of a nucleic acid encoding a bHLH11-like protein, which plants have increased seed yield relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modem rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigor has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defense mechanisms.

Surprisingly, it has now been found that increasing expression of a nucleic acid encoding a bHLH11-like (basic Helix-Loop-Helix 11) protein, which nucleic acid is under the control of a medium strength constitutive promoter, gives plants having increased seed yield relative to control plants.

According one embodiment, there is provided a method for increasing plant seed yield relative to control plants, comprising increasing expression in a plant of a nucleic acid encoding a bHLH11-like (basic Helix-Loop-Helix 11) protein which nucleic acid is under the control of a medium strength constitutive promoter.

**Background**

**bHLH11-like (basic Helix-Loop-Helix 11) protein**

Transcription factors are usually defined as proteins that show sequence-specific DNA binding and that are capable of activating and/or repressing transcription. The basic Helix-Loop-Helix transcription factor family is one of the largest families of transcription factors that have been characterised in *Arabidopsis thaliana* (Toledo-Ortiz et al., Plant Cell 15, 1749-1770, 2003; Bailey et al., Plant Cell 15, 2497-2501, 2003) and in rice (Li et al Plant Physiol. 141, 1167-1184, 2006). The distinguishing characteristic of the bHLH transcription factor family is the presence of a bipartite domain consisting of approximately 60 amino acids. This bipartite domain is comprised of a DNA-binding basic region, which binds to a consensus hexanucleotide E-box and two $\alpha$-helices separated by a variable loop region, located C-terminally of the basic domain. The two $\alpha$-helices promote dimerisation, allowing the formation of homo- and heterodimers between different family members. While the bHLH domain is evolutionarily conserved, there is little sequence similarity between clades beyond the domain. Li et al. (2006) classify the rice and Arabidopsis bHLH transcription factors into 22 subfamilies, based on the sequence of the bHLH domains.

Little is known about the function of bHLH11-like polypeptides in plants. So far, only one bHLH11-like polypeptide, OsPTF1 from rice, has been characterised. OsPTF1 is reported to be involved in tolerance to phosphate starvation (Yi et al., Plant Physiol. 138, 2087-2096). Rice plants overexpressing this gene under control of the 35S promoter did not show any different phenotype compared to control plants when grown under normal conditions, but under conditions of phosphate limitation, the plants had an improved phosphate uptake. Under phosphate limitation, the transgenic plants showed an increase in biomass, phosphate content, increased tillering and increased seed yield.

Surprisingly, it has now been found that increasing expression in a plant of a nucleic acid encoding a bHLH11-like polypeptide under the control of a medium strength constitutive promoter gives plants having increased seed yield relative to control plants. These effects were shown under growth conditions where phosphate was not limiting.

According one embodiment, there is provided a method for increasing plant seed yield relative to control plants, comprising increasing expression in a plant of a nucleic acid encoding a bHLH11-tike polypeptide- under the control of a medium strength constitutive promoter.

**Definitions**

Polypeptide(s)/Protein(s)

The terms "polypeptide" and "protein" are used interchangeably herein and refer to amino acids in a polymeric form of any length, linked together by peptide bonds.

Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break α-helical structures or β-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

Table 1: Examples of conserved amino acid substitutions

| Residue | Conservative Substitutions | Residue | Conservative Substitutions |
|---------|---------------------------|---------|---------------------------|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

Motif/Consensus sequence/Signature

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitrocellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1 °C per % base mismatch. The Tm may be calculated using the following equations, depending on the types of hybrids:

1) DNA-DNA hybrids (Meinkoth and Wahl, Anal. Biochem., 138: 267-284, 1984):

$$T_m= 81.5°C + 16.6 \times \log_{10}[Na^+]^a + 0.41 \times \%[G/C^b] - 500 \times [L^c]^{-1} - 0.61 \times \% \text{ formamide}$$

2) DNA-RNA or RNA-RNA hybrids:

$$T_m= 79.8 + 18.5 (\log_{10}[Na^+]^a) + 0.58 (\%G/C^b) + 11.8 (\%G/C^b)^2 - 820/L^c$$

3) oligo-DNA or oligo-RNA$^d$ hybrids:

For <20 nucleotides: $T_m= 2 (I_n)$
For 20-35 nucleotides: $T_m= 22 + 1.46 (I_n)$

[a] or for other monovalent cation, but only accurate in the 0.01-0.4 M range.

[b] only accurate for %GC in the 30% to 75% range.

[c] L = length of duplex in base pairs.

[d] oligo, oligonucleotide; $I_n$, = effective length of primer = $2 \times$(no. of G/C)+(no. of A/T).

Non-specific binding may be controlled using any one of a number of known techniques such as, for example, blocking the membrane with protein containing solutions, additions of heterologous RNA, DNA, and SDS to the hybridization buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. $1 \times$SSC is 0.15M NaCl and 15mM sodium citrate; the hybridisation solution and wash solutions may additionally include 5x Denhardt's reagent, 0.5-1.0% SDS, 100 $\mu$g/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position.

Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a:** Examples of constitutive promoters

| Gene Source | Reference |
| --- | --- |
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| ocs | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

**Table 2b:** Examples of root-specific promoters

| Gene Source | Reference |
|---|---|
| RCc3 | Plant Mol Biol. 1995 Jan;27(2):237-48 |
| Arabidopsis PHT1 | Kovama et al., 2005; Mudge et al. (2002, Plant J. 31:341) |
| Medicago phosphate transporter | Xiao et al., 2006 |
| Arabidopsis Pyk10 | Nitz et al. (2001) Plant Sci 161(2): 337-346 |
| root-expressible genes | Tingey et al., EMBO J. 6: 1, 1987. |
| tobacco auxin-inducible gene | Van der Zaal et al., Plant Mol. Biol. 16, 983, 1991. |
| β-tubulin | Oppenheimer, et al., Gene 63: 87, 1988. |
| tobacco root-specific genes | Conkling, et al., Plant Physiol. 93: 1203, 1990. |
| B. napus G1-3b gene | United States Patent No. 5, 401, 836 |
| SbPRP1 | Suzuki et al., Plant Mol. Biol. 21: 109-119, 1993. |
| LRX1 | Baumberger et al. 2001, Genes & Dev. 15:1128 |
| BTG-26 Brassica napus | US 20050044585 |
| LeAMT1 (tomato) | Lauter et al. (1996, PNAS 3:8139) |
| The LeNRT1-1 (tomato) | Lauter et al. (1996, PNAS 3:8139) |
| class I patatin gene (potato) | Liu et al., Plant Mol. Biol. 153:386-395, 1991. |
| KDC1 (Daucus carota) | Downey et al. (2000, J. Biol. Chem. 275:39420) |
| TobRB7 gene | W Song (1997) PhD Thesis, North Carolina State University, Raleigh, NC USA |
| OsRAB5a (rice) | Wang et al. 2002, Plant Sci. 163:273 |
| ALF5 (Arabidopsis) | Diener et al. (2001, Plant Cell 13:1625) |
| NRT2;1 Np (N. plumbaginifolia) | Quesada et al. (1997, Plant Mol. Biol. 34:265) |

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters (endosperm/aleurone/embryo specific) are shown in Table 2d, 2e, 2f. Further examples of seed-specific promoters are given in Qing Qu and Takaiwa (Plant Biotechnol. J. 2, 113-125, 2004), which disclosure is incorporated by reference herein as if fully set forth.

**Table 2c:** Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |

(continued)

| Gene source | Reference |
| --- | --- |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat $\alpha$, $\beta$, $\gamma$-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice $\alpha$-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice $\alpha$-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum $\alpha$-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | unpublished |
| PRO0147, trypsin inhibitor ITR1 (barley) | unpublished |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |
| $\alpha$-amylase (Amy32b) | Lanahan et al, Plant Cell 4:203-211, 1992; Skriver et al, Proc Natl Acad Sci USA 88:7266-7270, 1991 |
| cathepsin $\beta$-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 2d:** examples of endosperm-specific promoters

| Gene source | Reference |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 2e:** Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 2f:** Examples of aleurone-specific promoters:

| Gene source | Reference |
|---|---|
| α-amylase (Amy32b) | Lanahan et al, Plant Cell 4:203-211, 1992; Skriver et al, Proc Natl Acad Sci USA 88: 7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g.

Table 2g: Examples of green tissue-specific promoters

| Gene | Expression | Reference |
|---|---|---|
| Maize Orthophosphate dikinase | Leaf specific | Fukayama et al., 2001 |
| Maize Phosphoenolpyruvate carboxylase | Leaf specific | Kausch et al., 2001 |
| Rice Phosphoenolpyruvate carboxylase | Leaf specific | Liu et al., 2003 |
| Rice small subunit Rubisco | Leaf specific | Nomura et al., 2000 |
| rice beta expansin EXBP9 | Shoot specific | WO 2004/070039 |
| Pigeonpea small subunit Rubisco | Leaf specific | Panguluri et al., 2005 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of green meristem-specific promoters which may be used to perform the methods of the invention are shown in Table 2h below.

Table 2h: Examples of meristem-specific promoters

| Gene source | Expression pattern | Reference |
|---|---|---|
| rice OSH1 | Shoot apical meristem, from embryo globular stage to seedling stage | Sato et al. (1996) Proc. Natl. Acad. Sci. USA, 93: 8117-8122 |
| Rice metallothionein | Meristem specific | BAD87835.1 |
| WAK1 & WAK 2 | Shoot and root apical meristems, and in expanding leaves and sepals | Wagner & Kohom (2001) Plant Cell 13(2): 303-318 |

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Decreased expression

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonudeotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation(s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient toots for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptll that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof. This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via *Agrobacterium-mediated* transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for *Agrobacterium*-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis (Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8), and approaches exist that are generally applicable regardless of the target organism (Miller et al, Nature Biotechnol. 25, 778-785, 2007).

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The term "yield" of a plant may relate to vegetative biomass (root and/or shoot biomass), to reproductive organs, and/or to propagules (such as seeds) of that plant.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., Cocos spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Omithopus* spp., *Oryza* spp. *(e.g. Oryza sativa, Oryza latifolia),*

*Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Tripsacum oides, Triticosecale rimpaui, Triticum* spp. *(e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

## Detailed description of the invention

.Surprisingly, it has now been found that increasing expression in a plant of a nucleic acid encoding a bHLH11-like polypeptide gives plants having increased seed yield relative to control plants, which nucleic acid is under the control of a medium strength constitutive promoter. According to a first embodiment, the present invention provides a method for increasing seed yield in plants relative to control plants, comprising increasing expression in a plant of a nucleic acid encoding a bHLH11-like polypeptide under the control of a medium strength constitutive promoter.

A preferred method for expression of a nucleic acid encoding a bHLH11-like polypeptide is by introducing and expressing in a plant a nucleic acid encoding a bHLH11-like polypeptide, which nucleic acid is under the control of a medium strength constitutive promoter.

Any reference hereinafter to a "protein useful in the methods of the invention" is taken to mean a bHLH11-like polypeptide as defined herein. Any reference hereinafter to a "nucleic acid useful in the methods of the invention" is taken to mean a nucleic acid capable of encoding such a bHLH11-like polypeptide. The nucleic acid to be introduced into a plant (and therefore useful in performing the methods of the invention) is any nucleic acid encoding the type of protein which will now be described, hereafter also named "*bHLH11-like* nucleic acid" or "*bHLH11-like* gene".

A "bHLH11-like polypeptide" as defined herein refers to any polypeptide comprising a basic domain followed by a HLH domain (HMMPFam PF00010, ProfileScan PS50888, SMART SM00353) thereby forming a basic helix-loop-helix domain (bHLH) (Interpro IPR001092). Preferably, the bHLH11-like polypeptide comprises at least one, preferably two, more preferably three, most preferably four or more of the following motifs:

Motif 1 (SEQ ID NO: 246): (E/D)(D/S/E)(F/M)(UF)(D/E/Q/L)(Q/H/E)
Motif 2 (SEQ ID NO: 247): RA(R/I/Q)RG(Q/H)ATDPHSIAER
Motif 3 (SEQ ID NO: 248): (M/I/V/L)(K/R)(A/S/QID/N)LQ(E/D/V)LVP
Motif 4 (SEQ ID NO: 249): (M/I)(L/I)DEI(I/V/L)(D/E/G)Y(V/UI)(K/R)FL(Q/R)LQ(V/I)K
Motif 5 (SEQ ID NO: 250): (V/I)LSMSR(L/V)G
Motif 6 (SEQ ID NO: 251):

V(A/V/L/I)(K/R)(L/M)(M/L)(E/D)(E/D/S/K/T)(D/N/S)(M/V/I)(G/T/I)XAMQ(Y/L/F)L
wherein X can be any amino acid, but preferably one of S, T, A, M, K, N
I Motif 7 (SEQ ID NO: 252): (M/V)(P/S)(I/V)(S/A)LA.

Alternatively, the homologue of a bHLH11-like protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71 %, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 245, provided that the homologous protein comprises the conserved motifs as outlined above. The overall sequence identity is determined using a global alignment algorithm, such as the Needleman Wunsch algorithm in the program GAP (GCG Wisconsin Package, Accelrys), preferably with default parameters. Compared to overall sequence identity, the sequence identity will generally be higher when only conserved domains or motifs are considered. The sequence conservation is much higher in the region of the bHLH domain (see Table E3 in Example 453 and Figure 24). Therefore the bHLH domain is a good criterion for the defining the group of bHLH11-like proteins. Preferably, the bHLH11-like polypeptide comprises the sequence of Motif 8 (SEQ ID NO: 253): SIAERLRRERIAERMRALQELVPNTNKTDRAVMLDEILDYVKFLRLQVKVL, or a sequence that has, in increasing order of preference, at least 75%, 76%, 77%, 78%, 79%, 80%, 81%, 82%, 83%, 84%, 85%. 86%, 87%, 88%, 89%, 90%, 91%, 92%, 93%, 94%, 95%, 96%, 97%, 98%, or 99% sequence identity to SEQ ID NO: 253. The HLH domain as determined by SMART spans residue 132 to 181 in SEQ ID NO: 245 and is comprised in Motif 8.

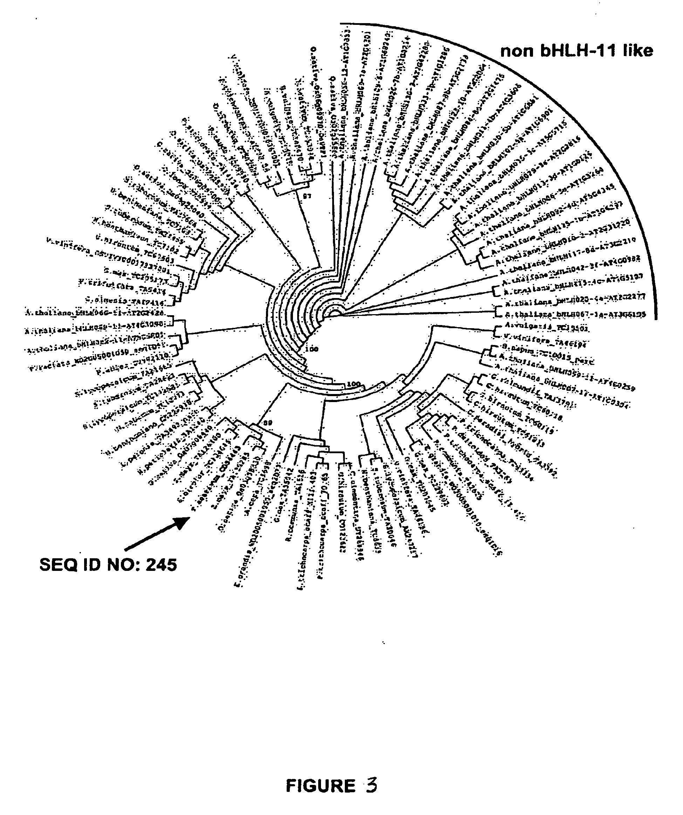

Preferably, the polypeptide sequence which when used in the construction of a phylogenetic tree, such as the one depicted in Figure 3, clusters within the group of bHLH11-like proteins, rather than with other bHLH proteins. Similarly, the bHLH11-like protein of choice will cluster within subgroup C when a tree is constructed according to Figure 6 in Li et al. (2006), rather than with any other group.

The terms "domain", "signature" and "motif" are defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137. (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

Furthermore, bHLH11-like polypeptides (at least in their native form) typically have DNA binding activity. Tools and techniques for measuring DNA binding activity are well known in the art. In addition, as shown in the present invention, a bHLH11-like protein, such as SEQ ID NO: 245, when overexpressed in rice under the control of a medium strength constitutive promoter, gives plants having increased seed yield, in particular increased fill rate. Further details are provided in Examples section.

The present invention is illustrated by transforming plants with the nucleic acid sequence represented by SEQ ID NO: 244, encoding the polypeptide sequence of SEQ ID NO: 245. However, performance of the invention is not restricted to these sequences; the methods of the invention may advantageously be performed using any bHLHl1-like-encoding nucleic acid or bHLH11-like polypeptide as defined herein.

Examples of nucleic acids encoding bHLH11-like polypeptides are given in Table E1 of Example 431 herein. Such nucleic acids are useful in performing the methods of the invention. The amino acid sequences given in Table E1 of Example 431 are example sequences of orthologues and paralogues of the bHLH11-like polypeptide represented by SEQ ID NO: 245, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table E1 of Example 41) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 244 or SEQ ID NO: 245, the second BLAST would therefore be against *Triticum aestivum* sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Nucleic acid variants may also be useful in practising the methods of the invention. Examples of such variants include nucleic acids encoding homologues and derivatives of any one of the amino acid sequences given in Table E1 of Example 41, the terms "homologue" and "derivative" being as defined herein. Also useful in the methods of the invention are nucleic acids encoding homologues and derivatives of orthologues or paralogues of any one of the amino acid sequences given in Table E1 of Example 431. Homologues and derivatives useful in the methods of the present invention have substantially the same biological and functional activity as the unmodified protein from which they are derived.

Further nucleic acid variants useful in practising the methods of the invention include portions of nucleic acids encoding bHLH11-like polypeptides, nucleic acids hybridising to nucleic acids encoding bHLH11-like polypeptides, splice variants of nucleic acids encoding bHLH11-like polypeptides, allelic variants of nucleic acids encoding bHLH11-like polypeptides and variants of nucleic acids encoding bHLHl1-like polypeptides obtained by gene shuffling. The terms hybridising sequence, splice variant, allelic variant and gene shuffling are as described herein.

Nucleic acids encoding bHLH11-like polypeptides need not be full-length nucleic acids, since performance of the methods of the invention does not rely on the use of full-length nucleic acid sequences. According to the present invention, there is provided a method for increasing seed yield in plants, comprising introducing and expressing in a plant a portion of any one of the nucleic acid sequences given in Table E1 of Example 431, or a portion of a nucleic acid encoding an orthologue, paralogue or homologue of any of the amino acid sequences given in Table E1 of Example 41, which portion is under the control of a medium strength constitutive promoter.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode a bHLH11-like polypeptide as defined herein, and have substantially the same biological activity as the amino acid sequences given in Table E1 of Example 41. Preferably, the portion is a portion of any one of the nucleic acids given in Table E1 of Example 431, or is a portion of a nucleic acid encoding an orthologue or paralogue of any one of the amino acid sequences given in Table E1 of Example 41. Preferably the portion is at least 500, 550, 600, 650, 700, 750, 800, 850, 900, 950, 1000, 1100, 1200, 1300, 1400, 1500, 1600, 1700, 1800, 1900, 2000, 2100, 2200, 2300, 2400, 2500 consecutive nucleotides in length, the consecutive nucleotides being of any one of the nucleic acid sequences given in Table E1 of Example 41, or of a nucleic acid encoding an orthologue or paralogue of any one of the amino acid sequences given in Table E1 of Example 431. Most preferably the portion is a portion of the nucleic acid of SEQ ID NO: 244. Preferably, the portion encodes a fragment of an amino acid sequence which, when used in the construction of a phylogenetic tree, such as the one depicted in Figure 3, clusters within the group of bHLH11-like proteins, rather than with other bHLH proteins. Similarly, the bHLH11-like protein of choice will cluster within subgroup C when a tree is constructed according to Figure 6 in Li et al. (2006), rather than with any other group.

Another nucleic acid variant useful in the methods of the invention is a nucleic acid capable of hybridising, under reduced stringency conditions, preferably under stringent conditions, with a nucleic acid encoding a bHLH11-like polypeptide as defined herein, or with a portion as defined herein.

According to the present invention, there is provided a method for increasing seed yield in plants, comprising introducing and expressing in a plant a nucleic acid capable of hybridizing to any one of the nucleic acids given in Table E1 of Example 1. or comprising introducing and expressing in a plant a nucleic acid capable of hybridising to a nucleic acid encoding an orthologue, paralogue or homologue of any of the nucleic acid sequences given in Table E1 of Example 1, which nucleic acid capable of hybridising is under the control of a medium strength constitutive promoter.

Hybridising sequences useful in the methods of the invention encode a bHLH11-like polypeptide as defined herein, having substantially the same biological activity as the amino acid sequences given in Table E1 of Example 1. Preferably, the hybridising sequence is capable of hybridising to the complement of any one of the nucleic acids given in Table E1 of Example 1, or to a portion of any of these sequences, a portion being as defined above, or the hybridising sequence is capable of hybridising to the complement of a nucleic acid encoding an orthologue or paralogue of any one of the amino acid sequences given in Table E1 of Example 1. Most preferably, the hybridising sequence is capable of hybridising to the complement of a nucleic acid as represented by SEQ ID NO: 244 or to a portion thereof.

Preferably, the hybridising sequence encodes a polypeptide with an amino acid sequence which, when full-length and used in the construction of a phylogenetic tree, such as the one depicted in Figure 3, clusters within the group of bHLH11-like proteins, rather than with other bHLH proteins. Similarly, the bHLH11-like protein of choice will cluster within subgroup C when a tree is constructed according to Figure 6 in Li et al. (2006), rather than with any other group.

Another nucleic acid variant useful in the methods of the invention is a splice variant encoding a bHLH 11-like polypeptide as defined hereinabove, a splice variant being as defined herein.

According to the present invention, there is provided a method for increasing seed yield in plants, comprising introducing and expressing in a plant a splice variant of any one of the nucleic acid sequences given in Table E1 of Example 1 or a splice variant of a nucleic acid encoding an orthologue, paralogue or homologue of any of the amino acid sequences given in Table E1 of Example 1, which splice variant is under the control of a medium strength constitutive promoter.

Preferred splice variants are splice variants of a nucleic acid represented by SEQ ID NO: 244, or a splice variant of a nucleic acid encoding an orthologue or paralogue of SEQ ID NO: 245. Preferably, the amino acid sequence encoded by the splice variant, when used in the construction of a phylogenetic tree, such as the one depicted in Figure 3, clusters within the group of bHLH11-like proteins, rather than with other bHLH proteins. Similarly, the bHLH11-like protein of choice will cluster within subgroup C when a tree is constructed according to Figure 6 in Li et al. (2006), rather than with any other group.

Another nucleic acid variant useful in performing the methods of the invention is an allelic variant of a nucleic acid encoding a bHLH11-like polypeptide as defined hereinabove, an allelic variant being as defined herein.

According to the present invention, there is provided a method for increasing seed yield in plants, comprising introducing and expressing in a plant an allelic variant of any one of the nucleic acids given in Table E1 of Example 431, or comprising introducing and expressing in a plant an allelic variant of a nucleic acid encoding an orthologue, paralogue or homologue of any of the amino acid sequences given in Table E1 of Example 1, which allelic variant is under the control of a medium strength constitutive promoter.

The polypeptides encoded by allelic variants useful in the methods of the present invention have substantially the same biological activity as the bHLH11-like polypeptide of SEQ ID NO: 244 and any of the amino acids depicted in Table E1 of Example 431. Allelic variants exist in nature, and encompassed within the methods of the present invention is the use of these natural alleles. Preferably, the allelic variant is an allelic variant of SEQ ID NO: 244 or an allelic variant of a nucleic acid encoding an orthologue or paralogue of SEQ ID NO: 245. Preferably, the amino acid sequence encoded by the allelic variant, when used in the construction of a phylogenetic tree, such as the one depicted in Figure 3, clusters within the group of bHLH11-like proteins, rather than with other bHLH proteins. Similarly, the bHLH11-like protein of choice will cluster within subgroup C when a tree is constructed according to Figure 6 in Li et al. (2006), rather than with any other group.

Gene shuffling or directed evolution may also be used to generate variants of nucleic acids encoding bHLH11-like polypeptides as defined above; the term "gene shuffling" being as defined herein.

According to the present invention, there is provided a method for increasing seed yield in plants, comprising introducing and expressing in a plant a variant of any one of the nucleic acid sequences given in Table E1 of Example 1, or comprising introducing and expressing in a plant a variant of a nucleic acid encoding an orthologue, paralogue or homologue of any of the amino acid sequences given in Table E1 of Example 1 which variant nucleic acid is obtained by gene shuffling, and which variant nucleic acid is under the control of a medium strength constitutive promoter.

The amino acid sequence encoded by the variant nucleic acid obtained by gene shuffling, when used in the construction of a phylogenetic tree, such as the one depicted in Figure 3, clusters within the group of bHLH11-like proteins, rather than with other bHLH proteins. Similarly, the bHLH11-like protein of choice will cluster within subgroup C when a tree is constructed according to Figure 6 in Li et al. (2006), rather than with any other group.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding bHLH11-like polypeptides may be derived from any natural or artificial source. The nucleic acid may be modified from its native form in composition and/or genomic environment through deliberate human manipulation. Preferably the bHLH11-like polypeptide-encoding nucleic acid is from a plant, further preferably from a monocotyledonous plant, more preferably from the family Poaceae, most preferably the nucleic acid is from *Triticum aestivum.*

Performance of the methods of the invention gives plants having increased seed yield relative to control plants. The terms "yield" and "seed yield" are described in more detail in the "definitions" section herein.

In a particular embodiment, harvestable parts of a plant are seeds, and performance of the methods of the invention results in plants having increased seed yield relative to the seed yield of control plants.

Taking corn as an example, a yield increase may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per square meter, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

The present invention provides a method for increasing seed yield of plants, relative to control plants, which method comprises increasing expression in a plant of a nucleic acid encoding a bHLH11-like polypeptide as defined herein, which nucleic acid is under the control of a medium strength constitutive promoter.

Since the transgenic plants according to the present invention have increased seed yield, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Also described herein is a method for increasing the growth rate of plants, which method comprises increasing expression in a plant of a nucleic acid encoding a bHLH11-like polypeptide as defined herein, which nucleic acid is under the control of a medium strength constitutive promoter.

An increase in seed yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11 % or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi, nematodes and insects.

In particular, the methods of the present invention may be performed under non-stress conditions or under conditions of mild drought to give plants having increased seed yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 90%, 87%, 85%, 83%, 80%, 77% or 75% of the average production of such plant in a given environment. Average production may be calculated on harvest and/or season basis. Persons skilled in the art are aware of average yield productions of a crop.

Performance of the methods of the invention gives plants grown under non-stress conditions or under mild drought conditions increased seed yield relative to control plants grown under comparable conditions. Therefore, according to the present invention, there is provided a method for increasing seed yield in plants grown under non-stress conditions or under mild drought conditions, which method comprises increasing expression in a plant of a nucleic acid encoding a bHLH11-like polypeptide, which nucleic acid is under the control of a medium strength constitutive promoter.

Performance of the methods of the invention gives plants grown under conditions of nutrient deficiency, particularly under conditions of nitrogen deficiency, increased yield relative to control plants grown under comparable conditions. Therefore, described herein is a method for increasing seed yield in plants grown under conditions of nutrient deficiency, which method comprises increasing expression in a plant of a nucleic acid encoding a bHLH11-like polypeptide under the control of a medium strength constitutive promoter, provided that the nutrient deficiency is not a phosphate deficiency. Nutrient deficiency may result from a lack of nutrients such as nitrogen, phosphates and other phosphorous-containing compounds, potassium, calcium, cadmium, magnesium, manganese, iron and boron, amongst others. However, the term "nutrient deficiency" as used in the context of the present invention does not encompass a deficiency in phosphate.

The present invention encompasses plants or parts thereof (including seeds) obtainable by the methods according to the present invention. The plants or parts thereof comprise a nucleic acid transgene encoding a bHLH11-like polypeptide as defined above, which nucleic acid is under the control of a medium strength constitutive promoter.

The invention also provides genetic constructs and vectors to facilitate introduction and/or expression in plants of nucleic acids encoding bHLH11-like polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding a bHLH11-like polypeptide as defined above;
(b) one or more control sequences capable of driving expression of the nucleic acid sequence of (a), wherein the one or more control sequences comprise a medium strength constitutive promoter; and optionally
(c) a transcription termination sequence.

Preferably, the nucleic acid encoding a bHLH11-like polypeptide is as defined above. The term "control sequence" and "termination sequence" are as defined herein.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a medium strength constitutive promoter).

The medium strength constitutive promoter may be any type of promoter, whether natural or synthetic, may be used to drive expression of the nucleic acid sequence, but preferably the promoter is of plant origin. A constitutive promoter is particularly useful in the methods. Preferably the constitutive promoter is also a ubiquitous promoter of medium strength. See the "Definitions" section herein for definitions of the various promoter types.

It should be clear that the applicability of the present invention is not restricted to the bHLH11-like polypeptide-encoding nucleic acid represented by SEQ ID NO: 1, nor is the applicability of the invention restricted to expression of a bHLH11-like polypeptide-encoding nucleic acid when driven by a constitutive GOS2 promoter.

The constitutive promoter is preferably a medium strength promoter, such as a GOS2 promoter, preferably the promoter is a GOS2 promoter from rice. Further preferably the constitutive promoter is represented by a nucleic acid sequence substantially similar to SEQ ID NO: 256, most preferably the constitutive promoter is as represented by SEQ ID NO: 256. See the "Definitions" section herein for further examples of constitutive promoters.

Optionally, one or more terminator sequences may be used in the construct introduced into a plant. Preferably, the construct comprises an expression cassette comprising the GOS2 promoter substantially similar to SEQ ID NO: 256 and the nucleic acid encoding the bHLH11-like polypeptide.

Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The invention also provides a method for the production of transgenic plants having increased seed yield relative to control plants, comprising introduction and expression in a plant of any nucleic acid encoding a bHLH11-like polypeptide as defined hereinabove, which nucleic acid is under the control of a medium strength constitutive promoter.

More specifically, the present invention provides a method for the production of transgenic plants having increased seed yield, which method comprises:

(i) introducing and expressing in a plant or plant cell a bHLH11-like polypeptide-encoding nucleic acid, wherein said nucleic acid is under the control of a medium strength constitutive promoter; and
(ii) cultivating the plant cell under conditions promoting plant growth and development.

The nucleic acid of (i) may be any of the nucleic acids capable of encoding a bHLH 11-like polypeptide as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding a bHLH11-like polypeptide as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, linseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum, emmer, spelt, secale, einkorn, teff, milo and oats.

The invention also extends to harvestable parts of a plant such as, but not limited to seeds, leaves, fruits, flowers, stems, roots, rhizomes, tubers and bulbs, which harvestable parts comprise a recombinant nucleic acid encoding a bHLH11-like polypeptide. The invention furthermore relates to products derived, preferably directly derived, from a harvestable part of such a plant, such as dry pellets or powders, oil, fat and fatty acids, starch or proteins.

According to the methods of the invention, the expression of a nucleic acid encoding a bHLH11-like polypeptide is increased expression. Methods for increasing expression of nucleic acids or genes, or gene products, are well documented in the art and examples are provided in the definitions section.

As mentioned above, a preferred method for increasing expression of a nucleic acid encoding a bHLH11-like polypeptide is by introducing and expressing in a plant a nucleic acid encoding a bHLH11-like polypeptide; however the effects of performing the method, i.e. increasing seed yield may also be achieved using other well known techniques, including but not limited to T-DNA activation tagging, TILLING, homologous recombination. A description of these techniques is provided in the definitions section.

The present invention also encompasses use of nucleic acids encoding bHLH11-like polypeptides as described herein and use of these bHLH11-like polypeptides in increasing seed yield in plants.

Nucleic acids encoding bHLH11-like polypeptide described herein, or the bHLH11-like polypeptides themselves, may find use in breeding programmes in which a DNA marker is identified which may be genetically linked to a bHLH11-like polypeptide-encoding gene. The nucleic acids/genes, or the bHLH11-like polypeptides themselves may be used to define a molecular marker. This DNA or protein marker may then be used in breeding programmes to select plants having increased seed yield as defined hereinabove in the methods of the invention.

Allelic variants of a bHLH11-like polypeptide-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding bHLH11-like polypeptides may also be used as probes for genetically and physically mapping the genes that they are a part of, and as markers for traits linked to those genes. Such information may be useful in plant breeding in order to develop lines with desired phenotypes. Such use of bHLH11-like polypeptide-encoding nucleic acids requires only a nucleic acid sequence of at least 15 nucleotides in length. The bHLH11-like polypeptide-encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the bHLH11-like-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the bHLH11-like polypeptide-encoding nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having increased seed yield, as described hereinbefore. These traits may also be combined with other economically advantageous traits, such as further yield-enhancing traits, tolerance to other abiotic and biotic stresses, traits modifying various architectural features and/or biochemical and/or physiological features.

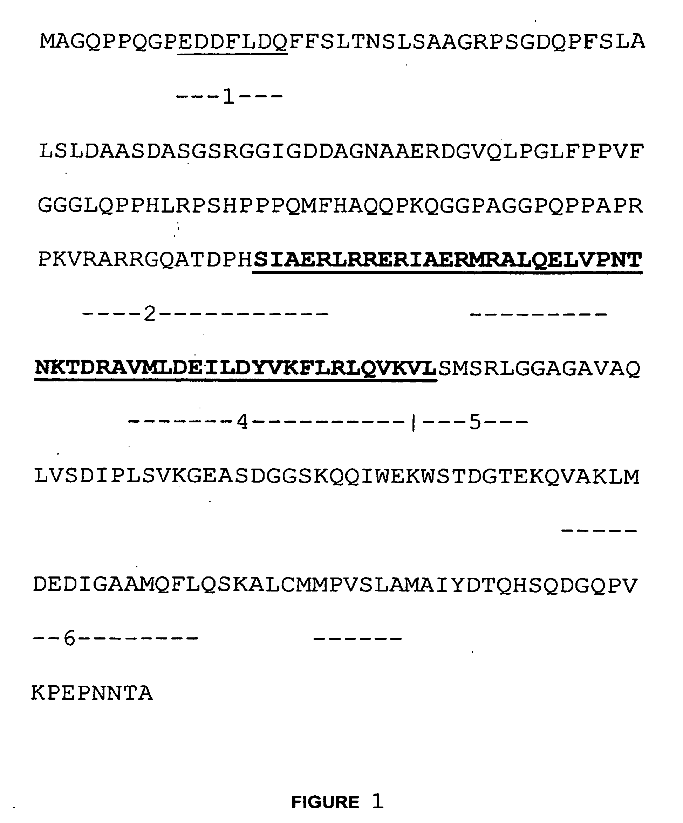

**Figure 1** represents the domain structure of SEQ ID NO: 245 with the conserved motifs indicated by underlining and their number. The HLH domain (motif 8) as determined by SMART is shown in bold underlined.

**Figure 2** represents a multiple alignment of various bHLH11-like proteins. A dot indicates conserved residues, a colon indicates highly conserved residues and an asterisk stands for perfectly conserved residues. The highest degree of sequence conservation is found in the region of the bHLH domain. The C-terminal part of AT2G24260 that extends beyond the other proteins in the alignment is deleted.

**Figure 3** Circular cladogram of selected bHLH proteins. bHLH11-like proteins and one Arabidopsis protein representing each of the other classes defined by Heim 2003 were used. The alignment was generated using "CLUSTALX", and a neighbour-joining tree was calculated. The circular cladogram was drawn using Dendroscope (Huson et al. BMC Bioinformatics 2007). Bootstrap results for 100 replicates is indicated for some major nodes; the boxed bootstrap value shows that the group of bHLH11-like proteins is clearly delineated from the other bHLH proteins.

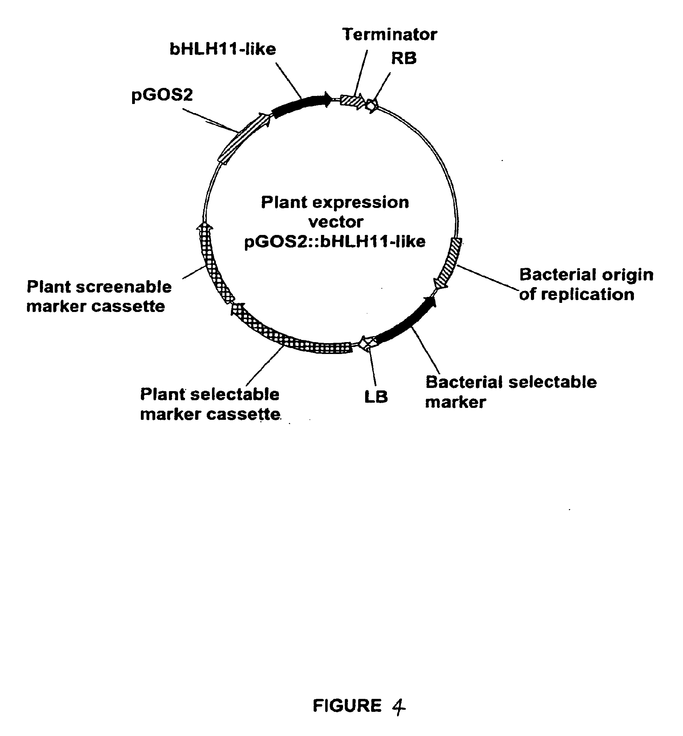

**Figure 4** represents the binary vector for increased expression in Oryza sativa of a bHLH11-like-encoding nucleic acid under the control of a rice GOS2 promoter (pGOS2).

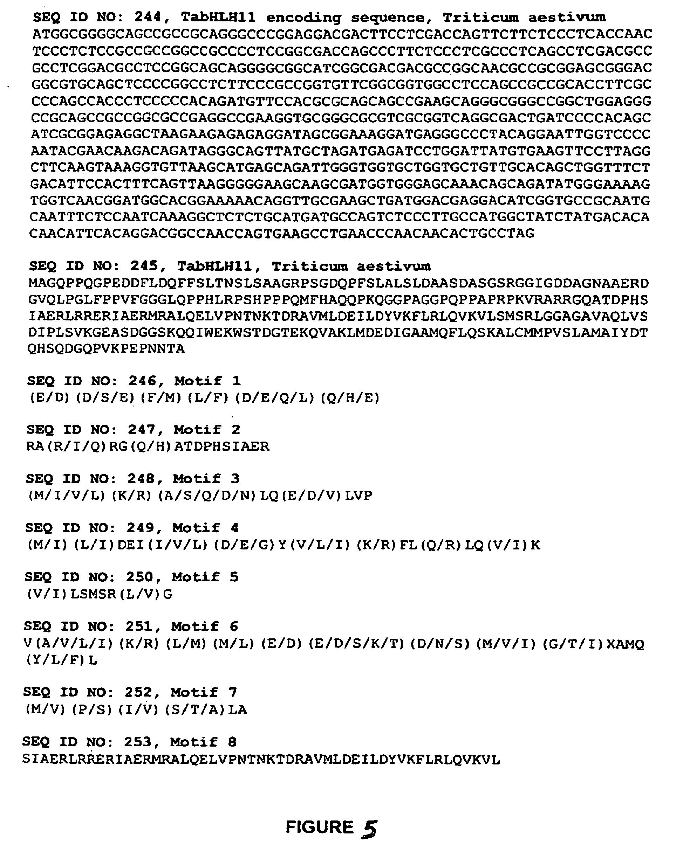

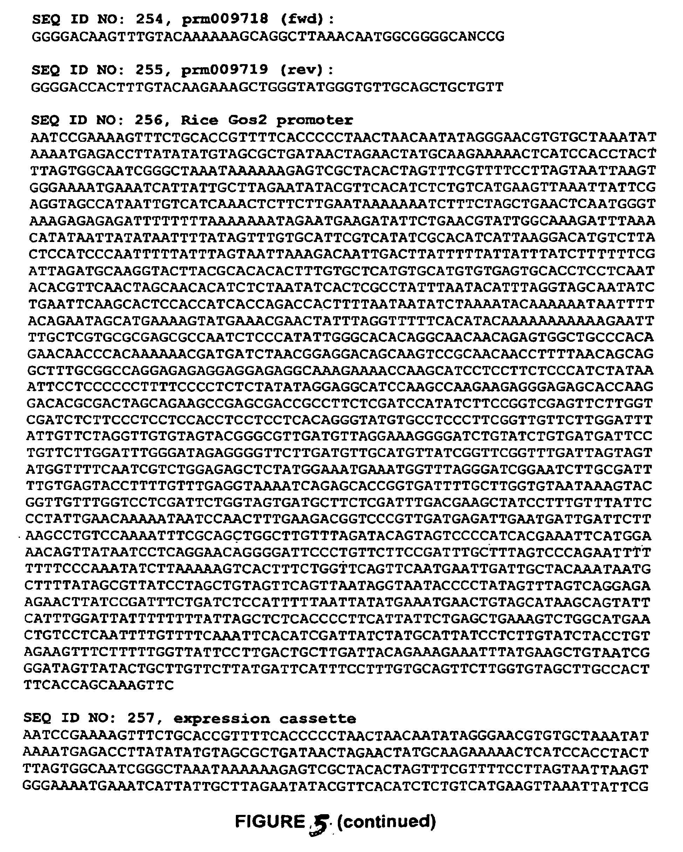

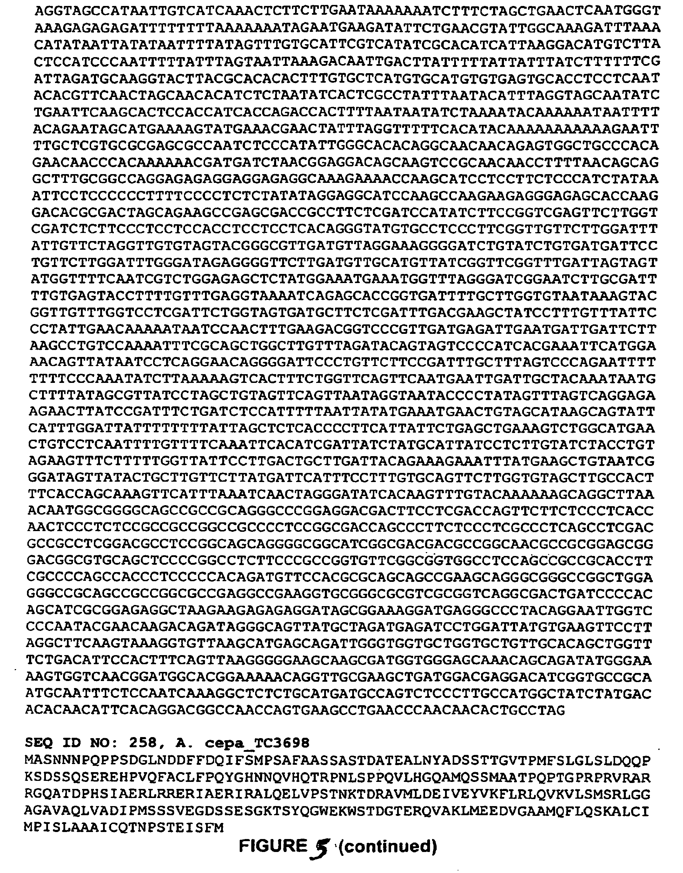

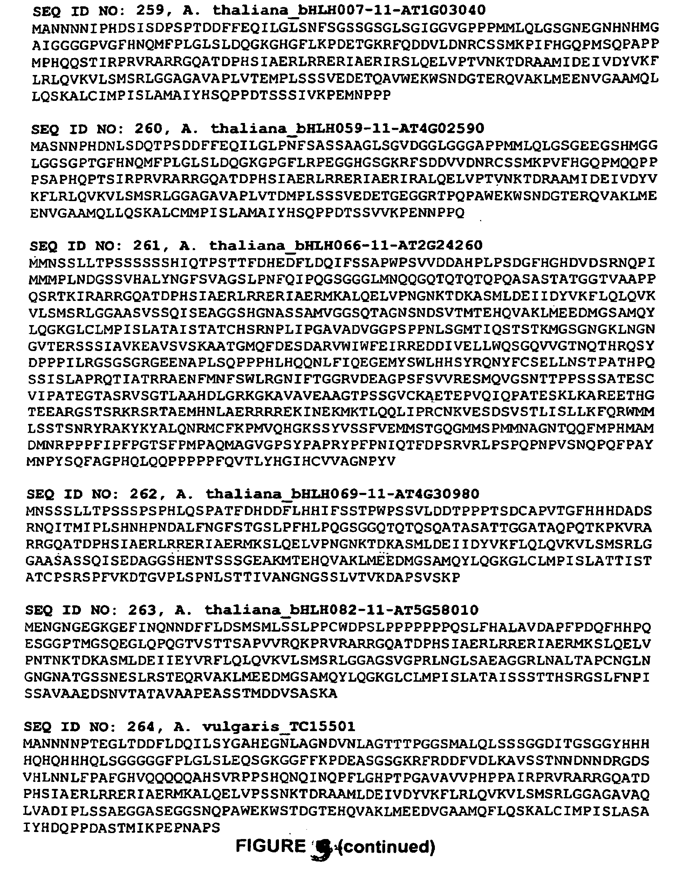

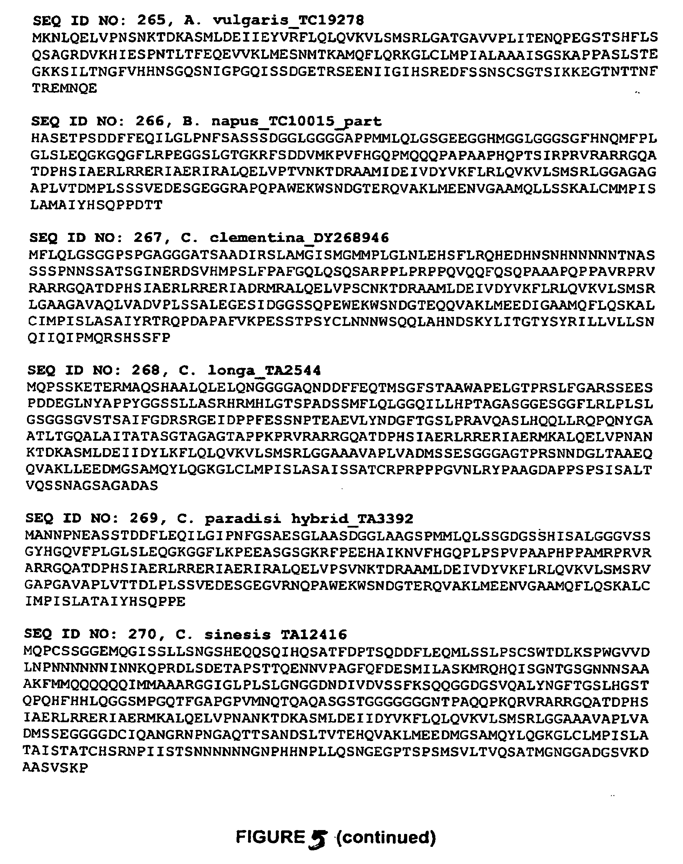

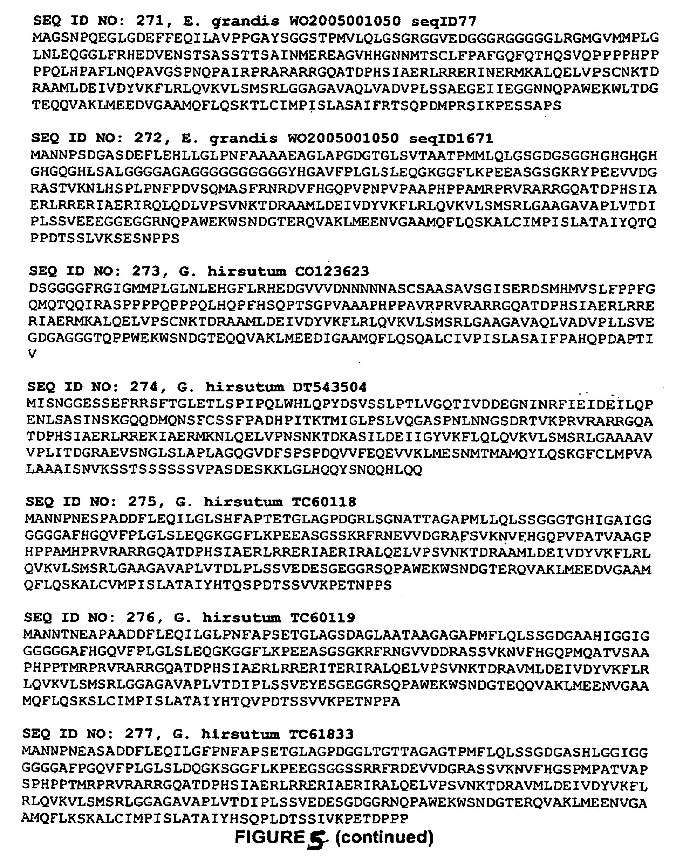

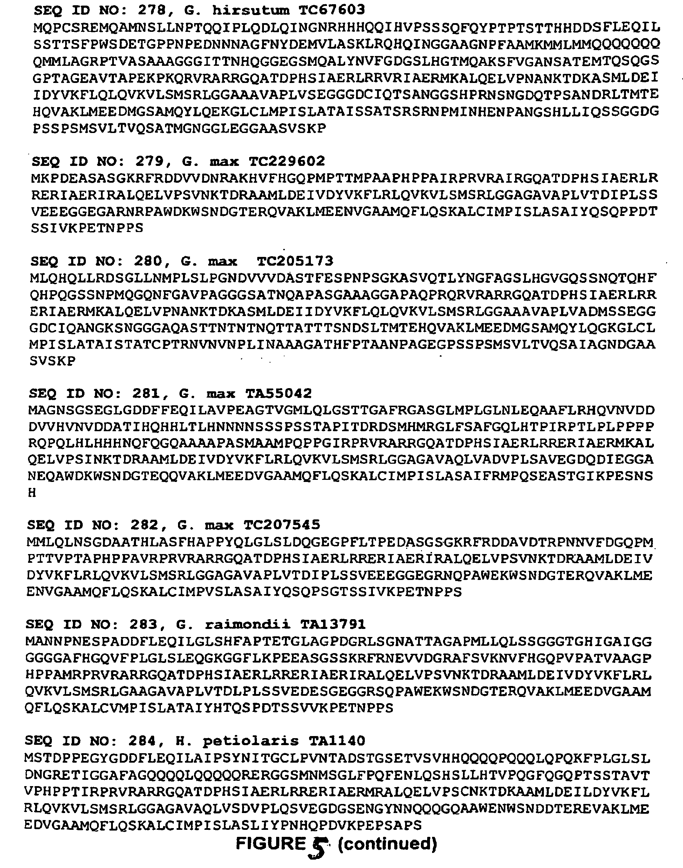

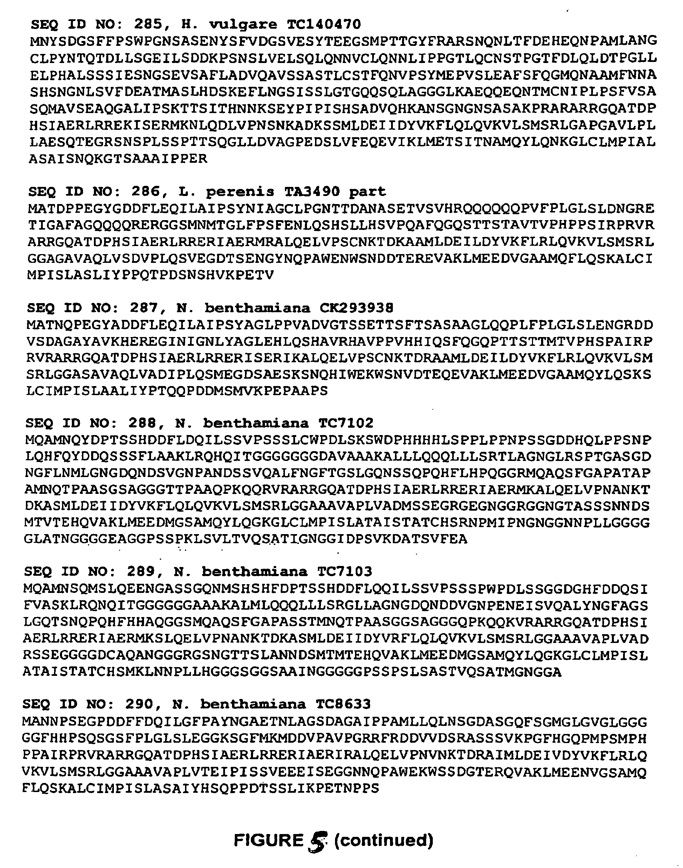

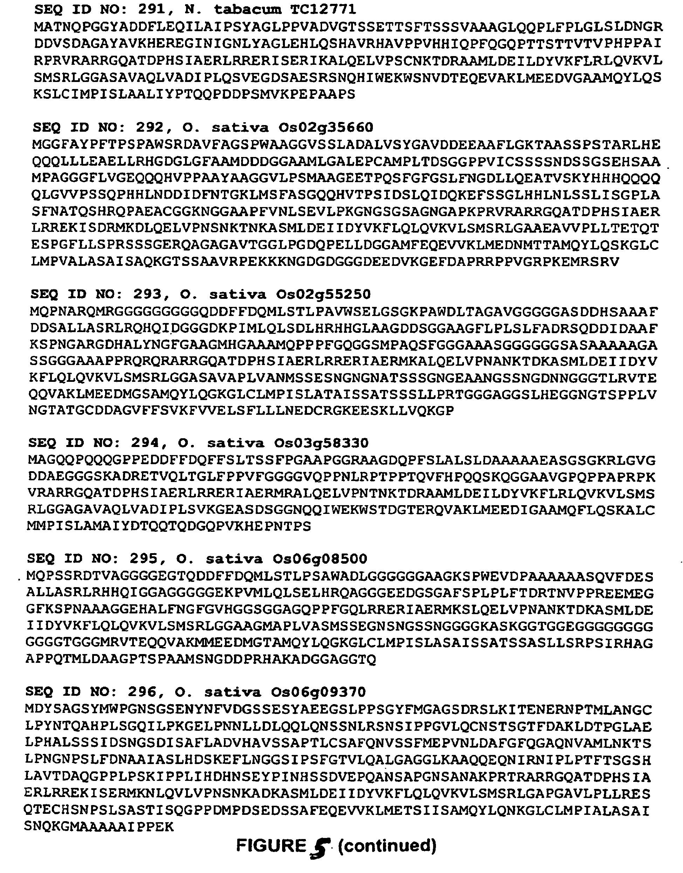

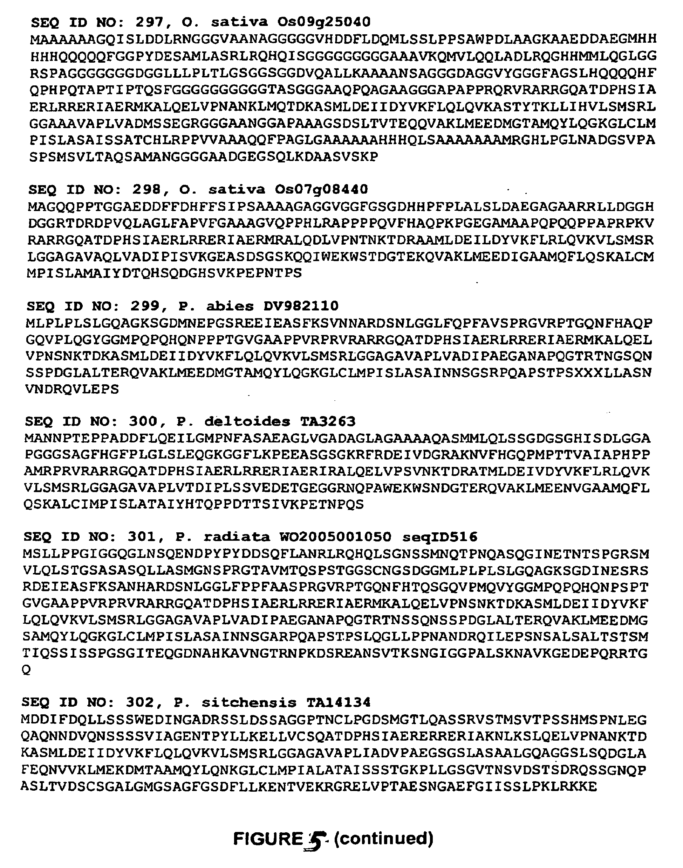

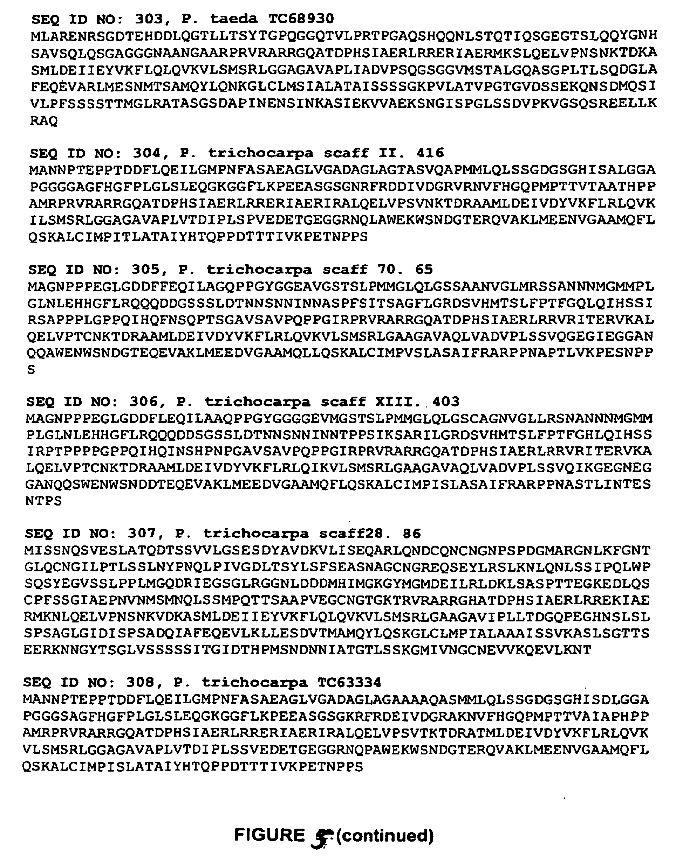

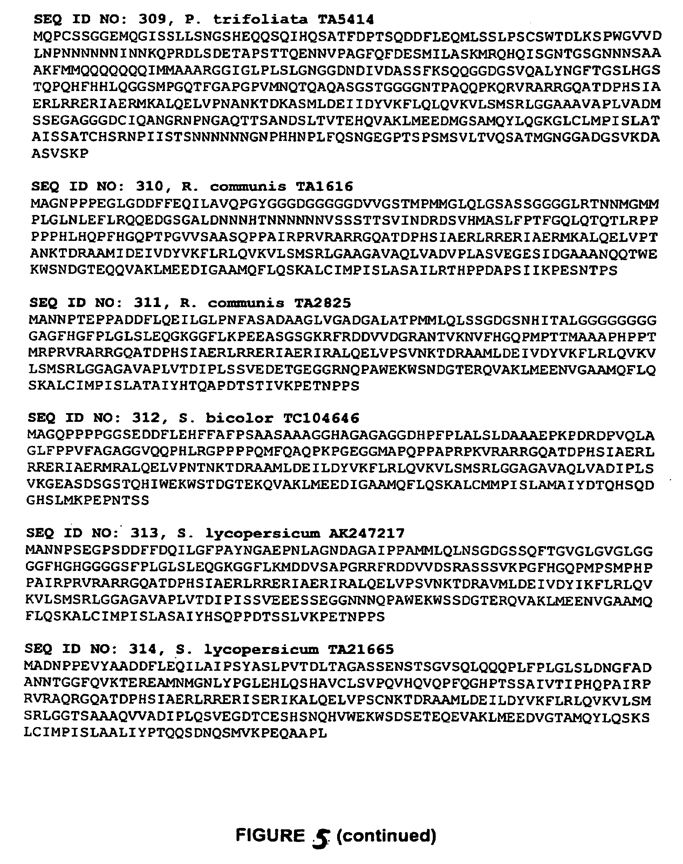

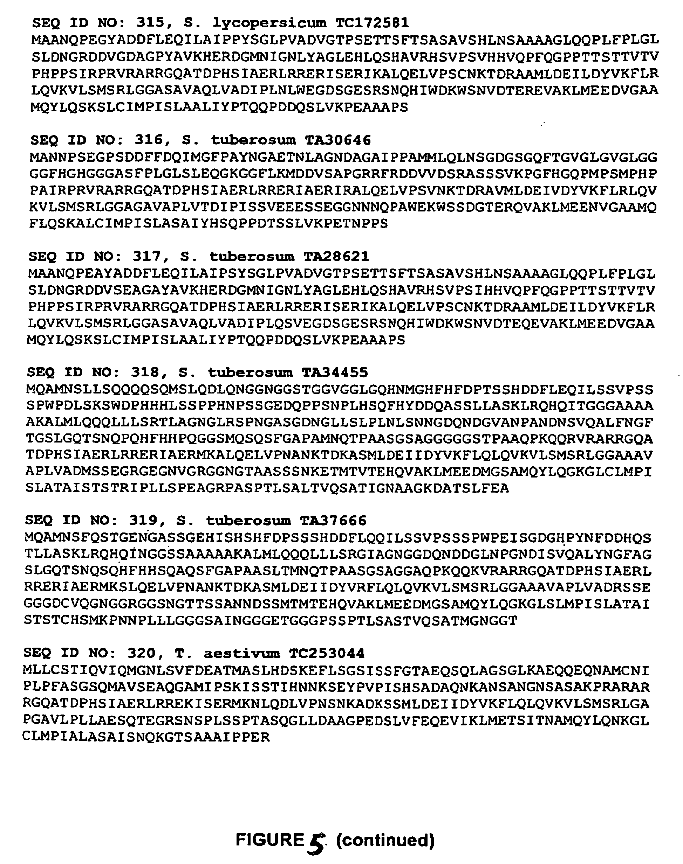

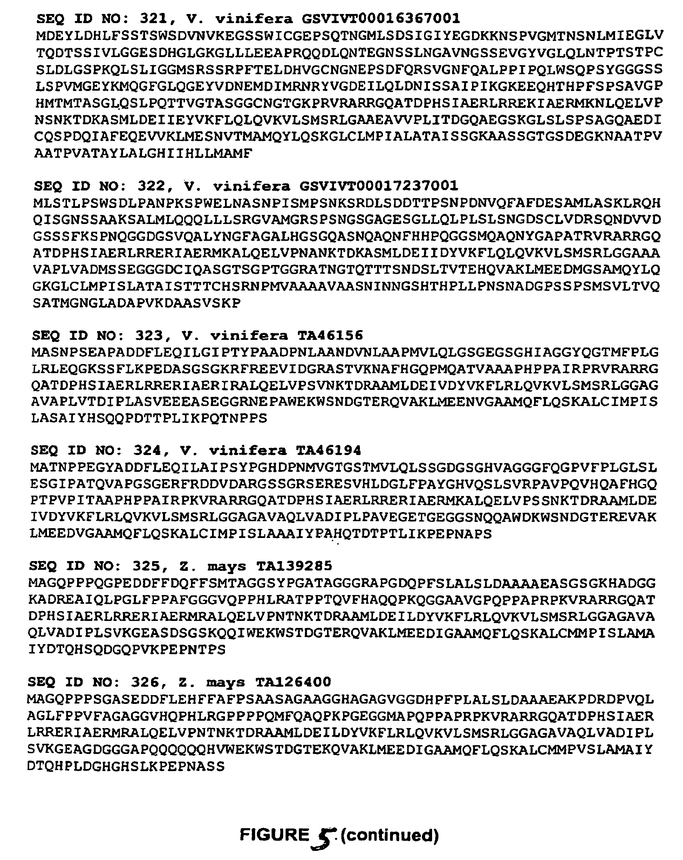

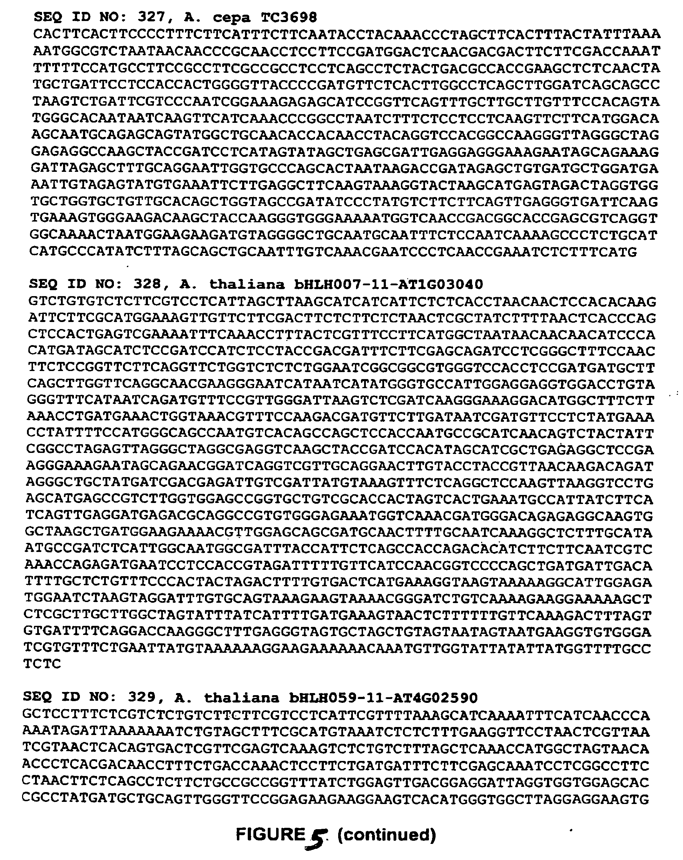

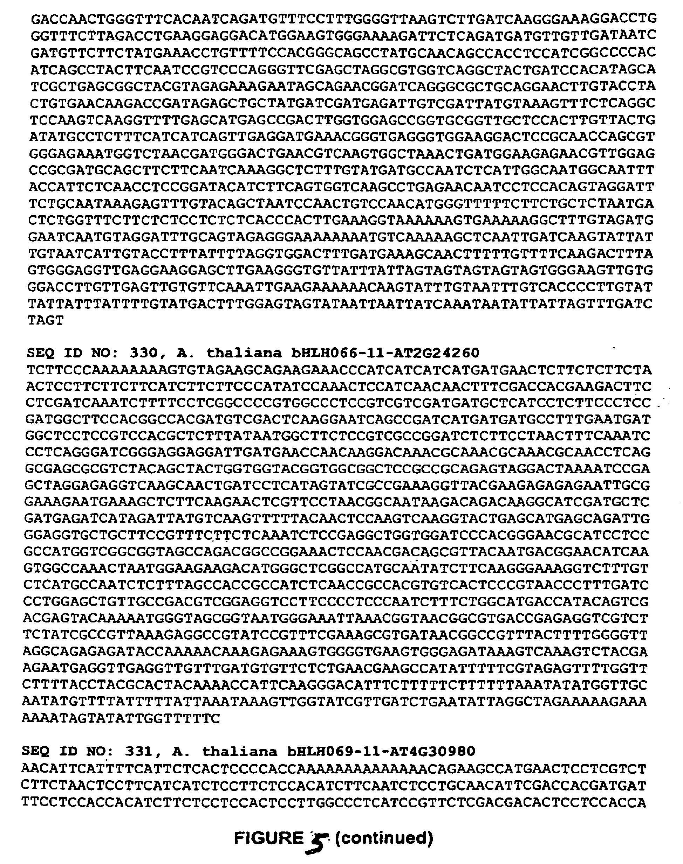

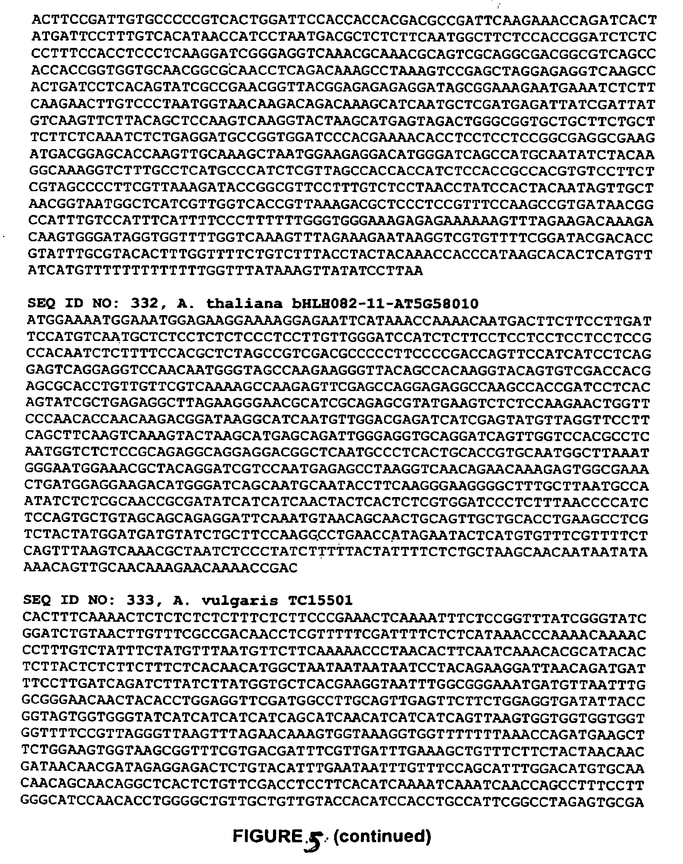

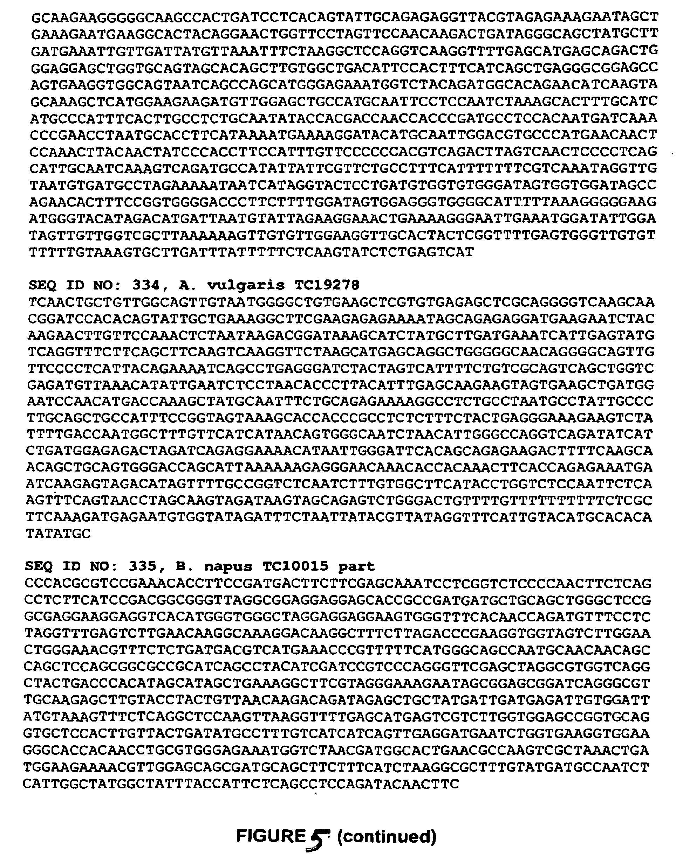

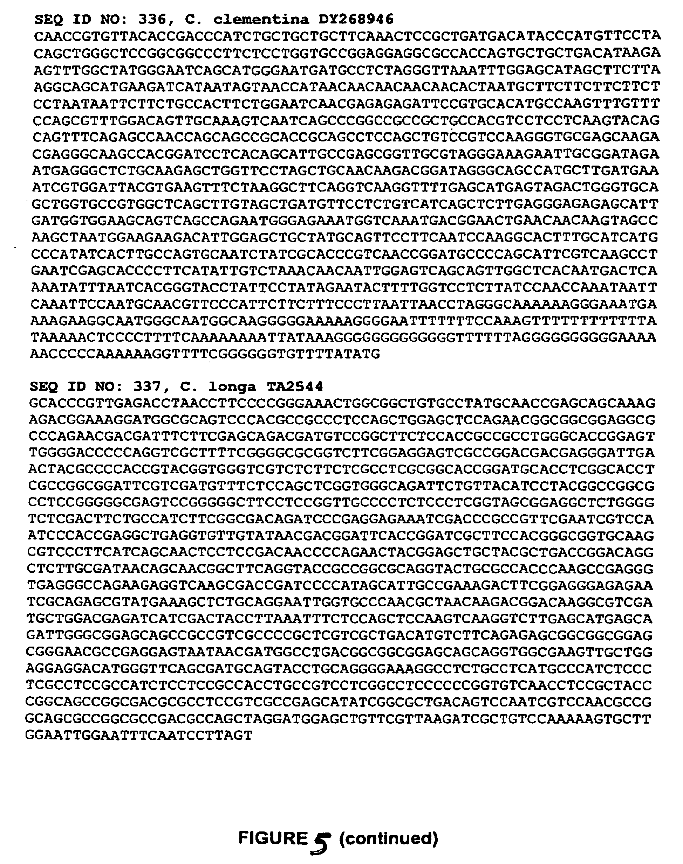

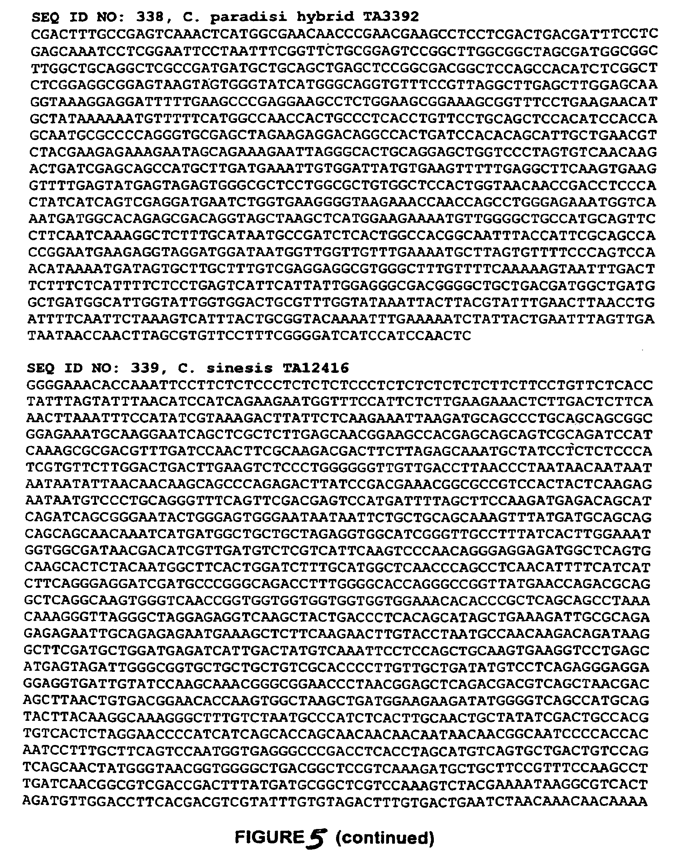

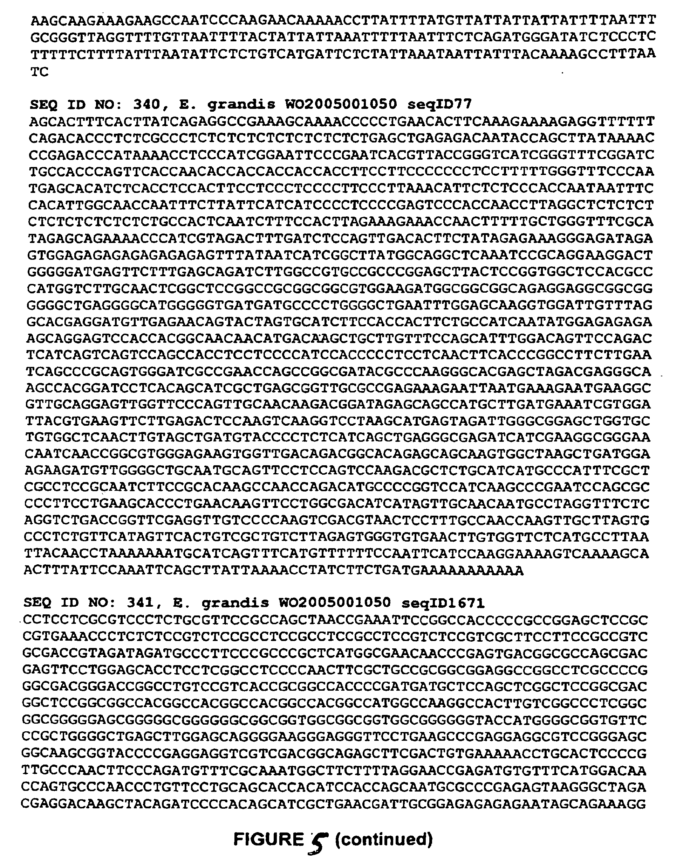

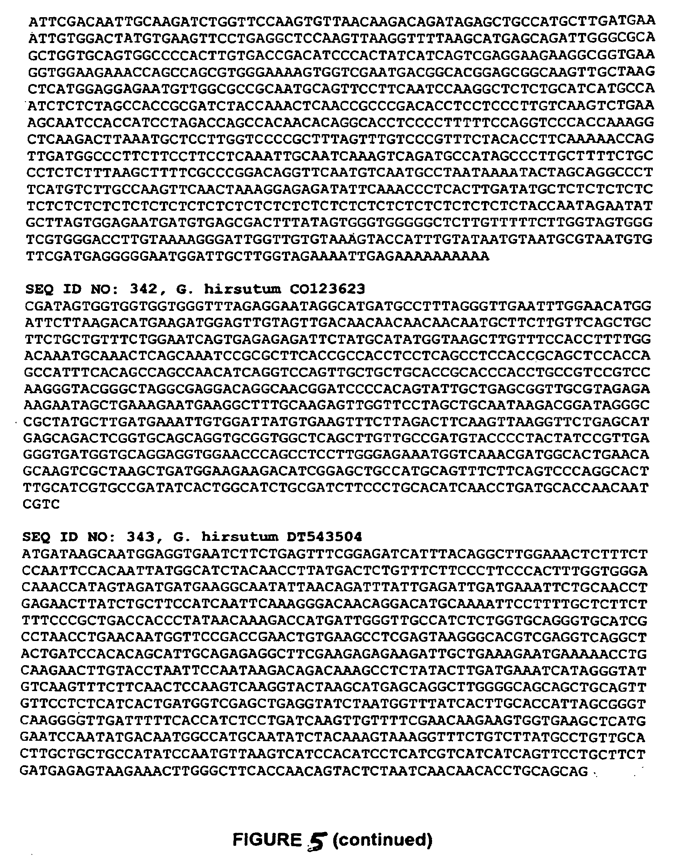

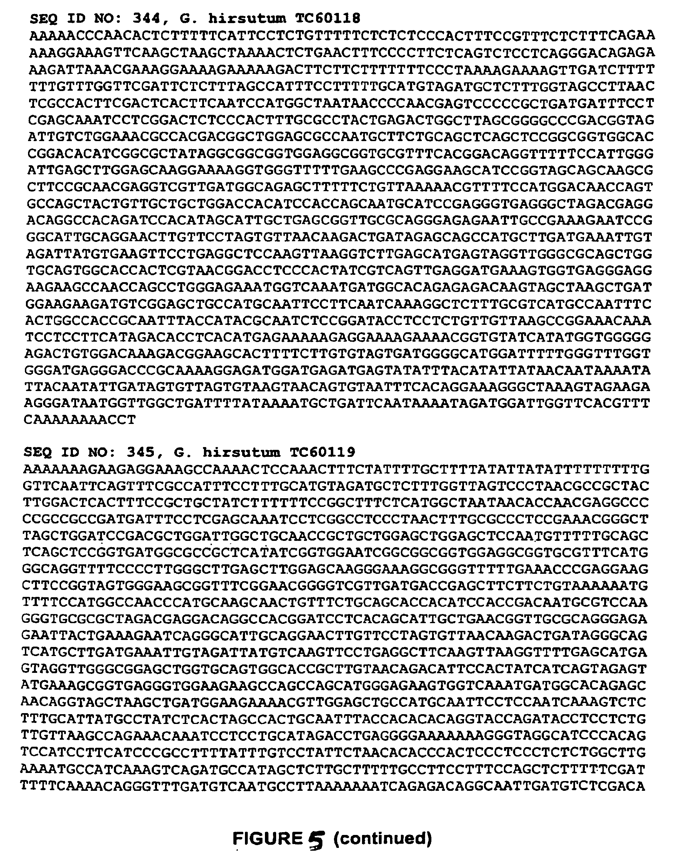

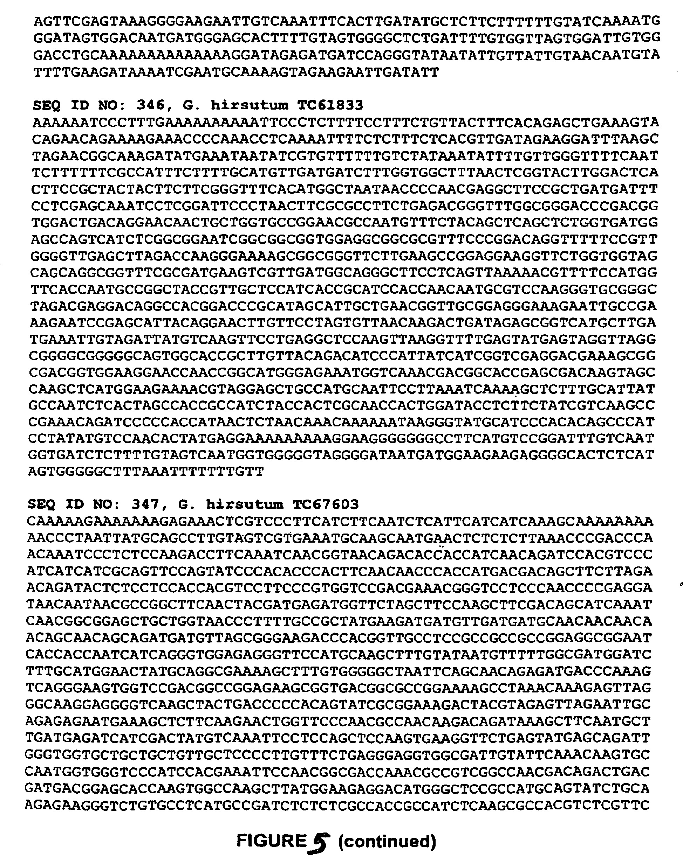

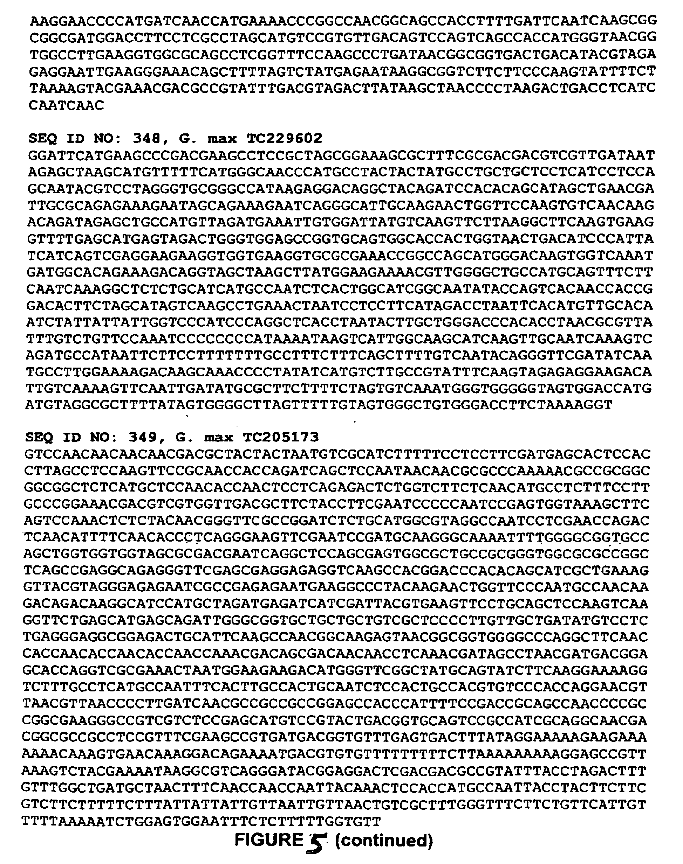

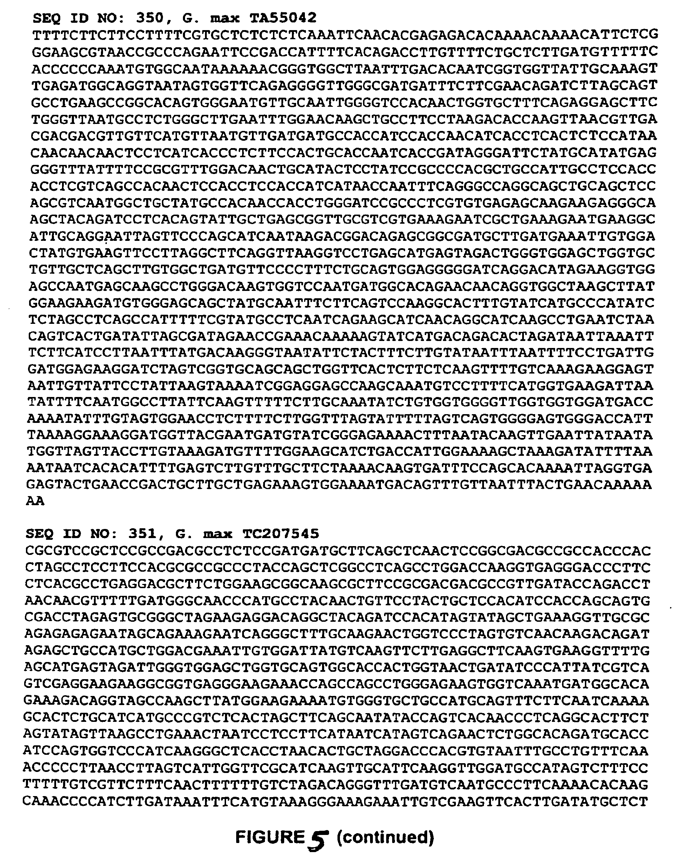

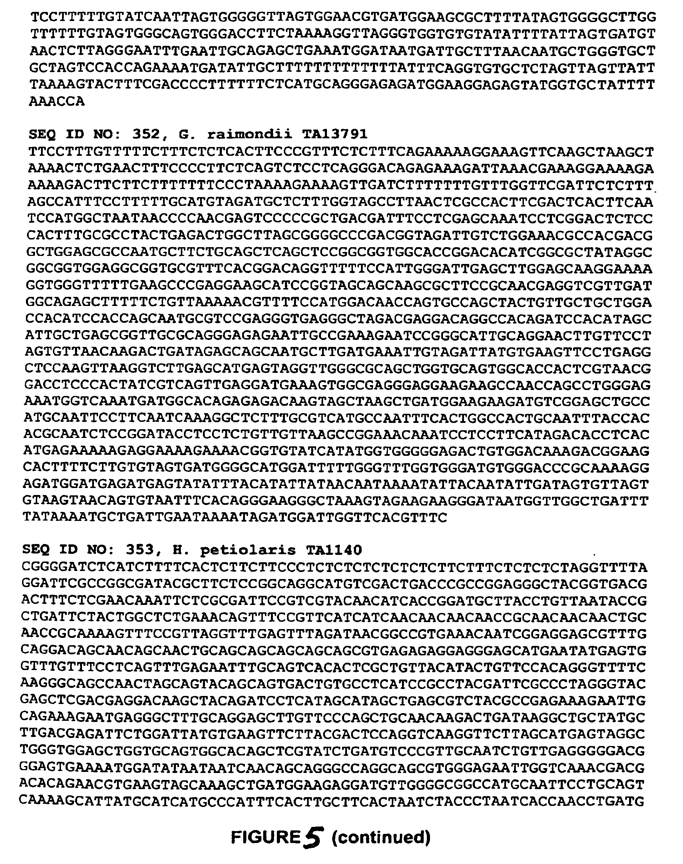

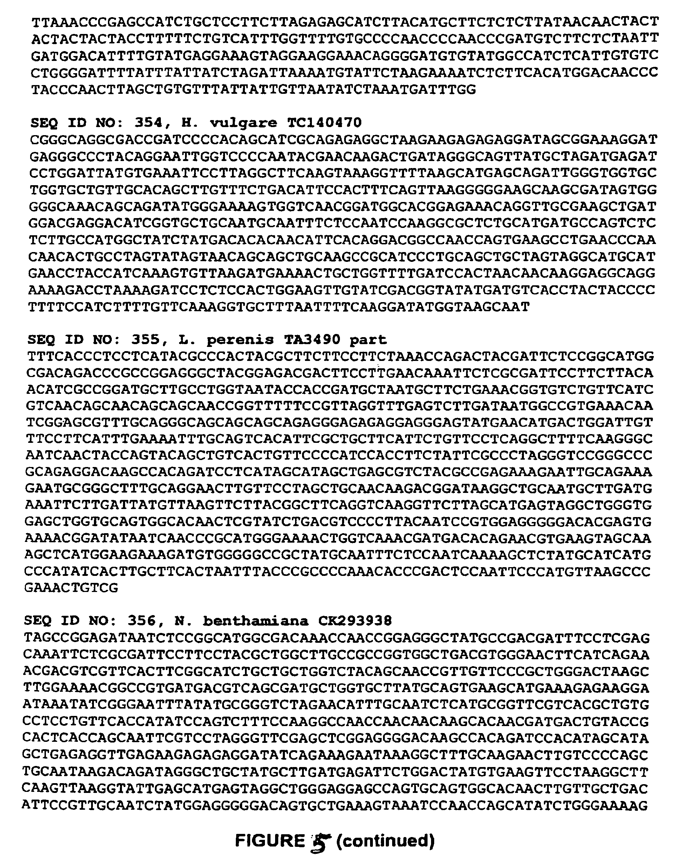

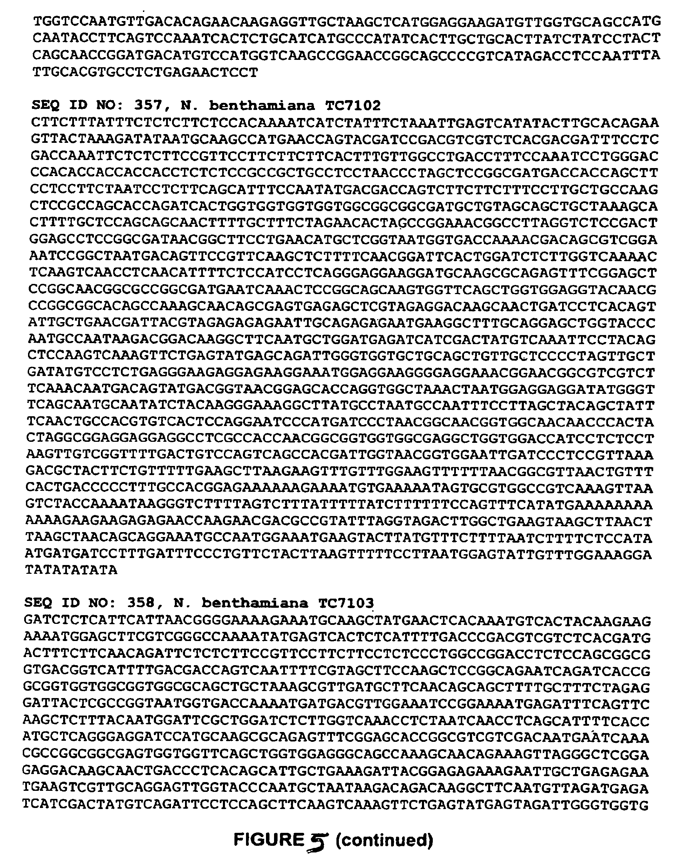

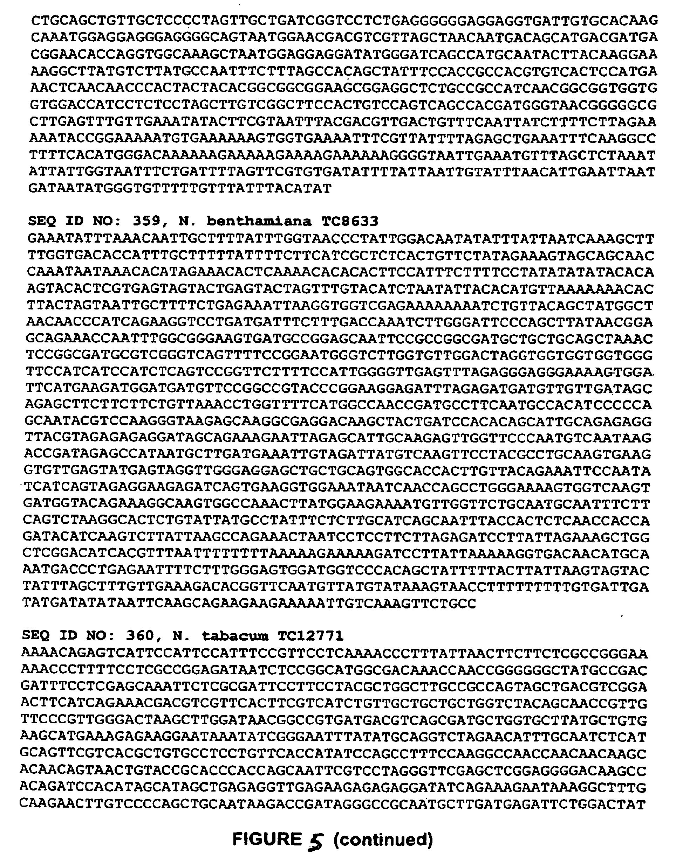

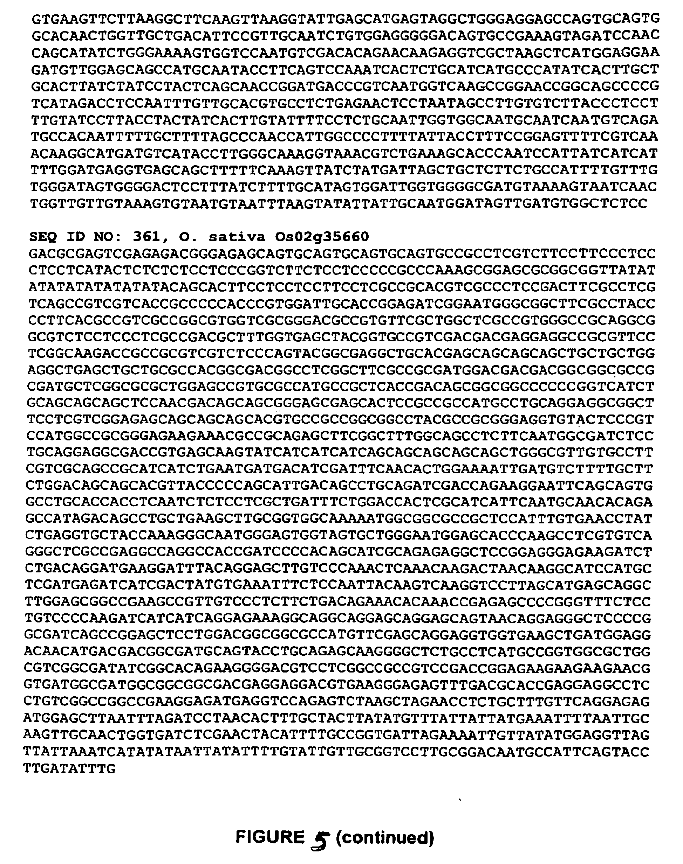

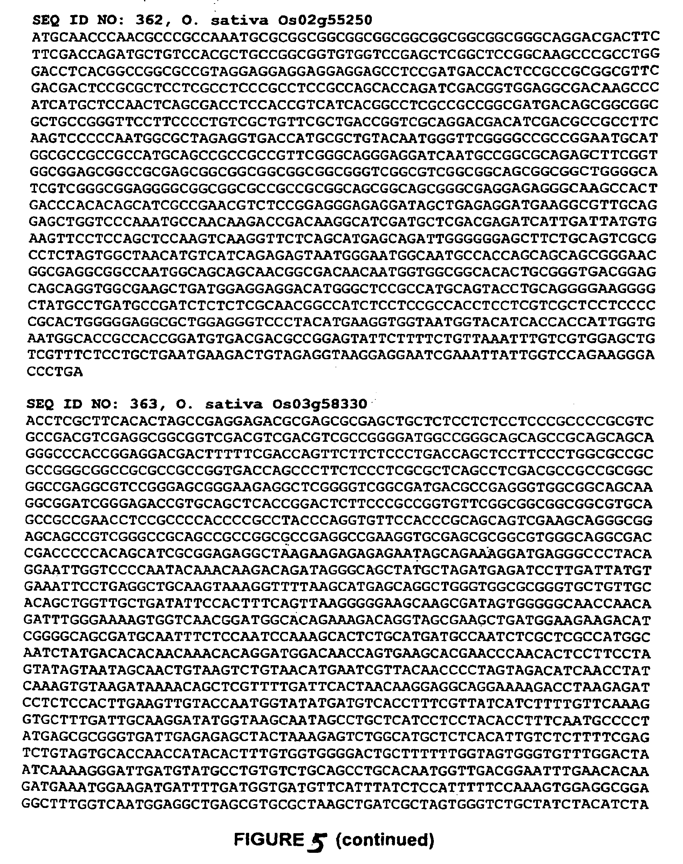

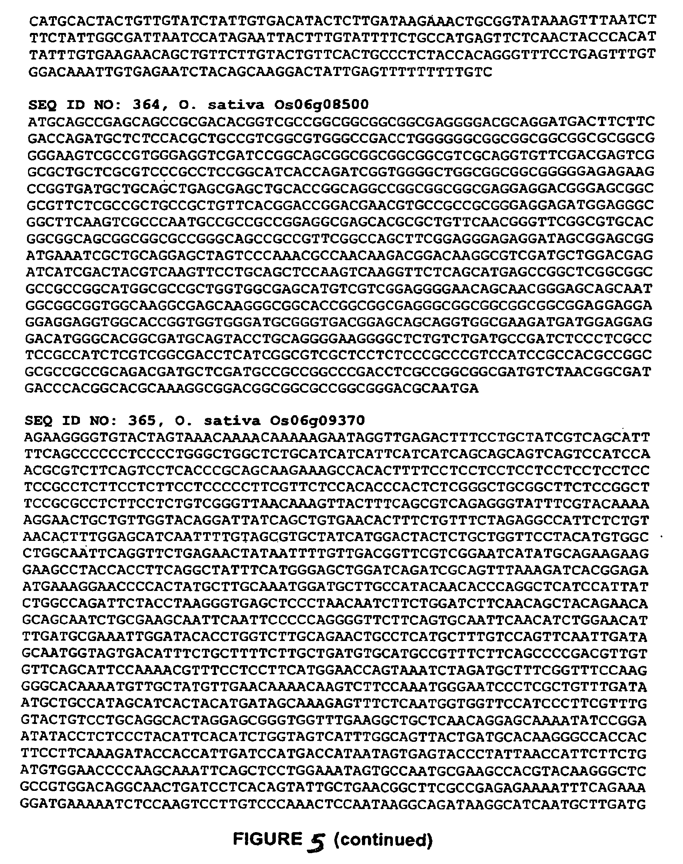

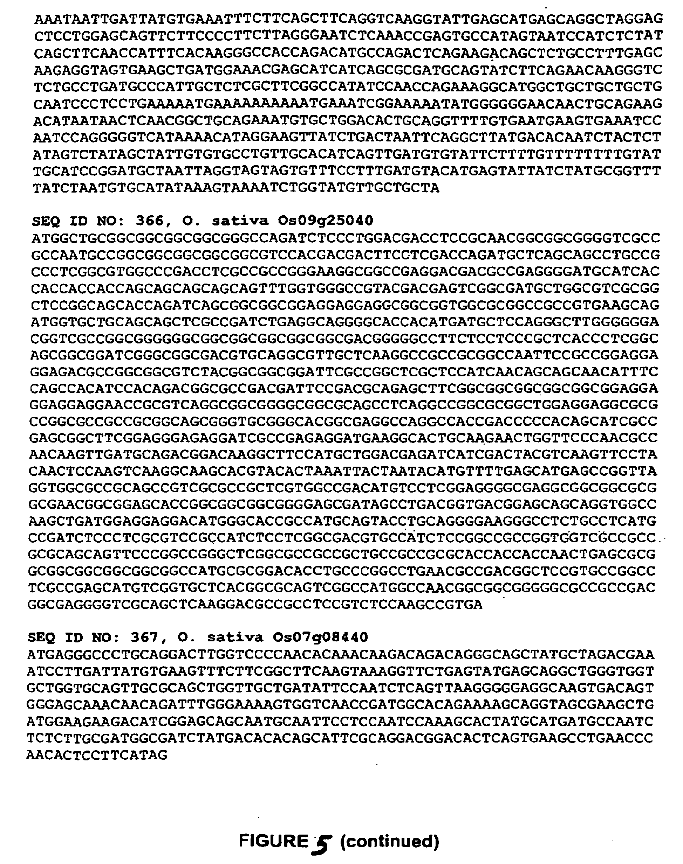

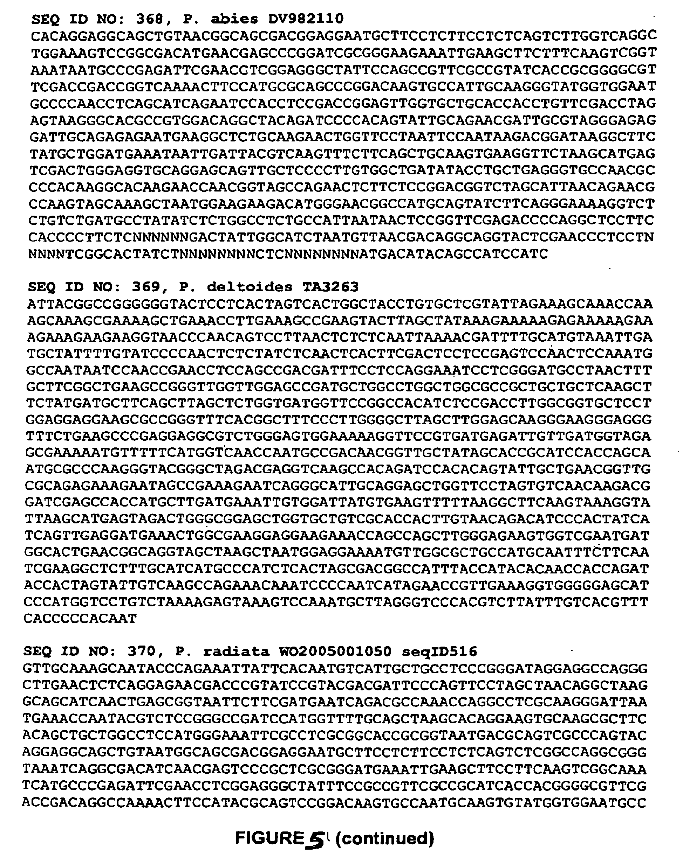

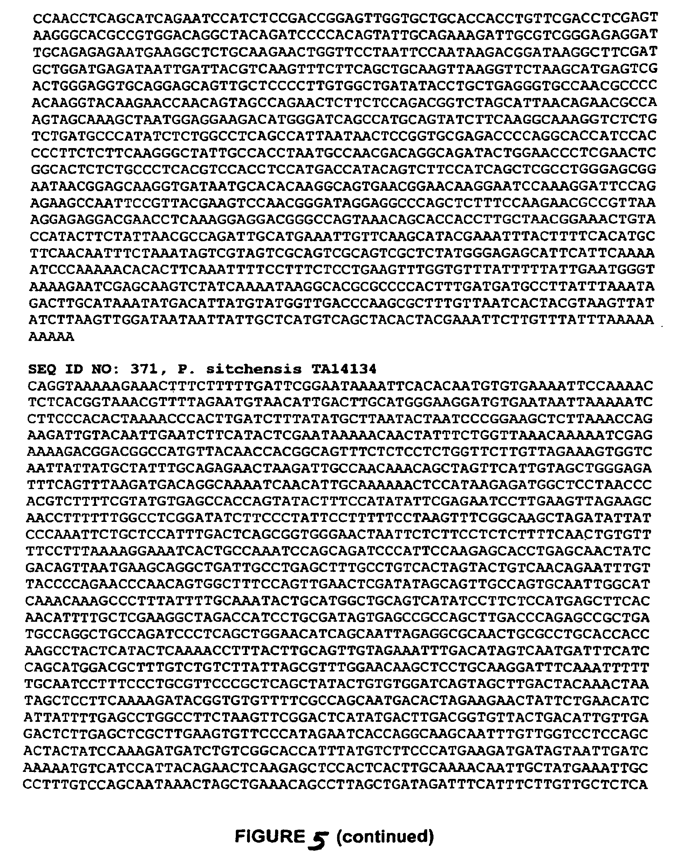

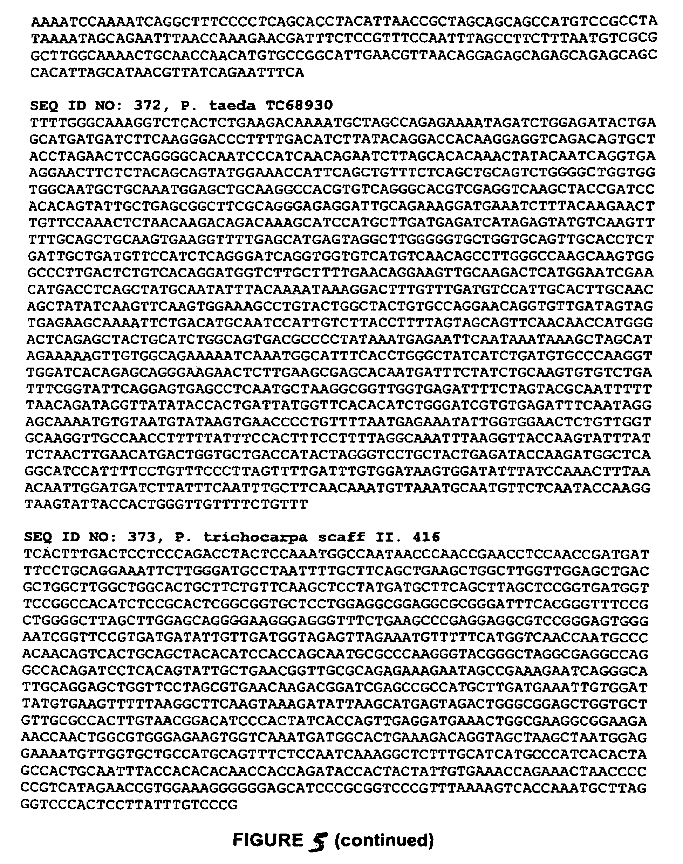

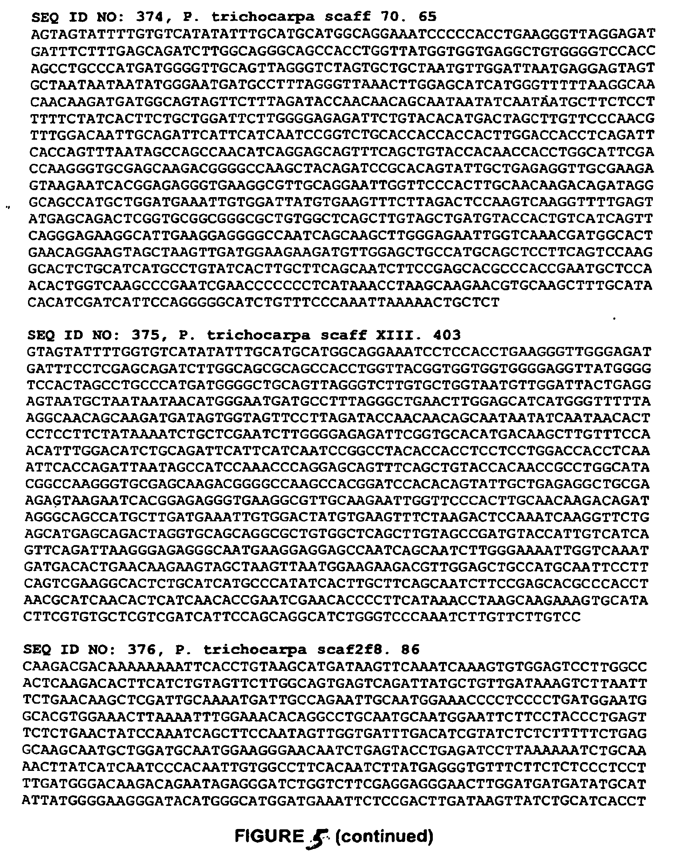

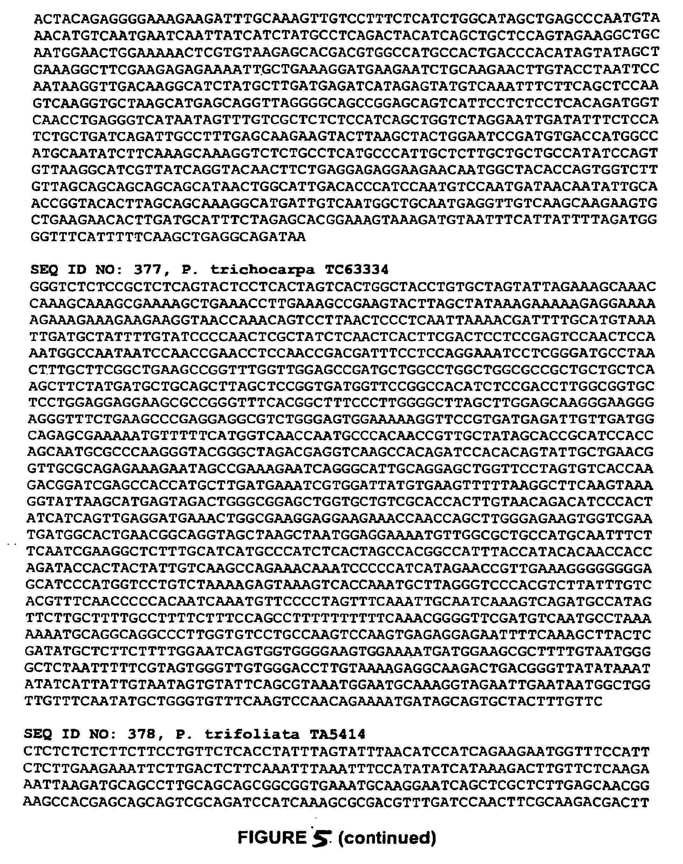

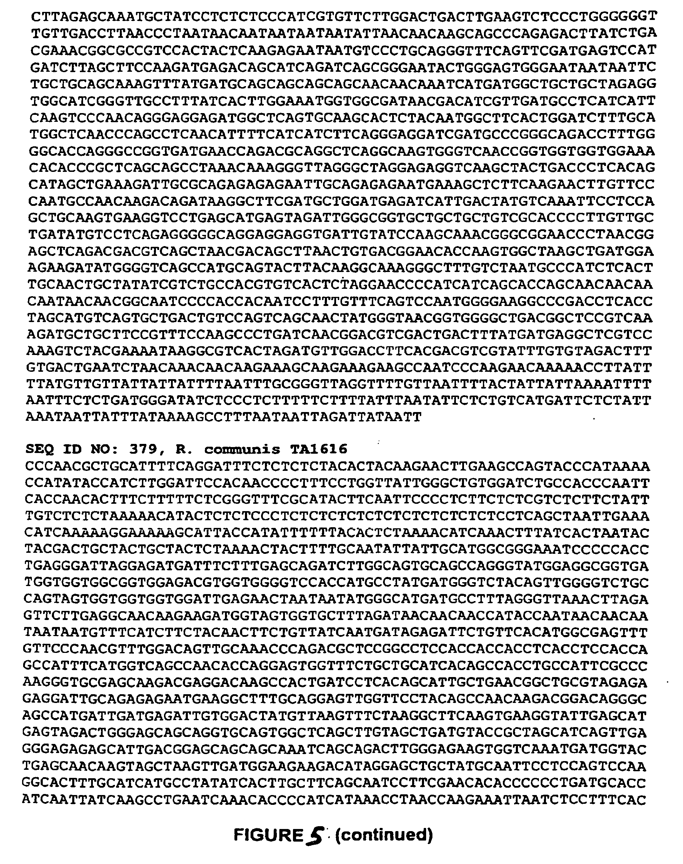

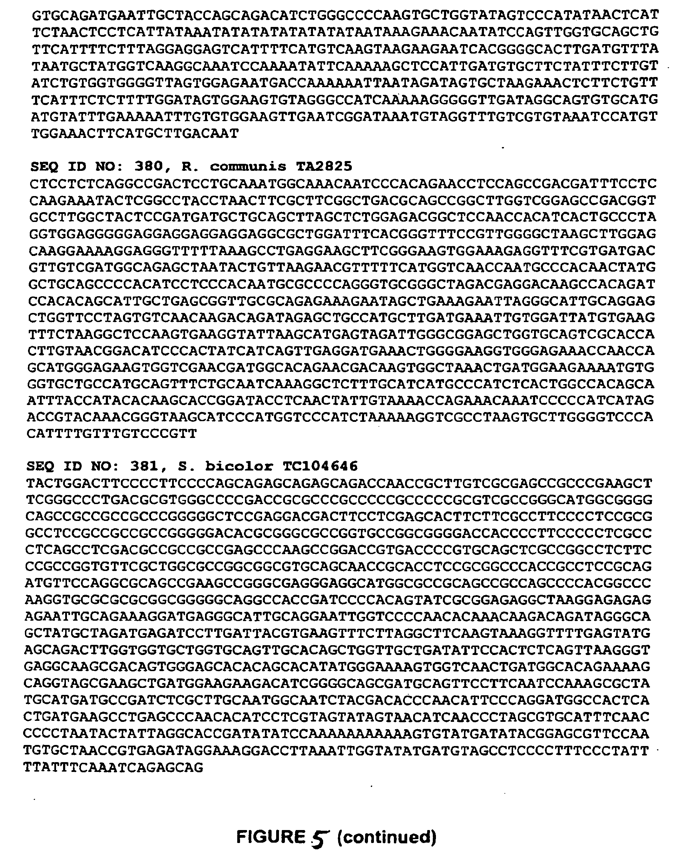

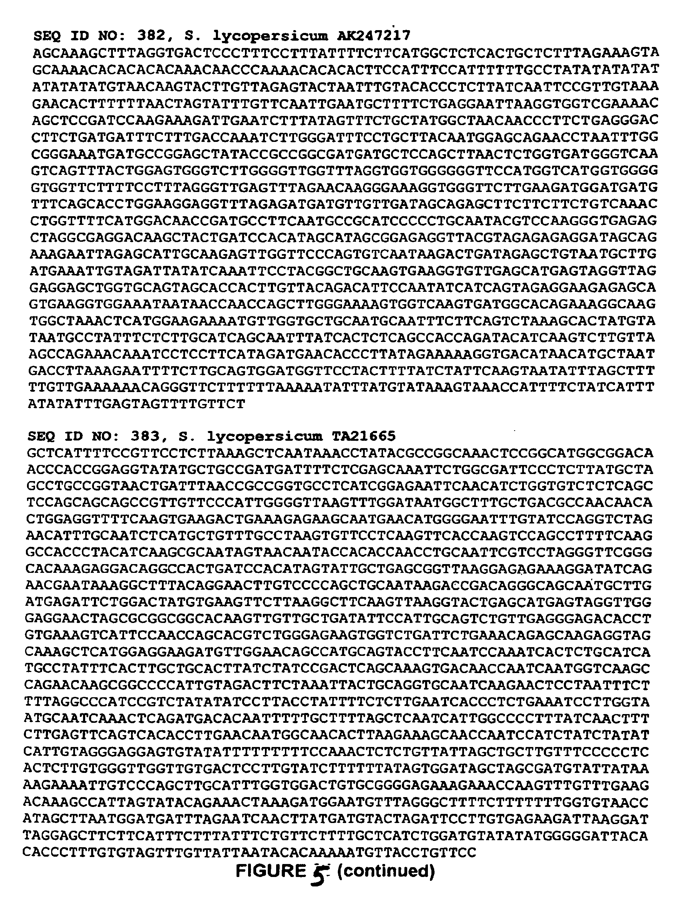

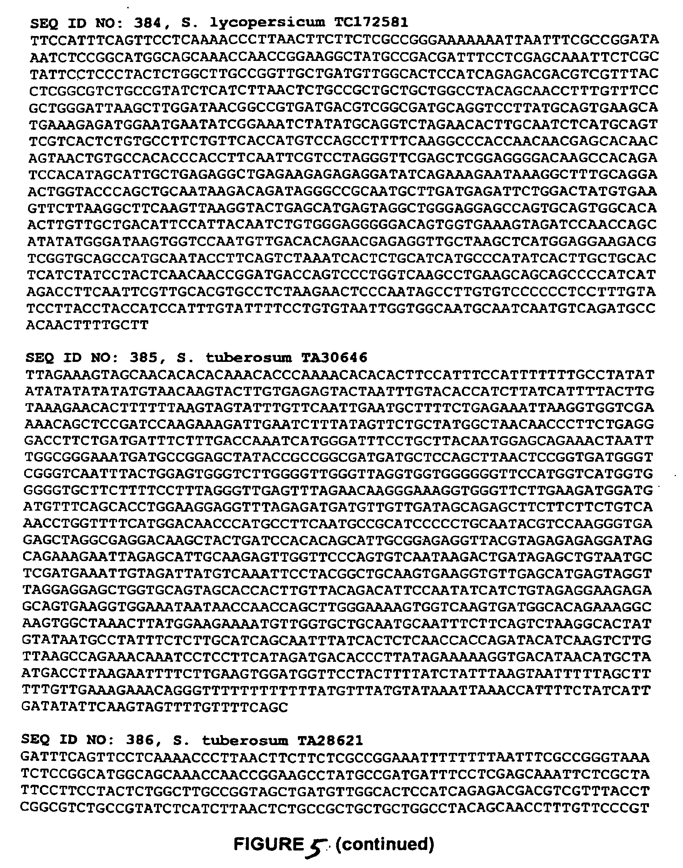

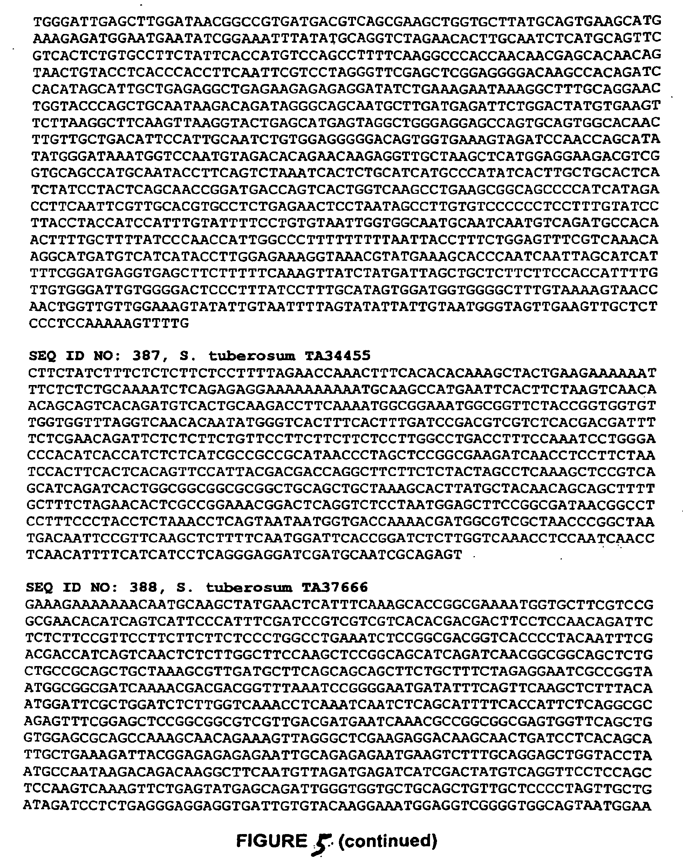

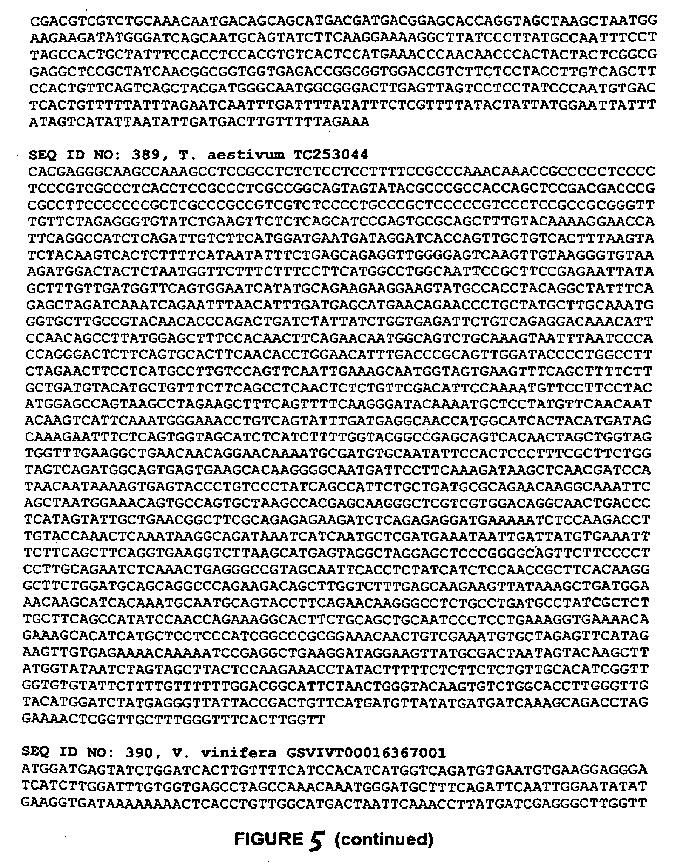

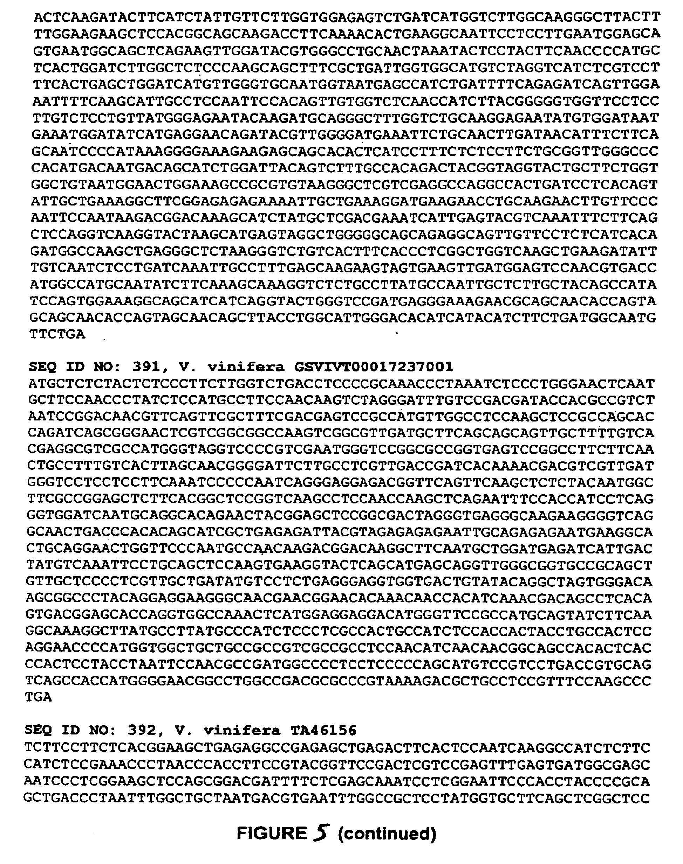

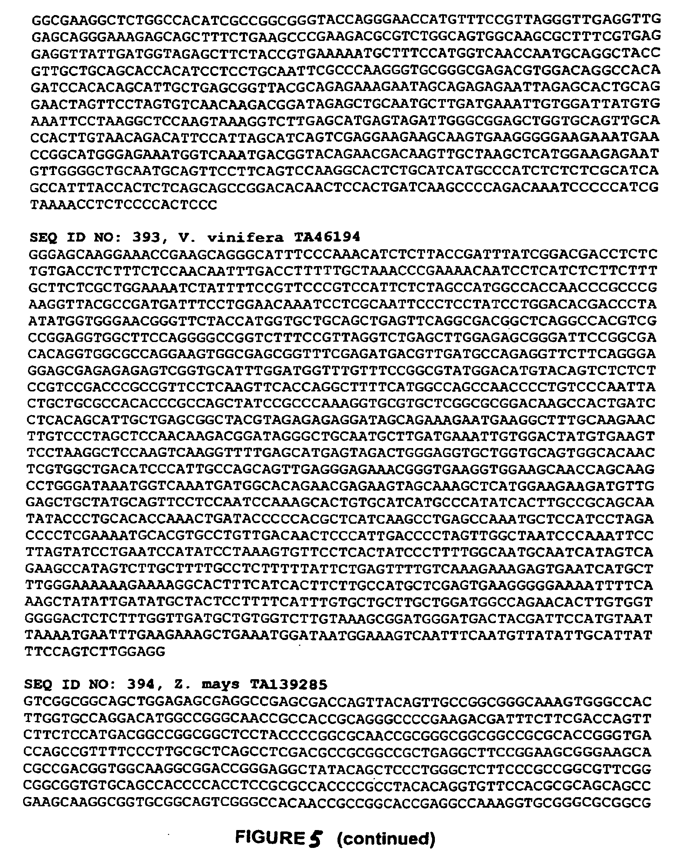

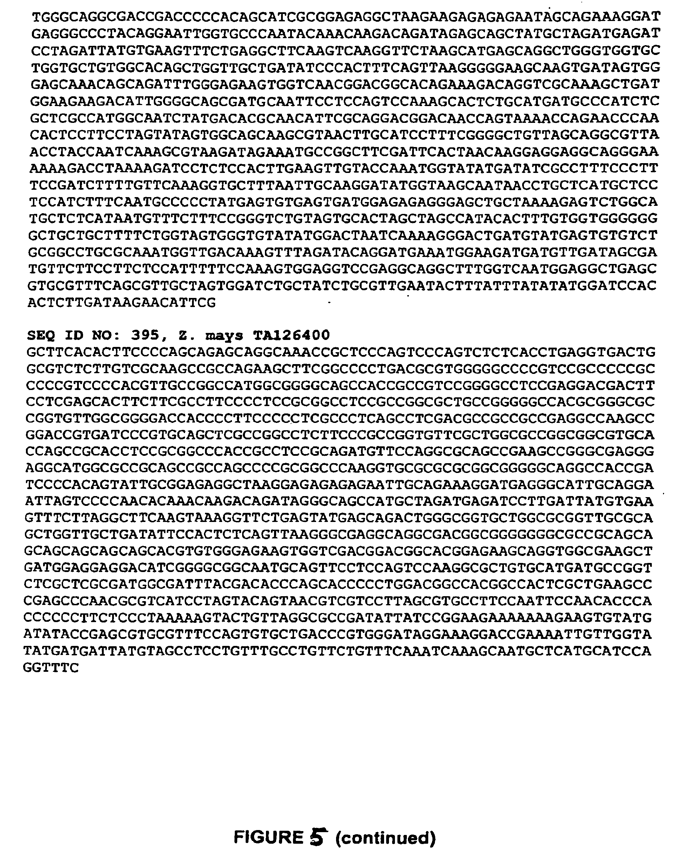

**Figure 5** details examples of bHLH11-like sequences useful in performing the methods according to the present invention.

**Examples**

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in Sambrook (2001) Molecular Cloning: a laboratory manual, 3rd Edition Cold Spring Harbor Laboratory Press, CSH, New York, or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

***Example 1: Identification of sequences related to the bHLH11-like nucleic acid sequence used in the methods of the invention***

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid used in the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified. Table E1 provides a list of nucleic acid sequences related to the nucleic acid sequence used in the methods of the present invention.

**Table E1**: Examples of bHLH11-like polypeptides:

| Plant Source | Nucleic acid SEQ ID NO: | Protein SEQ ID NO: |
|---|---|---|
| *Triticum aestivum* | 244 | 245 |
| *Allium cepa* | 258 | 327 |
| *Arabidopsis thaliana* | 259 | 328 |
| *Arabidopsis thaliana* | 260 | 329 |
| *Arabidopsis thaliana* | 261 | 330 |
| *Arabidopsis thaliana* | 262 | 331 |
| *Arabidopsis thaliana* | 263 | 332 |
| *Aquilegia vulgaris* | 264 | 333 |
| *Aquilegia vulgaris* | 265 | 334 |
| *Brassica napus* | 266 | 335 |
| *Citrus clementina* | 267 | 336 |
| *Curcuma longa* | 268 | 337 |
| *Citrus paridisi hybrid* | 269 | 338 |
| *Citrus sinensis* | 270 | 339 |
| *Eucalyptus grandis* | 271 | 340 |
| *Eucalyptus grandis* | 272 | 341 |
| *Gossypium hirsutum* | 273 | 342 |
| *Gossypium hirsutum* | 274 | 343 |
| *Gossypium hirsutum* | 275 | 344 |
| *Gossypium hirsutum* | 276 | 345 |
| *Gossypium hirsutum* | 277 | 346 |
| *Gossypium hirsutum* | 278 | 347 |
| *Glycine max* | 279 | 348 |
| *Glycine max* | 280 | 349 |
| *Glycine max* | 281 | 350 |
| *Glycine max* | 282 | 351 |
| *Gossypium raimondii* | 283 | 352 |
| *Helianthus petiolaris* | 284 | 353 |

(continued)

| Plant Source | Nucleic acid SEQ ID NO: | Protein SEQ ID NO: |
|---|---|---|
| *Hordeum vulgare* | 285 | 354 |
| *Lactuca perennis* | 286 | 355 |
| *Nicotiana benthamiana* | 287 | 356 |
| *Nicotiana benthamiana* | 288 | 357 |
| *Nicotiana benthamiana* | 289 | 358 |
| *Nicotiana benthamiana* | 290 | 359 |
| *Nicotiana tabacum* | 291 | 360 |
| *Oryza sativa* | 292 | 361 |
| *Oryza sativa* | 293 | 362 |
| *Oryza sativa* | 294 | 363 |
| *Oryza sativa* | 295 | 364 |
| *Oryza sativa* | 296 | 365 |
| *Oryza sativa* | 297 | 366 |
| *Oryza sativa* | 298 | 367 |
| *Picea abies* | 299 | 368 |
| *Populus deltoides* | 300 | 369 |
| *Pinus radiata* | 301 | 370 |
| *Picea sitchensis* | 302 | 371 |
| *Pinus taeda* | 303 | 372 |
| *Populus trichocarpa* | 304 | 373 |
| *Populus trichocarpa* | 305 | 374 |
| *Populus trichocarpa* | 306 | 375 |
| *Populus trichocarpa* | 307 | 376 |
| *Populus trichocarpa* | 308 | 377 |
| *Poncirus trifoliata* | 309 | 378 |
| *Ricinus communis* | 310 | 379 |
| *Ricinus communis* | 311 | 380 |
| *Sorghum bicolor* | 312 | 381 |
| *Solanum lycopersicum* | 313 | 382 |
| *Solanum lycopersicum* | 314 | 383 |
| *Solanum lycopersicum* | 315 | 384 |
| *Solanum tuberosum* | 316 | 385 |
| *Solanum tuberosum* | 317 | 386 |
| *Solanum tuberosum* | 318 | 387 |
| *Solanum tuberosum* | 319 | 388 |
| *Triticum aestivum* | 320 | 389 |
| *Vitis vinifera* | 321 | 390 |
| *Vitis vinifera* | 322 | 391 |

(continued)

| Plant Source | Nucleic acid SEQ ID NO: | Protein SEQ ID NO: |
|---|---|---|
| *Vitis vinifera* | 323 | 392 |
| *Vitis vinifera* | 324 | 393 |
| *Zea mays* | 325 | 394 |
| *Zea mays* | 326 | 395 |

In some instances, related sequences have tentatively been assembled and publicly disclosed by research institutions, such as The Institute for Genomic Research (TIGR). The Eukaryotic Gene Orthologs (EGO) database may be used to identify such related sequences, either by keyword search or by using the BLAST algorithm with the nucleic acid or polypeptide sequence of interest.

***Example 2: Alignment of bHLH11-like polypeptide sequences***

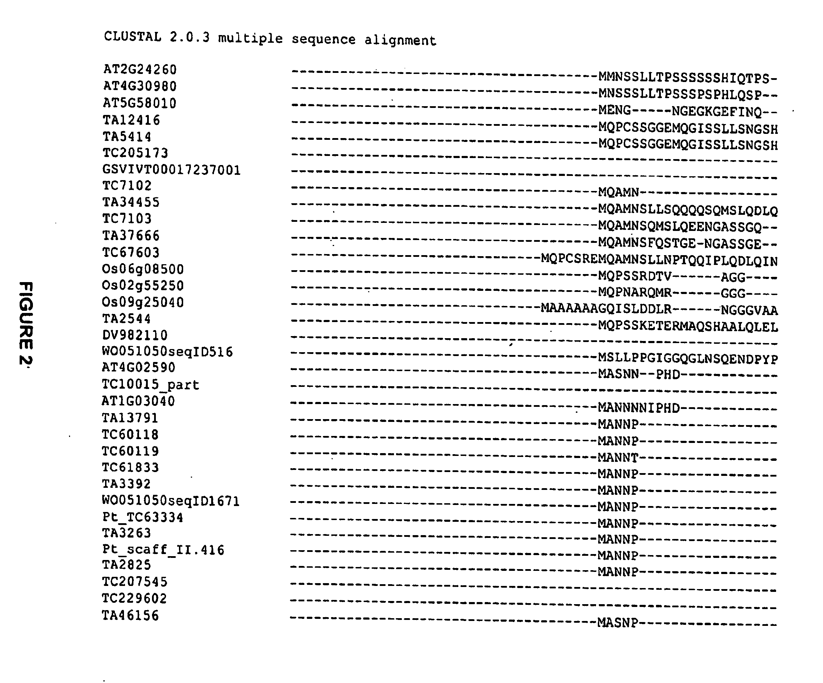

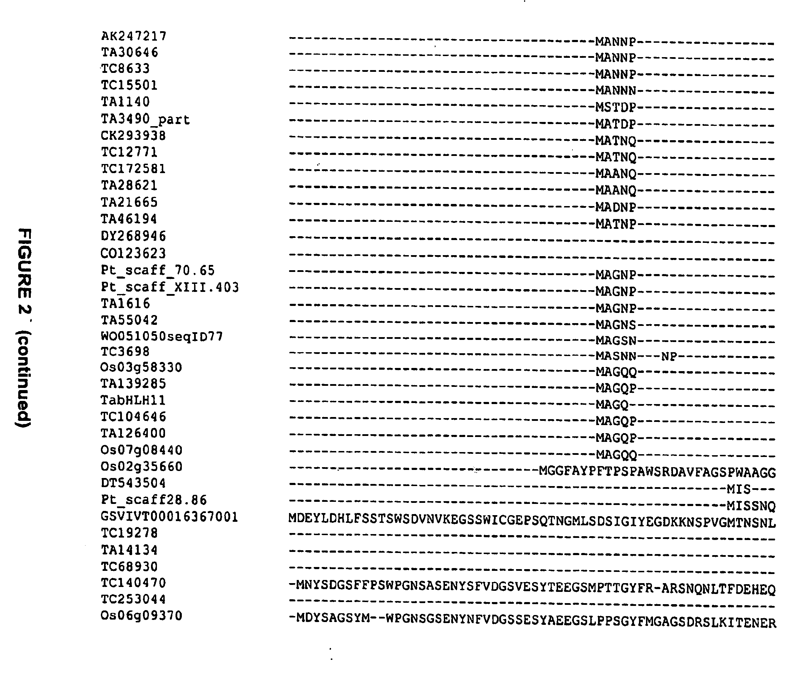

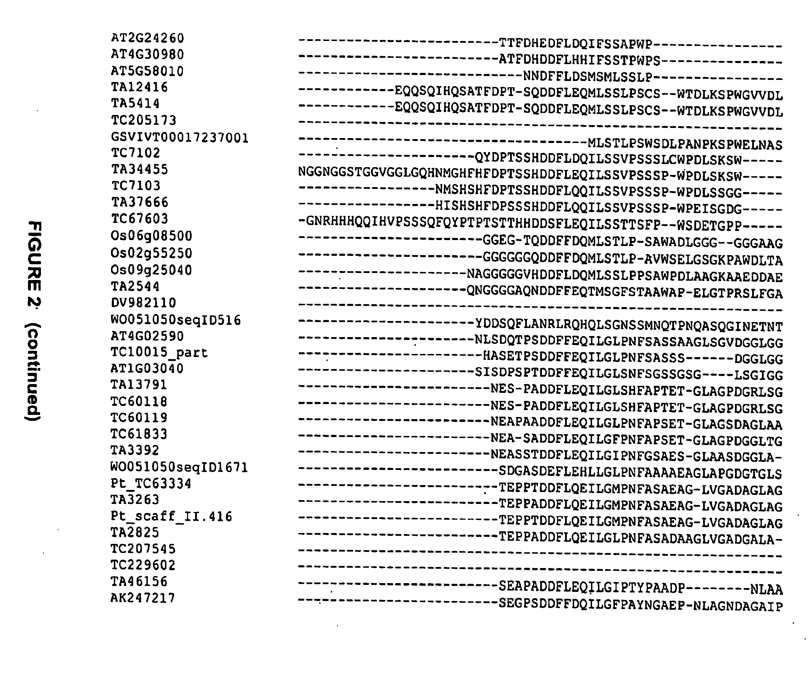

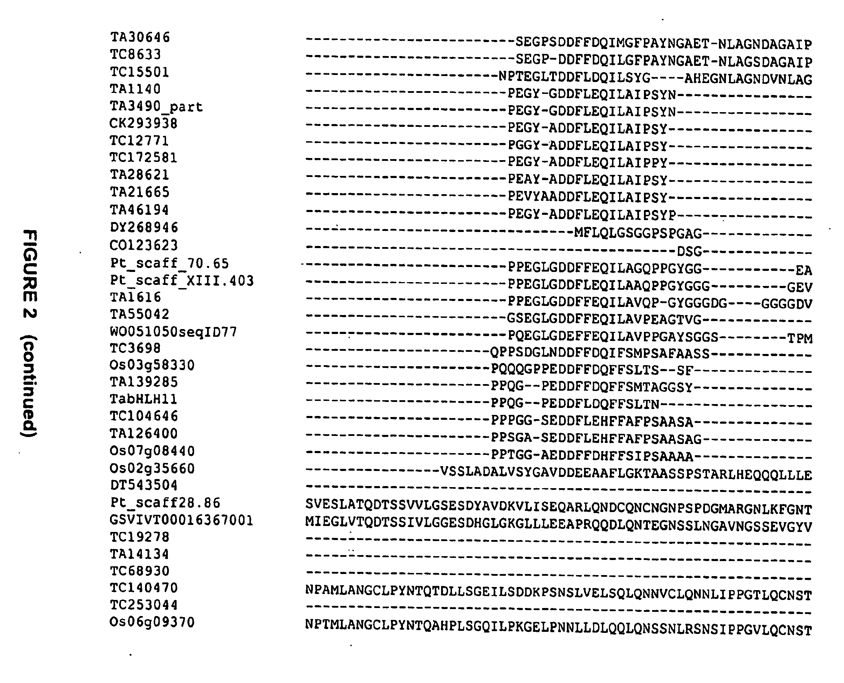

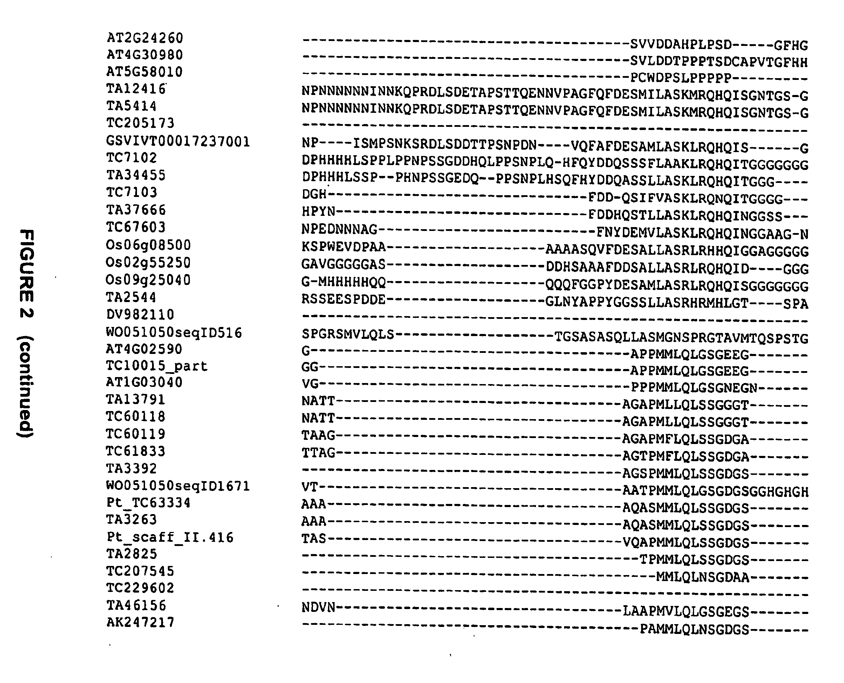

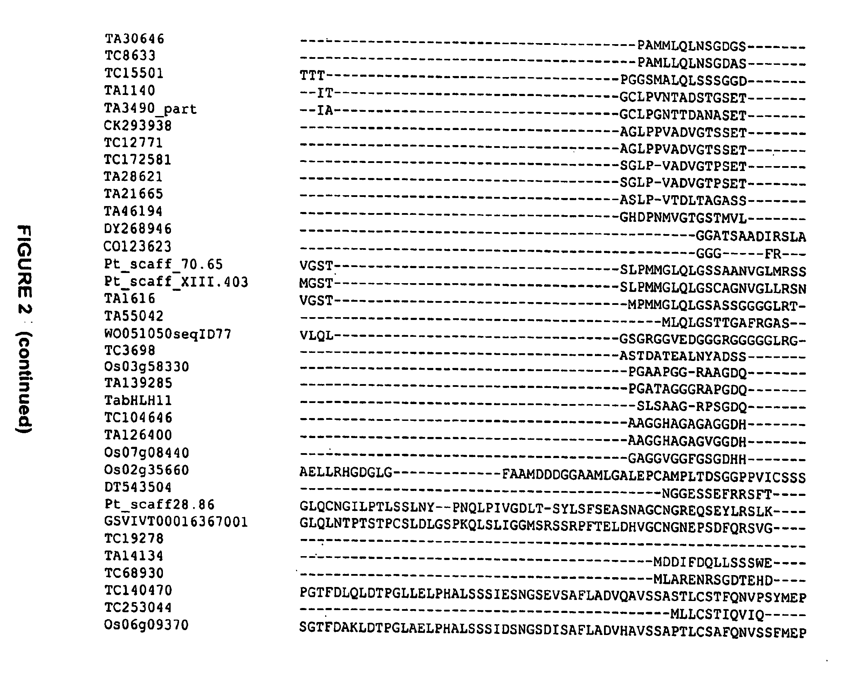

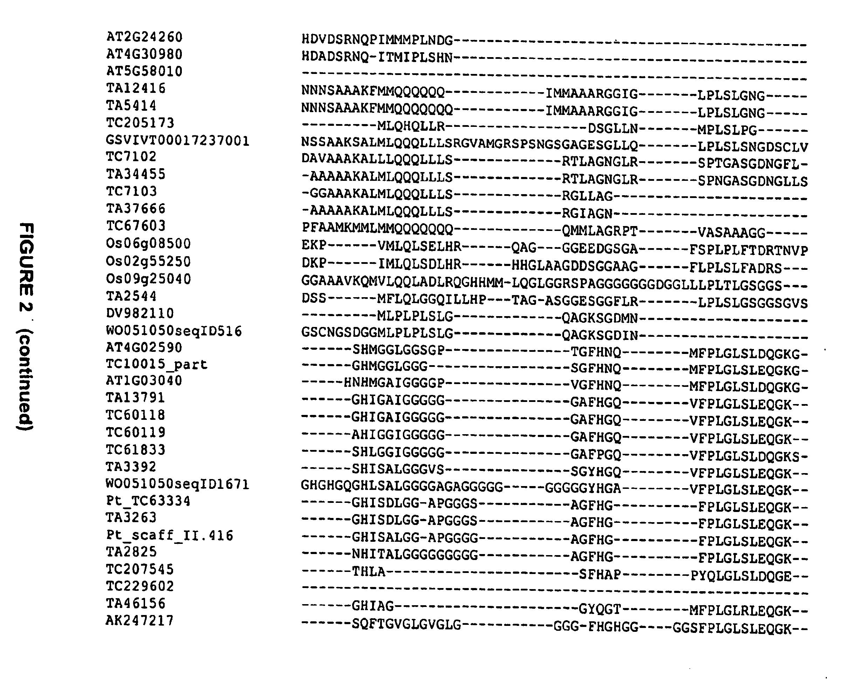

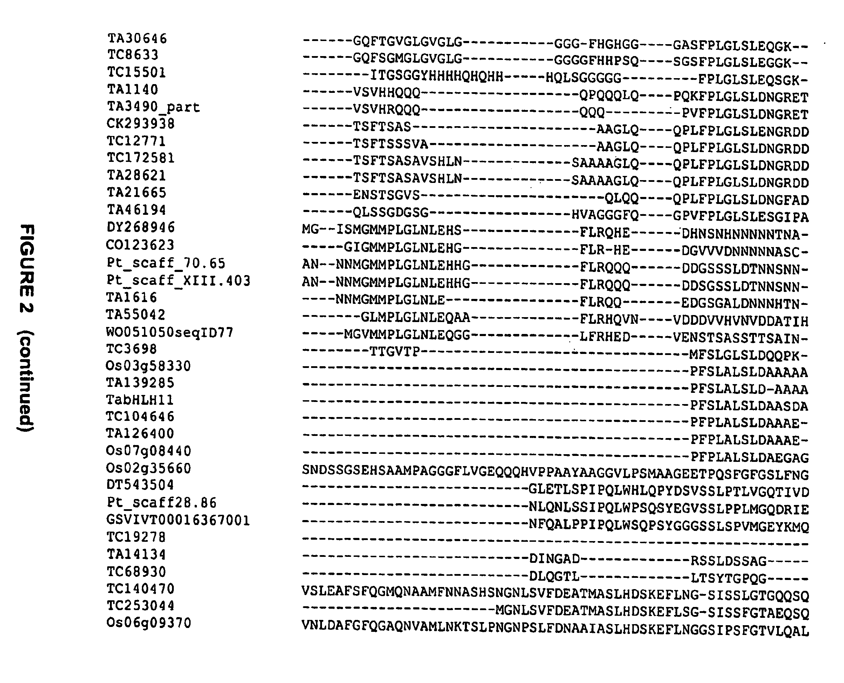

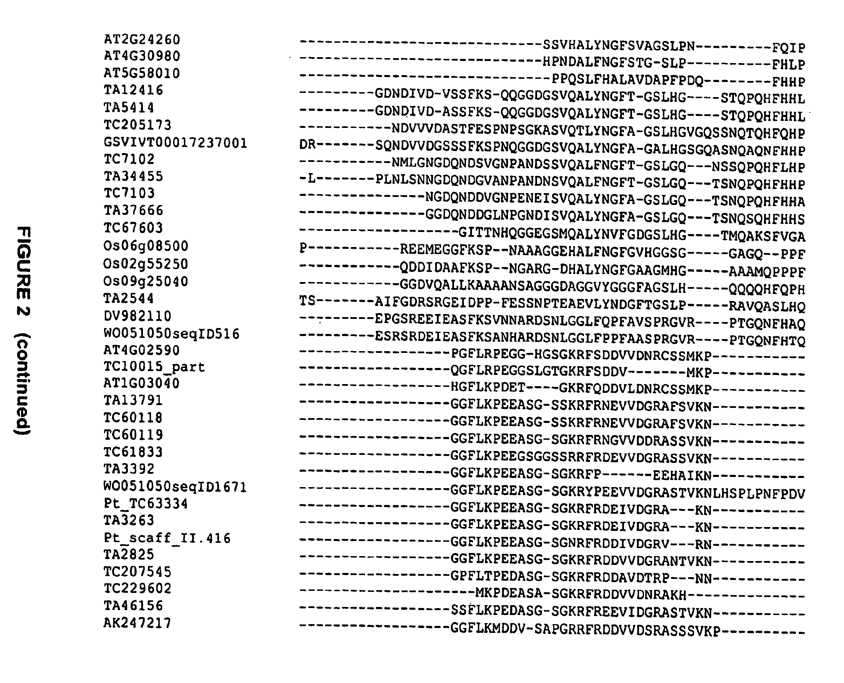

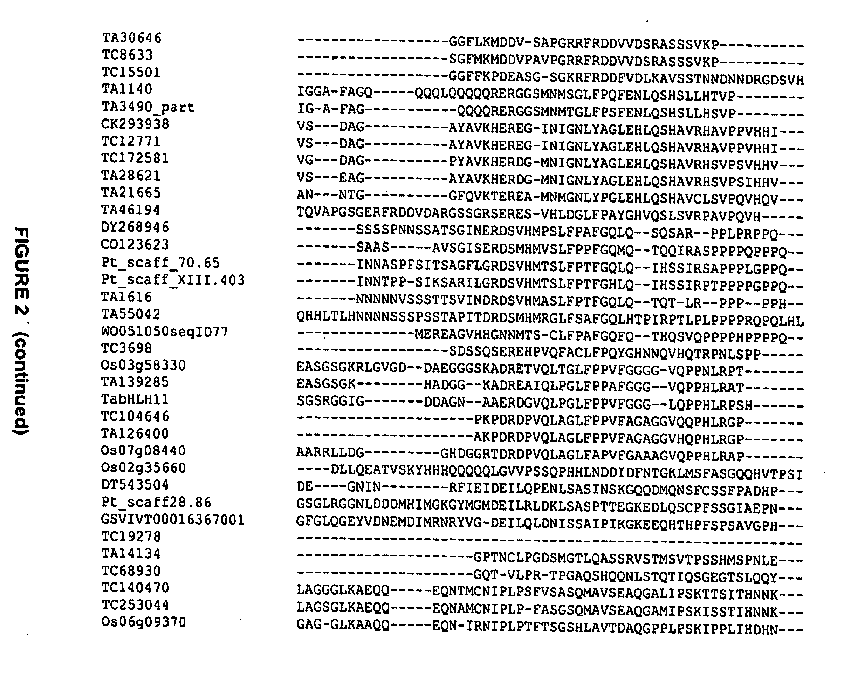

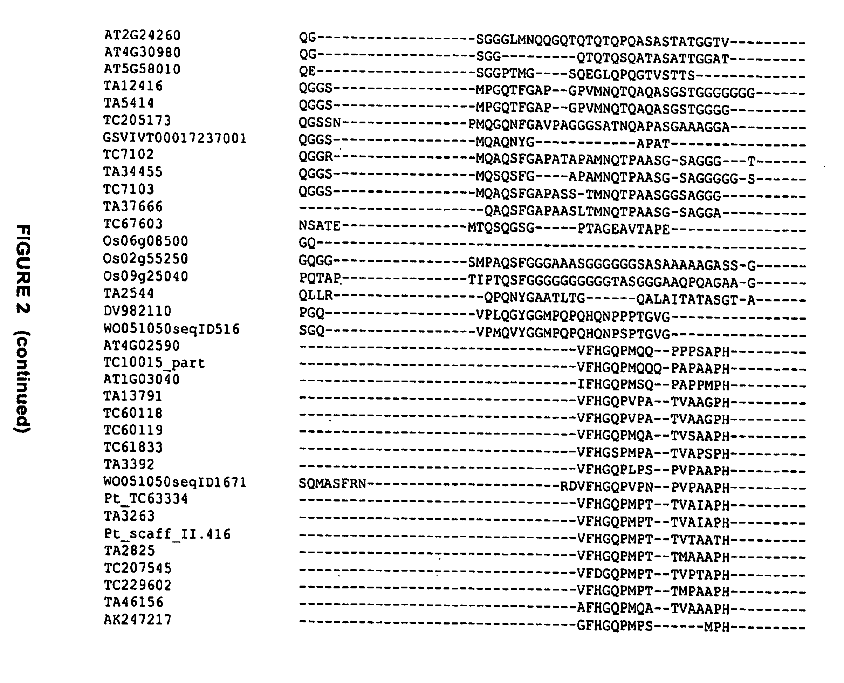

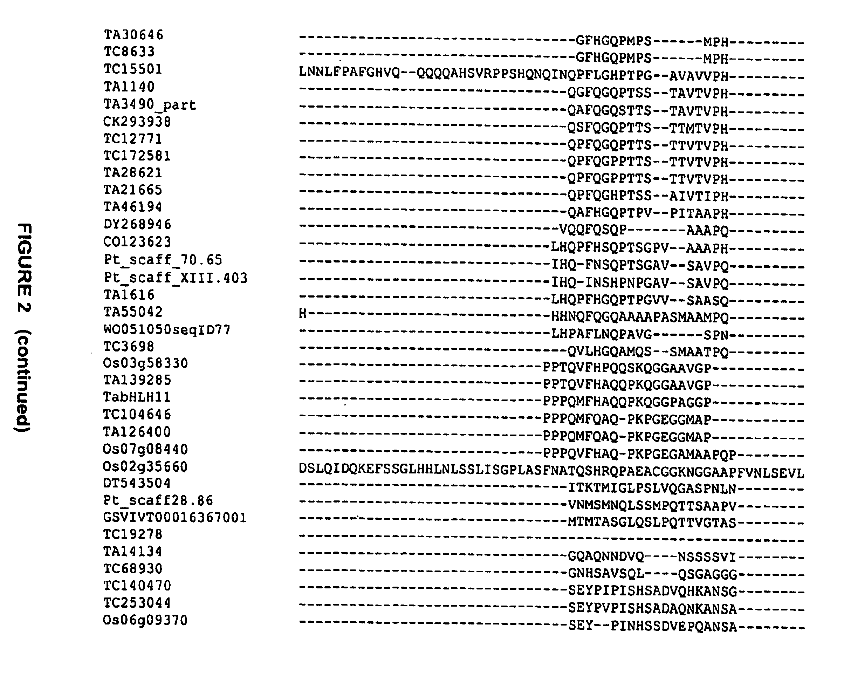

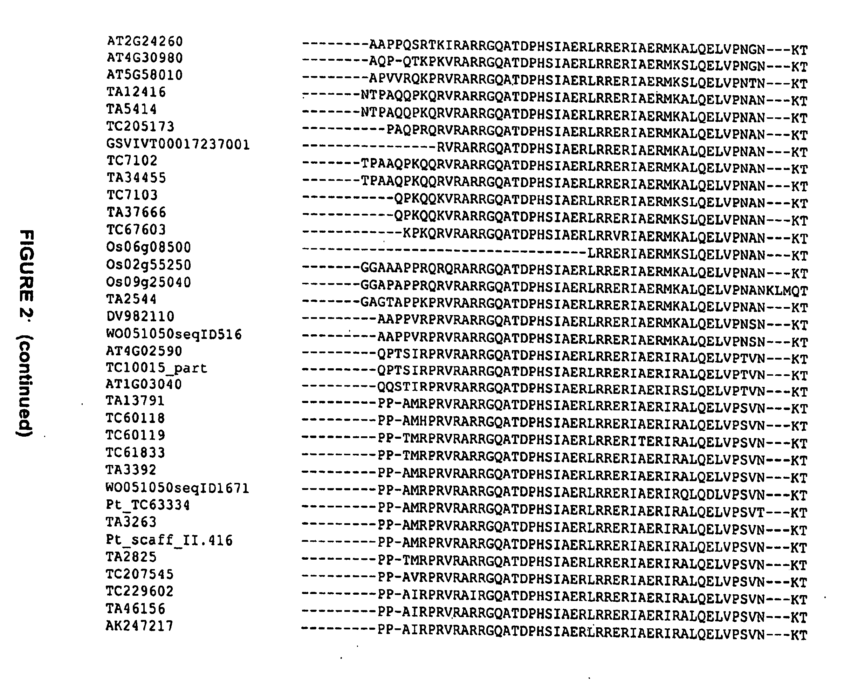

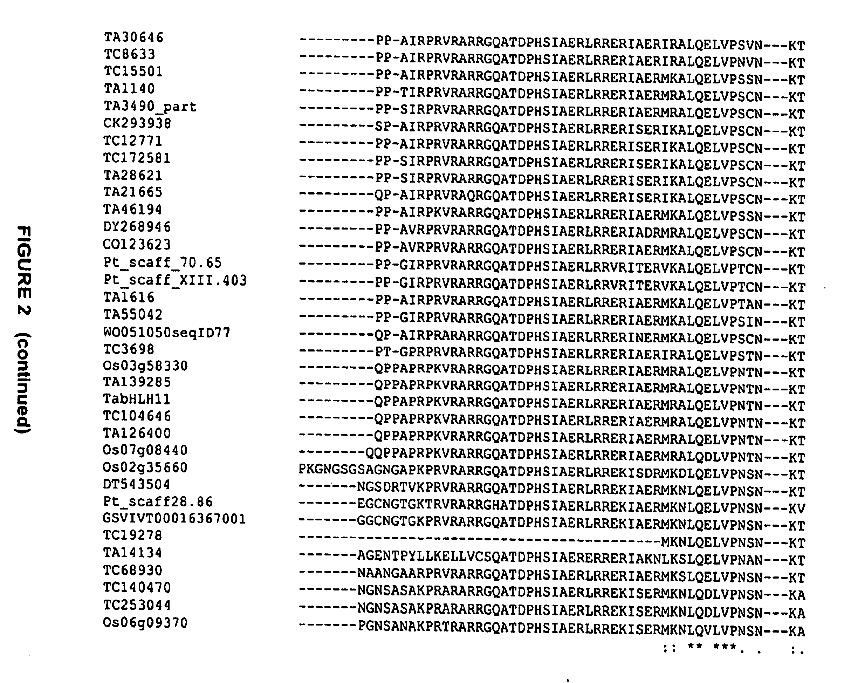

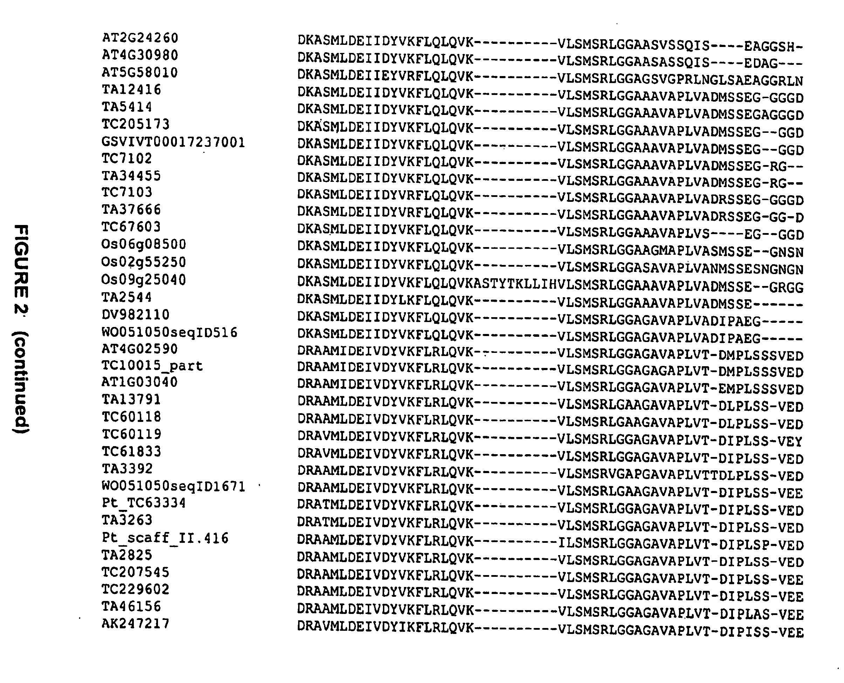

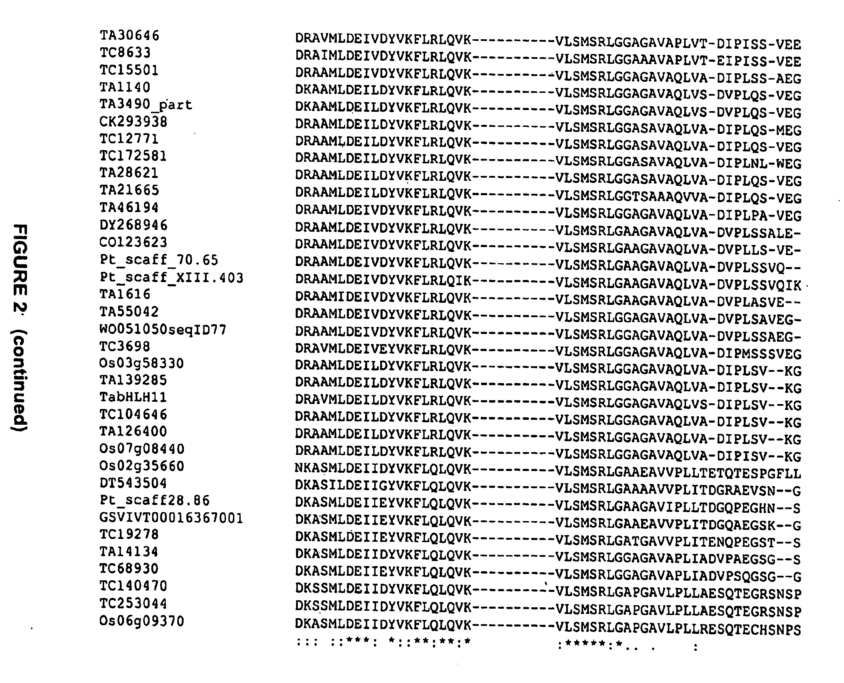

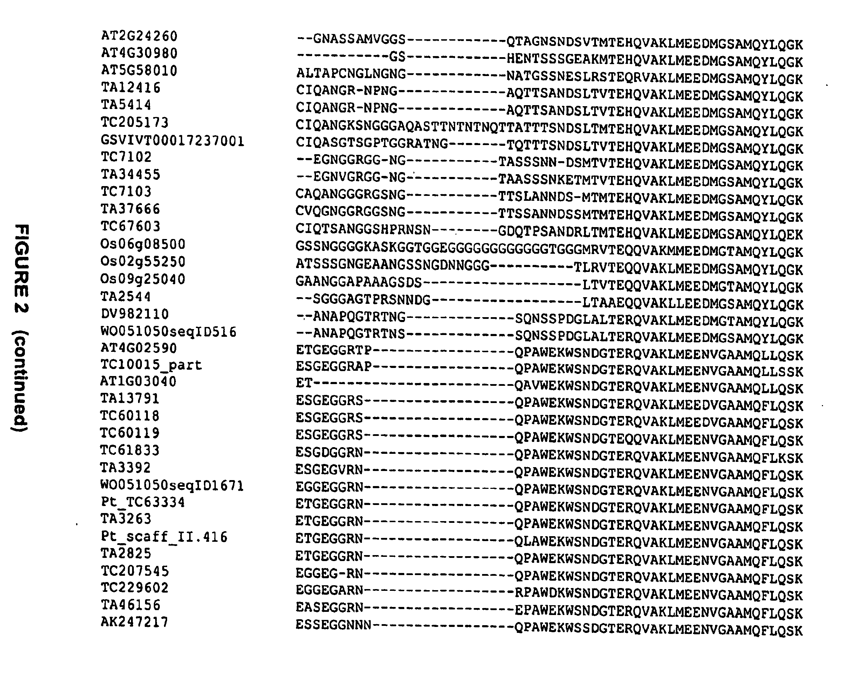

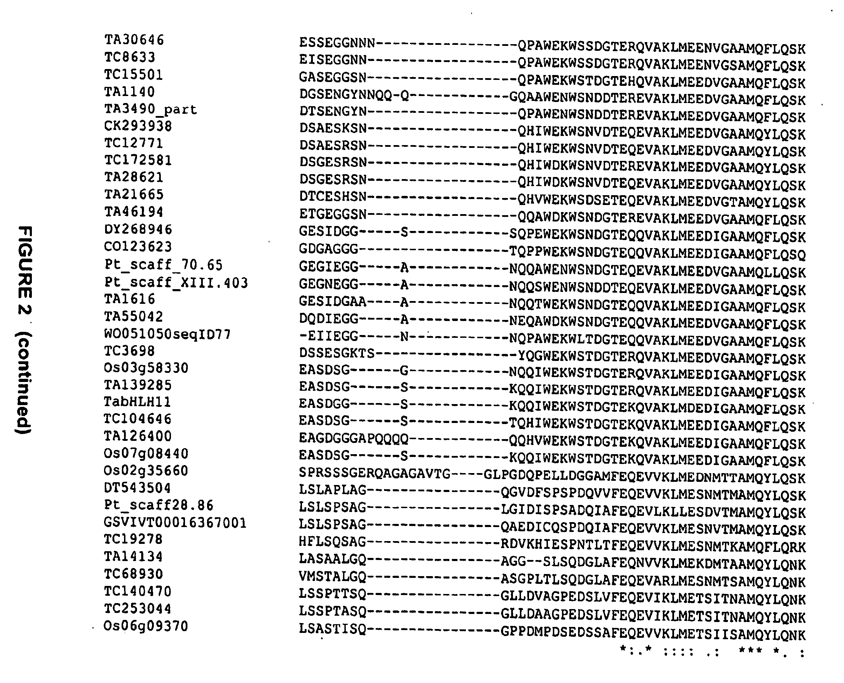

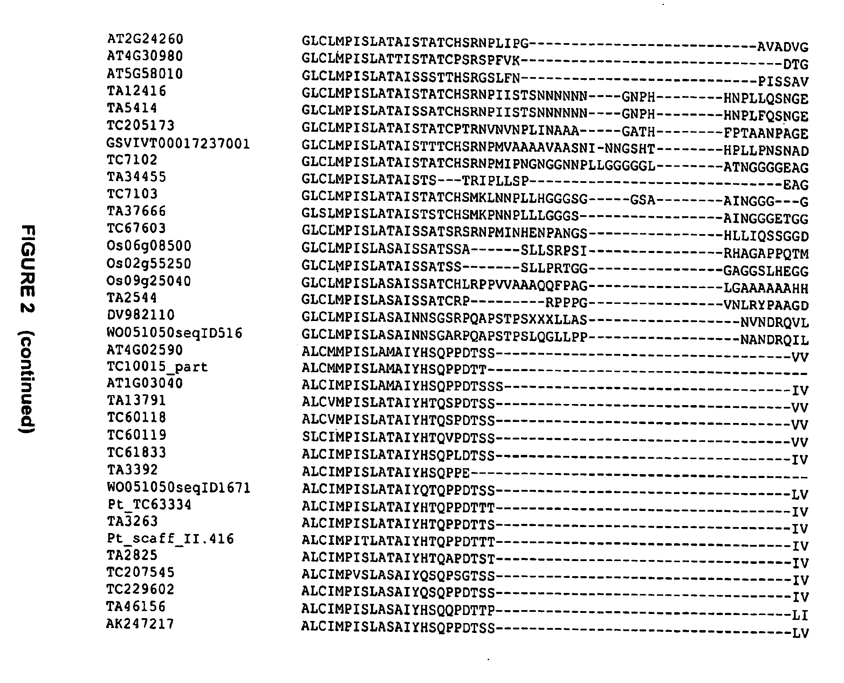

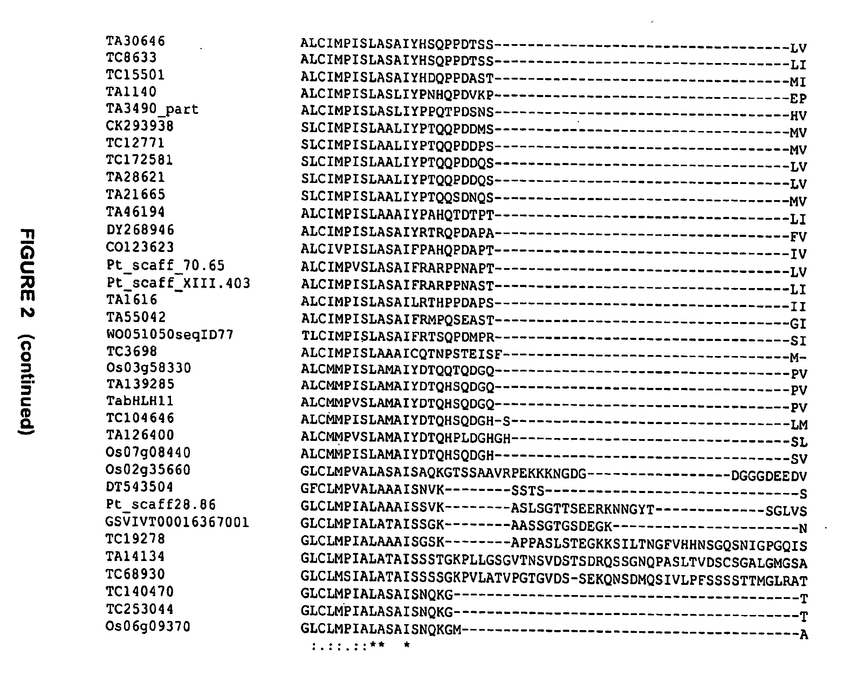

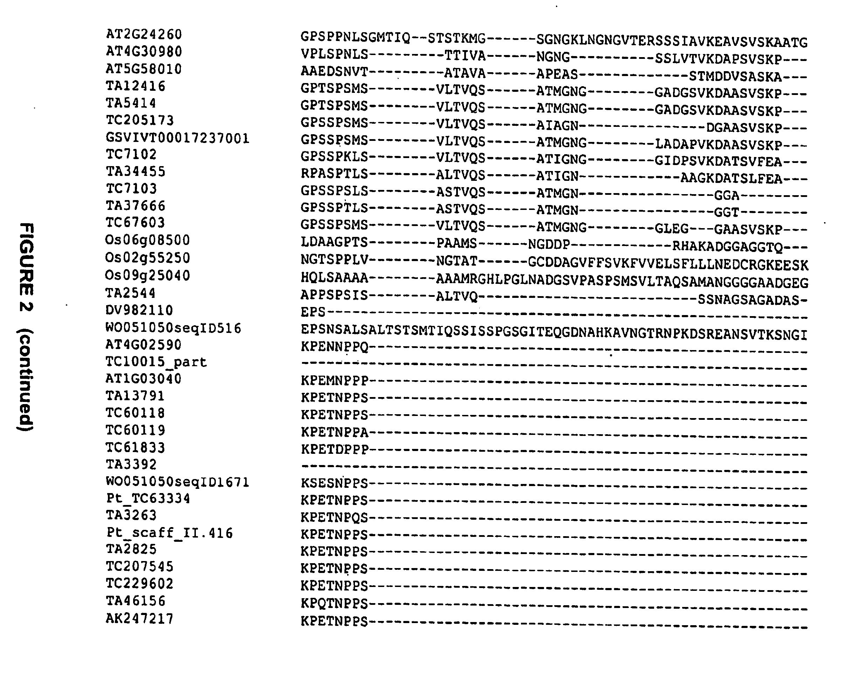

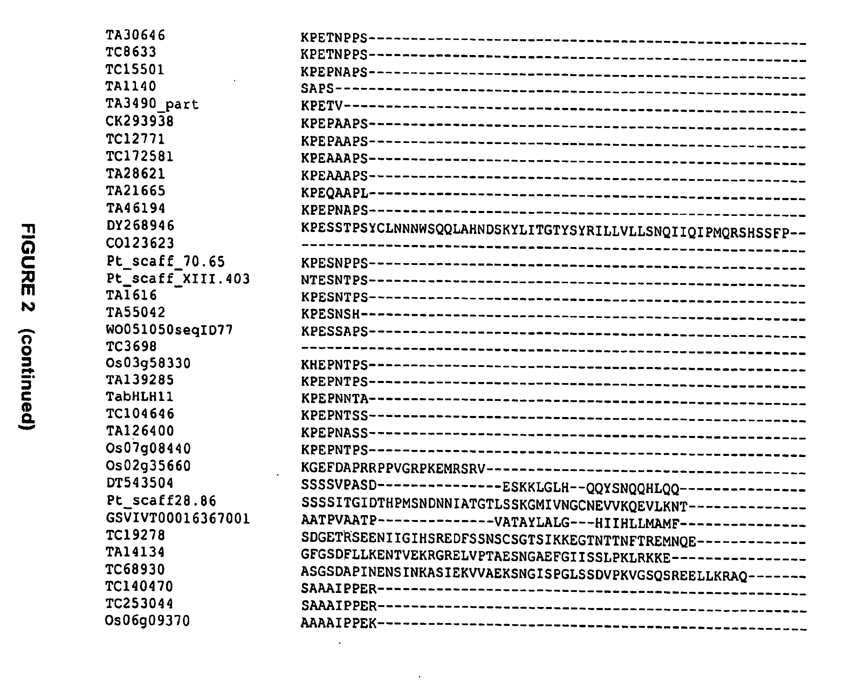

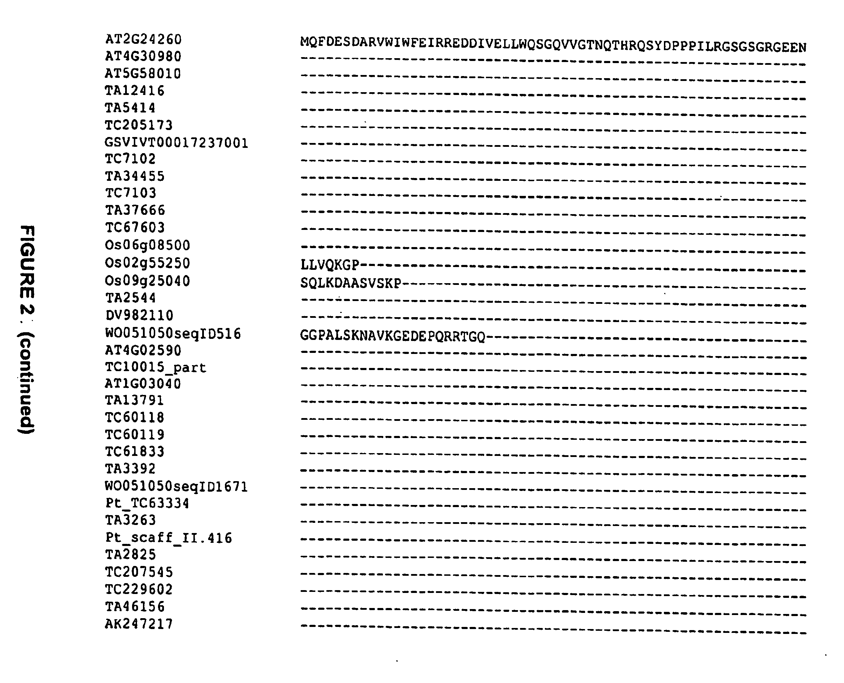

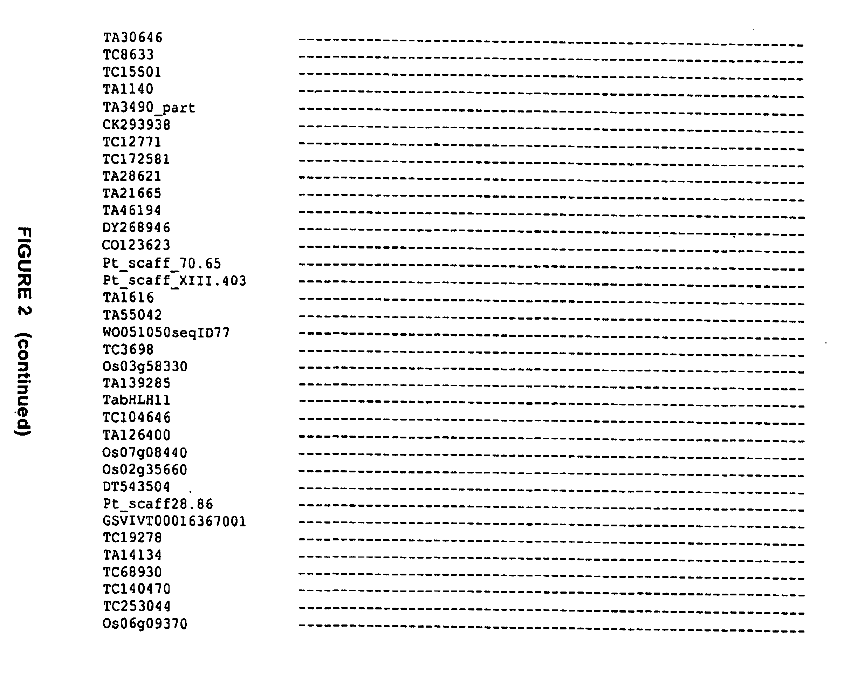

Alignment of polypeptide sequences was performed using the AlignX programme from the Vector NTI (Invitrogen) which is based on the popular Clustal W algorithm of progressive alignment (Thompson et al. (1997) Nucleic Acids Res 25:4876-4882; Chenna et al. (2003). Nucleic Acids Res 31:3497-3500). Default values are for the gap open penalty of 10, for the gap extension penalty of 0,1 and the selected weight matrix is Blosum 62 (if polypeptides are aligned). Minor manual editing was done to further optimise the alignment. Sequence conservation among bHLH11-like polypeptides is essentially in the C-terminal bHLH domain of the polypeptides, the N-terminal domain usually being more variable in sequence length and composition. The bHLH11-like polypeptides are aligned in Figure 2.

A phylogenetic tree of bHLH11-like polypeptides (Figure 3) was constructed using "CLUSTALX", and a neighbour-joining tree was calculated. The circular cladogram was drawn using Dendroscope (Huson et al., 2007).

***Example 3: Calculation of global percentage identity between polypeptide sequences useful in performing the methods of the invention***

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

Scoring matrix:     Blosum62
First Gap:          12
Extending gap:      2

Results of the software analysis are shown in Table E2 for the global similarity and identity over the full length of the polypeptide sequences. Percentage identity is given above the diagonal and percentage similarity is given below the diagonal. SEQ ID NO: 245 is represented as TabHLH11.

The percentage identity between the bHLH11-like polypeptide sequences useful in performing the methods of the invention can be as low as 20 % amino acid identity compared to SEQ ID NO: 245. The identity is however much higher when the HLH domains are compared (Table E3).

EP 2 230 310 B1

Table E2: MatGAT results for global similarity and identity over the full length of the bHLH11-like polypeptide sequences.

| | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|
| 1. TC3698 | | 51.1 | 53.5 | 15.9 | 39.5 | 37.1 | 49.4 | 19.5 | 53.8 | 45.8 | 36.3 | 53.7 | 30.6 | 52.0 | 46.5 | 51.0 | 52.2 | 51.7 | 51.2 | 34.6 |
| 2. AT1G03040 | 63.2 | | 81.0 | 16.3 | 37.8 | 36.3 | 52.4 | 19.9 | 72.3 | 40.5 | 35.9 | 64.6 | 31.1 | 50.7 | 59.5 | 45.9 | 58.4 | 68.1 | 45.3 | 37.0 |
| 3. AT4G02590 | 64.2 | 89.0 | | 17.0 | 37.0 | 35.1 | 55.2 | 18.8 | 82.4 | 43.0 | 35.4 | 69.5 | 33.8 | 53.5 | 64.6 | 47.6 | 62.6 | 72.6 | 47.0 | 36.4 |
| 4. AT2G24260 | 21.7 | 22.0 | 22.3 | | 27.7 | 19.7 | 16.4 | 12.7 | 15.6 | 17.0 | 19.0 | 15.8 | 23.0 | 15.8 | 15.8 | 16.8 | 15.9 | 16.8 | 15.4 | 17.2 |
| 5. AT4G30980 | 53.2 | 53.9 | 52.6 | 30.6 | | 46.4 | 36.0 | 22.0 | 35.5 | 38.7 | 40.6 | 35.1 | 41.3 | 36.3 | 32.6 | 36.2 | 37.7 | 36.1 | 37.4 | 37.2 |
| 6. AT5G58010 | 53.9 | 55.0 | 52.6 | 23.8 | 58.1 | | 32.6 | 24.2 | 34.0 | 35.4 | 37.1 | 36.0 | 35.7 | 31.6 | 32.3 | 35.5 | 38.8 | 36.0 | 38.1 | 39.2 |
| 7. TC15501 | 58.4 | 64.1 | 65.5 | 22.1 | 49.9 | 48.1 | | 17.5 | 52.8 | 47.0 | 37.0 | 56.0 | 35.1 | 57.8 | 59.2 | 52.8 | 52.4 | 60.9 | 51.0 | 31.4 |
| 8. TC19278 | 31.9 | 31.1 | 30.0 | 15.6 | 33.2 | 35.0 | 26.8 | | 18.8 | 21.1 | 16.4 | 19.5 | 18.9 | 17.8 | 17.6 | 17.5 | 23.0 | 20.2 | 19.4 | 27.6 |
| 9. TC10015_part | 64.9 | 80.5 | 85.8 | 19.9 | 50.6 | 52.2 | 60.4 | 31.7 | | 41.9 | 35.6 | 72.5 | 30.8 | 52.5 | 59.7 | 47.1 | 61.4 | 68.0 | 51.4 | 35.2 |
| 10. DY268946 | 56.8 | 55.9 | 59.1 | 23.8 | 52.5 | 46.4 | 61.0 | 31.6 | 53.0 | | 34.5 | 42.7 | 32.9 | 52.7 | 43.2 | 50.8 | 46.9 | 45.0 | 57.6 | 35.6 |
| 11. TA2544 | 47.3 | 46.8 | 47.8 | 26.1 | 49.3 | 46.3 | 49.0 | 23.9 | 45.6 | 47.6 | | 35.0 | 44.9 | 38.4 | 35.9 | 36.4 | 33.4 | 36.1 | 34.6 | 31.1 |
| 12. TA3392 | 68.1 | 76.8 | 79.4 | 21.7 | 51.9 | 52.2 | 63.0 | 32.7 | 84.0 | 56.2 | 43.7 | | 30.9 | 53.0 | 68.7 | 49.4 | 63.1 | 77.0 | 52.2 | 36.8 |
| 13. TA12416 | 38.0 | 38.2 | 40.7 | 30.4 | 48.4 | 43.3 | 46.3 | 25.8 | 37.3 | 43.7 | 54.6 | 40.3 | | 32.2 | 32.4 | 33.1 | 29.7 | 33.3 | 32.1 | 31.3 |
| 14. WO051050seqID77 | 61.3 | 63.8 | 66.3 | 20.8 | 51.3 | 50.9 | 66.4 | 28.1 | 61.9 | 65.8 | 45.4 | 61.3 | 39.4 | | 53.2 | 58.7 | 50.0 | 54.6 | 58.1 | 31.5 |
| 15. WO051050seqID1671 | 56.9 | 70.5 | 74.9 | 22.3 | 46.5 | 47.1 | 70.9 | 27.5 | 68.5 | 59.0 | 47.3 | 75.4 | 42.0 | 64.5 | | 46.8 | 58.7 | 71.0 | 44.8 | 33.5 |
| 16. TA55042 | 62.5 | 59.5 | 62.8 | 22.4 | 51.7 | 52.0 | 65.8 | 27.8 | 58.0 | 67.0 | 48.8 | 58.9 | 42.4 | 69.2 | 62.7 | | 48.1 | 48.6 | 58.1 | 33.8 |
| 17. TC207545 | 60.7 | 66.6 | 68.1 | 20.1 | 51.0 | 51.5 | 57.0 | 38.5 | 71.6 | 53.9 | 42.9 | 74.7 | 36.7 | 56.9 | 64.5 | 56.8 | | 64.7 | 56.0 | 37.4 |
| 18. TA13791 | 63.7 | 80.5 | 82.6 | 22.1 | 52.6 | 54.8 | 68.1 | 31.7 | 78.2 | 59.4 | 48.3 | 83.5 | 43.1 | 65.0 | 78.0 | 63.1 | 71.6 | | 50.6 | 35.4 |
| 19. CO123623 | 61.4 | 57.3 | 58.7 | 19.3 | 49.7 | 53.9 | 58.1 | 32.8 | 62.6 | 64.3 | 42.9 | 65.1 | 38.2 | 65.6 | 55.2 | 65.6 | 64.9 | 61.7 | | 36.8 |
| 20. DT543504 | 52.0 | 55.2 | 54.8 | 22.3 | 51.3 | 52.6 | 47.3 | 37.9 | 49.7 | 50.4 | 42.7 | 55.2 | 39.7 | 51.9 | 51.4 | 50.5 | 52.6 | 54.9 | 50.0 | |
| 21. TC60118 | 63.4 | 80.2 | 82.3 | 22.0 | 52.3 | 54.5 | 67.8 | 31.7 | 77.9 | 59.1 | 48.0 | 83.2 | 42.9 | 64.7 | 77.7 | 62.8 | 71.3 | 99.7 | 60.7 | 55.2 |
| 22. TC60119 | 65.1 | 78.9 | 82.9 | 21.1 | 51.9 | 53.3 | 67.0 | 30.6 | 77.3 | 58.6 | 47.6 | 82.6 | 40.5 | 65.6 | 76.6 | 60.7 | 71.1 | 91.1 | 58.6 | 53.6 |
| 23. TC61833 | 63.6 | 78.4 | 81.9 | 21.9 | 54.2 | 55.4 | 66.1 | 30.2 | 77.0 | 56.8 | 47.6 | 82.3 | 40.7 | 65.0 | 76.9 | 62.5 | 71.1 | 90.8 | 56.4 | 54.9 |
| 24. TC67603 | 40.3 | 43.3 | 42.4 | 28.8 | 48.7 | 45.9 | 47.5 | 26.9 | 40.7 | 45.7 | 56.7 | 42.9 | 67.0 | 43.1 | 42.9 | 45.7 | 38.6 | 42.9 | 40.0 | 41.2 |
| 25. TC229602 | 56.8 | 60.3 | 61.0 | 18.9 | 45.8 | 49.2 | 50.7 | 45.2 | 63.3 | 49.9 | 38.0 | 65.1 | 33.5 | 52.5 | 57.5 | 52.3 | 81.1 | 65.3 | 61.9 | 46.1 |
| 26. TC205173 | 50.4 | 51.0 | 48.7 | 25.3 | 58.5 | 54.6 | 53.3 | 34.3 | 47.2 | 52.5 | 52.2 | 47.5 | 54.6 | 51.3 | 48.0 | 54.6 | 49.0 | 51.0 | 49.6 | 49.9 |
| 27. TA1140 | 63.4 | 64.1 | 64.5 | 21.5 | 55.2 | 53.9 | 64.7 | 31.0 | 62.7 | 55.1 | 45.1 | 63.1 | 42.2 | 65.3 | 60.1 | 64.0 | 61.1 | 66.0 | 62.4 | 53.9 |
| 28. TC140470 | 37.6 | 38.5 | 38.0 | 26.6 | 35.8 | 36.4 | 41.2 | 22.2 | 34.9 | 37.0 | 43.7 | 37.4 | 48.0 | 38.0 | 40.3 | 40.3 | 33.5 | 37.8 | 32.8 | 43.9 |
| 29. TA3490_part | 67.6 | 64.6 | 64.2 | 22.0 | 53.2 | 54.5 | 62.1 | 32.4 | 65.2 | 58.0 | 45.6 | 67.9 | 41.4 | 63.4 | 59.5 | 63.4 | 63.1 | 68.6 | 63.1 | 52.6 |
| 30. CK293938 | 67.6 | 64.2 | 65.2 | 20.6 | 53.9 | 54.2 | 61.5 | 32.1 | 65.2 | 60.0 | 45.6 | 66.2 | 41.4 | 68.4 | 60.1 | 64.0 | 59.8 | 66.0 | 64.9 | 53.6 |
| 31. TC8633 | 64.6 | 78.8 | 79.0 | 22.5 | 53.9 | 56.3 | 68.1 | 31.8 | 74.5 | 56.5 | 45.1 | 75.5 | 41.8 | 65.3 | 71.1 | 62.5 | 69.9 | 80.9 | 59.3 | 54.9 |
| 32. TC7102 | 39.7 | 42.4 | 42.9 | 30.0 | 49.2 | 44.2 | 46.3 | 25.1 | 39.7 | 44.7 | 56.9 | 40.6 | 69.7 | 43.6 | 44.2 | 43.6 | 38.1 | 44.2 | 39.3 | 41.3 |

| | | | | | | | | | | | | | | | | | | | |
|---|---|---|---|---|---|---|---|---|---|---|---|---|---|---|---|---|---|---|---|
| 33. TC7103 | 45.0 | 46.0 | 46.3 | 26.7 | 53.2 | 51.2 | 48.8 | 27.9 | 45.0 | 46.5 | 57.1 | 45.2 | 65.0 | 46.3 | 50.4 | 49.4 | 42.9 | 49.1 | 41.6 | 45.5 |
| 34. TC12771 | 66.1 | 64.9 | 65.5 | 21.2 | 54.8 | 54.4 | 61.5 | 30.9 | 64.4 | 59.7 | 45.6 | 65.1 | 40.9 | 67.2 | 59.8 | 64.0 | 59.7 | 66.0 | 65.8 | 54.9 |
| 35. Os03g58330 | 67.7 | 61.9 | 61.3 | 21.3 | 53.9 | 54.2 | 58.7 | 32.7 | 62.6 | 55.9 | 45.4 | 61.6 | 39.2 | 62.2 | 53.8 | 61.9 | 59.2 | 63.7 | 62.9 | 51.3 |
| 36. Os06g08500 | 43.6 | 43.6 | 45.0 | 23.6 | 45.0 | 48.2 | 47.7 | 28.6 | 42.0 | 43.3 | 51.5 | 44.1 | 46.5 | 44.1 | 45.2 | 46.6 | 40.3 | 43.3 | 39.0 | 43.3 |
| 37. Os06g09370 | 37.7 | 37.4 | 38.1 | 26.8 | 35.1 | 34.1 | 40.0 | 22.2 | 34.1 | 40.6 | 44.1 | 38.5 | 44.4 | 38.5 | 40.6 | 42.7 | 33.1 | 38.9 | 36.2 | 40.8 |
| 38. Os02g55250 | 40.6 | 42.0 | 41.5 | 28.5 | 43.5 | 44.4 | 45.1 | 25.2 | 39.2 | 46.0 | 55.8 | 40.8 | 56.3 | 44.0 | 41.7 | 46.3 | 38.1 | 41.5 | 39.0 | 43.5 |
| 39. Os02g35660 | 35.7 | 35.1 | 36.6 | 29.2 | 36.5 | 34.4 | 39.3 | 22.9 | 33.6 | 38.5 | 43.1 | 34.9 | 45.4 | 36.6 | 39.5 | 37.0 | 32.3 | 37.4 | 34.0 | 39.7 |
| 40. Os07g08440 | 70.3 | 60.3 | 65.5 | 20.8 | 52.3 | 56.9 | 58.4 | 33.8 | 63.8 | 57.1 | 44.6 | 63.8 | 38.8 | 62.5 | 57.8 | 61.9 | 58.4 | 63.7 | 65.9 | 51.0 |
| 41. Os09g25040 | 37.3 | 35.7 | 38.5 | 30.0 | 40.1 | 38.5 | 41.5 | 24.4 | 36.1 | 38.7 | 54.3 | 37.7 | 60.7 | 39.3 | 41.5 | 39.3 | 33.9 | 38.3 | 35.7 | 37.7 |
| 42. Pt_scaff_II.416 | 62.7 | 79.5 | 81.9 | 22.4 | 53.5 | 53.7 | 66.7 | 31.3 | 78.7 | 58.3 | 47.3 | 84.3 | 42.0 | 65.6 | 78.3 | 62.2 | 73.3 | 89.4 | 61.0 | 55.2 |
| 43. Pt_scaff_70.65 | 60.7 | 62.2 | 65.9 | 21.9 | 52.3 | 49.2 | 65.8 | 27.5 | 60.7 | 69.9 | 44.9 | 60.7 | 40.3 | 71.9 | 64.5 | 74.6 | 56.5 | 63.4 | 66.2 | 50.8 |
| 44. Pt_scaff_XIII.403 | 59.9 | 62.0 | 62.3 | 21.2 | 50.6 | 49.7 | 66.1 | 28.1 | 58.7 | 67.0 | 45.4 | 59.0 | 42.2 | 69.2 | 65.9 | 73.1 | 55.4 | 64.4 | 64.7 | 49.7 |
| 45. Pt_scaff28.86 | 39.5 | 39.3 | 40.8 | 27.2 | 39.3 | 38.8 | 42.5 | 29.4 | 36.2 | 43.4 | 45.6 | 39.9 | 48.0 | 40.1 | 42.1 | 42.3 | 36.0 | 39.5 | 36.8 | 50.2 |
| 46. Pt_TC63334 | 65.0 | 79.8 | 81.6 | 22.1 | 54.8 | 52.3 | 68.7 | 31.3 | 78.7 | 58.0 | 47.6 | 84.3 | 42.6 | 65.0 | 77.2 | 62.5 | 73.7 | 89.1 | 62.0 | 53.6 |
| 47. TA14134 | 46.2 | 49.8 | 50.8 | 24.7 | 50.5 | 50.2 | 49.3 | 37.5 | 43.7 | 52.5 | 44.6 | 45.2 | 41.2 | 50.2 | 47.1 | 51.4 | 45.5 | 50.2 | 46.8 | 50.8 |
| 48. TA5414 | 38.0 | 38.5 | 41.2 | 30.1 | 48.5 | 42.7 | 45.9 | 25.9 | 37.8 | 42.9 | 55.8 | 41.2 | 98.9 | 41.2 | 42.1 | 42.7 | 37.0 | 42.9 | 39.1 | 39.7 |
| 49. TA3263 | 65.3 | 79.5 | 81.6 | 22.3 | 54.8 | 54.0 | 68.4 | 31.7 | 79.0 | 58.3 | 48.3 | 84.7 | 42.6 | 65.0 | 77.2 | 62.8 | 73.7 | 89.4 | 62.3 | 54.2 |
| 50. TA2825 | 65.2 | 80.1 | 83.9 | 22.0 | 53.2 | 54.2 | 66.7 | 32.1 | 80.3 | 55.9 | 47.3 | 86.0 | 41.6 | 66.6 | 79.5 | 63.7 | 74.6 | 90.4 | 60.9 | 55.9 |
| 51. TA1616 | 61.0 | 65.3 | 66.3 | 22.3 | 52.1 | 50.3 | 70.4 | 28.8 | 60.4 | 70.4 | 47.8 | 62.9 | 41.6 | 74.2 | 63.9 | 73.4 | 57.7 | 66.6 | 68.4 | 54.0 |
| 52. TA21665 | 66.2 | 65.2 | 63.9 | 21.3 | 52.3 | 53.2 | 60.4 | 31.1 | 63.5 | 58.3 | 44.4 | 67.6 | 42.9 | 64.4 | 59.5 | 61.0 | 60.1 | 66.0 | 62.5 | 52.3 |
| 53. TC172581 | 64.5 | 63.8 | 67.1 | 21.1 | 52.9 | 53.9 | 61.0 | 30.6 | 62.2 | 60.0 | 46.6 | 66.1 | 41.8 | 66.3 | 61.3 | 63.4 | 59.5 | 67.1 | 62.2 | 54.2 |
| 54. AK247217 | 64.6 | 80.5 | 80.3 | 22.9 | 52.3 | 52.0 | 70.1 | 31.5 | 75.2 | 56.2 | 45.1 | 78.1 | 42.0 | 67.8 | 72.5 | 64.4 | 70.5 | 82.8 | 60.6 | 52.6 |
| 55. TC104646 | 69.5 | 61.6 | 61.9 | 20.7 | 51.0 | 53.5 | 58.7 | 35.0 | 67.3 | 55.7 | 42.0 | 63.3 | 39.2 | 60.3 | 56.1 | 59.8 | 63.5 | 63.0 | 65.3 | 49.7 |
| 56. DV982110 | 59.3 | 55.6 | 53.5 | 22.6 | 55.8 | 57.9 | 51.0 | 38.0 | 55.8 | 51.3 | 44.9 | 61.6 | 41.6 | 53.4 | 49.1 | 51.7 | 56.9 | 58.1 | 62.0 | 56.2 |
| 57. WO051050seqID516 | 40.2 | 42.5 | 42.3 | 28.0 | 44.1 | 42.3 | 46.4 | 27.0 | 39.3 | 46.9 | 53.3 | 42.5 | 52.5 | 44.1 | 43.6 | 43.8 | 37.8 | 43.2 | 39.5 | 41.9 |
| 58. TC68930 | 48.3 | 49.2 | 49.5 | 25.9 | 49.5 | 53.5 | 47.0 | 38.4 | 45.6 | 51.3 | 47.3 | 47.4 | 42.0 | 50.5 | 46.2 | 52.0 | 48.3 | 50.2 | 45.0 | 50.5 |
| 59. TA30646 | 64.6 | 79.8 | 80.0 | 22.1 | 52.6 | 52.3 | 68.7 | 31.5 | 75.8 | 55.7 | 44.6 | 78.1 | 41.2 | 67.2 | 72.8 | 64.4 | 70.2 | 83.5 | 60.6 | 54.2 |
| 60. TA28621 | 65.8 | 64.1 | 67.1 | 21.3 | 53.2 | 52.3 | 61.5 | 30.3 | 62.8 | 60.9 | 46.8 | 67.1 | 41.6 | 65.6 | 61.8 | 64.4 | 59.5 | 68.1 | 63.5 | 54.2 |
| 61. TA34455 | 40.3 | 41.6 | 41.2 | 29.5 | 49.7 | 43.4 | 45.9 | 23.0 | 38.0 | 45.6 | 59.3 | 41.4 | 67.0 | 43.4 | 43.8 | 44.7 | 37.1 | 42.5 | 36.9 | 41.2 |
| 62. TA37666 | 45.5 | 46.6 | 48.7 | 27.4 | 53.9 | 48.2 | 51.8 | 28.3 | 43.7 | 50.3 | 60.7 | 47.1 | 63.1 | 48.4 | 48.4 | 49.0 | 42.1 | 49.0 | 45.3 | 44.5 |
| 63. TC253044 | 56.6 | 54.0 | 51.6 | 19.7 | 50.3 | 53.9 | 49.3 | 36.9 | 53.4 | 45.8 | 42.9 | 55.5 | 39.0 | 50.9 | 48.8 | 51.1 | 53.4 | 53.1 | 52.8 | 59.8 |
| 64. TabHLH11 | 71.2 | 60.9 | 61.0 | 19.9 | 50.6 | 56.6 | 57.0 | 34.2 | 66.2 | 55.9 | 42.7 | 66.2 | 38.8 | 59.7 | 55.8 | 60.1 | 61.9 | 62.7 | 66.9 | 50.3 |
| 65. TA46156 | 67.5 | 76.8 | 78.4 | 22.3 | 54.8 | 55.2 | 66.4 | 33.6 | 78.9 | 58.6 | 45.9 | 84.1 | 40.5 | 64.7 | 74.6 | 64.0 | 74.4 | 85.5 | 64.4 | 56.2 |
| 66. GSVIVT00017237001 | 43.5 | 42.1 | 43.1 | 29.4 | 48.1 | 45.2 | 47.4 | 27.5 | 40.4 | 47.8 | 59.1 | 40.4 | 69.5 | 44.0 | 45.5 | 45.7 | 40.9 | 45.9 | 39.7 | 43.8 |

| | 21 | 22 | 23 | 24 | 25 | 26 | 27 | 28 | 29 | 30 | 31 | 32 | 33 | 34 | 35 | 36 | 37 | 38 | 39 | 40 |
|---|---|---|---|---|---|---|---|---|---|---|---|---|---|---|---|---|---|---|---|---|
| 67. TA46194 | 66.1 | 69.0 | 71.9 | 22.3 | 52.1 | 53.4 | 70.9 | 29.7 | 67.4 | 63.5 | 49.0 | 69.0 | 41.8 | 72.5 | 68.8 | 68.3 | 64.5 | 73.5 | 66.8 | 53.4 |
| 68. GSVIVT00016367001 | 35.7 | 37.7 | 36.5 | 24.5 | 38.1 | 36.9 | 41.6 | 24.1 | 34.4 | 41.9 | 44.5 | 35.5 | 46.2 | 37.7 | 40.8 | 38.6 | 32.8 | 37.1 | 34.6 | 45.4 |
| 69. TA139285 | 70.2 | 59.9 | 61.3 | 20.3 | 51.6 | 56.2 | 59.5 | 34.4 | 62.1 | 58.0 | 43.4 | 63.2 | 37.5 | 62.5 | 54.6 | 61.9 | 60.7 | 61.1 | 66.0 | 48.0 |
| 70. TA126400 | 69.5 | 61.9 | 62.6 | 20.9 | 54.5 | 52.5 | 57.3 | 34.0 | 66.7 | 56.8 | 44.9 | 65.3 | 41.4 | 61.6 | 56.1 | 61.0 | 62.5 | 63.0 | 63.2 | 50.0 |
| 1. TC3698 | 51.4 | 53.4 | 51.7 | 31.6 | 48.5 | 38.8 | 52.7 | 25.4 | 54.5 | 53.0 | 53.0 | 32.7 | 36.2 | 53.3 | 56.0 | 31.1 | 26.5 | 32.4 | 26.5 | 56.9 |
| 2. AT1G03040 | 67.8 | 67.2 | 67.6 | 33.9 | 54.3 | 35.3 | 49.2 | 26.3 | 50.9 | 48.0 | 65.5 | 33.7 | 35.4 | 48.3 | 51.4 | 30.9 | 25.8 | 34.1 | 27.9 | 49.8 |
| 3. AT4G02590 | 72.3 | 73.6 | 73.3 | 32.8 | 57.1 | 37.7 | 50.3 | 27.5 | 52.6 | 49.5 | 67.5 | 34.3 | 35.4 | 50.8 | 52.7 | 32.8 | 27.8 | 33.6 | 28.7 | 51.2 |
| 4. AT2G24260 | 16.7 | 16.3 | 16.6 | 22.0 | 15.0 | 21.5 | 16.1 | 15.4 | 16.6 | 16.1 | 16.8 | 23.1 | 21.8 | 16.3 | 16.7 | 17.5 | 15.5 | 21.2 | 16.4 | 16.4 |
| 5. AT4G30980 | 36.1 | 35.9 | 36.2 | 40.8 | 34.8 | 44.8 | 36.2 | 26.7 | 36.5 | 36.1 | 35.4 | 41.8 | 44.3 | 35.6 | 39.8 | 33.9 | 25.5 | 36.2 | 28.6 | 37.6 |
| 6. AT5G58010 | 36.0 | 36.4 | 36.3 | 37.2 | 36.9 | 47.3 | 37.7 | 28.1 | 37.8 | 36.9 | 37.9 | 36.0 | 39.7 | 36.4 | 37.4 | 37.7 | 26.9 | 36.4 | 26.1 | 37.4 |
| 7. TC15501 | 60.7 | 59.0 | 58.0 | 36.2 | 47.9 | 37.1 | 54.0 | 30.8 | 53.8 | 53.8 | 58.7 | 38.2 | 37.6 | 53.0 | 52.5 | 32.7 | 29.4 | 35.0 | 30.7 | 52.0 |
| 8. TC19278 | 20.2 | 20.2 | 19.6 | 18.4 | 27.3 | 23.1 | 18.0 | 15.6 | 19.0 | 19.3 | 20.5 | 17.9 | 20.2 | 18.6 | 19.5 | 20.8 | 15.6 | 19.1 | 17.9 | 19.8 |
| 9. TC10015_part | 67.6 | 69.0 | 68.9 | 31.7 | 55.3 | 38.5 | 51.3 | 25.5 | 53.4 | 50.0 | 64.1 | 32.8 | 35.7 | 50.6 | 51.8 | 32.9 | 26.0 | 32.9 | 26.7 | 52.8 |
| 10. DY268946 | 44.7 | 43.8 | 44.2 | 32.7 | 43.2 | 38.9 | 44.6 | 24.4 | 45.8 | 45.4 | 43.5 | 34.2 | 33.5 | 45.1 | 46.9 | 31.6 | 26.5 | 32.9 | 26.6 | 47.9 |
| 11. TA2544 | 36.1 | 35.7 | 36.5 | 41.8 | 29.6 | 41.3 | 35.3 | 30.2 | 35.4 | 33.8 | 35.1 | 45.9 | 42.6 | 35.0 | 35.1 | 39.3 | 31.1 | 42.5 | 32.1 | 35.3 |
| 12. TA3392 | 76.7 | 76.1 | 75.2 | 33.9 | 56.2 | 36.2 | 52.2 | 25.6 | 54.3 | 51.6 | 64.5 | 33.0 | 35.2 | 52.1 | 53.0 | 30.4 | 28.9 | 32.7 | 26.2 | 51.5 |
| 13. TA12416 | 33.3 | 33.3 | 34.1 | 56.2 | 27.5 | 48.0 | 33.7 | 30.2 | 33.3 | 31.2 | 32.6 | 56.3 | 55.6 | 31.8 | 32.2 | 36.9 | 28.7 | 45.2 | 30.6 | 31.4 |
| 14. WO051050seqID77 | 54.3 | 54.0 | 52.5 | 33.3 | 47.2 | 36.7 | 54.2 | 27.7 | 54.3 | 56.0 | 53.4 | 34.7 | 35.6 | 55.9 | 52.0 | 31.9 | 28.8 | 34.5 | 27.4 | 51.6 |
| 15. WO051050seqID1671 | 70.7 | 70.3 | 68.9 | 32.0 | 53.2 | 34.4 | 47.6 | 27.4 | 49.4 | 48.5 | 62.0 | 34.8 | 35.0 | 48.7 | 48.7 | 32.1 | 28.7 | 33.1 | 29.3 | 48.6 |
| 16. TA55042 | 48.4 | 47.8 | 49.9 | 32.6 | 46.4 | 37.7 | 50.1 | 29.4 | 53.1 | 50.9 | 49.4 | 35.2 | 36.8 | 50.3 | 51.3 | 33.9 | 32.2 | 36.3 | 27.3 | 51.0 |
| 17. TC207545 | 64.4 | 65.1 | 65.6 | 30.9 | 77.6 | 37.8 | 52.6 | 23.9 | 55.6 | 52.0 | 61.8 | 32.0 | 32.9 | 53.0 | 54.1 | 28.6 | 23.9 | 31.3 | 24.9 | 53.2 |
| 18. TA13791 | 99.7 | 87.2 | 84.9 | 33.7 | 59.7 | 39.4 | 54.0 | 26.2 | 56.7 | 53.3 | 69.0 | 35.6 | 38.5 | 53.3 | 54.6 | 33.2 | 27.1 | 33.8 | 28.2 | 51.7 |
| 19. CO123623 | 50.5 | 49.5 | 49.2 | 30.9 | 52.2 | 39.3 | 51.1 | 25.4 | 53.2 | 50.3 | 47.7 | 31.5 | 33.6 | 52.0 | 50.6 | 31.1 | 29.0 | 30.1 | 27.3 | 50.9 |
| 20. DT543504 | 35.7 | 34.8 | 36.1 | 34.0 | 34.0 | 36.3 | 34.2 | 31.0 | 36.1 | 37.1 | 35.7 | 31.9 | 33.4 | 38.1 | 34.5 | 31.8 | 28.8 | 32.3 | 30.8 | 32.5 |
| 21. TC60118 | | 86.8 | 84.6 | 33.7 | 59.4 | 39.1 | 53.7 | 26.2 | 56.4 | 53.0 | 68.7 | 35.6 | 38.5 | 53.0 | 54.3 | 33.2 | 27.5 | 33.6 | 28.2 | 51.4 |
| 22. TC60119 | 90.8 | | 86.9 | 32.7 | 59.5 | 39.1 | 53.3 | 26.1 | 54.7 | 53.5 | 70.1 | 36.7 | 37.3 | 54.4 | 53.1 | 33.1 | 27.0 | 34.2 | 28.2 | 51.1 |
| 23. TC61833 | 90.5 | 92.5 | | 33.5 | 59.3 | 37.7 | 52.6 | 26.5 | 55.9 | 53.3 | 71.1 | 36.4 | 36.8 | 53.9 | 51.1 | 34.5 | 25.4 | 34.7 | 27.4 | 52.0 |
| 24. TC67603 | 42.6 | 42.2 | 42.2 | | 28.5 | 47.1 | 35.0 | 27.3 | 34.9 | 33.2 | 33.2 | 49.6 | 52.5 | 33.7 | 34.3 | 35.6 | 28.3 | 40.6 | 30.7 | 32.6 |
| 25. TC229602 | 65.0 | 64.5 | 63.9 | 35.8 | | 38.2 | 47.7 | 22.5 | 50.5 | 50.7 | 58.2 | 29.0 | 32.3 | 50.0 | 50.8 | 28.2 | 21.3 | 28.4 | 22.7 | 49.3 |
| 26. TC205173 | 50.7 | 51.9 | 51.6 | 56.4 | 45.7 | | 39.2 | 25.3 | 37.8 | 37.4 | 39.8 | 47.3 | 53.1 | 38.0 | 38.7 | 40.3 | 26.6 | 41.2 | 27.9 | 38.6 |
| 27. TA1140 | 65.7 | 66.0 | 65.4 | 45.2 | 54.2 | 52.2 | | 27.1 | 84.2 | 66.0 | 51.2 | 35.8 | 37.5 | 67.0 | 52.5 | 33.3 | 27.2 | 31.8 | 28.2 | 52.1 |
| 28. TC140470 | 37.6 | 37.4 | 37.8 | 42.8 | 29.7 | 36.4 | 38.0 | | 29.1 | 26.1 | 27.9 | 28.7 | 27.7 | 27.0 | 26.6 | 24.4 | 71.1 | 28.1 | 35.1 | 26.1 |

| | | | | | | | | | | | | | | | | | | | | |
|---|---|---|---|---|---|---|---|---|---|---|---|---|---|---|---|---|---|---|---|---|
| 29. TA3490_part | 68.3 | 66.4 | 66.6 | 44.7 | 57.2 | 49.6 | 87.3 | 37.4 | | 68.0 | 53.2 | 34.4 | 36.5 | 68.5 | 54.0 | 32.7 | 28.3 | 33.3 | 27.6 | 53.7 |
| 30. CK293938 | 65.7 | 66.8 | 65.6 | 42.2 | 57.4 | 49.6 | 78.1 | 36.2 | 79.4 | | 51.5 | 34.2 | 37.5 | 96.3 | 53.8 | 32.4 | 28.0 | 33.9 | 27.5 | 53.5 |
| 31. TC8633 | 80.5 | 81.6 | 82.3 | 44.3 | 63.2 | 51.3 | 67.3 | 38.5 | 68.9 | 67.5 | | 36.0 | 34.9 | 52.5 | 53.8 | 33.0 | 29.2 | 33.6 | 26.9 | 52.6 |
| 32. TC7102 | 44.0 | 44.0 | 45.1 | 62.1 | 34.8 | 55.5 | 44.7 | 43.0 | 42.7 | 44.0 | 44.7 | | 64.6 | 34.5 | 35.9 | 37.5 | 28.8 | 47.3 | 32.6 | 34.1 |
| 33. TC7103 | 48.8 | 49.1 | 48.3 | 65.8 | 40.1 | 62.5 | 48.8 | 41.0 | 47.5 | 47.0 | 46.5 | 72.9 | | 38.2 | 37.8 | 41.1 | 28.9 | 46.2 | 29.6 | 38.3 |
| 34. TC12771 | 65.7 | 65.8 | 64.6 | 43.1 | 55.7 | 50.7 | 78.1 | 37.4 | 78.5 | 98.0 | 67.5 | 44.5 | 48.1 | | 54.6 | 32.2 | 28.6 | 34.9 | 26.9 | 54.7 |
| 35. Os03g58330 | 63.4 | 63.5 | 60.3 | 43.6 | 56.1 | 50.1 | 64.7 | 36.8 | 67.0 | 66.6 | 66.9 | 42.7 | 46.0 | 66.1 | | 36.6 | 27.1 | 35.3 | 28.0 | 74.9 |
| 36. Os06g08500 | 43.3 | 45.2 | 47.4 | 46.4 | 37.1 | 51.2 | 47.7 | 36.2 | 44.4 | 42.5 | 42.5 | 49.2 | 55.6 | 45.0 | 47.4 | | 24.0 | 46.8 | 29.1 | 36.2 |
| 37. Os06g09370 | 39.1 | 37.4 | 38.1 | 41.4 | 28.7 | 36.6 | 36.4 | 81.5 | 37.2 | 38.7 | 38.3 | 43.9 | 41.2 | 38.5 | 35.8 | 37.4 | | 28.6 | 34.3 | 26.6 |
| 38. Os02g55250 | 41.3 | 41.3 | 42.2 | 56.0 | 35.1 | 50.1 | 42.0 | 45.3 | 44.0 | 41.5 | 44.0 | 60.5 | 59.0 | 43.5 | 42.2 | 59.2 | 46.4 | | 31.8 | 35.3 |
| 39. Os02g35660 | 37.2 | 36.3 | 35.9 | 44.3 | 29.6 | 36.5 | 37.4 | 49.8 | 36.5 | 35.9 | 35.9 | 44.1 | 40.3 | 36.5 | 36.1 | 36.6 | 47.9 | 45.6 | | 25.7 |
| 40. Os07g08440 | 63.4 | 62.8 | 63.9 | 41.0 | 55.3 | 49.6 | 63.4 | 35.6 | 64.5 | 67.6 | 66.6 | 41.8 | 45.7 | 68.1 | 82.0 | 46.0 | 35.8 | 41.7 | 34.0 | |
| 41. Os09g25040 | 38.1 | 38.1 | 38.7 | 54.5 | 31.1 | 47.9 | 38.7 | 42.5 | 36.3 | 36.3 | 37.5 | 58.5 | 54.9 | 37.1 | 37.3 | 47.3 | 43.7 | 56.3 | 44.8 | 37.5 |
| 42. Pt_scaff_II.416 | 89.1 | 88.2 | 85.6 | 42.4 | 66.0 | 50.4 | 65.0 | 37.0 | 67.3 | 67.0 | 82.1 | 44.5 | 48.3 | 66.7 | 63.3 | 44.7 | 39.5 | 43.3 | 36.6 | 64.3 |
| 43. Pt_scaff_70.65 | 63.1 | 63.7 | 63.7 | 43.3 | 52.3 | 50.7 | 63.7 | 38.7 | 63.4 | 65.6 | 63.1 | 44.5 | 47.0 | 65.9 | 60.7 | 44.7 | 42.9 | 44.2 | 37.8 | 60.7 |
| 44. Pt_scaff_XIII.403 | 64.1 | 62.3 | 63.2 | 44.0 | 50.6 | 54.0 | 62.9 | 41.0 | 65.0 | 63.5 | 63.5 | 44.9 | 47.5 | 63.2 | 59.9 | 43.6 | 41.8 | 44.9 | 38.2 | 59.0 |
| 45. Pt_scaff28.86 | 39.3 | 39.9 | 39.5 | 47.8 | 31.8 | 42.1 | 39.9 | 47.6 | 38.6 | 40.6 | 41.4 | 47.6 | 45.8 | 40.1 | 36.4 | 41.2 | 49.6 | 47.6 | 45.6 | 35.3 |
| 46. Pt_TC63334 | 88.8 | 88.2 | 86.2 | 42.4 | 66.7 | 51.0 | 63.1 | 37.4 | 67.0 | 64.3 | 82.8 | 45.1 | 48.8 | 64.7 | 64.0 | 44.1 | 38.3 | 43.5 | 35.3 | 65.0 |
| 47. TA14134 | 50.2 | 48.9 | 46.5 | 43.6 | 42.8 | 54.9 | 50.2 | 35.1 | 49.2 | 48.9 | 51.7 | 43.6 | 45.5 | 49.8 | 47.7 | 46.6 | 36.8 | 44.7 | 36.8 | 46.8 |
| 48. TA5414 | 42.7 | 41.0 | 41.7 | 67.1 | 33.5 | 55.1 | 42.1 | 48.6 | 41.5 | 42.1 | 41.2 | 69.4 | 63.9 | 40.8 | 38.5 | 47.2 | 44.8 | 57.1 | 45.4 | 39.3 |
| 49. TA3263 | 89.1 | 88.5 | 86.6 | 43.1 | 66.7 | 51.0 | 63.1 | 37.4 | 67.3 | 64.7 | 82.8 | 45.6 | 49.1 | 65.0 | 64.0 | 44.4 | 38.7 | 43.8 | 35.1 | 64.3 |
| 50. TA2825 | 90.1 | 90.1 | 89.2 | 43.1 | 67.2 | 51.0 | 66.0 | 35.8 | 67.2 | 66.2 | 83.4 | 46.0 | 49.4 | 66.2 | 63.2 | 45.0 | 37.9 | 43.3 | 36.6 | 65.2 |
| 51. TA1616 | 66.3 | 65.0 | 65.0 | 42.9 | 55.2 | 51.0 | 65.6 | 38.3 | 63.2 | 65.6 | 66.0 | 47.2 | 47.8 | 66.6 | 63.2 | 48.5 | 40.8 | 46.3 | 36.3 | 66.0 |
| 52. TA21665 | 65.7 | 66.4 | 66.6 | 42.9 | 54.4 | 51.3 | 73.5 | 36.6 | 76.4 | 84.8 | 64.2 | 43.8 | 48.6 | 84.9 | 65.9 | 44.7 | 35.6 | 43.3 | 37.2 | 65.9 |
| 53. TC172581 | 66.8 | 67.4 | 65.6 | 42.2 | 55.6 | 50.7 | 76.1 | 38.5 | 76.0 | 92.1 | 66.1 | 43.8 | 48.3 | 92.8 | 65.8 | 44.4 | 38.5 | 41.5 | 36.6 | 64.5 |
| 54. AK247217 | 82.5 | 84.2 | 84.3 | 43.3 | 63.9 | 49.6 | 67.6 | 38.0 | 67.9 | 69.2 | 95.0 | 42.7 | 47.3 | 67.5 | 64.6 | 43.6 | 37.9 | 44.2 | 35.9 | 64.6 |
| 55. TC104646 | 62.7 | 61.5 | 61.6 | 43.1 | 59.2 | 49.3 | 62.1 | 35.1 | 66.6 | 67.6 | 62.9 | 40.2 | 44.2 | 67.1 | 77.6 | 45.2 | 35.8 | 40.1 | 34.0 | 85.0 |
| 56. DV982110 | 57.8 | 57.9 | 57.7 | 44.5 | 56.2 | 58.2 | 58.5 | 35.3 | 57.9 | 55.7 | 55.6 | 43.6 | 50.4 | 54.4 | 57.5 | 46.0 | 35.6 | 43.5 | 34.9 | 59.0 |
| 57. WO051050seqID516 | 43.0 | 43.6 | 43.6 | 50.5 | 35.0 | 46.0 | 41.9 | 45.9 | 41.3 | 41.9 | 43.2 | 52.9 | 48.8 | 42.1 | 42.5 | 44.3 | 44.6 | 51.6 | 42.6 | 41.9 |
| 58. TC68930 | 50.5 | 48.0 | 49.5 | 45.2 | 43.5 | 57.9 | 50.2 | 37.0 | 48.9 | 47.1 | 47.4 | 45.1 | 50.4 | 47.1 | 49.8 | 47.7 | 37.0 | 45.8 | 35.9 | 46.8 |
| 59. TA30646 | 83.2 | 84.2 | 84.3 | 42.9 | 63.9 | 49.6 | 68.3 | 37.6 | 67.5 | 68.5 | 95.4 | 42.2 | 46.8 | 68.5 | 62.3 | 43.1 | 39.3 | 45.6 | 36.3 | 65.9 |
| 60. TA28621 | 67.8 | 68.4 | 66.6 | 42.4 | 54.9 | 51.3 | 77.1 | 38.7 | 76.6 | 93.8 | 66.8 | 43.6 | 48.6 | 94.4 | 66.8 | 45.0 | 38.5 | 41.5 | 36.5 | 64.1 |
| 61. TA34455 | 42.3 | 43.4 | 41.8 | 63.1 | 34.2 | 53.0 | 44.5 | 45.3 | 42.3 | 44.3 | 42.7 | 83.2 | 71.4 | 43.6 | 44.3 | 47.4 | 44.4 | 60.0 | 43.1 | 41.6 |
| 62. TA37666 | 48.7 | 48.7 | 47.4 | 63.7 | 39.8 | 61.0 | 49.5 | 42.4 | 48.2 | 49.5 | 49.5 | 71.1 | 88.9 | 50.3 | 48.2 | 54.7 | 41.8 | 55.8 | 38.5 | 47.6 |

| | 41 | 42 | 43 | 44 | 45 | 46 | 47 | 48 | 49 | 50 | 51 | 52 | 53 | 54 | 55 | 56 | 57 | 58 | 59 | 60 |
|---|---|---|---|---|---|---|---|---|---|---|---|---|---|---|---|---|---|---|---|---|
| 63. TC253044 | 52.8 | 54.9 | 50.8 | 40.3 | 47.6 | 48.4 | 50.3 | 56.5 | 55.9 | 54.7 | 54.0 | 41.3 | 45.7 | 55.7 | 52.7 | 42.5 | 50.0 | 40.6 | 38.4 | 52.9 |
| 64. TabHLH11 | 62.4 | 62.2 | 61.6 | 40.5 | 58.7 | 50.4 | 63.1 | 35.8 | 66.2 | 66.9 | 65.6 | 41.8 | 45.2 | 67.8 | 86.4 | 43.3 | 36.8 | 41.0 | 36.3 | 80.5 |
| 65. TA46156 | 85.1 | 85.2 | 83.3 | 44.7 | 67.8 | 53.4 | 67.3 | 38.0 | 70.0 | 70.9 | 81.1 | 43.3 | 48.3 | 70.8 | 66.0 | 44.1 | 37.0 | 42.4 | 35.7 | 66.2 |
| 66. GSVIVT00017237001 | 45.9 | 45.0 | 45.9 | 64.9 | 35.9 | 61.2 | 44.0 | 42.6 | 42.1 | 42.8 | 46.2 | 68.2 | 65.1 | 43.8 | 43.3 | 52.4 | 43.1 | 58.5 | 42.4 | 44.7 |
| 67. TA46194 | 73.2 | 72.5 | 72.2 | 44.3 | 57.8 | 54.3 | 70.6 | 38.7 | 72.8 | 74.8 | 73.2 | 44.9 | 49.6 | 75.1 | 68.4 | 45.8 | 39.1 | 45.1 | 37.6 | 68.7 |
| 68. GSVIVT00016367001 | 36.9 | 36.5 | 38.1 | 44.3 | 29.9 | 41.6 | 39.0 | 50.3 | 36.9 | 35.3 | 38.8 | 45.4 | 44.3 | 35.5 | 36.5 | 39.6 | 50.5 | 46.2 | 49.4 | 35.9 |
| 69. TA139285 | 60.7 | 61.5 | 61.0 | 40.7 | 56.1 | 49.0 | 64.7 | 35.6 | 66.2 | 68.9 | 64.2 | 41.1 | 46.8 | 68.1 | 90.5 | 45.5 | 38.1 | 40.4 | 34.0 | 84.0 |
| 70. TA126400 | 62.7 | 61.8 | 63.6 | 42.6 | 57.9 | 49.3 | 62.4 | 34.9 | 65.9 | 66.9 | 62.3 | 40.0 | 45.2 | 66.1 | 76.2 | 44.1 | 35.6 | 40.8 | 35.3 | 82.9 |

| | 41 | 42 | 43 | 44 | 45 | 46 | 47 | 48 | 49 | 50 | 51 | 52 | 53 | 54 | 55 | 56 | 57 | 58 | 59 | 60 |
|---|---|---|---|---|---|---|---|---|---|---|---|---|---|---|---|---|---|---|---|---|
| 1. TC3698 | 30.0 | 50.8 | 48.7 | 47.8 | 28.7 | 50.8 | 31.8 | 29.7 | 51.4 | 54.0 | 50.6 | 52.3 | 50.2 | 54.4 | 58.0 | 45.1 | 33.3 | 33.4 | 54.7 | 50.8 |
| 2. AT1G03040 | 29.5 | 65.7 | 48.6 | 47.5 | 29.9 | 66.7 | 31.1 | 31.1 | 66.7 | 70.0 | 52.4 | 46.8 | 49.2 | 66.5 | 50.6 | 40.2 | 33.0 | 31.8 | 67.0 | 49.2 |
| 3. AT4G02590 | 31.7 | 71.8 | 52.1 | 49.3 | 30.9 | 72.8 | 31.6 | 33.6 | 73.1 | 75.2 | 54.0 | 49.4 | 53.2 | 70.2 | 53.4 | 43.1 | 33.3 | 32.5 | 69.9 | 52.7 |
| 4. AT2G24260 | 21.4 | 16.7 | 16.6 | 15.5 | 18.1 | 17.0 | 18.9 | 23.0 | 17.0 | 16.4 | 17.3 | 15.6 | 15.4 | 17.0 | 16.6 | 18.5 | 20.8 | 19.9 | 16.5 | 15.7 |
| 5. AT4G30980 | 33.8 | 35.5 | 36.1 | 33.6 | 29.6 | 36.4 | 34.9 | 41.6 | 36.8 | 37.5 | 36.4 | 36.1 | 36.9 | 36.4 | 41.0 | 42.2 | 34.9 | 36.4 | 36.6 | 35.7 |
| 6. AT5G58010 | 31.9 | 34.6 | 34.2 | 34.1 | 31.3 | 35.8 | 36.3 | 35.6 | 35.8 | 35.1 | 33.9 | 36.2 | 35.1 | 35.4 | 38.3 | 46.5 | 33.5 | 39.9 | 36.2 | 34.7 |
| 7. TC15501 | 33.5 | 59.7 | 55.6 | 55.5 | 30.7 | 62.0 | 30.8 | 35.2 | 61.5 | 61.2 | 59.3 | 51.0 | 51.4 | 61.1 | 51.6 | 41.5 | 36.4 | 30.0 | 60.3 | 51.7 |
| 8. TC19278 | 17.0 | 19.7 | 17.0 | 18.0 | 22.7 | 19.7 | 29.4 | 19.0 | 20.5 | 20.3 | 18.3 | 18.8 | 18.4 | 19.8 | 21.0 | 25.1 | 20.2 | 30.4 | 20.1 | 18.4 |
| 9. TC10015_part | 30.6 | 67.5 | 50.4 | 48.7 | 27.4 | 68.5 | 30.9 | 31.0 | 68.8 | 70.7 | 53.3 | 48.7 | 50.0 | 65.9 | 51.8 | 42.4 | 32.8 | 31.8 | 66.2 | 50.0 |
| 10. DY268946 | 31.5 | 42.9 | 54.2 | 51.6 | 29.6 | 44.0 | 36.8 | 33.1 | 44.0 | 43.7 | 57.3 | 42.4 | 43.9 | 41.7 | 47.0 | 40.7 | 35.5 | 35.9 | 44.5 | 44.9 |
| 11. TA2544 | 43.3 | 36.2 | 36.9 | 36.4 | 33.1 | 36.4 | 32.5 | 45.2 | 36.6 | 36.1 | 37.0 | 33.5 | 35.9 | 35.7 | 35.1 | 37.6 | 40.8 | 34.2 | 34.5 | 36.1 |
| 12. TA3392 | 30.8 | 76.3 | 49.3 | 48.6 | 28.3 | 78.0 | 30.2 | 31.0 | 78.0 | 76.7 | 52.6 | 53.2 | 52.5 | 68.3 | 51.8 | 42.8 | 33.4 | 31.3 | 68.0 | 53.2 |
| 13. TA12416 | 47.5 | 32.7 | 32.7 | 33.3 | 32.5 | 33.5 | 31.2 | 98.3 | 32.8 | 33.5 | 33.5 | 30.3 | 32.2 | 32.6 | 32.9 | 35.2 | 37.7 | 31.7 | 32.2 | 32.2 |
| 14. WO051050seqID77 | 32.3 | 54.0 | 63.0 | 61.3 | 29.3 | 54.6 | 32.1 | 32.0 | 54.6 | 54.3 | 63.7 | 54.0 | 54.0 | 55.3 | 52.0 | 41.4 | 34.8 | 31.2 | 55.0 | 53.5 |
| 15. WO051050seqID1671 | 33.4 | 70.4 | 50.5 | 50.7 | 30.9 | 70.3 | 28.9 | 32.9 | 70.6 | 73.4 | 51.6 | 46.5 | 48.3 | 65.7 | 48.6 | 38.5 | 33.5 | 29.4 | 66.0 | 48.6 |
| 16. TA55042 | 31.1 | 47.8 | 61.3 | 60.1 | 30.4 | 48.1 | 30.9 | 33.3 | 48.1 | 50.0 | 62.5 | 47.4 | 50.3 | 49.2 | 51.5 | 40.4 | 31.6 | 33.0 | 49.5 | 50.9 |
| 17. TC207545 | 28.8 | 67.3 | 48.3 | 44.8 | 27.2 | 68.3 | 32.8 | 30.2 | 68.7 | 69.8 | 48.5 | 50.7 | 51.6 | 64.1 | 56.7 | 49.3 | 31.8 | 35.2 | 64.4 | 51.3 |
| 18. TA13791 | 32.5 | 80.7 | 50.1 | 50.7 | 30.7 | 82.0 | 32.6 | 33.3 | 82.7 | 82.1 | 54.9 | 53.4 | 54.2 | 71.8 | 54.9 | 43.3 | 35.4 | 32.8 | 72.4 | 54.2 |
| 19. CO123623 | 29.6 | 50.3 | 58.4 | 56.5 | 26.2 | 51.5 | 32.1 | 33.1 | 51.5 | 48.1 | 61.3 | 47.6 | 48.1 | 45.8 | 53.4 | 47.8 | 32.2 | 33.1 | 46.1 | 49.7 |
| 20. DT543504 | 29.0 | 33.3 | 33.3 | 32.7 | 40.8 | 32.9 | 36.1 | 30.8 | 33.5 | 35.1 | 33.1 | 35.0 | 36.4 | 34.1 | 34.6 | 41.2 | 31.0 | 37.2 | 35.3 | 37.2 |
| 21. TC60118 | 32.3 | 80.4 | 49.9 | 50.4 | 30.7 | 81.7 | 32.6 | 33.3 | 82.4 | 81.8 | 54.7 | 53.1 | 53.9 | 71.5 | 54.6 | 43.0 | 35.2 | 32.3 | 72.1 | 53.9 |
| 22. TC60119 | 31.4 | 80.5 | 49.9 | 48.6 | 30.1 | 81.8 | 31.2 | 33.6 | 82.4 | 82.8 | 53.3 | 53.8 | 54.1 | 73.5 | 51.7 | 42.9 | 35.1 | 32.4 | 73.8 | 55.0 |
| 23. TC61833 | 31.2 | 78.9 | 49.1 | 48.4 | 30.1 | 80.5 | 30.2 | 34.7 | 81.2 | 81.6 | 53.3 | 53.1 | 55.0 | 74.3 | 52.3 | 43.2 | 35.2 | 33.3 | 74.3 | 54.9 |
| 24. TC67603 | 43.3 | 33.3 | 33.2 | 32.0 | 33.3 | 34.0 | 31.5 | 56.8 | 33.9 | 32.8 | 34.2 | 32.5 | 33.0 | 32.7 | 33.9 | 37.6 | 36.3 | 33.2 | 32.5 | 33.6 |

| | | | | | | | | | | | | | | | | | | | | |
|---|---|---|---|---|---|---|---|---|---|---|---|---|---|---|---|---|---|---|---|---|
| 25. TC229602 | 26.2 | 61.0 | 43.8 | 41.6 | 25.1 | 62.3 | 32.9 | 27.7 | 62.7 | 64.2 | 47.9 | 47.0 | 49.0 | 59.8 | 52.5 | 48.2 | 29.4 | 34.5 | 60.1 | 49.0 |
| 26. TC205173 | 41.4 | 38.3 | 36.5 | 36.4 | 30.9 | 38.3 | 36.8 | 47.9 | 38.3 | 39.6 | 36.5 | 37.0 | 38.1 | 38.2 | 38.2 | 48.5 | 36.9 | 40.7 | 38.0 | 37.3 |
| 27. TA1140 | 31.2 | 54.7 | 54.0 | 50.8 | 27.7 | 52.7 | 31.9 | 34.3 | 52.7 | 52.9 | 53.7 | 61.7 | 64.3 | 55.4 | 52.8 | 41.1 | 31.4 | 32.8 | 54.0 | 64.9 |
| 28. TC140470 | 28.2 | 26.2 | 28.1 | 28.7 | 31.9 | 25.2 | 24.0 | 31.2 | 25.3 | 24.9 | 27.4 | 27.6 | 27.4 | 29.0 | 26.1 | 27.3 | 27.7 | 25.0 | 28.6 | 27.4 |
| 29. TA3490_part | 30.3 | 54.9 | 53.0 | 52.6 | 29.0 | 54.3 | 31.7 | 33.3 | 54.9 | 55.0 | 53.7 | 66.0 | 67.0 | 55.5 | 56.8 | 45.3 | 32.3 | 33.6 | 55.5 | 67.3 |
| 30. CK293938 | 30.0 | 53.0 | 52.7 | 50.9 | 28.6 | 52.0 | 30.9 | 31.2 | 52.6 | 54.0 | 54.1 | 76.4 | 87.9 | 53.3 | 56.8 | 41.2 | 32.7 | 32.7 | 53.5 | 88.9 |
| 31. TC8633 | 31.4 | 69.9 | 52.7 | 50.7 | 30.5 | 71.2 | 34.0 | 32.1 | 71.5 | 72.4 | 52.6 | 51.5 | 51.9 | 90.8 | 52.7 | 43.9 | 35.0 | 31.9 | 91.1 | 51.9 |
| 32. TC7102 | 48.4 | 35.3 | 33.5 | 34.4 | 32.9 | 35.7 | 32.8 | 56.1 | 35.7 | 36.7 | 36.8 | 33.2 | 35.6 | 35.7 | 34.3 | 36.2 | 38.0 | 33.4 | 35.2 | 35.3 |
| 33. TC7103 | 44.7 | 34.3 | 34.8 | 36.7 | 32.9 | 35.7 | 34.1 | 55.3 | 36.2 | 37.2 | 37.7 | 36.6 | 37.8 | 36.2 | 37.0 | 41.0 | 37.6 | 37.0 | 36.0 | 38.5 |
| 34. TC12771 | 30.0 | 53.6 | 53.0 | 50.6 | 28.8 | 51.7 | 31.4 | 31.9 | 52.3 | 54.0 | 54.7 | 77.7 | 89.2 | 54.1 | 57.4 | 40.9 | 32.1 | 33.1 | 53.8 | 90.5 |
| 35. Os03g58330 | 32.3 | 54.3 | 50.1 | 49.0 | 27.4 | 51.2 | 33.5 | 31.8 | 51.2 | 55.0 | 52.8 | 51.1 | 52.1 | 53.6 | 72.9 | 44.2 | 34.1 | 34.6 | 54.0 | 52.7 |
| 36. Os06g08500 | 38.0 | 32.9 | 32.1 | 31.3 | 27.7 | 34.3 | 32.3 | 37.9 | 34.6 | 33.9 | 34.5 | 29.6 | 33.1 | 33.1 | 33.3 | 36.6 | 32.8 | 32.0 | 32.8 | 32.7 |
| 37. Os06g09370 | 28.6 | 27.6 | 31.2 | 30.4 | 32.1 | 27.9 | 23.9 | 27.8 | 28.1 | 26.7 | 30.1 | 26.3 | 28.6 | 28.5 | 27.4 | 28.2 | 27.5 | 25.4 | 28.4 | 27.6 |
| 38. Os02g55250 | 46.4 | 33.4 | 34.2 | 34.4 | 32.8 | 33.7 | 33.5 | 45.8 | 34.7 | 34.1 | 35.8 | 33.3 | 34.2 | 33.5 | 34.0 | 36.1 | 36.8 | 34.3 | 34.6 | 34.0 |
| 39. Os02g35660 | 30.4 | 26.4 | 29.1 | 27.2 | 31.9 | 26.1 | 25.7 | 30.5 | 26.4 | 28.2 | 27.7 | 25.5 | 27.8 | 27.3 | 26.5 | 27.9 | 29.1 | 26.6 | 27.6 | 28.1 |
| 40. Os07g08440 | 31.4 | 53.4 | 50.0 | 47.8 | 27.7 | 52.3 | 31.7 | 31.7 | 52.3 | 56.2 | 55.8 | 51.9 | 50.8 | 51.4 | 81.3 | 45.6 | 33.6 | 31.9 | 52.6 | 50.8 |
| 41. Os09g25040 | | 30.6 | 31.2 | 31.2 | 31.1 | 30.9 | 29.1 | 47.7 | 31.7 | 31.5 | 31.6 | 29.4 | 30.5 | 31.7 | 31.1 | 32.5 | 37.2 | 31.3 | 31.5 | 30.4 |
| 42. Pt_scaff_II.416 | 38.3 | | 50.3 | 49.6 | 29.1 | 93.7 | 30.5 | 33.0 | 93.0 | 88.9 | 52.1 | 52.5 | 54.9 | 72.7 | 53.4 | 44.4 | 36.5 | 32.4 | 73.0 | 54.5 |
| 43. Pt_scaff_70.65 | 38.1 | 63.1 | | 88.4 | 29.5 | 50.0 | 31.0 | 32.4 | 49.7 | 51.6 | 73.2 | 51.5 | 51.7 | 52.3 | 49.4 | 42.3 | 34.6 | 31.6 | 52.0 | 52.0 |
| 44. Pt_scaff_XIII.403 | 38.5 | 63.2 | 90.7 | | 27.9 | 49.6 | 28.2 | 32.3 | 49.3 | 49.6 | 71.3 | 49.9 | 50.4 | 50.6 | 48.8 | 40.0 | 34.8 | 30.5 | 50.6 | 51.3 |
| 45. Pt_scaff28.86 | 46.1 | 39.3 | 40.4 | 40.6 | | 29.8 | 31.8 | 33.2 | 30.3 | 28.9 | 29.8 | 28.7 | 27.9 | 30.1 | 27.6 | 31.1 | 33.6 | 32.2 | 30.1 | 28.5 |
| 46. Pt_TC63334 | 38.1 | 95.3 | 63.4 | 61.4 | 40.4 | | 30.8 | 33.6 | 98.7 | 89.2 | 52.3 | 52.5 | 53.9 | 74.0 | 53.7 | 44.6 | 35.2 | 33.1 | 74.3 | 53.9 |
| 47. TA14134 | 38.1 | 48.3 | 49.2 | 47.6 | 42.1 | 49.2 | | 30.1 | 30.8 | 31.4 | 31.4 | 29.6 | 30.5 | 33.2 | 31.4 | 39.1 | 33.7 | 49.4 | 33.4 | 30.0 |
| 48. TA5414 | 60.5 | 42.1 | 41.0 | 42.1 | 47.9 | 42.7 | 39.7 | | 32.7 | 33.8 | 32.4 | 30.3 | 32.5 | 32.4 | 31.9 | 35.3 | 38.1 | 30.2 | 31.8 | 32.5 |
| 49. TA3263 | 38.7 | 95.0 | 63.4 | 61.4 | 41.0 | 99.0 | 49.8 | 42.1 | | 89.2 | 52.6 | 53.1 | 54.5 | 74.3 | 54.3 | 44.9 | 35.8 | 33.6 | 74.6 | 54.5 |
| 50. TA2825 | 39.1 | 93.3 | 64.7 | 62.9 | 39.0 | 93.0 | 50.2 | 41.2 | 93.3 | | 53.6 | 53.6 | 54.9 | 76.3 | 54.0 | 44.8 | 35.0 | 32.0 | 76.3 | 54.9 |
| 51. TA1616 | 39.5 | 65.6 | 83.7 | 80.5 | 41.2 | 64.1 | 50.3 | 40.6 | 64.1 | 66.3 | | 51.1 | 52.4 | 54.1 | 54.8 | 42.5 | 35.7 | 32.1 | 54.1 | 53.6 |
| 52. TA21665 | 37.7 | 67.0 | 63.7 | 63.2 | 37.7 | 66.7 | 48.0 | 41.2 | 67.0 | 66.6 | 65.0 | | 75.2 | 52.3 | 53.8 | 43.0 | 32.0 | 32.5 | 52.0 | 76.5 |
| 53. TC172581 | 38.1 | 68.4 | 64.7 | 63.5 | 39.9 | 66.8 | 49.8 | 42.1 | 67.1 | 68.4 | 65.6 | 82.2 | | 53.3 | 54.0 | 43.5 | 34.0 | 31.8 | 52.1 | 96.7 |
| 54. AK247217 | 37.9 | 83.8 | 65.3 | 63.8 | 42.8 | 84.4 | 50.8 | 41.9 | 84.4 | 86.1 | 66.9 | 65.6 | 66.8 | | 52.8 | 44.3 | 35.0 | 32.8 | 98.3 | 53.8 |
| 55. TC104646 | 37.1 | 63.3 | 57.4 | 57.8 | 36.0 | 63.7 | 44.6 | 38.9 | 64.0 | 62.9 | 61.7 | 65.9 | 65.8 | 62.9 | | 48.5 | 34.2 | 34.4 | 53.5 | 55.0 |
| 56. DV982110 | 38.9 | 58.3 | 54.1 | 50.3 | 40.1 | 58.3 | 51.1 | 41.7 | 58.7 | 57.5 | 53.7 | 57.4 | 53.3 | 55.0 | 60.3 | | 54.2 | 40.7 | 45.0 | 43.2 |
| 57. WO051050seqID516 | 52.3 | 44.7 | 44.5 | 44.7 | 49.5 | 42.5 | 42.8 | 51.7 | 43.0 | 43.0 | 46.0 | 41.7 | 43.0 | 42.3 | 42.3 | 55.7 | | 37.2 | 34.6 | 33.7 |
| 58. TC68930 | 40.9 | 49.8 | 48.0 | 46.1 | 45.0 | 50.8 | 63.7 | 40.4 | 51.1 | 48.9 | 50.2 | 48.3 | 50.2 | 49.5 | 47.7 | 53.2 | 47.9 | | 32.9 | 31.7 |

EP 2 230 310 B1

| | | | | | | | | | | | | | | | | | | | | |
|---|---|---|---|---|---|---|---|---|---|---|---|---|---|---|---|---|---|---|---|---|
| 59. TA30646 | 37.9 | 84.1 | 65.3 | 63.8 | 42.8 | 84.8 | 49.8 | 41.7 | 84.8 | 85.8 | 66.9 | 65.2 | 66.8 | 99.0 | 63.6 | 56.6 | 42.1 | 49.2 | | 53.0 |
| 60. TA28621 | 37.7 | 68.8 | 65.3 | 63.8 | 40.4 | 67.4 | 48.3 | 41.2 | 67.8 | 69.1 | 66.0 | 83.6 | 97.7 | 67.1 | 65.5 | 53.6 | 43.8 | 48.3 | 67.4 | |
| 61. TA34455 | 59.5 | 42.5 | 44.7 | 46.5 | 46.1 | 41.2 | 42.7 | 66.9 | 40.9 | 42.5 | 43.0 | 40.9 | 43.2 | 41.4 | 40.0 | 42.5 | 53.8 | 44.5 | 41.2 | 42.7 |
| 62. TA37666 | 52.3 | 49.7 | 51.0 | 49.2 | 43.9 | 50.0 | 47.1 | 63.5 | 50.3 | 47.6 | 48.2 | 49.7 | 48.4 | 47.6 | 44.5 | 46.1 | 48.8 | 51.0 | 48.2 | 49.5 |
| 63. TC253044 | 36.5 | 54.0 | 49.8 | 50.3 | 41.4 | 53.3 | 46.8 | 39.1 | 54.0 | 54.5 | 49.1 | 54.4 | 53.6 | 51.7 | 50.0 | 55.2 | 40.0 | 51.7 | 51.0 | 53.9 |
| 64. TabHLH11 | 36.5 | 62.3 | 58.3 | 59.0 | 36.4 | 63.0 | 45.5 | 38.2 | 63.3 | 63.2 | 59.5 | 65.9 | 66.1 | 65.6 | 83.3 | 60.5 | 43.0 | 46.2 | 65.2 | 64.5 |
| 65. TA46156 | 37.5 | 85.0 | 64.7 | 61.4 | 39.0 | 85.3 | 50.5 | 40.2 | 85.7 | 87.0 | 65.0 | 66.9 | 67.8 | 82.5 | 65.1 | 60.2 | 42.5 | 48.9 | 82.5 | 69.4 |
| 66. GSVIVT00017237001 | 56.5 | 44.3 | 45.9 | 48.1 | 47.6 | 45.0 | 44.0 | 69.2 | 45.2 | 44.7 | 45.0 | 43.1 | 44.7 | 44.7 | 41.4 | 45.7 | 55.9 | 47.4 | 44.5 | 45.2 |
| 67. TA46194 | 39.7 | 73.8 | 70.4 | 69.2 | 40.1 | 73.5 | 49.5 | 42.1 | 73.8 | 73.5 | 74.8 | 72.5 | 73.2 | 74.4 | 66.8 | 56.9 | 44.3 | 50.5 | 74.4 | 73.5 |
| 68. GSVIVT00016367001 | 45.9 | 38.8 | 40.6 | 41.2 | 63.9 | 36.7 | 39.0 | 45.6 | 36.3 | 37.9 | 37.9 | 36.7 | 37.3 | 38.8 | 34.6 | 37.3 | 44.5 | 40.6 | 38.6 | 37.1 |
| 69. TA139285 | 37.7 | 61.7 | 58.3 | 58.4 | 38.4 | 61.7 | 47.1 | 38.2 | 61.7 | 61.2 | 64.1 | 66.2 | 65.5 | 64.2 | 82.5 | 59.6 | 42.8 | 48.6 | 64.6 | 65.8 |
| 70. TA126400 | 36.7 | 64.0 | 58.0 | 55.7 | 36.4 | 64.7 | 45.8 | 41.5 | 65.0 | 64.9 | 62.0 | 66.2 | 64.5 | 61.9 | 92.3 | 59.3 | 42.5 | 48.0 | 61.9 | 65.5 |

| | 61 | 62 | 63 | 64 | 65 | 66 | 67 | 68 | 69 | 70 |
|---|---|---|---|---|---|---|---|---|---|---|
| 1. TC3698 | 31.4 | 35.3 | 37.5 | 56.1 | 53.5 | 34.9 | 54.6 | 25.8 | 58.1 | 55.7 |
| 2. AT1G03040 | 32.8 | 36.4 | 37.3 | 50.6 | 63.2 | 33.5 | 53.4 | 28.0 | 50.8 | 49.4 |
| 3. AT4G02590 | 33.4 | 38.9 | 37.0 | 53.7 | 69.0 | 34.8 | 56.3 | 28.5 | 53.7 | 50.7 |
| 4. AT2G24260 | 20.6 | 21.8 | 14.8 | 17.0 | 16.3 | 22.1 | 16.5 | 16.3 | 16.8 | 16.5 |
| 5. AT4G30980 | 41.3 | 43.2 | 35.3 | 39.5 | 38.6 | 39.7 | 37.3 | 29.5 | 40.2 | 40.5 |
| 6. AT5G58010 | 36.6 | 40.5 | 36.6 | 37.3 | 36.1 | 36.2 | 35.9 | 28.6 | 38.4 | 37.5 |
| 7. TC15501 | 37.0 | 37.5 | 34.0 | 51.1 | 59.6 | 34.8 | 64.3 | 30.7 | 53.4 | 49.7 |
| 8. TC19278 | 17.0 | 20.6 | 24.1 | 19.9 | 21.3 | 20.0 | 20.1 | 19.5 | 20.2 | 20.1 |
| 9. TC10015_part | 32.7 | 35.1 | 36.8 | 53.9 | 68.5 | 33.2 | 54.4 | 26.9 | 51.8 | 51.6 |
| 10. DY268946 | 34.0 | 34.6 | 33.1 | 49.0 | 45.3 | 36.4 | 50.0 | 30.2 | 46.1 | 45.5 |
| 11. TA2544 | 46.6 | 45.3 | 31.7 | 32.9 | 36.3 | 42.9 | 37.1 | 33.9 | 36.2 | 35.0 |
| 12. TA3392 | 34.1 | 36.5 | 37.5 | 53.7 | 73.3 | 34.2 | 57.6 | 28.0 | 54.4 | 52.6 |
| 13. TA12416 | 53.7 | 52.9 | 28.8 | 32.9 | 33.3 | 58.2 | 33.3 | 31.9 | 31.8 | 33.3 |
| 14. WO051050seqID77 | 35.8 | 33.9 | 36.7 | 49.2 | 56.2 | 33.4 | 60.3 | 26.9 | 52.6 | 51.2 |
| 15. WO051050seqID1671 | 33.8 | 34.8 | 33.8 | 48.9 | 66.8 | 34.0 | 57.0 | 29.8 | 49.6 | 48.9 |
| 16. TA55042 | 35.3 | 36.8 | 35.1 | 49.0 | 51.4 | 34.2 | 56.0 | 28.4 | 52.0 | 51.2 |
| 17. TC207545 | 29.6 | 32.8 | 39.7 | 54.5 | 67.5 | 33.2 | 55.4 | 26.1 | 54.6 | 55.6 |
| 18. TA13791 | 36.0 | 38.0 | 36.3 | 54.7 | 75.6 | 36.5 | 59.8 | 29.1 | 54.6 | 53.7 |
| 19. CO123623 | 29.8 | 33.3 | 36.7 | 53.0 | 49.4 | 30.9 | 55.6 | 27.3 | 52.5 | 51.9 |
| 20. DT543504 | 32.0 | 33.9 | 41.4 | 37.9 | 36.0 | 31.4 | 34.5 | 37.8 | 34.8 | 35.2 |

| | | | | | | | | | | |
|---|---|---|---|---|---|---|---|---|---|---|
| 21. TC60118 | 36.0 | 38.0 | 36.3 | 54.4 | 75.2 | 36.5 | 59.5 | 29.1 | 54.2 | 53.4 |
| 22. TC60119 | 34.8 | 37.5 | 36.4 | 53.9 | 75.7 | 35.6 | 59.4 | 29.0 | 53.9 | 51.2 |
| 23. TC61833 | 34.0 | 36.7 | 34.1 | 53.1 | 73.4 | 35.2 | 59.2 | 28.6 | 53.8 | 52.5 |
| 24. TC67603 | 46.9 | 51.7 | 30.2 | 32.6 | 34.9 | 51.4 | 34.0 | 31.0 | 32.4 | 33.9 |
| 25. TC229602 | 27.7 | 31.3 | 34.7 | 51.4 | 63.3 | 29.4 | 53.2 | 23.3 | 51.4 | 50.0 |
| 26. TC205173 | 42.1 | 48.7 | 35.1 | 38.1 | 39.7 | 52.3 | 40.4 | 30.7 | 37.6 | 38.8 |
| 27. TA1140 | 34.5 | 37.4 | 36.7 | 52.1 | 54.3 | 35.5 | 61.0 | 27.7 | 54.6 | 52.8 |
| 28. TC140470 | 30.3 | 28.1 | 55.1 | 25.1 | 24.9 | 29.7 | 28.1 | 34.4 | 25.2 | 25.5 |
| 29. TA3490_part | 33.6 | 36.7 | 36.5 | 54.6 | 58.5 | 34.0 | 61.3 | 26.3 | 55.1 | 54.5 |
| 30. CK293938 | 34.3 | 37.7 | 36.6 | 52.6 | 55.9 | 34.3 | 65.0 | 27.8 | 55.8 | 54.2 |
| 31. TC8633 | 33.7 | 36.8 | 35.2 | 53.7 | 71.7 | 36.0 | 59.3 | 29.4 | 54.1 | 50.9 |
| 32. TC7102 | 71.8 | 63.0 | 31.4 | 36.8 | 34.2 | 54.6 | 35.6 | 30.0 | 34.8 | 34.0 |
| 33. TC7103 | 62.4 | 83.3 | 33.6 | 39.1 | 36.0 | 53.1 | 38.0 | 29.6 | 38.5 | 36.9 |
| 34. TC12771 | 34.6 | 38.1 | 36.6 | 53.7 | 57.2 | 33.9 | 66.2 | 28.7 | 55.8 | 54.2 |
| 35. Os03g58330 | 36.6 | 37.6 | 34.9 | 80.3 | 53.5 | 34.5 | 58.3 | 27.8 | 87.2 | 69.4 |
| 36. Os06g08500 | 39.0 | 40.0 | 30.1 | 34.1 | 32.6 | 40.3 | 33.5 | 27.5 | 35.9 | 32.3 |
| 37. Os06g09370 | 30.3 | 28.4 | 45.0 | 26.8 | 27.4 | 29.2 | 27.7 | 34.1 | 28.0 | 25.9 |
| 38. Os02g55250 | 46.1 | 44.2 | 31.3 | 33.8 | 33.6 | 46.3 | 34.4 | 30.3 | 34.6 | 34.8 |
| 39. Os02g35660 | 31.3 | 29.8 | 29.5 | 27.1 | 25.9 | 31.3 | 27.7 | 33.7 | 27.2 | 27.6 |
| 40. Os07g08440 | 33.6 | 36.3 | 35.3 | 74.9 | 53.0 | 35.8 | 57.1 | 27.0 | 79.7 | 76.2 |
| 41. Os09g25040 | 45.6 | 43.6 | 27.9 | 31.1 | 30.5 | 47.3 | 31.6 | 30.4 | 32.3 | 31.4 |
| 42. Pt_scaff_II.416 | 34.1 | 35.6 | 35.5 | 53.9 | 74.1 | 34.4 | 61.5 | 28.9 | 55.3 | 52.2 |
| 43. Pt_scaff_70.65 | 34.1 | 37.6 | 35.0 | 48.8 | 53.6 | 35.0 | 58.9 | 28.0 | 49.5 | 50.1 |
| 44. Pt_scaff_XIII.403 | 34.7 | 36.5 | 34.2 | 47.5 | 50.3 | 35.8 | 57.4 | 29.5 | 47.6 | 48.1 |
| 45. Pt_scaff28.86 | 32.2 | 32.3 | 31.2 | 28.1 | 28.3 | 34.3 | 31.0 | 49.3 | 29.8 | 26.4 |
| 46. Pt_TC63334 | 33.9 | 37.2 | 34.8 | 54.8 | 76.6 | 35.1 | 60.0 | 28.5 | 55.6 | 53.1 |
| 47. TA14134 | 30.4 | 35.1 | 32.5 | 32.0 | 32.4 | 33.7 | 32.5 | 29.4 | 32.9 | 32.2 |
| 48. TA5414 | 52.7 | 53.3 | 29.2 | 32.9 | 33.8 | 57.9 | 33.8 | 32.1 | 32.6 | 32.8 |
| 49. TA3263 | 33.8 | 37.7 | 34.8 | 55.1 | 76.9 | 35.6 | 60.0 | 28.3 | 55.6 | 53.4 |
| 50. TA2825 | 34.6 | 35.8 | 35.6 | 55.8 | 77.7 | 35.8 | 62.0 | 28.0 | 55.5 | 53.7 |
| 51. TA1616 | 35.0 | 36.8 | 34.7 | 51.4 | 56.6 | 34.4 | 62.5 | 29.2 | 55.0 | 54.4 |
| 52. TA21665 | 32.3 | 36.3 | 35.6 | 52.1 | 52.9 | 33.2 | 60.5 | 27.6 | 53.6 | 52.7 |
| 53. TC172581 | 34.2 | 37.7 | 37.1 | 51.9 | 57.3 | 35.3 | 64.2 | 28.6 | 53.1 | 51.6 |
| 54. AK247217 | 34.2 | 36.4 | 34.4 | 54.0 | 73.9 | 35.3 | 60.2 | 30.1 | 53.3 | 50.9 |

| 55. TC104646 | 34.4 | 36.3 | 35.7 | 74.4 | 54.6 | 34.2 | 58.1 | 26.3 | 77.6 | 91.2 |
|---|---|---|---|---|---|---|---|---|---|---|
| 56. DV982110 | 34.7 | 38.2 | 41.9 | 45.8 | 45.4 | 37.3 | 43.8 | 29.7 | 46.7 | 45.9 |
| 57. WO051050seqID516 | 38.0 | 37.3 | 29.6 | 33.4 | 35.4 | 40.4 | 36.1 | 29.6 | 34.8 | 33.3 |
| 58. TC68930 | 31.5 | 36.6 | 35.7 | 32.2 | 32.1 | 35.4 | 32.3 | 30.7 | 34.3 | 34.6 |
| 59. TA30646 | 32.0 | 36.7 | 34.4 | 54.7 | 73.9 | 35.5 | 60.2 | 29.5 | 54.9 | 51.2 |
| 60. TA28621 | 33.6 | 39.1 | 37.4 | 51.4 | 56.2 | 35.3 | 63.5 | 28.7 | 53.4 | 52.2 |
| 61. TA34455 | | 61.4 | 30.3 | 35.9 | 33.5 | 50.6 | 35.4 | 32.2 | 34.9 | 34.6 |
| 62. TA37666 | 70.7 | | 32.2 | 39.2 | 36.2 | 52.3 | 38.4 | 30.4 | 37.4 | 37.9 |
| 63. TC253044 | 40.9 | 47.1 | | 34.6 | 36.5 | 33.0 | 37.2 | 30.6 | 35.8 | 35.8 |
| 64. TabHLH11 | 41.6 | 45.5 | 51.4 | | 52.5 | 33.3 | 55.7 | 27.7 | 82.7 | 70.7 |
| 65. TA46156 | 41.6 | 47.6 | 53.8 | 66.4 | | 35.1 | 65.7 | 27.0 | 55.3 | 52.9 |
| 66. GSVIVT00017237001 | 67.1 | 64.4 | 43.5 | 41.9 | 45.9 | | 36.0 | 31.6 | 32.5 | 35.6 |
| 67. TA46194 | 45.2 | 50.5 | 51.1 | 66.1 | 75.7 | 45.0 | | 29.3 | 57.5 | 56.5 |
| 68. GSVIVT00016367001 | 46.8 | 42.7 | 41.4 | 35.1 | 36.1 | 44.5 | 37.5 | | 26.9 | 26.1 |
| 69. TA139285 | 43.2 | 44.0 | 54.8 | 89.5 | 66.4 | 40.0 | 68.1 | 37.3 | | 72.8 |
| 70. TA126400 | 40.7 | 46.1 | 53.8 | 79.6 | 65.1 | 41.9 | 66.5 | 35.7 | 80.4 | |

Table E3: MatGAT results for global similarity and identity over the HLH domains of the bHLH11-like polypeptide sequences.

| | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|
| 1. AT2G24260 | | 98.0 | 92.2 | 98.0 | 98.0 | 98.0 | 98.0 | 98.0 | 98.0 | 94.1 | 94.1 | 96.1 | 86.3 | 98.0 | 88.9 | 96.1 | 98.0 | 98.0 |
| 2. AT4G30980 | 100.0 | | 94.1 | 96.1 | 96.1 | 96.1 | 96.1 | 96.1 | 96.1 | 96.1 | 96.1 | 94.1 | 88.2 | 96.1 | 87.0 | 94.1 | 96.1 | 96.1 |
| 3. AT5G58010 | 98.0 | 98.0 | | 92.2 | 92.2 | 92.2 | 92.2 | 92.2 | 92.2 | 96.1 | 96.1 | 90.2 | 84.3 | 92.2 | 83.3 | 90.2 | 92.2 | 92.2 |
| 4. TA12416 | 98.0 | 98.0 | 98.0 | | 100.0 | 100.0 | 100.0 | 100.0 | 100.0 | 96.1 | 96.1 | 98.0 | 88.2 | 100.0 | 90.7 | 98.0 | 98.0 | 98.0 |
| 5. TA5414 | 98.0 | 98.0 | 98.0 | 100.0 | | 100.0 | 100.0 | 100.0 | 100.0 | 96.1 | 96.1 | 98.0 | 88.2 | 100.0 | 90.7 | 98.0 | 98.0 | 98.0 |
| 6. TC205173 | 98.0 | 98.0 | 98.0 | 100.0 | 100.0 | | 100.0 | 100.0 | 100.0 | 96.1 | 96.1 | 98.0 | 88.2 | 100.0 | 90.7 | 98.0 | 98.0 | 98.0 |
| 7. GSVIVT00017237001 | 98.0 | 98.0 | 98.0 | 100.0 | 100.0 | 100.0 | | 100.0 | 100.0 | 96.1 | 96.1 | 98.0 | 88.2 | 100.0 | 90.7 | 98.0 | 98.0 | 98.0 |
| 8. TC7102 | 98.0 | 98.0 | 98.0 | 100.0 | 100.0 | 100.0 | 100.0 | | 100.0 | 96.1 | 96.1 | 98.0 | 88.2 | 100.0 | 90.7 | 98.0 | 98.0 | 98.0 |
| 9. TA34455 | 98.0 | 98.0 | 98.0 | 100.0 | 100.0 | 100.0 | 100.0 | 100.0 | | 96.1 | 96.1 | 98.0 | 88.2 | 100.0 | 90.7 | 98.0 | 98.0 | 98.0 |
| 10. TC7103 | 98.0 | 98.0 | 98.0 | 100.0 | 100.0 | 100.0 | 100.0 | 100.0 | 100.0 | | 100.0 | 94.1 | 88.2 | 96.1 | 87.0 | 94.1 | 94.1 | 94.1 |
| 11. TA37666 | 98.0 | 98.0 | 98.0 | 100.0 | 100.0 | 100.0 | 100.0 | 100.0 | 100.0 | 100.0 | | 94.1 | 88.2 | 96.1 | 87.0 | 94.1 | 94.1 | 94.1 |
| 12. TC67603 | 96.1 | 96.1 | 96.1 | 98.0 | 98.0 | 98.0 | 98.0 | 98.0 | 98.0 | 98.0 | 98.0 | | 86.3 | 98.0 | 88.9 | 96.1 | 96.1 | 96.1 |
| 13. Os06g08500 | 90.2 | 90.2 | 90.2 | 92.2 | 92.2 | 92.2 | 92.2 | 92.2 | 92.2 | 92.2 | 92.2 | 90.2 | | 88.2 | 79.6 | 86.3 | 86.3 | 86.3 |
| 14. Os02g55250 | 98.0 | 98.0 | 98.0 | 100.0 | 100.0 | 100.0 | 100.0 | 100.0 | 100.0 | 100.0 | 100.0 | 98.0 | 92.2 | | 90.7 | 98.0 | 98.0 | 98.0 |
| 15. Os09g25040 | 88.9 | 88.9 | 88.9 | 90.7 | 90.7 | 90.7 | 90.7 | 90.7 | 90.7 | 90.7 | 90.7 | 88.9 | 83.3 | 90.7 | | 88.9 | 88.9 | 88.9 |

EP 2 230 310 B1

| | | | | | | | | | | | | | | | | | | |
|---|---|---|---|---|---|---|---|---|---|---|---|---|---|---|---|---|---|---|
| 16. TA2544 | 98.0 | 98.0 | 98.0 | 100.0 | 100.0 | 100.0 | 100.0 | 100.0 | 100.0 | 100.0 | 100.0 | 98.0 | 92.2 | 100.0 | 90.7 | | 96.1 | 96.1 |
| 17. DV982110 | 98.0 | 98.0 | 100.0 | 100.0 | 100.0 | 100.0 | 100.0 | 100.0 | 100.0 | 100.0 | 100.0 | 98.0 | 92.2 | 100.0 | 90.7 | 100.0 | | 100.0 |
| 18. WO051050seqID516 | 98.0 | 98.0 | 100.0 | 100.0 | 100.0 | 100.0 | 100.0 | 100.0 | 100.0 | 100.0 | 100.0 | 98.0 | 92.2 | 100.0 | 90.7 | 100.0 | 100.0 | |
| 19. AT4G02590 | 96.1 | 96.1 | 96.1 | 96.1 | 96.1 | 96.1 | 96.1 | 96.1 | 96.1 | 96.1 | 96.1 | 94.1 | 88.2 | 96.1 | 87.0 | 96.1 | 96.1 | 96.1 |
| 20. TC10015_part | 96.1 | 96.1 | 96.1 | 96.1 | 96.1 | 96.1 | 96.1 | 96.1 | 96.1 | 96.1 | 96.1 | 94.1 | 88.2 | 96.1 | 87.0 | 96.1 | 96.1 | 96.1 |
| 21. AT1G03040 | 96.1 | 96.1 | 96.1 | 96.1 | 96.1 | 96.1 | 96.1 | 96.1 | 96.1 | 96.1 | 96.1 | 94.1 | 88.2 | 96.1 | 87.0 | 96.1 | 96.1 | 96.1 |
| 22. TA13791 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 23. TC60118 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 24. TC60119 | 94.1 | 94.1 | 94.1 | 94.1 | 94.1 | 94.1 | 94.1 | 94.1 | 94.1 | 94.1 | 94.1 | 92.2 | 86.3 | 94.1 | 85.2 | 94.1 | 94.1 | 94.1 |
| 25. TC61833 | 96.1 | 96.1 | 96.1 | 96.1 | 96.1 | 96.1 | 96.1 | 96.1 | 96.1 | 96.1 | 96.1 | 94.1 | 88.2 | 96.1 | 87.0 | 96.1 | 96.1 | 96.1 |
| 26. TA3392 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 27. WO051050seqID1671 | 96.1 | 96.1 | 96.1 | 96.1 | 96.1 | 96.1 | 96.1 | 96.1 | 96.1 | 96.1 | 96.1 | 94.1 | 88.2 | 96.1 | 87.0 | 96.1 | 96.1 | 96.1 |
| 28. Pt_TC63334 | 96.1 | 96.1 | 96.1 | 96.1 | 96.1 | 96.1 | 96.1 | 96.1 | 96.1 | 96.1 | 96.1 | 94.1 | 88.2 | 96.1 | 87.0 | 96.1 | 96.1 | 96.1 |
| 29. TA3263 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 30. Pt_scaff_II.416 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 31. TA2825 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 32. TC207545 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 33. TC229602 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 34. TA46156 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 35. AK247217 | 96.1 | 96.1 | 96.1 | 96.1 | 96.1 | 96.1 | 96.1 | 96.1 | 96.1 | 96.1 | 96.1 | 94.1 | 88.2 | 96.1 | 87.0 | 96.1 | 96.1 | 96.1 |
| 36. TA30646 | 96.1 | 96.1 | 96.1 | 96.1 | 96.1 | 96.1 | 96.1 | 96.1 | 96.1 | 96.1 | 96.1 | 94.1 | 88.2 | 96.1 | 87.0 | 96.1 | 96.1 | 96.1 |
| 37. TC8633 | 96.1 | 96.1 | 96.1 | 96.1 | 96.1 | 96.1 | 96.1 | 96.1 | 96.1 | 96.1 | 96.1 | 94.1 | 88.2 | 96.1 | 87.0 | 96.1 | 96.1 | 96.1 |
| 38. TC15501 | 98.0 | 98.0 | 100.0 | 100.0 | 100.0 | 100.0 | 100.0 | 100.0 | 100.0 | 100.0 | 100.0 | 98.0 | 92.2 | 100.0 | 90.7 | 100.0 | 100.0 | 100.0 |
| 39. TA1140 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 40. TA3490_part | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 41. CK293938 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 42. TC12771 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 43. TC172581 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 44. TA28621 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 45. TA21665 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 46. TA46194 | 98.0 | 98.0 | 100.0 | 100.0 | 100.0 | 100.0 | 100.0 | 100.0 | 100.0 | 100.0 | 100.0 | 98.0 | 92.2 | 100.0 | 90.7 | 100.0 | 100.0 | 100.0 |
| 47. DY268946 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 48. CO123623 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 49. Pt_scaff_70.65 | 92.2 | 92.2 | 92.2 | 92.2 | 92.2 | 92.2 | 92.2 | 92.2 | 92.2 | 92.2 | 92.2 | 94.1 | 84.3 | 92.2 | 83.3 | 92.2 | 92.2 | 92.2 |

EP 2 230 310 B1

| | | | | | | | | | | | | | | | | | | |
|---|---|---|---|---|---|---|---|---|---|---|---|---|---|---|---|---|---|---|
| 50. Pt_scaff_XIII.403 | 92.2 | 92.2 | 92.2 | 92.2 | 92.2 | 92.2 | 92.2 | 92.2 | 92.2 | 92.2 | 92.2 | 94.1 | 84.3 | 92.2 | 83.3 | 92.2 | 92.2 | 92.2 |
| 51. TA1616 | 96.1 | 96.1 | 96.1 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 52. TA55042 | 98.0 | 98.0 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 53. WO051050seqID77 | 96.1 | 96.1 | 96.1 | 96.1 | 96.1 | 96.1 | 96.1 | 96.1 | 96.1 | 96.1 | 96.1 | 94.1 | 88.2 | 96.1 | 87.0 | 96.1 | 96.1 | 96.1 |
| 54. TC3698 | 96.1 | 96.1 | 98.0 | 96.1 | 96.1 | 96.1 | 96.1 | 96.1 | 96.1 | 96.1 | 96.1 | 94.1 | 88.2 | 96.1 | 87.0 | 96.1 | 98.0 | 98.0 |
| 55. Os03g58330 | 98.0 | 98.0 | 100.0 | 98.0 | 98.0 | 98.0 | 98.0 | 98.0 | 98.0 | 98.0 | 98.0 | 96.1 | 90.2 | 98.0 | 88.9 | 98.0 | 100.0 | 100.0 |
| 56. TA139285 | 98.0 | 98.0 | 100.0 | 98.0 | 98.0 | 98.0 | 98.0 | 98.0 | 98.0 | 98.0 | 98.0 | 96.1 | 90.2 | 98.0 | 88.9 | 98.0 | 100.0 | 100.0 |
| 57. TabHLH11 | 96.1 | 96.1 | 98.0 | 96.1 | 96.1 | 96.1 | 96.1 | 96.1 | 96.1 | 96.1 | 96.1 | 94.1 | 88.2 | 96.1 | 87.0 | 96.1 | 98.0 | 98.0 |
| 58. TC104646 | 98.0 | 98.0 | 100.0 | 98.0 | 98.0 | 98.0 | 98.0 | 98.0 | 98.0 | 98.0 | 98.0 | 96.1 | 90.2 | 98.0 | 88.9 | 98.0 | 100.0 | 100.0 |
| 59. TA126400 | 98.0 | 98.0 | 100.0 | 98.0 | 98.0 | 98.0 | 98.0 | 98.0 | 98.0 | 98.0 | 98.0 | 96.1 | 90.2 | 98.0 | 88.9 | 98.0 | 100.0 | 100.0 |
| 60. Os07g08440 | 98.0 | 98.0 | 100.0 | 98.0 | 98.0 | 98.0 | 98.0 | 98.0 | 98.0 | 98.0 | 98.0 | 96.1 | 90.2 | 98.0 | 88.9 | 98.0 | 100.0 | 100.0 |
| 61. Os02g35660 | 96.1 | 96.1 | 98.0 | 98.0 | 98.0 | 98.0 | 98.0 | 98.0 | 98.0 | 98.0 | 98.0 | 96.1 | 90.2 | 98.0 | 88.9 | 98.0 | 98.0 | 98.0 |
| 62. DT543504 | 94.1 | 96.1 | 98.0 | 96.1 | 96.1 | 96.1 | 96.1 | 96.1 | 96.1 | 98.0 | 98.0 | 94.1 | 90.2 | 96.1 | 87.0 | 96.1 | 96.1 | 96.1 |
| 63. Pt_scaff28.86 | 94.1 | 96.1 | 98.0 | 96.1 | 96.1 | 96.1 | 96.1 | 96.1 | 96.1 | 98.0 | 98.0 | 94.1 | 90.2 | 96.1 | 88.9 | 96.1 | 96.1 | 96.1 |
| 64. GSVIVT00016367001 | 96.1 | 98.0 | 100.0 | 98.0 | 98.0 | 98.0 | 98.0 | 98.0 | 98.0 | 100.0 | 100.0 | 96.1 | 92.2 | 98.0 | 88.9 | 98.0 | 98.0 | 98.0 |
| 65. TC19278 | 68.6 | 70.6 | 72.5 | 70.6 | 70.6 | 70.6 | 70.6 | 70.6 | 70.6 | 72.5 | 72.5 | 70.6 | 72.5 | 70.6 | 63.0 | 70.6 | 70.6 | 70.6 |
| 66. TA14134 | 94.1 | 94.1 | 94.1 | 96.1 | 96.1 | 96.1 | 96.1 | 96.1 | 96.1 | 96.1 | 96.1 | 94.1 | 88.2 | 96.1 | 87.0 | 96.1 | 96.1 | 96.1 |
| 67. TC68930 | 98.0 | 98.0 | 100.0 | 100.0 | 100.0 | 100.0 | 100.0 | 100.0 | 100.0 | 100.0 | 100.0 | 98.0 | 92.2 | 100.0 | 90.7 | 100.0 | 100.0 | 100.0 |
| 68. TC140470 | 94.1 | 96.1 | 98.0 | 96.1 | 96.1 | 96.1 | 96.1 | 96.1 | 96.1 | 98.0 | 98.0 | 94.1 | 90.2 | 96.1 | 87.0 | 96.1 | 96.1 | 96.1 |
| 69. TC253044 | 94.1 | 96.1 | 98.0 | 96.1 | 96.1 | 96.1 | 96.1 | 96.1 | 96.1 | 98.0 | 98.0 | 94.1 | 90.2 | 96.1 | 87.0 | 96.1 | 96.1 | 96.1 |
| 70. Os06g09370 | 92.2 | 94.1 | 96.1 | 94.1 | 94.1 | 94.1 | 94.1 | 94.1 | 94.1 | 96.1 | 96.1 | 92.2 | 88.2 | 94.1 | 85.2 | 94.1 | 94.1 | 94.1 |

| | 19 | 20 | 21 | 22 | 23 | 24 | 25 | 26 | 27 | 28 | 29 | 30 | 31 | 32 | 33 | 34 | 35 | 36 |
|---|---|---|---|---|---|---|---|---|---|---|---|---|---|---|---|---|---|---|
| 1. AT2G24260 | 82.4 | 82.4 | 80.4 | 84.3 | 84.3 | 82.4 | 84.3 | 84.3 | 80.4 | 82.4 | 84.3 | 82.4 | 84.3 | 84.3 | 84.3 | 84.3 | 82.4 | 84.3 |
| 2. AT4G30980 | 80.4 | 80.4 | 82.4 | 82.4 | 82.4 | 80.4 | 82.4 | 82.4 | 80.4 | 80.4 | 82.4 | 80.4 | 82.4 | 82.4 | 82.4 | 82.4 | 80.4 | 82.4 |
| 3. AT5G58010 | 76.5 | 76.5 | 78.4 | 78.4 | 78.4 | 76.5 | 78.4 | 78.4 | 76.5 | 76.5 | 78.4 | 76.5 | 78.4 | 78.4 | 78.4 | 78.4 | 76.5 | 78.4 |
| 4. TA12416 | 82.4 | 82.4 | 80.4 | 84.3 | 84.3 | 82.4 | 84.3 | 84.3 | 80.4 | 82.4 | 84.3 | 82.4 | 84.3 | 84.3 | 84.3 | 84.3 | 82.4 | 84.3 |
| 5. TA5414 | 82.4 | 82.4 | 80.4 | 84.3 | 84.3 | 82.4 | 84.3 | 84.3 | 80.4 | 82.4 | 84.3 | 82.4 | 84.3 | 84.3 | 84.3 | 84.3 | 82.4 | 84.3 |
| 6. TC205173 | 82.4 | 82.4 | 80.4 | 84.3 | 84.3 | 82.4 | 84.3 | 84.3 | 80.4 | 82.4 | 84.3 | 82.4 | 84.3 | 84.3 | 84.3 | 84.3 | 82.4 | 84.3 |
| 7. GSVIVT00017237001 | 82.4 | 82.4 | 80.4 | 84.3 | 84.3 | 82.4 | 84.3 | 84.3 | 80.4 | 82.4 | 84.3 | 82.4 | 84.3 | 84.3 | 84.3 | 84.3 | 82.4 | 84.3 |
| 8. TC7102 | 82.4 | 82.4 | 80.4 | 84.3 | 84.3 | 82.4 | 84.3 | 84.3 | 80.4 | 82.4 | 84.3 | 82.4 | 84.3 | 84.3 | 84.3 | 84.3 | 82.4 | 84.3 |
| 9. TA34455 | 82.4 | 82.4 | 80.4 | 84.3 | 84.3 | 82.4 | 84.3 | 84.3 | 80.4 | 82.4 | 84.3 | 82.4 | 84.3 | 84.3 | 84.3 | 84.3 | 82.4 | 84.3 |
| 10. TC7103 | 78.4 | 78.4 | 80.4 | 80.4 | 80.4 | 78.4 | 80.4 | 80.4 | 78.4 | 78.4 | 80.4 | 78.4 | 80.4 | 80.4 | 80.4 | 80.4 | 78.4 | 80.4 |
| 11. TA37666 | 78.4 | 78.4 | 80.4 | 80.4 | 80.4 | 78.4 | 80.4 | 80.4 | 78.4 | 78.4 | 80.4 | 78.4 | 80.4 | 80.4 | 80.4 | 80.4 | 78.4 | 80.4 |

| | | | | | | | | | | | | | | | | | | |
|---|---|---|---|---|---|---|---|---|---|---|---|---|---|---|---|---|---|---|
| 12. TC67603 | 80.4 | 80.4 | 78.4 | 82.4 | 82.4 | 80.4 | 82.4 | 82.4 | 78.4 | 80.4 | 82.4 | 80.4 | 82.4 | 82.4 | 82.4 | 82.4 | 80.4 | 82.4 |
| 13. Os06g08500 | 70.6 | 70.6 | 72.5 | 72.5 | 72.5 | 70.6 | 72.5 | 72.5 | 70.6 | 70.6 | 72.5 | 70.6 | 72.5 | 72.5 | 72.5 | 72.5 | 70.6 | 72.5 |
| 14. Os02g55250 | 82.4 | 82.4 | 80.4 | 84.3 | 84.3 | 82.4 | 84.3 | 84.3 | 80.4 | 82.4 | 84.3 | 82.4 | 84.3 | 84.3 | 84.3 | 84.3 | 82.4 | 84.3 |
| 15. Os09g25040 | 74.1 | 74.1 | 72.2 | 75.9 | 75.9 | 74.1 | 75.9 | 75.9 | 72.2 | 74.1 | 75.9 | 75.9 | 75.9 | 75.9 | 75.9 | 75.9 | 74.1 | 75.9 |
| 16. TA2544 | 80.4 | 80.4 | 78.4 | 82.4 | 82.4 | 80.4 | 82.4 | 82.4 | 78.4 | 80.4 | 82.4 | 80.4 | 82.4 | 82.4 | 82.4 | 82.4 | 82.4 | 82.4 |
| 17. DV982110 | 82.4 | 82.4 | 80.4 | 84.3 | 84.3 | 82.4 | 84.3 | 84.3 | 80.4 | 82.4 | 84.3 | 82.4 | 84.3 | 84.3 | 84.3 | 84.3 | 82.4 | 84.3 |
| 18. WO051050seqID516 | 82.4 | 82.4 | 80.4 | 84.3 | 84.3 | 82.4 | 84.3 | 84.3 | 80.4 | 82.4 | 84.3 | 82.4 | 84.3 | 84.3 | 84.3 | 84.3 | 82.4 | 84.3 |
| 19. AT4G02590 | | 100.0 | 98.0 | 96.1 | 96.1 | 92.2 | 94.1 | 96.1 | 92.2 | 92.2 | 94.1 | 94.1 | 96.1 | 96.1 | 96.1 | 96.1 | 92.2 | 94.1 |
| 20. TC10015_part | 100.0 | | 98.0 | 96.1 | 96.1 | 92.2 | 94.1 | 96.1 | 92.2 | 92.2 | 94.1 | 94.1 | 96.1 | 96.1 | 96.1 | 96.1 | 92.2 | 94.1 |
| 21. AT1G03040 | 100.0 | 100.0 | | 94.1 | 94.1 | 90.2 | 92.2 | 94.1 | 92.2 | 90.2 | 92.2 | 92.2 | 94.1 | 94.1 | 94.1 | 94.1 | 90.2 | 92.2 |
| 22. TA13791 | 100.0 | 100.0 | 100.0 | | 100.0 | 96.1 | 98.0 | 100.0 | 96.1 | 96.1 | 98.0 | 98.0 | 100.0 | 100.0 | 100.0 | 100.0 | 96.1 | 98.0 |
| 23. TC60118 | 100.0 | 100.0 | 100.0 | 100.0 | | 96.1 | 98.0 | 100.0 | 96.1 | 96.1 | 98.0 | 98.0 | 100.0 | 100.0 | 100.0 | 100.0 | 96.1 | 98.0 |
| 24. TC60119 | 96.1 | 96.1 | 96.1 | 96.1 | 96.1 | | 98.0 | 96.1 | 92.2 | 94.1 | 96.1 | 94.1 | 96.1 | 96.1 | 96.1 | 96.1 | 96.1 | 98.0 |
| 25. TC61833 | 98.0 | 98.0 | 98.0 | 98.0 | 98.0 | 98.0 | | 98.0 | 94.1 | 96.1 | 98.0 | 96.1 | 98.0 | 98.0 | 98.0 | 98.0 | 98.0 | 100.0 |
| 26. TA3392 | 100.0 | 100.0 | 100.0 | 100.0 | 100.0 | 96.1 | 98.0 | | 96.1 | 96.1 | 98.0 | 98.0 | 100.0 | 100.0 | 100.0 | 100.0 | 96.1 | 98.0 |
| 27. WO051050seqID1671 | 98.0 | 98.0 | 98.0 | 98.0 | 98.0 | 94.1 | 96.1 | 98.0 | | 92.2 | 94.1 | 94.1 | 96.1 | 96.1 | 96.1 | 96.1 | 92.2 | 94.1 |
| 28. Pt_TC63334 | 96.1 | 96.1 | 96.1 | 96.1 | 96.1 | 94.1 | 96.1 | 96.1 | 94.1 | | 98.0 | 94.1 | 96.1 | 96.1 | 96.1 | 96.1 | 94.1 | 96.1 |
| 29. TA3263 | 98.0 | 98.0 | 98.0 | 98.0 | 98.0 | 96.1 | 98.0 | 98.0 | 96.1 | 98.0 | | 96.1 | 98.0 | 98.0 | 98.0 | 98.0 | 96.1 | 98.0 |
| 30. Pt_scaff_II.416 | 100.0 | 100.0 | 100.0 | 100.0 | 100.0 | 96.1 | 98.0 | 100.0 | 98.0 | 96.1 | 98.0 | | 98.0 | 98.0 | 98.0 | 98.0 | 94.1 | 96.1 |
| 31. TA2825 | 100.0 | 100.0 | 100.0 | 100.0 | 100.0 | 96.1 | 98.0 | 100.0 | 98.0 | 96.1 | 98.0 | 100.0 | | 100.0 | 100.0 | 100.0 | 96.1 | 98.0 |
| 32. TC207545 | 100.0 | 100.0 | 100.0 | 100.0 | 100.0 | 96.1 | 98.0 | 100.0 | 98.0 | 96.1 | 98.0 | 100.0 | 100.0 | | 100.0 | 100.0 | 96.1 | 98.0 |
| 33. TC229602 | 100.0 | 100.0 | 100.0 | 100.0 | 100.0 | 96.1 | 98.0 | 100.0 | 98.0 | 96.1 | 98.0 | 100.0 | 100.0 | 100.0 | | 100.0 | 96.1 | 98.0 |
| 34. TA46156 | 100.0 | 100.0 | 100.0 | 100.0 | 100.0 | 96.1 | 98.0 | 100.0 | 98.0 | 96.1 | 98.0 | 100.0 | 100.0 | 100.0 | 100.0 | | 96.1 | 98.0 |
| 35. AK247217 | 98.0 | 98.0 | 98.0 | 98.0 | 98.0 | 98.0 | 100.0 | 98.0 | 96.1 | 96.1 | 98.0 | 98.0 | 98.0 | 98.0 | 98.0 | 98.0 | | 98.0 |
| 36. TA30646 | 98.0 | 98.0 | 98.0 | 98.0 | 98.0 | 98.0 | 100.0 | 98.0 | 96.1 | 96.1 | 98.0 | 98.0 | 98.0 | 98.0 | 98.0 | 98.0 | 100.0 | |
| 37. TC8633 | 96.1 | 96.1 | 96.1 | 98.0 | 98.0 | 98.0 | 100.0 | 98.0 | 96.1 | 96.1 | 98.0 | 98.0 | 98.0 | 98.0 | 98.0 | 98.0 | 100.0 | 100.0 |
| 38. TC15501 | 98.0 | 98.0 | 98.0 | 98.0 | 98.0 | 94.1 | 96.1 | 98.0 | 96.1 | 94.1 | 96.1 | 98.0 | 98.0 | 98.0 | 98.0 | 98.0 | 96.1 | 96.1 |
| 39. TA1140 | 98.0 | 98.0 | 98.0 | 98.0 | 98.0 | 94.1 | 96.1 | 98.0 | 96.1 | 94.1 | 96.1 | 98.0 | 98.0 | 98.0 | 98.0 | 98.0 | 96.1 | 96.1 |
| 40. TA3490_part | 98.0 | 98.0 | 98.0 | 98.0 | 98.0 | 94.1 | 96.1 | 98.0 | 96.1 | 94.1 | 96.1 | 98.0 | 98.0 | 98.0 | 98.0 | 98.0 | 96.1 | 96.1 |
| 41. CK293938 | 98.0 | 98.0 | 98.0 | 98.0 | 98.0 | 96.1 | 96.1 | 98.0 | 96.1 | 94.1 | 96.1 | 98.0 | 98.0 | 98.0 | 98.0 | 98.0 | 96.1 | 96.1 |
| 42. TC12771 | 98.0 | 98.0 | 98.0 | 98.0 | 98.0 | 96.1 | 96.1 | 98.0 | 96.1 | 94.1 | 96.1 | 98.0 | 98.0 | 98.0 | 98.0 | 98.0 | 96.1 | 96.1 |
| 43. TC172581 | 98.0 | 98.0 | 98.0 | 98.0 | 98.0 | 96.1 | 96.1 | 98.0 | 96.1 | 94.1 | 96.1 | 98.0 | 98.0 | 98.0 | 98.0 | 98.0 | 96.1 | 96.1 |
| 44. TA28621 | 98.0 | 98.0 | 98.0 | 98.0 | 98.0 | 96.1 | 96.1 | 98.0 | 96.1 | 94.1 | 96.1 | 98.0 | 98.0 | 98.0 | 98.0 | 98.0 | 96.1 | 96.1 |
| 45. TA21665 | 98.0 | 98.0 | 98.0 | 98.0 | 98.0 | 96.1 | 96.1 | 98.0 | 96.1 | 94.1 | 96.1 | 98.0 | 98.0 | 98.0 | 98.0 | 98.0 | 96.1 | 96.1 |

EP 2 230 310 B1

| | 55 | 50 | 45 | 40 | 35 | 30 | 25 | 20 | 15 | 10 | 5 | | | | | | | |
|---|---|---|---|---|---|---|---|---|---|---|---|---|---|---|---|---|---|---|
| 46. TA46194 | 98.0 | 98.0 | 98.0 | 98.0 | 98.0 | 94.1 | 96.1 | 98.0 | 96.1 | 94.1 | 96.1 | 98.0 | 98.0 | 98.0 | 98.0 | 98.0 | 96.1 | 96.1 |
| 47. DY268946 | 98.0 | 98.0 | 98.0 | 98.0 | 98.0 | 94.1 | 96.1 | 98.0 | 96.1 | 94.1 | 96.1 | 98.0 | 98.0 | 98.0 | 98.0 | 98.0 | 96.1 | 96.1 |
| 48. CO123623 | 98.0 | 98.0 | 98.0 | 98.0 | 98.0 | 94.1 | 96.1 | 98.0 | 96.1 | 94.1 | 96.1 | 98.0 | 98.0 | 98.0 | 98.0 | 98.0 | 96.1 | 96.1 |
| 49. Pt_scaff_70.65 | 94.1 | 94.1 | 94.1 | 94.1 | 94.1 | 94.1 | 92.2 | 94.1 | 92.2 | 90.2 | 92.2 | 94.1 | 94.1 | 94.1 | 94.1 | 94.1 | 92.2 | 92.2 |
| 50. Pt_scaff_XIII.403 | 94.1 | 94.1 | 94.1 | 94.1 | 94.1 | 94.1 | 92.2 | 94.1 | 92.2 | 90.2 | 92.2 | 94.1 | 94.1 | 94.1 | 94.1 | 94.1 | 92.2 | 92.2 |
| 51. TA1616 | 98.0 | 98.0 | 98.0 | 98.0 | 98.0 | 94.1 | 96.1 | 98.0 | 96.1 | 94.1 | 96.1 | 98.0 | 98.0 | 98.0 | 98.0 | 98.0 | 96.1 | 96.1 |
| 52. TA55042 | 100.0 | 100.0 | 100.0 | 100.0 | 100.0 | 96.1 | 98.0 | 100.0 | 98.0 | 96.1 | 98.0 | 100.0 | 100.0 | 100.0 | 100.0 | 100.0 | 98.0 | 98.0 |
| 53. WO051050seqID77 | 96.1 | 96.1 | 96.1 | 96.1 | 96.1 | 94.1 | 94.1 | 96.1 | 94.1 | 92.2 | 94.1 | 96.1 | 96.1 | 96.1 | 96.1 | 96.1 | 94.1 | 94.1 |
| 54. TC3698 | 96.1 | 96.1 | 96.1 | 96.1 | 96.1 | 96.1 | 98.0 | 96.1 | 94.1 | 94.1 | 96.1 | 96.1 | 96.1 | 96.1 | 96.1 | 96.1 | 98.0 | 98.0 |
| 55. Os03g58330 | 96.1 | 96.1 | 96.1 | 98.0 | 98.0 | 94.1 | 96.1 | 98.0 | 96.1 | 94.1 | 96.1 | 98.0 | 98.0 | 98.0 | 98.0 | 98.0 | 96.1 | 96.1 |
| 56. TA139285 | 96.1 | 96.1 | 96.1 | 98.0 | 98.0 | 94.1 | 96.1 | 98.0 | 96.1 | 94.1 | 96.1 | 98.0 | 98.0 | 98.0 | 98.0 | 98.0 | 96.1 | 96.1 |
| 57. TabHLH11 | 94.1 | 94.1 | 94.1 | 96.1 | 96.1 | 96.1 | 98.0 | 96.1 | 94.1 | 94.1 | 96.1 | 96.1 | 96.1 | 96.1 | 96.1 | 96.1 | 98.0 | 98.0 |
| 58. TC104646 | 96.1 | 96.1 | 96.1 | 98.0 | 98.0 | 94.1 | 96.1 | 98.0 | 96.1 | 94.1 | 96.1 | 98.0 | 98.0 | 98.0 | 98.0 | 98.0 | 96.1 | 96.1 |
| 59. TA126400 | 96.1 | 96.1 | 96.1 | 98.0 | 98.0 | 94.1 | 96.1 | 98.0 | 96.1 | 94.1 | 96.1 | 98.0 | 98.0 | 98.0 | 98.0 | 98.0 | 96.1 | 96.1 |
| 60. Os07g08440 | 96.1 | 96.1 | 96.1 | 98.0 | 98.0 | 94.1 | 96.1 | 98.0 | 96.1 | 94.1 | 96.1 | 98.0 | 98.0 | 98.0 | 98.0 | 98.0 | 96.1 | 96.1 |
| 61. Os02g35660 | 94.1 | 94.1 | 94.1 | 96.1 | 96.1 | 94.1 | 94.1 | 96.1 | 96.1 | 94.1 | 96.1 | 96.1 | 96.1 | 96.1 | 96.1 | 96.1 | 94.1 | 94.1 |
| 62. DT543504 | 92.2 | 92.2 | 94.1 | 94.1 | 94.1 | 90.2 | 92.2 | 94.1 | 94.1 | 92.2 | 94.1 | 94.1 | 94.1 | 94.1 | 94.1 | 94.1 | 92.2 | 92.2 |
| 63. Pt_scaff28.86 | 92.2 | 92.2 | 94.1 | 94.1 | 94.1 | 90.2 | 92.2 | 94.1 | 94.1 | 92.2 | 94.1 | 94.1 | 94.1 | 94.1 | 94.1 | 94.1 | 92.2 | 92.2 |
| 64. GSVIVT00016367001 | 94.1 | 94.1 | 96.1 | 96.1 | 96.1 | 92.2 | 94.1 | 96.1 | 96.1 | 94.1 | 96.1 | 96.1 | 96.1 | 96.1 | 96.1 | 96.1 | 94.1 | 94.1 |
| 65. TC19278 | 66.7 | 66.7 | 68.6 | 68.6 | 68.6 | 66.7 | 66.7 | 68.6 | 68.6 | 66.7 | 68.6 | 68.6 | 68.6 | 68.6 | 68.6 | 68.6 | 66.7 | 66.7 |
| 66. TA14134 | 92.2 | 92.2 | 92.2 | 94.1 | 94.1 | 90.2 | 92.2 | 94.1 | 92.2 | 92.2 | 94.1 | 94.1 | 94.1 | 94.1 | 94.1 | 94.1 | 92.2 | 92.2 |
| 67. TC68930 | 96.1 | 96.1 | 96.1 | 98.0 | 98.0 | 94.1 | 96.1 | 98.0 | 96.1 | 96.1 | 98.0 | 98.0 | 98.0 | 98.0 | 98.0 | 98.0 | 96.1 | 96.1 |
| 68. TC140470 | 92.2 | 92.2 | 94.1 | 94.1 | 94.1 | 92.2 | 92.2 | 94.1 | 94.1 | 92.2 | 94.1 | 94.1 | 94.1 | 94.1 | 94.1 | 94.1 | 92.2 | 92.2 |
| 69. TC253044 | 92.2 | 92.2 | 94.1 | 94.1 | 94.1 | 92.2 | 92.2 | 94.1 | 94.1 | 92.2 | 94.1 | 94.1 | 94.1 | 94.1 | 94.1 | 94.1 | 92.2 | 92.2 |
| 70. Os06g09370 | 90.2 | 90.2 | 92.2 | 92.2 | 92.2 | 90.2 | 90.2 | 92.2 | 92.2 | 90.2 | 92.2 | 92.2 | 92.2 | 92.2 | 92.2 | 92.2 | 90.2 | 90.2 |

| | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|
| 1. AT2G24260 | 86.3 | 88.2 | 88.2 | 88.2 | 84.3 | 84.3 | 84.3 | 84.3 | 84.3 | 88.2 | 84.3 | 88.2 | 82.4 | 80.4 | 86.3 | 88.2 | 86.3 | 82.4 |
| 2. AT4G30980 | 84.3 | 86.3 | 86.3 | 86.3 | 82.4 | 82.4 | 82.4 | 82.4 | 82.4 | 86.3 | 82.4 | 86.3 | 80.4 | 78.4 | 84.3 | 86.3 | 84.3 | 80.4 |
| 3. AT5G58010 | 80.4 | 82.4 | 82.4 | 82.4 | 78.4 | 78.4 | 78.4 | 78.4 | 78.4 | 82.4 | 78.4 | 82.4 | 76.5 | 74.5 | 80.4 | 82.4 | 80.4 | 82.4 |
| 4. TA12416 | 86.3 | 88.2 | 88.2 | 88.2 | 84.3 | 84.3 | 84.3 | 84.3 | 84.3 | 88.2 | 84.3 | 88.2 | 82.4 | 80.4 | 88.2 | 88.2 | 86.3 | 82.4 |
| 5. TA5414 | 86.3 | 88.2 | 88.2 | 88.2 | 84.3 | 84.3 | 84.3 | 84.3 | 84.3 | 88.2 | 84.3 | 88.2 | 82.4 | 80.4 | 88.2 | 88.2 | 86.3 | 82.4 |
| 6. TC205173 | 86.3 | 88.2 | 88.2 | 88.2 | 84.3 | 84.3 | 84.3 | 84.3 | 84.3 | 88.2 | 84.3 | 88.2 | 82.4 | 80.4 | 88.2 | 88.2 | 86.3 | 82.4 |
| 7. GSVIVT00017237001 | 86.3 | 88.2 | 88.2 | 88.2 | 84.3 | 84.3 | 84.3 | 84.3 | 84.3 | 88.2 | 84.3 | 88.2 | 82.4 | 80.4 | 88.2 | 88.2 | 86.3 | 82.4 |

47

| Sequence | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|
| 8. TC7102 | 86.3 | 88.2 | 88.2 | 88.2 | 84.3 | 84.3 | 84.3 | 84.3 | 84.3 | 88.2 | 84.3 | 88.2 | 82.4 | 80.4 | 88.2 | 88.2 | 86.3 | 82.4 |
| 9. TA34455 | 86.3 | 88.2 | 88.2 | 88.2 | 84.3 | 84.3 | 84.3 | 84.3 | 84.3 | 88.2 | 84.3 | 88.2 | 82.4 | 80.4 | 88.2 | 88.2 | 86.3 | 82.4 |
| 10. TC7103 | 82.4 | 84.3 | 84.3 | 84.3 | 80.4 | 80.4 | 80.4 | 80.4 | 80.4 | 84.3 | 80.4 | 84.3 | 78.4 | 76.5 | 84.3 | 84.3 | 82.4 | 78.4 |
| 11. TA37666 | 82.4 | 84.3 | 84.3 | 84.3 | 80.4 | 80.4 | 80.4 | 80.4 | 80.4 | 84.3 | 80.4 | 84.3 | 78.4 | 76.5 | 84.3 | 84.3 | 82.4 | 78.4 |
| 12. TC67603 | 84.3 | 86.3 | 86.3 | 86.3 | 82.4 | 82.4 | 82.4 | 82.4 | 82.4 | 86.3 | 82.4 | 86.3 | 84.3 | 82.4 | 86.3 | 86.3 | 84.3 | 80.4 |
| 13. Os06g08500 | 74.5 | 76.5 | 76.5 | 76.5 | 72.5 | 72.5 | 72.5 | 72.5 | 72.5 | 76.5 | 72.5 | 76.5 | 70.6 | 68.6 | 76.5 | 76.5 | 74.5 | 70.6 |
| 14. Os02g55250 | 86.3 | 88.2 | 88.2 | 88.2 | 84.3 | 84.3 | 84.3 | 84.3 | 84.3 | 88.2 | 84.3 | 88.2 | 82.4 | 80.4 | 88.2 | 88.2 | 86.3 | 82.4 |
| 15. Os09g25040 | 77.8 | 79.6 | 79.6 | 79.6 | 75.9 | 75.9 | 75.9 | 75.9 | 75.9 | 79.6 | 75.9 | 79.6 | 74.1 | 72.2 | 79.6 | 79.6 | 77.8 | 74.1 |
| 16. TA2544 | 84.3 | 86.3 | 86.3 | 86.3 | 82.4 | 82.4 | 82.4 | 82.4 | 82.4 | 86.3 | 82.4 | 86.3 | 80.4 | 78.4 | 86.3 | 86.3 | 84.3 | 80.4 |
| 17. DV982110 | 86.3 | 90.2 | 88.2 | 88.2 | 84.3 | 84.3 | 84.3 | 84.3 | 84.3 | 90.2 | 84.3 | 88.2 | 82.4 | 80.4 | 86.3 | 88.2 | 86.3 | 82.4 |
| 18. WO051050seqID516 | 86.3 | 90.2 | 88.2 | 88.2 | 84.3 | 84.3 | 84.3 | 84.3 | 84.3 | 90.2 | 84.3 | 88.2 | 82.4 | 80.4 | 86.3 | 88.2 | 86.3 | 82.4 |
| 19. AT4G02590 | 94.1 | 90.2 | 88.2 | 88.2 | 88.2 | 88.2 | 88.2 | 88.2 | 88.2 | 90.2 | 90.2 | 90.2 | 88.2 | 86.3 | 94.1 | 90.2 | 88.2 | 90.2 |
| 20. TC10015_part | 94.1 | 90.2 | 88.2 | 88.2 | 88.2 | 88.2 | 88.2 | 88.2 | 88.2 | 90.2 | 90.2 | 90.2 | 88.2 | 86.3 | 94.1 | 90.2 | 88.2 | 90.2 |
| 21. AT1G03040 | 92.2 | 88.2 | 86.3 | 86.3 | 86.3 | 86.3 | 86.3 | 86.3 | 86.3 | 88.2 | 88.2 | 88.2 | 86.3 | 84.3 | 92.2 | 88.2 | 86.3 | 88.2 |
| 22. TA13791 | 96.1 | 94.1 | 92.2 | 92.2 | 92.2 | 92.2 | 92.2 | 92.2 | 92.2 | 94.1 | 94.1 | 94.1 | 88.2 | 86.3 | 90.2 | 94.1 | 92.2 | 94.1 |
| 23. TC60118 | 96.1 | 94.1 | 92.2 | 92.2 | 92.2 | 92.2 | 92.2 | 92.2 | 92.2 | 94.1 | 94.1 | 94.1 | 88.2 | 86.3 | 90.2 | 94.1 | 92.2 | 94.1 |
| 24. TC60119 | 94.1 | 90.2 | 88.2 | 88.2 | 90.2 | 90.2 | 90.2 | 90.2 | 90.2 | 90.2 | 90.2 | 90.2 | 88.2 | 86.3 | 86.3 | 90.2 | 90.2 | 94.1 |
| 25. TC61833 | 96.1 | 92.2 | 90.2 | 90.2 | 90.2 | 90.2 | 90.2 | 90.2 | 90.2 | 92.2 | 92.2 | 92.2 | 86.3 | 84.3 | 88.2 | 92.2 | 90.2 | 96.1 |
| 26. TA3392 | 96.1 | 94.1 | 92.2 | 92.2 | 92.2 | 92.2 | 92.2 | 92.2 | 92.2 | 94.1 | 94.1 | 94.1 | 88.2 | 86.3 | 90.2 | 94.1 | 92.2 | 94.1 |
| 27. WO051050seqID1671 | 92.2 | 90.2 | 88.2 | 88.2 | 88.2 | 88.2 | 88.2 | 88.2 | 88.2 | 90.2 | 90.2 | 90.2 | 84.3 | 82.4 | 86.3 | 90.2 | 88.2 | 90.2 |
| 28. Pt_TC63334 | 94.1 | 90.2 | 88.2 | 88.2 | 88.2 | 88.2 | 88.2 | 88.2 | 88.2 | 90.2 | 90.2 | 90.2 | 84.3 | 82.4 | 86.3 | 90.2 | 88.2 | 92.2 |
| 29. TA3263 | 96.1 | 92.2 | 90.2 | 90.2 | 90.2 | 90.2 | 90.2 | 90.2 | 90.2 | 92.2 | 92.2 | 92.2 | 86.3 | 84.3 | 88.2 | 92.2 | 90.2 | 94.1 |
| 30. Pt_scaff_II.416 | 94.1 | 92.2 | 90.2 | 90.2 | 90.2 | 90.2 | 90.2 | 90.2 | 90.2 | 92.2 | 92.2 | 92.2 | 86.3 | 84.3 | 88.2 | 92.2 | 90.2 | 92.2 |
| 31. TA2825 | 96.1 | 94.1 | 92.2 | 92.2 | 92.2 | 92.2 | 92.2 | 92.2 | 92.2 | 94.1 | 94.1 | 94.1 | 88.2 | 86.3 | 90.2 | 94.1 | 92.2 | 94.1 |
| 32. TC207545 | 96.1 | 94.1 | 92.2 | 92.2 | 92.2 | 92.2 | 92.2 | 92.2 | 92.2 | 94.1 | 94.1 | 94.1 | 88.2 | 86.3 | 90.2 | 94.1 | 92.2 | 94.1 |
| 33. TC229602 | 96.1 | 94.1 | 92.2 | 92.2 | 92.2 | 92.2 | 92.2 | 92.2 | 92.2 | 94.1 | 94.1 | 94.1 | 88.2 | 86.3 | 90.2 | 94.1 | 92.2 | 94.1 |
| 34. TA46156 | 96.1 | 94.1 | 92.2 | 92.2 | 92.2 | 92.2 | 92.2 | 92.2 | 92.2 | 94.1 | 94.1 | 94.1 | 88.2 | 86.3 | 90.2 | 94.1 | 92.2 | 94.1 |
| 35. AK247217 | 94.1 | 90.2 | 88.2 | 88.2 | 88.2 | 88.2 | 88.2 | 88.2 | 88.2 | 90.2 | 90.2 | 90.2 | 84.3 | 82.4 | 86.3 | 90.2 | 88.2 | 94.1 |
| 36. TA30646 | 96.1 | 92.2 | 90.2 | 90.2 | 90.2 | 90.2 | 90.2 | 90.2 | 90.2 | 92.2 | 92.2 | 92.2 | 86.3 | 84.3 | 88.2 | 92.2 | 90.2 | 96.1 |
| 37. TC8633 |  | 90.2 | 88.2 | 88.2 | 88.2 | 88.2 | 88.2 | 88.2 | 88.2 | 90.2 | 90.2 | 90.2 | 86.3 | 84.3 | 88.2 | 90.2 | 88.2 | 92.2 |
| 38. TC15501 | 96.1 |  | 92.2 | 92.2 | 92.2 | 92.2 | 92.2 | 92.2 | 92.2 | 100.0 | 94.1 | 98.0 | 90.2 | 88.2 | 94.1 | 98.0 | 96.1 | 90.2 |
| 39. TA1140 | 96.1 | 98.0 |  | 100.0 | 92.2 | 92.2 | 92.2 | 92.2 | 92.2 | 92.2 | 94.1 | 94.1 | 86.3 | 84.3 | 88.2 | 92.2 | 92.2 | 88.2 |
| 40. TA3490_part | 96.1 | 98.0 | 100.0 |  | 92.2 | 92.2 | 92.2 | 92.2 | 92.2 | 92.2 | 94.1 | 94.1 | 86.3 | 84.3 | 88.2 | 92.2 | 92.2 | 88.2 |
| 41. CK293938 | 96.1 | 98.0 | 100.0 | 100.0 |  | 100.0 | 100.0 | 100.0 | 100.0 | 92.2 | 90.2 | 94.1 | 90.2 | 88.2 | 88.2 | 92.2 | 94.1 | 88.2 |

| | | | | | | | | | | | | | | | | | | |
|---|---|---|---|---|---|---|---|---|---|---|---|---|---|---|---|---|---|---|
| 42. TC12771 | 96.1 | 98.0 | 100.0 | 100.0 | 100.0 | | 100.0 | 100.0 | 100.0 | 92.2 | 90.2 | 94.1 | 90.2 | 88.2 | 88.2 | 92.2 | 94.1 | 88.2 |
| 43. TC172581 | 96.1 | 98.0 | 100.0 | 100.0 | 100.0 | 100.0 | | 100.0 | 100.0 | 92.2 | 90.2 | 94.1 | 90.2 | 88.2 | 88.2 | 92.2 | 94.1 | 88.2 |
| 44. TA28621 | 96.1 | 98.0 | 100.0 | 100.0 | 100.0 | 100.0 | 100.0 | | 100.0 | 92.2 | 90.2 | 94.1 | 90.2 | 88.2 | 88.2 | 92.2 | 94.1 | 88.2 |
| 45. TA21665 | 96.1 | 98.0 | 100.0 | 100.0 | 100.0 | 100.0 | 100.0 | 100.0 | | 92.2 | 90.2 | 94.1 | 90.2 | 88.2 | 88.2 | 92.2 | 94.1 | 88.2 |
| 46. TA46194 | 96.1 | 100.0 | 98.0 | 98.0 | 98.0 | 98.0 | 98.0 | 98.0 | 98.0 | | 94.1 | 98.0 | 90.2 | 88.2 | 94.1 | 98.0 | 96.1 | 90.2 |
| 47. DY268946 | 96.1 | 98.0 | 100.0 | 100.0 | 100.0 | 100.0 | 100.0 | 100.0 | 100.0 | 98.0 | | 96.1 | 88.2 | 86.3 | 90.2 | 94.1 | 94.1 | 90.2 |
| 48. CO123623 | 96.1 | 98.0 | 100.0 | 100.0 | 100.0 | 100.0 | 100.0 | 100.0 | 100.0 | 98.0 | 100.0 | | 92.2 | 90.2 | 94.1 | 98.0 | 98.0 | 90.2 |
| 49. Pt_scaff_70.65 | 90.2 | 94.1 | 96.1 | 96.1 | 98.0 | 98.0 | 98.0 | 98.0 | 98.0 | 94.1 | 96.1 | 96.1 | | 98.0 | 90.2 | 90.2 | 92.2 | 84.3 |
| 50. Pt_scaff_XIII.403 | 90.2 | 94.1 | 96.1 | 96.1 | 98.0 | 98.0 | 98.0 | 98.0 | 98.0 | 94.1 | 96.1 | 96.1 | 100.0 | | 88.2 | 88.2 | 90.2 | 82.4 |
| 51. TA1616 | 94.1 | 100.0 | 98.0 | 98.0 | 98.0 | 98.0 | 98.0 | 98.0 | 98.0 | 100.0 | 98.0 | 98.0 | 94.1 | 94.1 | | 94.1 | 92.2 | 86.3 |
| 52. TA55042 | 98.0 | 98.0 | 98.0 | 98.0 | 98.0 | 98.0 | 98.0 | 98.0 | 98.0 | 98.0 | 98.0 | 98.0 | 94.1 | 94.1 | 98.0 | | 96.1 | 90.2 |
| 53. WO051050seqID77 | 94.1 | 96.1 | 98.0 | 98.0 | 100.0 | 100.0 | 100.0 | 100.0 | 100.0 | 96.1 | 98.0 | 98.0 | 96.1 | 96.1 | 96.1 | 96.1 | | 88.2 |
| 54. TC3698 | 98.0 | 98.0 | 96.1 | 96.1 | 96.1 | 96.1 | 96.1 | 96.1 | 96.1 | 98.0 | 96.1 | 96.1 | 92.2 | 92.2 | 96.1 | 96.1 | 94.1 | |
| 55. Os03g58330 | 96.1 | 100.0 | 98.0 | 98.0 | 98.0 | 98.0 | 98.0 | 98.0 | 98.0 | 100.0 | 98.0 | 98.0 | 92.2 | 92.2 | 96.1 | 98.0 | 96.1 | 98.0 |
| 56. TA139285 | 96.1 | 100.0 | 98.0 | 98.0 | 98.0 | 98.0 | 98.0 | 98.0 | 98.0 | 100.0 | 98.0 | 98.0 | 92.2 | 92.2 | 96.1 | 98.0 | 96.1 | 98.0 |
| 57. TabHLH11 | 98.0 | 98.0 | 96.1 | 96.1 | 96.1 | 96.1 | 96.1 | 96.1 | 96.1 | 98.0 | 96.1 | 96.1 | 90.2 | 90.2 | 94.1 | 96.1 | 94.1 | 100.0 |
| 58. TC104646 | 96.1 | 100.0 | 98.0 | 98.0 | 98.0 | 98.0 | 98.0 | 98.0 | 98.0 | 100.0 | 98.0 | 98.0 | 92.2 | 92.2 | 96.1 | 98.0 | 96.1 | 98.0 |
| 59. TA126400 | 96.1 | 100.0 | 98.0 | 98.0 | 98.0 | 98.0 | 98.0 | 98.0 | 98.0 | 100.0 | 98.0 | 98.0 | 92.2 | 92.2 | 96.1 | 98.0 | 96.1 | 98.0 |
| 60. Os07g08440 | 96.1 | 100.0 | 98.0 | 98.0 | 98.0 | 98.0 | 98.0 | 98.0 | 98.0 | 100.0 | 98.0 | 98.0 | 92.2 | 92.2 | 96.1 | 98.0 | 96.1 | 98.0 |
| 61. Os02g35660 | 94.1 | 98.0 | 96.1 | 96.1 | 96.1 | 96.1 | 96.1 | 96.1 | 96.1 | 98.0 | 96.1 | 96.1 | 92.2 | 92.2 | 96.1 | 96.1 | 96.1 | 96.1 |
| 62. DT543504 | 92.2 | 96.1 | 94.1 | 94.1 | 94.1 | 94.1 | 94.1 | 94.1 | 94.1 | 96.1 | 94.1 | 94.1 | 88.2 | 88.2 | 94.1 | 94.1 | 92.2 | 94.1 |
| 63. Pt_scaff28.86 | 92.2 | 96.1 | 94.1 | 94.1 | 94.1 | 94.1 | 94.1 | 94.1 | 94.1 | 96.1 | 94.1 | 94.1 | 88.2 | 88.2 | 94.1 | 94.1 | 92.2 | 94.1 |
| 64. GSVIVT00016367001 | 94.1 | 98.0 | 96.1 | 96.1 | 96.1 | 96.1 | 96.1 | 96.1 | 96.1 | 98.0 | 96.1 | 96.1 | 90.2 | 90.2 | 96.1 | 96.1 | 94.1 | 96.1 |
| 65. TC19278 | 66.7 | 70.6 | 68.6 | 68.6 | 68.6 | 68.6 | 68.6 | 68.6 | 68.6 | 70.6 | 68.6 | 68.6 | 66.7 | 66.7 | 68.6 | 68.6 | 68.6 | 68.6 |
| 66. TA14134 | 92.2 | 96.1 | 94.1 | 94.1 | 94.1 | 94.1 | 94.1 | 94.1 | 94.1 | 96.1 | 92.2 | 94.1 | 88.2 | 88.2 | 94.1 | 94.1 | 92.2 | 92.2 |
| 67. TC68930 | 96.1 | 100.0 | 98.0 | 98.0 | 98.0 | 98.0 | 98.0 | 98.0 | 98.0 | 100.0 | 98.0 | 98.0 | 92.2 | 92.2 | 98.0 | 98.0 | 96.1 | 98.0 |
| 68. TC140470 | 92.2 | 96.1 | 94.1 | 94.1 | 94.1 | 94.1 | 94.1 | 94.1 | 94.1 | 96.1 | 94.1 | 94.1 | 90.2 | 90.2 | 94.1 | 94.1 | 94.1 | 94.1 |
| 69. TC253044 | 92.2 | 96.1 | 94.1 | 94.1 | 94.1 | 94.1 | 94.1 | 94.1 | 94.1 | 96.1 | 94.1 | 94.1 | 90.2 | 90.2 | 94.1 | 94.1 | 94.1 | 94.1 |
| 70. Os06g09370 | 90.2 | 94.1 | 92.2 | 92.2 | 92.2 | 92.2 | 92.2 | 92.2 | 92.2 | 94.1 | 92.2 | 92.2 | 88.2 | 88.2 | 92.2 | 92.2 | 92.2 | 92.2 |

| | 55 | 56 | 57 | 58 | 59 | 60 | 61 | 62 | 63 | 64 | 65 | 66 | 67 | 68 | 69 | 70 |
|---|---|---|---|---|---|---|---|---|---|---|---|---|---|---|---|---|
| 1. AT2G24260 | 88.2 | 88.2 | 88.2 | 88.2 | 88.2 | 86.3 | 88.2 | 90.2 | 90.2 | 92.2 | 64.7 | 88.2 | 94.1 | 86.3 | 86.3 | 88.2 |
| 2. AT4G30980 | 86.3 | 86.3 | 86.3 | 86.3 | 86.3 | 84.3 | 88.2 | 90.2 | 90.2 | 92.2 | 64.7 | 90.2 | 96.1 | 86.3 | 86.3 | 88.2 |
| 3. AT5G58010 | 84.3 | 84.3 | 84.3 | 84.3 | 84.3 | 82.4 | 84.3 | 88.2 | 90.2 | 92.2 | 68.6 | 86.3 | 96.1 | 82.4 | 82.4 | 84.3 |

EP 2 230 310 B1

| | | | | | | | | | | | | | | | | |
|---|---|---|---|---|---|---|---|---|---|---|---|---|---|---|---|---|
| 4. TA12416 | 88.2 | 88.2 | 88.2 | 88.2 | 88.2 | 86.3 | 88.2 | 90.2 | 90.2 | 92.2 | 64.7 | 90.2 | 94.1 | 86.3 | 86.3 | 88.2 |
| 5. TA5414 | 88.2 | 88.2 | 88.2 | 88.2 | 88.2 | 86.3 | 88.2 | 90.2 | 90.2 | 92.2 | 64.7 | 90.2 | 94.1 | 86.3 | 86.3 | 88.2 |
| 6. TC205173 | 88.2 | 88.2 | 88.2 | 88.2 | 88.2 | 86.3 | 88.2 | 90.2 | 90.2 | 92.2 | 64.7 | 90.2 | 94.1 | 86.3 | 86.3 | 88.2 |
| 7. GSVIVT00017237001 | 88.2 | 88.2 | 88.2 | 88.2 | 88.2 | 86.3 | 88.2 | 90.2 | 90.2 | 92.2 | 64.7 | 90.2 | 94.1 | 86.3 | 86.3 | 88.2 |
| 8. TC7102 | 88.2 | 88.2 | 88.2 | 88.2 | 88.2 | 86.3 | 88.2 | 90.2 | 90.2 | 92.2 | 64.7 | 90.2 | 94.1 | 86.3 | 86.3 | 88.2 |
| 9. TA34455 | 88.2 | 88.2 | 88.2 | 88.2 | 88.2 | 86.3 | 88.2 | 90.2 | 90.2 | 92.2 | 64.7 | 90.2 | 94.1 | 86.3 | 86.3 | 88.2 |
| 10. TC7103 | 84.3 | 84.3 | 84.3 | 84.3 | 84.3 | 82.4 | 86.3 | 88.2 | 88.2 | 90.2 | 66.7 | 90.2 | 94.1 | 84.3 | 84.3 | 86.3 |
| 11. TA37666 | 84.3 | 84.3 | 84.3 | 84.3 | 84.3 | 82.4 | 86.3 | 88.2 | 88.2 | 90.2 | 66.7 | 90.2 | 94.1 | 84.3 | 84.3 | 86.3 |
| 12. TC67603 | 86.3 | 86.3 | 86.3 | 86.3 | 86.3 | 84.3 | 86.3 | 88.2 | 88.2 | 90.2 | 64.7 | 88.2 | 92.2 | 84.3 | 84.3 | 86.3 |
| 13. Os06g08500 | 76.5 | 76.5 | 76.5 | 76.5 | 76.5 | 74.5 | 78.4 | 80.4 | 80.4 | 82.4 | 64.7 | 82.4 | 86.3 | 76.5 | 76.5 | 78.4 |
| 14. Os02g55250 | 88.2 | 88.2 | 88.2 | 88.2 | 88.2 | 86.3 | 88.2 | 90.2 | 90.2 | 92.2 | 64.7 | 90.2 | 94.1 | 86.3 | 86.3 | 88.2 |
| 15. Os09g25040 | 79.6 | 79.6 | 79.6 | 79.6 | 79.6 | 77.8 | 79.6 | 81.5 | 81.5 | 83.3 | 57.4 | 81.5 | 85.2 | 77.8 | 77.8 | 79.6 |
| 16. TA2544 | 86.3 | 86.3 | 86.3 | 86.3 | 86.3 | 84.3 | 86.3 | 88.2 | 88.2 | 90.2 | 62.7 | 88.2 | 92.2 | 84.3 | 84.3 | 86.3 |
| 17. DV982110 | 88.2 | 88.2 | 88.2 | 88.2 | 88.2 | 86.3 | 90.2 | 92.2 | 92.2 | 94.1 | 66.7 | 88.2 | 96.1 | 88.2 | 88.2 | 90.2 |
| 18. WO051050seqID516 | 88.2 | 88.2 | 88.2 | 88.2 | 88.2 | 86.3 | 90.2 | 92.2 | 92.2 | 94.1 | 66.7 | 88.2 | 96.1 | 88.2 | 88.2 | 90.2 |
| 19. AT4G02590 | 90.2 | 90.2 | 88.2 | 90.2 | 90.2 | 88.2 | 72.5 | 74.5 | 74.5 | 76.5 | 49.0 | 74.5 | 78.4 | 70.6 | 70.6 | 72.5 |
| 20. TC10015_part | 90.2 | 90.2 | 88.2 | 90.2 | 90.2 | 88.2 | 72.5 | 74.5 | 74.5 | 76.5 | 49.0 | 74.5 | 78.4 | 70.6 | 70.6 | 72.5 |
| 21. AT1G03040 | 88.2 | 88.2 | 86.3 | 88.2 | 88.2 | 86.3 | 72.5 | 74.5 | 74.5 | 76.5 | 49.0 | 76.5 | 80.4 | 70.6 | 70.6 | 72.5 |
| 22. TA13791 | 92.2 | 92.2 | 90.2 | 92.2 | 92.2 | 90.2 | 74.5 | 76.5 | 76.5 | 78.4 | 51.0 | 76.5 | 80.4 | 72.5 | 72.5 | 74.5 |
| 23. TC60118 | 92.2 | 92.2 | 90.2 | 92.2 | 92.2 | 90.2 | 74.5 | 76.5 | 76.5 | 78.4 | 51.0 | 76.5 | 80.4 | 72.5 | 72.5 | 74.5 |
| 24. TC60119 | 88.2 | 88.2 | 90.2 | 88.2 | 88.2 | 86.3 | 74.5 | 74.5 | 74.5 | 76.5 | 51.0 | 74.5 | 78.4 | 72.5 | 72.5 | 74.5 |
| 25. TC61833 | 90.2 | 90.2 | 92.2 | 90.2 | 90.2 | 88.2 | 74.5 | 76.5 | 76.5 | 78.4 | 51.0 | 76.5 | 80.4 | 72.5 | 72.5 | 74.5 |
| 26. TA3392 | 92.2 | 92.2 | 90.2 | 92.2 | 92.2 | 90.2 | 74.5 | 76.5 | 76.5 | 78.4 | 51.0 | 76.5 | 80.4 | 72.5 | 72.5 | 74.5 |
| 27. WO051050seqID1671 | 88.2 | 88.2 | 86.3 | 88.2 | 88.2 | 90.2 | 72.5 | 74.5 | 74.5 | 76.5 | 49.0 | 74.5 | 78.4 | 74.5 | 74.5 | 74.5 |
| 28. Pt_TC63334 | 88.2 | 88.2 | 88.2 | 88.2 | 88.2 | 86.3 | 72.5 | 74.5 | 74.5 | 76.5 | 49.0 | 74.5 | 78.4 | 70.6 | 70.6 | 72.5 |
| 29. TA3263 | 90.2 | 90.2 | 90.2 | 90.2 | 90.2 | 88.2 | 74.5 | 76.5 | 76.5 | 78.4 | 51.0 | 76.5 | 80.4 | 72.5 | 72.5 | 74.5 |
| 30. Pt_scaff_II.416 | 90.2 | 90.2 | 88.2 | 90.2 | 90.2 | 88.2 | 72.5 | 74.5 | 74.5 | 76.5 | 49.0 | 74.5 | 78.4 | 70.6 | 70.6 | 72.5 |
| 31. TA2825 | 92.2 | 92.2 | 90.2 | 92.2 | 92.2 | 90.2 | 74.5 | 76.5 | 76.5 | 78.4 | 51.0 | 76.5 | 80.4 | 72.5 | 72.5 | 74.5 |
| 32. TC207545 | 92.2 | 92.2 | 90.2 | 92.2 | 92.2 | 90.2 | 74.5 | 76.5 | 76.5 | 78.4 | 51.0 | 76.5 | 80.4 | 72.5 | 72.5 | 74.5 |
| 33. TC229602 | 92.2 | 92.2 | 90.2 | 92.2 | 92.2 | 90.2 | 74.5 | 76.5 | 76.5 | 78.4 | 51.0 | 76.5 | 80.4 | 72.5 | 72.5 | 74.5 |
| 34. TA46156 | 92.2 | 92.2 | 90.2 | 92.2 | 92.2 | 90.2 | 74.5 | 76.5 | 76.5 | 78.4 | 51.0 | 76.5 | 80.4 | 72.5 | 72.5 | 74.5 |
| 35. AK247217 | 88.2 | 88.2 | 90.2 | 88.2 | 88.2 | 86.3 | 72.5 | 74.5 | 74.5 | 76.5 | 49.0 | 74.5 | 78.4 | 70.6 | 70.6 | 72.5 |
| 36. TA30646 | 90.2 | 90.2 | 92.2 | 90.2 | 90.2 | 88.2 | 74.5 | 76.5 | 76.5 | 78.4 | 51.0 | 76.5 | 80.4 | 72.5 | 72.5 | 74.5 |
| 37. TC8633 | 92.2 | 92.2 | 92.2 | 92.2 | 92.2 | 90.2 | 76.5 | 78.4 | 78.4 | 80.4 | 52.9 | 78.4 | 82.4 | 74.5 | 74.5 | 76.5 |

| Seq | | | | | | | | | | | | | | | | | | | |
|---|---|---|---|---|---|---|---|---|---|---|---|---|---|---|---|---|---|---|---|
| 38. TC15501 | 92.2 | 92.2 | 92.2 | 92.2 | 90.2 | 92.2 | 90.2 | 80.4 | 82.4 | 82.4 | 82.4 | 80.4 | 84.3 | 56.9 | 78.4 | 86.3 | 78.4 | 78.4 | 80.4 |
| 39. TA1140 | 94.1 | 94.1 | 94.1 | 92.2 | 92.2 | 94.1 | 92.2 | 78.4 | 80.4 | 80.4 | 80.4 | 78.4 | 82.4 | 54.9 | 78.4 | 84.3 | 76.5 | 76.5 | 78.4 |
| 40. TA3490_part | 94.1 | 94.1 | 94.1 | 92.2 | 92.2 | 94.1 | 92.2 | 78.4 | 80.4 | 80.4 | 80.4 | 78.4 | 82.4 | 54.9 | 78.4 | 84.3 | 76.5 | 76.5 | 78.4 |
| 41. CK293938 | 90.2 | 90.2 | 90.2 | 88.2 | 88.2 | 90.2 | 88.2 | 78.4 | 76.5 | 76.5 | 76.5 | 78.4 | 78.4 | 52.9 | 76.5 | 80.4 | 76.5 | 76.5 | 78.4 |
| 42. TC12771 | 90.2 | 90.2 | 90.2 | 88.2 | 88.2 | 90.2 | 88.2 | 78.4 | 76.5 | 76.5 | 76.5 | 78.4 | 78.4 | 52.9 | 76.5 | 80.4 | 76.5 | 76.5 | 78.4 |
| 43. TC172581 | 90.2 | 90.2 | 90.2 | 88.2 | 88.2 | 90.2 | 88.2 | 78.4 | 76.5 | 76.5 | 76.5 | 78.4 | 78.4 | 52.9 | 76.5 | 80.4 | 76.5 | 76.5 | 78.4 |
| 44. TA28621 | 90.2 | 90.2 | 90.2 | 88.2 | 88.2 | 90.2 | 88.2 | 78.4 | 76.5 | 76.5 | 76.5 | 78.4 | 78.4 | 52.9 | 76.5 | 80.4 | 76.5 | 76.5 | 78.4 |
| 45. TA21665 | 90.2 | 90.2 | 90.2 | 88.2 | 88.2 | 90.2 | 88.2 | 78.4 | 76.5 | 76.5 | 76.5 | 78.4 | 78.4 | 52.9 | 76.5 | 80.4 | 76.5 | 76.5 | 78.4 |
| 46. TA46194 | 92.2 | 92.2 | 92.2 | 90.2 | 90.2 | 92.2 | 90.2 | 80.4 | 82.4 | 82.4 | 82.4 | 80.4 | 84.3 | 56.9 | 78.4 | 86.3 | 78.4 | 78.4 | 80.4 |
| 47. DY268946 | 92.2 | 92.2 | 92.2 | 90.2 | 90.2 | 92.2 | 90.2 | 78.4 | 76.5 | 76.5 | 76.5 | 78.4 | 76.5 | 52.9 | 76.5 | 80.4 | 72.5 | 72.5 | 74.5 |
| 48. CO123623 | 92.2 | 92.2 | 92.2 | 90.2 | 90.2 | 92.2 | 90.2 | 78.4 | 80.4 | 80.4 | 80.4 | 78.4 | 82.4 | 54.9 | 78.4 | 84.3 | 76.5 | 76.5 | 78.4 |
| 49. Pt_scaff_70.65 | 86.3 | 86.3 | 86.3 | 84.3 | 84.3 | 86.3 | 84.3 | 74.5 | 74.5 | 74.5 | 74.5 | 74.5 | 76.5 | 52.9 | 74.5 | 78.4 | 72.5 | 72.5 | 74.5 |
| 50. Pt_scaff_XIII.403 | 84.3 | 84.3 | 84.3 | 82.4 | 82.4 | 84.3 | 82.4 | 72.5 | 72.5 | 72.5 | 72.5 | 72.5 | 74.5 | 51.0 | 72.5 | 76.5 | 70.6 | 70.6 | 72.5 |
| 51. TA1616 | 90.2 | 90.2 | 90.2 | 88.2 | 88.2 | 90.2 | 88.2 | 76.5 | 78.4 | 78.4 | 78.4 | 76.5 | 80.4 | 52.9 | 78.4 | 82.4 | 74.5 | 74.5 | 76.5 |
| 52. TA55042 | 92.2 | 92.2 | 92.2 | 90.2 | 90.2 | 92.2 | 90.2 | 78.4 | 80.4 | 80.4 | 80.4 | 78.4 | 82.4 | 54.9 | 78.4 | 84.3 | 76.5 | 76.5 | 78.4 |
| 53. WO051050seqID77 | 90.2 | 90.2 | 90.2 | 88.2 | 88.2 | 90.2 | 88.2 | 78.4 | 78.4 | 78.4 | 78.4 | 76.5 | 80.4 | 54.9 | 76.5 | 82.4 | 76.5 | 76.5 | 78.4 |
| 54. TC3698 | 90.2 | 90.2 | 90.2 | 92.2 | 92.2 | 90.2 | 88.2 | 72.5 | 74.5 | 74.5 | 76.5 | 74.5 | 80.4 | 52.9 | 74.5 | 82.4 | 70.6 | 70.6 | 72.5 |
| 55. Os03g58330 | 100.0 | 100.0 | 100.0 | 98.0 | 98.0 | 100.0 | 98.0 | 78.4 | 80.4 | 80.4 | 80.4 | 78.4 | 82.4 | 54.9 | 78.4 | 84.3 | 76.5 | 76.5 | 78.4 |
| 56. TA139285 | 100.0 | | 100.0 | 98.0 | 98.0 | 100.0 | 98.0 | 78.4 | 80.4 | 80.4 | 80.4 | 78.4 | 82.4 | 54.9 | 78.4 | 84.3 | 76.5 | 76.5 | 78.4 |
| 57. TabHLH11 | 98.0 | 98.0 | 98.0 | | 98.0 | 98.0 | 96.1 | 96.1 | 94.1 | 94.1 | 96.1 | 98.0 | 94.1 | 66.7 | 82.4 | 88.2 | 88.2 | 88.4 | 90.2 |
| 58. TC104646 | 100.0 | 100.0 | 100.0 | 98.0 | | 100.0 | 98.0 | 98.0 | 98.0 | 98.0 | 96.1 | 98.0 | 96.1 | 68.6 | 82.4 | 94.1 | 88.2 | 88.2 | 90.2 |
| 59. TA126400 | 100.0 | 100.0 | 100.0 | 98.0 | 100.0 | | 98.0 | 100.0 | 98.0 | 98.0 | 98.0 | 98.0 | 98.0 | 70.6 | 84.3 | 96.1 | 82.4 | 82.4 | 92.2 |
| 60. Os07g08440 | 100.0 | 100.0 | 100.0 | 98.0 | 100.0 | 100.0 | | 98.0 | 98.0 | 98.0 | 98.0 | 100.0 | 100.0 | | 82.4 | 88.6 | 70.6 | 70.6 | 64.7 |
| 61. Os02g35660 | 98.0 | 98.0 | 98.0 | 96.1 | 98.0 | 98.0 | 98.0 | | 98.0 | 98.0 | 98.0 | 98.0 | 98.0 | 72.5 | 84.3 | 88.2 | 80.4 |
| 62. DT543504 | 96.1 | 96.1 | 96.1 | 94.1 | 96.1 | 96.1 | 96.1 | 96.1 | | 96.1 | 96.1 | 96.1 | 96.1 | 72.5 | 84.3 | 86.3 |
| 63. Pt_scaff28.86 | 96.1 | 96.1 | 96.1 | 94.1 | 96.1 | 96.1 | 96.1 | 96.1 | 96.1 | | 98.0 | 98.0 | 96.1 | 70.6 | 84.3 |
| 64. GSVIVT00016367001 | 98.0 | 98.0 | 98.0 | 96.1 | 98.0 | 98.0 | 98.0 | 98.0 | 98.0 | 98.0 | | 100.0 | 98.0 | 70.6 |
| 65. TC19278 | 70.6 | 70.6 | 70.6 | 68.6 | 70.6 | 70.6 | 70.6 | 70.6 | 70.6 | 70.6 | 72.5 | | 72.5 |
| 66. TA14134 | 94.1 | 94.1 | 94.1 | 92.2 | 94.1 | 94.1 | 94.1 | 94.1 | 92.2 | 94.1 | 96.1 | 96.1 |
| 67. TC68930 | 100.0 | 100.0 | 100.0 | 98.0 | 100.0 | 100.0 | 100.0 | 100.0 | 96.1 | | 80.4 |
| 68. TC140470 | 96.1 | 96.1 | 96.1 | 94.1 | 96.1 | 96.1 | 96.1 | 98.0 | | 88.2 |
| 69. TC253044 | 96.1 | 96.1 | 96.1 | 94.1 | 96.1 | 96.1 | 96.1 | | 86.3 | 96.1 |
| 70. Os06g09370 | 94.1 | 94.1 | 92.2 | 94.1 | 96.1 | 94.1 | | 98.0 | 96.1 |

**Example 4: Identification of domains comprised in polypeptide sequences useful in performing the methods of the invention**

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

The results of the InterPro scan of the polypeptide sequence as represented by SEQ ID NO: 245 are presented in Table E4.

**Table E4:** InterPro scan results (major accession numbers) of the polypeptide sequence as represented by SEQ ID NO: 245.

| Database | Accession number | Accession name | Amino acid coordinates on SEQ ID NO 245; e-value |
|---|---|---|---|
| InterPro | IPR001092 | Basic helix-loop-helix dimerisation region bHLH | |
| HMMPfam | PF00010.14 | Helix-loop-helix DNA-binding domain | T[127-176] 1.6e-06 |
| HMMSmart | SM00353 | no description | T[132-181]1.5e-09 |
| ProfileScan | PS50888 | HLH | T[120-176] 12.451 |
| InterPro | IPR011598 | Helix-loop-helix DNA-binding | |
| superfamily | SSF47459 | Helix-loop-helix DNA-binding domain | T[122-195] 1.8e-14 |

***Example 5: Topology prediction of the polypeptide sequences useful in performing the methods of the invention***

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no).

The results of TargetP 1.1 analysis of the polypeptide sequence as represented by SEQ ID NO: 245 are presented Table E5. The "plant organism group has been selected, no cutoffs defined, and the predicted length of the transit peptide requested. The subcellular localization of the polypeptide sequence as represented by SEQ ID NO: 245 may be the cytoplasrn or nucleus, no transit peptide is predicted.

**Table E5:** TargetP 1.1 analysis of the polypeptide sequence as represented by SEQ ID NO: 245

| | |
|---|---|
| Length (AA) | 281 |
| Chloroplastic transit peptide | 0.094 |
| Mitochondrial transit peptide | 0.166 |
| Secretory pathway signal peptide | 0.034 |
| Other subcellular targeting | 0.855 |
| Predicted Location | / |
| Reliability class | 2 |

(continued)

| Length (AA) | 281 |
|---|---|
| Predicted transit peptide length | / |

When analysed with PLOC (Park and Kanehisa, Bioinformatics, 19, 1656-1663, 2003) or with PSORT (URL: psort.org), the protein is predicted to have a nuclear localisation.

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark;

### Example 6: Functional assay for the bHLH11-like polypeptide

A DNA-binding assay for AtbHLH6 is provided in Dombrecht et al. (2007), this approach can be used for any bHLH11-like protein. Briefly, a 1000-bp fragment of the AtbHLH6 coding sequence encompassing codons 285 to 623 was amplified from genomic DNA and cloned into the NheI-BamHI-digested pTacLCELD6-His vector (Xue, Plant J. 41, 638-649, 2005). The resulting construct encoded the last 338 amino acids of AtbHLH6 (including the bHLH region) in-frame with the reporter protein CelD and a 6xHis tag. Correct amplification and cloning were verified by DNA sequencing.

Determination of the consensus sequence of the MYC2 DNA binding motif and the relative binding affinity of these sites was done according to Xue (2005), wherein a fusion of a DNA-binding protein (DBP) to 6·His-tagged cellulase D (CELD) serves both as a means for affinity purification of DBP-DNA complex in the selection of binding sites from a pool of biotinylated random-sequence oligonucleotides and as a reporter for measurement of DNA-binding activity.

For the selection of binding-sites using cellulose paper as an affinity matrix, DBP-CELD was incubated at room temperature for 1 h with 20 ng of a biotin-labelled Bio-RS-Oligo in 40 $\mu$l of binding/washing buffer (described above) containing 1 mM EDTA, 0.25 $\mu$g $\mu$l$^{-1}$ poly d(AC-TG), 1 mg ml$^{-1}$BSA and 10% glycerol. An appropriate amount of crude DBP-CELD used for binding-site selection was that achieving 20-30% relative cellulose binding efficiency (percentage cellulose activity bound to cellulose after washing). DBP-CELD/Bio-RS-Oligo mixtures were transferred to 96-well microplate wells containing Whatman 1 filter paper (4 mm$^2$) which was pre-soaked with 10 $\mu$l blocking solution [binding/washing buffer containing 0.5 $\mu$g $\mu$l$^{-1}$poly d(AC-TG) and 10% glycerol]. After incubation at 0°C for 1 hr with gentle shaking, the cellulose paper was washed six times with binding/washing buffer containing 1 mM EDTA and 0.1 mg ml$^{-1}$ BSA. The use of extensive washing in the target site selection was based on observations of the relatively high stability of DBP-oligonudeotide complex immobilized on solid matrix. DBP-CELD carrying target binding sites were eluted at 40°C for 15 min with 40 $\mu$l of cellulase eluting buffer [10 mM HEPES, pH7, 50 mM KCl, 0.2 mM EDTA, 4 mM cellobiose, 0.05 mg ml$^{-1}$ BSA and 10 $\mu$g ml$^{-1}$ of a sense sequence-specific primer SP-S]. The eluate was used for PCR amplification of selected oligonucleotides. The PCR product (0.1 $\mu$l) without purification was used for the next round of site selection.

Alternatively, 6·His tagged DBP-CELD (10-25 $\mu$g crude protein) was incubated in 60 $\mu$l of PNT buffer (50 mM sodium phosphate, pH8.0, 300 mM NaCl and 0.05% Tween 20) containing 10 mM imidazole and 350 $\mu$g Ni-NTA magnetic agarose beads (Qiagen) at room temperature for 45-60 min with gentle shaking in a micro-tube mixer (Tomy Seiko Co., Tokyo, Japan). The Ni-NTA magnetic beads were collected at the side of the tubes by placing tubes in a 12-tube magnet (Qiagen). Unbound proteins were removed by washing twice with 150-200 $\mu$l of PNT containing 20 mM imidazole and once with 60 $\mu$l of binding/washing buffer containing 2 mM MgCl$_2$, 1 mg ml$^{-1}$ BSA and 10% glycerol. Washed beads were suspended in 40 $\mu$l of binding/washing buffer containing 2 mM MgCl$_2$, 0.25 $\mu$g $\mu$l$^{-1}$ poly d(AC-TG), 1 mg ml$^{-1}$ BSA, 0.0025% Nonidet P-40, 10% glycerol and biotin-labelled oligonucleotides (50 ng Bio-RS-Oligo or 1 $\mu$l of PCR product amplified from previously selected oligonucleotides). The suspension was incubated at room temperature for 1.5 h with gentle shaking in the micro-tube mixer. After washing four times (3-4 min each washing) with 150-200 $\mu$l of binding/washing buffer containing 2 mM MgCl$_2$, 0.1 mg mr$^1$ BSA and 0.0025% Nonidet P-40, the beads carrying target site-bound BDP-CELD were resuspended in 8 $\mu$l of 5mM Tris-Cl (pH 8.0)/0.5 mM EDTA containing 5 $\mu$g ml$^{-1}$ of a sense sequence-specific primer (SP-S) and the suspension was transferred to a clean tube and used for PCR amplification of selected oligonucleotides. The PCR product (1 $\mu$l) without purification was used for the next round of site selection.

For EMSA assays, 6-His-tagged DBP-CELD proteins were purified using Ni-NTA magnetic agarose beads using a high stringent binding buffer (50 mMsodium phosphate, pH8.0, 1 MNaCl, 10% glycerol, 1% Tween 20 and 10 mM imidazole) and the rest of the procedure followed the manufacturer's instruction. Double-stranded synthetic oligonucleotides (30-45 fmol) labelled with digoxigenin at the 3'-end were incubated with a purified DBP (30-75 ng) in 15 $\mu$l of binding buffer [25 mM HEPES/KOH, pH 7.0, 50 mM KCl, 0.5 mM DTT, 2 mM MgCl$_2$, 0.2 $\mu$g $\mu$l$^{-1}$ poly d(AC-TG), 0.3 mg ml$^{-1}$ BSA and 10% glycerol]. After incubation at room temperature for 30 min, DBP/DNA complexes were separated from free probes on a 6% polyacrylamide gel in a 40-mM Tris-acetate buffer (pH 7.5) containing 5 mM Na acetate, 0.5 mM EDTA and 5% glycerol. The DBP/DNA complexes and free probes in the gels after electrophoresis were transferred to a Hybond Np membrane. Alkaline phosphatase-conjugated anti-digoxigenin antibody and a chemiluminescent substrate, CDP-Star (Roche Diagnostics) was used for detection of digoxigenin according to the manufacturer's instructions. Further details are provided in Xue (2005).

***Example 7: Cloning of the nucleic acid sequence used in the methods of the invention***

The nucleic acid sequence used in the methods of the invention and comprising SEQ ID NO: 244 was amplified by PCR using as template a custom-made *Triticum aestivum* seedlings cDNA library (in pCMV Sport 6.0; Invitrogen, Paisley, UK). PCR was performed using Hifi Taq DNA polymerase in standard conditions, using 200 ng of template in a 50 $\mu$l PCR mix. The primers used were prm009718 (SEQ ID NO: 254; sense, start codon in bold):

5'-ggggacaagtttgtacaaaaaagcaggcttaaacaatggcggggcanccg-3' and prm009719 (SEQ ID NO: 255; reverse, complementary):
5'-ggggaccactttgtacaagaaagctgggtatgggtgttgcagctgctgtt-3',

which include the AttB sites for Gateway recombination. The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone", pbHLH11-like. Plasmid pDONR201 was purchased from Invitrogen, as part of the Gateway® technology.

The entry clone comprising SEQ ID NO: 244 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 256) for constitutive expression was located upstream of this Gateway cassette.

After the LR recombination step, the resulting expression vector pGOS2::bHLH11-like (Figure 23) was transformed into *Agrobacterium* strain LBA4044 according to methods well known in the art.

***Example 8: Plant transformation***

<u>Rice transformation</u>

The *Agrobacterium* containing the expression vector was used to transform *Oryza sativa* plants. Mature dry seeds of the rice iaponica cultivar Nipponbare were dehusked. Sterilization was carried out by incubatino for one minute in 70% ethanol, followed by 30 minutes in 0.2% HgCl$_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and Propagated on the same medium. After two weeks, the calli were multiplied or Propagated by subculture on the same medium for another 2 weeks. Embryogenic callus pieces were subcultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density (OD$_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryonenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges 1999, Chan et al. 1993. Hiei et al. 1994).

*Corn transformation*

Transformation of maize (*Zea mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Acrobacterium tumefaciens* containing the expression vector, and transgenic plants are recovered through organogenesis. After incubation with *Agrobacterium, the* embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mo/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mo/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa *(Medicago sativa)* is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

*Cotton transformation*

Cotton (Gossypium *hirsutum* L.) transformation is performed using *Agrobacterium tumefaciens,* on hypocotyls explants. The commercial cultivars such as Coker 130 or Coker 312 (SeedCo, Lubbock, TX) are standard varieties used for transformation, but other varieties can also be used. The seeds are surface sterilized and germinated in the dark. Hypocotyl explants are cut from the germinated seedlings to lengths of about 1-1.5 centimeter. The hypotocyl explant is submersed in the *Agrobacterium tumefaciens* inoculum containing the expression vector, for 5 minutes then cocultivated for about 48 hours on MS +1.8 mg/l KNO3 + 2% glucose at 24° C, in the dark. The explants are transferred the same medium containing appropriate bacterial and plant selectable markers (renewed several times), until embryogenic calli is seen. The calli are separated and subcultured until somatic embryos appear. Plantlets derived from the somatic embryos are matured on rooting medium until roots develop. The rooted shoots are transplanted to potting soil in the greenhouse. T1 seeds are produced from plants that exhibit tolerance to the selection agent and that contain a single copy of the T-DNA insert.

*Example 9: Phenotypic evaluation procedure*

9.1 Evaluation setup

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions are watered at regular intervals to ensure that water and nutrients are not limiting to satisfy plant needs to complete growth and development.
From the stage of sowing until the stage of maturity the plants were passed several times through a digital imaging cabinet. At each time point digital images (2048x1536 pixels, 16 million colours) were taken of each plant from at least 6 different angles.

*Drought screen*

Plants from T2 seeds are grown in potting soil under normal conditions until they approach the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level is reached again. The plants are then re-transferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

*Salt stress screen*

Plants are grown on a substrate made of coco fibers and argex (3 to 1 ratio). A normal nutrient solution are used during the first two weeks after transplanting the plantlets in the greenhouse. After the first two weeks, 25 mM of salt (NaCl) is added to the nutrient solution, until the plants are harvested. Seed-related parameters are then measured.

9.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

9.3 Parameters measured

*Biomass-related parameter measurement*

From the stage of sowing until the stage of maturity the plants were passed several times through a digital imaging cabinet. At each time point digital images (2048x1536 pixels, 16 million colours) were taken of each plant from at least 6 different angles.
The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).
Early vigour was determined by counting the total number of pixels from aboveground plant parts discriminated from the background. This value was averaged for the pictures taken on the same time point from different angles and was converted to a physical surface value expressed in square mm by calibration. The results described below are for plants three weeks post-germination.

*Seed-related parameter measurements*

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^8$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

*Example 10: Results of the phenotypic evaluation of the transgenic plants*

The results of the evaluation of transgenic rice plants expressing a *bHLH11-like* nucleic acid under non-stress conditions are presented below. An increase of more than 5 % in at least 2 lines was observed for total seed yield, number of filled seeds, fill rate, harvest index, number of first panicles and of at least 3% for thousand kernel weight. Data on the overall increase (measured over all tested lines) for each parameter are presented in Table E6:

Table E6:

| Parameter | Overall increase (%) |
|---|---|
| Total weight of seeds (total seed yield) | 20 |
| Number of filled seeds | 20 |
| Fill rate | 18 |
| Harvest Index | 21 |

**Claims**

1. A method for increasing seed yield in plants relative to control plants, comprising increasing expression in a plant of a nucleic acid encoding a bHLH11-like polypeptide under the control of a medium strength constitutive promoter, wherein said bHLH-11 like polypeptide comprises a Helix-Loop-Helix domain, and one or more of the following motifs:

    (i) Motif 1 (SEQ ID NO: 246);
    (ii) Motif 2 (SEQ ID NO: 247);
    (iii) Motif 3 (SEQ ID NO: 248);
    (iv) Motif 4 (SEQ ID NO: 249);
    (v) Motif 5 (SEQ ID NO: 250);
    (vi) Motif 6 (SEQ ID NO: 251);
    (vii) Motif 7 (SEQ ID NO: 252).

2. Method according to claim 1, wherein said increased expression is effected by introducing and expressing in a plant a nucleic acid encoding a bHLH11-like polypeptide.

3. Method according to any preceding claim, wherein said nucleic acid encoding a bHLH11-like polypeptide encodes any one of the proteins listed in Table E1 or is a portion of such a nucleic acid capable of hybridizing with such a nucleic acid.

4. Method according to any preceding claim, wherein said nucleic acid sequence encodes an orthologue or paralogue of any of the proteins given in Table E1.

5. Method according to any one of claims 1 to 4, wherein said increased seed yield is obtained under non-stress conditions.

6. Method according to any one of claims 2 to 5, wherein said constitutive promoter of medium strength is a GOS2 promoter, preferably a GOS2 promoter from rice.

7. Method according to any preceding claim, wherein said nucleic acid encoding a bHLH11-like polypeptide is of plant origin, preferably from a monocotyledonous plant, further preferably from the family Poaceae, more preferably from the genus *Triticum,* most preferably from *Triticum aestivum.*

8. Plant or part thereof, including seeds, obtainable by a method according to any preceding claim, wherein said plant or part thereof comprises a recombinant nucleic acid encoding a bHLH11-like polypeptide as defined in claim 1.

9. Construct comprising:

(i) nucleic acid encoding a bHLH11-like polypeptide as defined in claim 1;
(ii) One or more control sequences capable of driving expression of the nucleic acid sequence of (i), wherein said one or more control sequences comprise a medium strength constitutive promoter; and optionally
(iii) A transcription termination sequence.

10. Construct according to claim 9, wherein said constitutive promoter of medium strength is a GOS2 promoter, preferably a GOS2 promoter from rice.

11. Use of a construct according to claim 9 or 10 in a method for making plants having increased seed yield relative to control plants.

12. Plant, plant part or plant cell transformed with a construct according to claim 9 or 10.

13. Method for the production of a transgenic plant having increased seed yield relative to control plants, comprising:

(i) introducing and expressing in a plant a nucleic acid encoding a bHLH11-lke polypeptide as defined in claim 1, wherein said nucleic acid is under the control of a medium strength constitutive promoter; and
(ii) cultivating the plant cell under conditions promoting plant growth and development.

14. Transgenic plant having increased seed yield, relative to control plants, resulting from increased expression of a nucleic acid encoding a bHLH11-like polypeptide as defined in claim 1, wherein said nucleic acid is under the control of a medium strength constitutive promoter, or a transgenic plant cell derived from said transgenic plant.

15. Transgenic plant according to claim 8, 12 or 14, or a transgenic plant cell derived thereof, wherein said plant is a crop plant or monocot or a cereal, such as rice, maize, wheat, barley, millet, rye, triticale, sorghum emmer, spelt, secale, einkorn, teff, milo and oats.

16. Harvestable parts of a plant according to claim 15 comprising a construct according to claim 9 or 10, wherein said harvestable parts are preferably seeds.

17. Use ofa nucleic acid encoding a bHLH11-like polypeptide as defined in claim 1, wherein said nucleic acid is under the control of a medium strength constitutive promoter for increasing seed yield in plants, relative to control plants.

**Patentansprüche**

1. Verfahren zur Erhöhung des Samenertrags in Pflanzen im Vergleich zu Kontrollpflanzen, bei dem man in einer Pflanze die Expression einer für ein bHLH11-ähnliches Polypeptid unter der Kontrolle eines konstitutiven Promotors mittlerer Stärke kodierenden Nukleinsäure erhöht, wobei dieses bHLH11-ähnliche Polypeptid eine Helix-Schleife-Helix-Domäne und eines oder mehrere der folgenden Motive umfasst:

(i) Motiv 1 (SEQ ID NO: 246),
(ii) Motiv 2 (SEQ ID NO: 247),
(iii) Motiv 3 (SEQ ID NO: 248),
(iv) Motiv 4 (SEQ ID NO: 249),
(v) Motiv 5 (SEQ ID NO: 250),
(vi) Motiv 6 (SEQ ID NO: 251),
(vii) Motiv 7 (SEQ ID NO: 252).

2. Verfahren nach Anspruch 1, wobei die erhöhte Expression durch Einführen und Exprimieren einer Nukleinsäure, die ein bHLH11-ähnliches Polypeptid kodiert, in eine(r) Pflanze herbeigeführt wird.

3. Verfahren nach einem vorhergehenden Anspruch, wobei die Nukleinsäure, die ein bHLH11-ähnliches Polypeptid kodiert, eines der in Tabelle E1 aufgeführten Proteine kodiert, oder ein Teil einer solchen Nukleinsäure ist, die mit einer solchen Nukleinsäure hybridisieren kann.

4. Verfahren nach einem vorhergehenden Anspruch, wobei die Nukleinsäuresequenz ein Orthologon oder Paralogon von einem der in Tabelle E1 angegebenen Proteine kodiert.

5. Verfahren nach einem der Ansprüche 1 bis 4, wobei der erhöhte Samenertrag unter Nichtstressbedingungen erhalten wird.

6. Verfahren nach einem der Ansprüche 2 bis 5, wobei der konstitutive Promotor mittlerer Stärke ein GOS2-Promotor, bevorzugt ein GOS2 Promotor aus Reis, ist.

7. Verfahren nach einem vorhergehenden Anspruch, wobei die Nukleinsäure, die ein bHLH11-ähnliches Polypeptid kodiert, aus einer Pflanze, vorzugsweise aus einer monokotylen Pflanze, weiter bevorzugt aus der Familie Poqceae, stärker bevorzugt aus der Gattung *Triticum,* am stärksten bevorzugt aus *Triticum aestiuum,* stammt.

8. Pflanze oder Teil davon, einschließlich Samen, die durch ein Verfahren nach einem vorhergehenden Anspruch erhältlich sind, wobei die Pflanze oder das Teil davon eine rekombinante Nukleinsäure umfasst, die ein bHLH11-ähnliches Polypeptid kodiert, wie in Anspruch 1 definiert.

9. Konstrukt, umfassend:

    (i) eine Nukleinsäure, die ein bHLH11-ähnliches Polypeptid nach Anspruch 1 kodiert;
    (ii) eine oder mehrere Kontrollsequenzen, die die Expression der Nukleinsäuresequenz von (i) steuern können, wobei die eine oder die mehreren Kontrollsequenzen einen konstitutiven Promotor mittlerer Stärke umfassen; und gegebenenfalls
    (iii) eine Transkriptionsterminationssequenz.

10. Konstrukt nach Anspruch 9, wobei der konstitutive Promotor mittlerer Stärke ein GOS2-Promotor, bevorzugt ein GOS2-Promotor aus Reis, ist.

11. Verwendung eines Konstrukts nach Anspruch 9 oder 10 in einem Verfahren zur Herstellung von Pflanzen mit gesteigertem Samenertrag im Vergleich zu Kontrollpflanzen.

12. Pflanze, Pflanzenteil oder Pflanzenzelle, die mit einem Konstrukt nach Anspruch 9 oder 10 transformiert ist.

13. Verfahren zur Produktion einer transgenen Pflanze mit gesteigertem Samenertrag im Vergleich zu Kontrollpflanzen, umfassend:

    (i) Einführen und Exprimieren einer Nukleinsäure, die ein bHLH11-ähnliches Polypeptid nach Anspruch 1 kodiert, wobei diese Nukleinsäure unter der Kontrolle eines konstitutiven Promotors mittlerer Stärke ist, in eine(r) Pflanze; und
    (ii) Züchten der Pflanzenzelle unter Bedingungen, die das Wachstum und die Entwicklung der Pflanze fördern.

14. Transgene Pflanze mit gesteigertem Samenertrag im Vergleich zu Kontrollpflanzen, der sich aus einer gesteigerten Expression einer Nukleinsäure, die ein bHLH11-ähnliches Polypeptid nach Anspruch 1 kodiert, ergibt, wobei diese Nukleinsäure unter der Kontrolle eines konstitutiven Promotors mittlerer Stärke ist, oder eine transgene Pflanzenzelle, die aus der transgenen Pflanze stammt.

15. Transgene Pflanze nach Anspruch 8, 12 oder 14, oder eine daraus stammende transgene Pflanzenzelle, wobei die Pflanze eine Kulturpflanze oder eine Monokotyle oder ein Getreide, wie Reis, Mais, Weizen, Gerste, Hirse, Roggen, Triticale, Sorghum, Emmer, Dinkel, Sekale, Einkorn, Teff, Milo und Hafer ist.

16. Erntefähige Teile einer Pflanze nach Anspruch 15, umfassend ein Konstrukt nach Anspruch 9 oder 10, wobei die erntefähigen Teile vorzugsweise Samen sind.

17. Verwendung einer wie in Anspruch 1 definierten, für ein bHLH11-ähnliches Polypeptid kodierenden Nukleinsäure, wobei die Nukleinsäure unter der Kontrolle eines konstitutiven Promotors mittlerer Stärke steht, zum Steigern des Samenertrags in Pflanzen im Vergleich zu Kontrollpflanzen.

**Revendications**

1. Procédé pour augmenter le rendement en graines dans des plantes par rapport à celui de plantes témoins, comprenant l'augmentation de l'expression, dans une plante, d'un acide nucléique codant pour un polypeptide bHLH11-like sous le contrôle d'un promoteur constitutif de force moyenne, dans lequel ledit polypeptide bHLH11-like comprend un domaine hélice-boucle-hélice, et un ou plusieurs des motifs suivants :

(i) Motif 1 (SEQ ID NO:246) ;
(ii) Motif 2 (SEQ ID NO:247) ;
(iii) Motif 3 (SEQ ID NO:248) ;
(iv) Motif 4 (SEQ ID NO:249) ;
(v) Motif 5 (SEQ ID NO:250) ;
(vi) Motif 6 (SEQ ID NO:251) ;
(vii) Motif 7 (SEQ ID NO:252).

2. Procédé selon la revendication 1, dans lequel ladite augmentation de l'expression est réalisée par introduction et expression, dans une plante, d'un acide nucléique codant pour un polypeptide bHLH11-like.

3. Procédé selon l'une quelconque des revendications précédentes, dans lequel ledit acide nucléique codant pour un polypeptide bHLH11-like code pour l'une quelconque des protéines listées dans le Tableau E1 ou est une portion d'un tel acide nucléique, capable de s'hybrider à un tel acide nucléique.

4. Procédé selon l'une quelconque des revendications précédentes, dans lequel ladite séquence d'acide nucléique code pour un orthologue ou un paralogue de l'une quelconque des protéines consignées dans le Tableau E1.

5. Procédé selon l'une quelconque des revendications 1 à 4, dans lequel ladite augmentation du rendement en graines est obtenue dans des conditions sans stress.

6. Procédé selon l'une quelconque des revendications 2 à 5, dans lequel ledit promoteur constitutif de force moyenne est un promoteur GOS2, de préférence un promoteur GOS2 du riz.

7. Procédé selon l'une quelconque des revendications précédentes, dans lequel ledit acide nucléique codant pour un polypeptide bHLH11-like provient d'une plante, de préférence d'une plante monocotylédone, d'une manière plus préférée de la famille des Poaceae, plus particulièrement du genre *Triticum,* tout spécialement de *Triticum aestivum.*

8. Plante ou partie de plante, y compris les graines, pouvant être obtenue par un procédé selon l'une quelconque des revendications précédentes, ladite plante ou partie de plante comprenant un acide nucléique recombinant codant pour un polypeptide bHLH11-like tel que défini dans la revendication 1.

9. Construction comprenant :

(i) un acide nucléique codant pour un polypeptide bHLH11-like tel que défini dans la revendication 1 ;
(ii) une ou plusieurs séquences régulatrices capable de commander l'expression de la séquence d'acide nucléique de (i), ladite ou lesdites séquences régulatrices comprenant un promoteur constitutif de force moyenne, et, en option,
(iii) une séquence terminatrice de transcription.

10. Construction selon la revendication 9, dans laquelle ledit promoteur constitutif de force moyenne est un promoteur GOS2, de préférence un promoteur GOS2 du riz.

11. Utilisation d'une construction selon la revendication 9 ou 10, dans un procédé permettant d'obtenir des plantes ayant un rendement en graines augmenté par rapport à celui de plantes témoins.

12. Plante, partie de plante ou cellule végétale transformée avec une construction selon la revendication 9 ou 10.

13. Procédé de production d'une plante transgénique présentant un rendement en graines augmenté par rapport à celui de plantes témoins, comprenant

(i) l'introduction et l'expression, dans une plante, d'un acide nucléique codant pour un polypeptide bHLH11-like tel que défini dans la revendication 1, ledit acide nucléique étant sous le contrôle d'un promoteur constitutif de force moyenne ; et

(ii) la culture de la cellule végétale dans des conditions favorisant la croissance et le développement de la plante.

14. Plante transgénique ayant un rendement en graines augmenté par rapport à celui de plantes témoins, résultant de l'augmentation de l'expression d'un acide nucléique codant pour un polypeptide bHLH11-like tel que défini dans la revendication 1, ledit acide nucléique étant sous le contrôle d'un promoteur constitutif de force moyenne, ou cellule de plante transgénique dérivant de ladite plante transgénique.

15. Plante transgénique selon la revendication 8, 12 ou 14, ou cellule de plante transgénique qui en dérive, ladite plante étant une plante de culture ou une monocotylédone ou une céréale telle que le riz, le maïs, le blé, l'orge, le millet, le seigle, le triticale, le sorgo, l'amidonnier, l'épeautre, le sécale, l'engrain, le tef, le milo et l'avoine.

16. Parties récoltables d'une plante selon la revendication 15, comprenant une construction selon la revendication 9 ou 10, lesdites parties récoltables étant de préférence les graines.

17. Utilisation d'un acide nucléique codant pour un polypeptide bHLH11-like tel que défini dans la revendication 1, pour laquelle ledit acide nucléique est sous le contrôle d'un promoteur constitutif de force moyenne pour augmenter le rendement en graines de plantes par rapport à celui de plantes témoins.

MAGQPPQGPE<u>DDFLDQ</u>FFSLTNSLSAAGRPSGDQPFSLA

        ---1---

LSLDAASDASGSRGGIGDDAGNAAERDGVQLPGLFPPVF

GGGLQPPHLRPSHPPPQMFHAQQPKQGGPAGGPQPPAPR

PKVRARRGQATDPH**<u>SIAERLRRERIAERMRALQELVPNT</u>**

    ----2-----------        ---------

**<u>NKTDRAVMLDEILDYVKFLRLQVKVL</u>**SMSRLGGAGAVAQ

        -------4-----------|---5---

LVSDIPLSVKGEASDGGSKQQIWEKWSTDGTEKQVAKLM

                              -----

DEDIGAAMQFLQSKALCMMPVSLAMAIYDTQHSQDGQPV

--6---------        ------

KPEPNNTA

**FIGURE 1**

CLUSTAL 2.0.3 multiple sequence alignment

```
AT2G24260              ------------------------------------MMNSSLLTPSSSSSSHIQTPS-
AT4G30980              ----------------------------------MNSSSLLTPSSSPSPHLQSP--
AT5G58010             -----------------------------------MENG-----NGEGKGEFINQ--
TA12416               ------------------------------------MQPCSSGGEMQGISSLLSNGSH
TA5414                ------------------------------------MQPCSSGGEMQGISSLLSNGSH
TC205173             ----------------------------------------------------------
GSVIVT00017237001    ----------------------------------------------------------
TC7102               -----------------------------------MQAMN-----------------
TA34455              -----------------------------------MQAMNSLLSQQQQSQMSLQDLQ
TC7103               -----------------------------------MQAMNSQMSLQEENGASSGQ--
TA37666              -----------------------------------MQAMNSFQSTGE-NGASSGE--
TC67603              ----------------------------------MQPCSREMQAMNSLLNPTQQIPLQDLQIN
Os06g08500           ------------------------------------MQPSSRDTV------AGG----
Os02g55250           ------------------------------------MQPNARQMR------GGG----
Os09g25040           -----------------------------------MAAAAAGQISLDDLR------NGGGVAA
TA2544               ------------------------------------MQPSSKETERMAQSHAALQLEL
DV982110             ----------------------------------------------------------
WO051050seqID516     ------------------------------------MSLLPPGIGGQGLNSQENDPYP
AT4G02590            ------------------------------------MASNN--PHD-----------
TC10015_part         ----------------------------------------------------------
AT1G03040            ------------------------------------MANNNNIPHD-----------
TA13791              ------------------------------------MANNP---------------
TC60118              ------------------------------------MANNP---------------
TC60119              ------------------------------------MANNP---------------
TC61833              ------------------------------------MANNT---------------
TA3392               ------------------------------------MANNP---------------
WO051050seqID1671    ------------------------------------MANNP---------------
Pt_TC63334           ------------------------------------MANNP---------------
TA3263               ------------------------------------MANNP---------------
Pt_scaff_II.416      ------------------------------------MANNP---------------
TA2825               ------------------------------------MANNP---------------
TC207545             ----------------------------------------------------------
TC229602             ----------------------------------------------------------
TA46156              ------------------------------------MASNP---------------
```

FIGURE 2

EP 2 230 310 B1

EP 2 230 310 B1

FIGURE 2 (continued)

```
AK247217           ------------------------------------MANNP----------------
TA30646            ------------------------------------MANNP----------------
TC8633             ------------------------------------MANNP----------------
TC15501            ------------------------------------MANNN----------------
TA1140             ------------------------------------MSTDP----------------
TA3490_part        ------------------------------------MATDP----------------
CK293938           ------------------------------------MATNQ----------------
TC12771            ------------------------------------MATNQ----------------
TC172581           ------------------------------------MAANQ----------------
TA28621            ------------------------------------MAANQ----------------
TA21665            ------------------------------------MADNP----------------
TA46194            ------------------------------------MATNP----------------
DY268946           ---------------------------------------------------------
CO123623           ---------------------------------------------------------
Pt_scaff_70.65     ------------------------------------MAGNP----------------
Pt_scaff_XIII.403  ------------------------------------MAGNP----------------
TA1616             ------------------------------------MAGNP----------------
TA55042            ------------------------------------MAGNS----------------
WOO51050seqID77    ------------------------------------MAGSN----------------
TC3698             ------------------------------------MASNN---NP------------
Os03g58330         ------------------------------------MAGQQ----------------
TA139285           ------------------------------------MAGQP----------------
TabHLH11           ------------------------------------MAGQ-----------------
TC104646           ------------------------------------MAGQP----------------
TA126400           ------------------------------------MAGQP----------------
Os07g08440         ------------------------------------MAGQQ----------------
Os02g35660         ---------------------------MGGFAYPFTPSPAWSRDAVFAGSPWAAGG
DTS43504           -------------------------------------------------MIS---
Pt_scaff28.86      -------------------------------------------------MISSNQ
GSVIVT00016367001  MDEYLDHLFSSTSWSDVNVKEGSSWICGEPSQTNGMLSDSIGIYEGDKKNSPVGMTNSNL
TC19278            ---------------------------------------------------------
TA14134            ---------------------------------------------------------
TC68930            ---------------------------------------------------------
TC140470           -MNYSDGSFFPSWPGNSASENYSFVDGSVESYTEEGSMPTTGYFR-ARSNQNLTFDEHEQ
TC253044           ---------------------------------------------------------
Os06g09370         -MDYSAGSYM--WPGNSGSENYNFVDGSSESYAEEGSLPPSGYFMGAGSDRSLKITENER
```

FIGURE 2 (continued)

```
AT2G24260               -----------------------------TTFDHEDFLDQIFSSAPWP----------------
AT4G30980               ------------------------ATFDHDDFLHHIFSSTPWPS---------------
AT5G58010               ----------------------NNDFFLDSMSMLSSLP---------------
TA12416                 ----------EQQSQIHQSATFDPT-SQDDFLEQMLSSLPSCS--WTDLKSPWGVVDL
TA5414                  ----------EQQSQIHQSATFDPT-SQDDFLEQMLSSLPSCS--WTDLKSPWGVVDL
TC205173                -------------------------------------------------
GSVIVT00017237001       --------------------------MLSTLPSWSDLPANPKSPWELNAS
TC7102                  ----------------------QYDPTSSHDDFLDQILSSVPSSSLCWPDLSKSW-----
TA34455                 NGGNGGSTGGVGGLGQHNMGHFHFDPTSSHDDFLEQILSSVPSSSP-WPDLSKSW-----
TC7103                  -----------------NMSHSHFDPTSSHDDFLQQILSSVPSSSP-WPDLSSGG-----
TA37666                 -----------------HISHSHFDPSSSHDDFLQQILSSVPSSSP-WPEISGDG-----
TC67603                 -GNRHHHQQIHVPSSSQFQYPTPTSTTHHDDSFLEQILSSTTSFP--WSDETGPP-----
Os06g08500              ---------------------GGEG-TQDDFFDQMLSTLP-SAWADLGGG--GGGAAG
Os02g55250              ---------------------GGGGGGQDDFFDQMLSTLP-AVWSELGSGKPAWDLTA
Os09g25040              -----------------NAGGGGGVHDDFLDQMLSSLPPSAWPDLAAGKAAEDDAE
TA2544                  -----------------QNGGGGAQNDDFFEQTMSGFSTAAWAP-ELGTPRSLFGA
DV982110                -------------------------------------------------
WO051050seqID516        -----------------YDDSQFLANRLRQHQLSGNSSMNQTPNQASQGINETNT
AT4G02590               ------------------NLSDQTPSDDFFEQILGLPNFSASSAAGLSGVDGGLGG
TC10015_part            ------------------HASETPSDDFFEQILGLPNFSASSS------DGGLGG
AT1G03040               ------------------SISDPSPTDDFFEQILGLSNFSGSSSGSG----LSGIGG
TA13791                 ------------------NES-PADDFLEQILGLSHFAPTET-GLAGPDGRLSG
TC60118                 ------------------NES-PADDFLEQILGLSHFAPTET-GLAGPDGRLSG
TC60119                 ------------------NEAPAADDFLEQILGLPNFAPSET-GLAGSDAGLAA
TC61833                 ------------------NEA-SADDFLEQILGFPNFAPSET-GLAGPDGGLTG
TA3392                  ------------------NEASSTDDFLEQILGIPNFGSAES-GLAASDGGLA-
WO051050seqID1671       ------------------SDGASDEFLEHLLGLPNFAAAAEAGLAPGDGTGLS
Pt_TC63334              -----------------TEPPTDDFLQEILGMPNFASAEAG-LVGADAGLAG
TA3263                  -----------------TEPPADDFLQEILGMPNFASAEAG-LVGADAGLAG
Pt_scaff_II.416         -----------------TEPPTDDFLQEILGMPNFASAEAG-LVGADAGLAG
TA2825                  -----------------TEPPADDFLQEILGLPNFASADAAGLVGADGALA-
TC207545                -------------------------------------------------
TC229602                -------------------------------------------------
TA46156                 -----------------SEAPADDFLEQILGIPTYPAADP-------NLAA
AK247217               -----------------SEGPSDDFFDQILGFPAYNGAEP-NLAGNDAGAIP
```

EP 2 230 310 B1

```
TA30646              -------------------------------SEGPSDDFFDQIMGFPAYNGAET-NLAGNDAGAIP
TC8633               -------------------------------SEGP-DDFFDQILGFPAYNGAET-NLAGSDAGAIP
TC15501              ----------------------------NPTEGLTDDFLDQILSYG----AHEGNLAGNDVNLAG
TA1140               ----------------------------PEGY-GDDFLEQILAIPSYN----------------
TA3490_part          ----------------------------PEGY-GDDFLEQILAIPSYN----------------
CK293938             ----------------------------PEGY-ADDFLEQILAIPSY-----------------
TC12771              ----------------------------PGGY-ADDFLEQILAIPSY-----------------
TC172581             ----------------------------PEGY-ADDFLEQILAIPPY-----------------
TA28621              ----------------------------PEAY-ADDFLEQILAIPSY-----------------
TA21665              ----------------------------PEVYAADDFLEQILAIPSY-----------------
TA46194              ----------------------------PEGY-ADDFLEQILAIPSYP----------------
DY268946             -------------------------------MFLQLGSGGPSPGAG------------------
CO123623             -----------------------------------------DSG-------------------
Pt_scaff_70.65       ----------------------------PPEGLGDDFFEQILAGQPPGYGG----------EA
Pt_scaff_XIII.403    ----------------------------PPEGLGDDFLEQILAAQPPGYGGG--------GEV
TA1616               ----------------------------PPEGLGDDFFEQILAVQP-GYGGGDG----GGGGDV
TA55042              ----------------------------GSEGLGDDFFEQILAVPEAGTVG-------------
WO051050seqID77      ----------------------------PQEGLGDEFFEQILAVPPGAYSGGS-------TPM
TC3698               ------------------------QPPSDGLNDDFFDQIFSMPSAFAASS-------------
Os03g58330           ------------------------PQQQGPPEDDFFDQFFSLTS--SF--------------
TA139285             ------------------------PPQG--PEDDFFDQFFSMTAGGSY-------------
TabHLH11             ------------------------PPQG--PEDDFLDQFFSLTN----------------
TC104646             ------------------------PPPGG-SEDDFLEHFFAFPSAASA-------------
TA126400             ------------------------PPSGA-SEDDFLEHFFAFPSAASAG-----------
Os07g08440           ------------------------PPTGG-AEDDFFDHFFSIPSAAAA------------
Os02g35660           ------------------VSSLADALVSYGAVDDEEAAFLGKTAASSPSTARLHEQQQLLLE
DT543504             ----------------------------------------------------------------
Pt_scaff28.86        SVESLATQDTSSVVLGSESDYAVDKVLISEQARLQNDCQNCNGNPSPDGMARGNLKFGNT
GSVIVT00016367001    MIEGLVTQDTSSIVLGGESDHGLGKGLLLEEAPRQQDLQNTEGNSSLNGAVNGSSEVGYV
TC19278              ----------------------------------------------------------------
TA14134              ----------------------------------------------------------------
TC68930              ----------------------------------------------------------------
TC140470             NPAMLANGCLPYNTQTDLLSGEILSDDKPSNSLVELSQLQNNVCLQNNLIPPGTLQCNST
TC253044             ----------------------------------------------------------------
Os06g09370           NPTMLANGCLPYNTQAHPLSGQILPKGELPNNLLDLQQLQNSSNLRSNSIPPGVLQCNST
```

FIGURE 2 (continued)

FIGURE 2 (continued)

```
AT2G24260              --------------------------------------SVVDDAHPLPSD-----GFHG
AT4G30980              ------------------------------------SVLDDTPPPTSDCAPVTGFHH
AT5G58010              ------------------------------------PCWDPSLPPPPP---------
TA12416               NPNNNNNNINNKQPRDLSDETAPSTTQENNVPAGFQFDESMILASKMRQHQISGNTGS-G
TA5414                NPNNNNNNINNKQPRDLSDETAPSTTQENNVPAGFQFDESMILASKMRQHQISGNTGS-G
TC205173              --------------------------------------------------------
GSVIVT00017237001     NP----ISMPSNKSRDLSDDTTPSNPDN----VQFAFDESAMLASKLRQHQIS------G
TC7102                DPHHHHLSPPLPPNPSSGDDHQLPPSNPLQ-HFQYDDQSSSFLAAKLRQHQITGGGGGGG
TA34455               DPHHHLSSP--PHNPSSGEDQ--PPSNPLHSQFHYDDQASSLLASKLRQHQITGGG----
TC7103                DGH-----------------------FDD-QSIFVASKLRQNQITGGGG---
TA37666               HPYN-------------------------FDDHQSTLLASKLRQHQINGGSS---
TC67603               NPEDNNNAG-------------------------FNYDEMVLASKLRQHQINGGAAG-N
Os06g08500            KSPWEVDPAA-----------------AAAASQVFDESALLASRLRHHQIGGAGGGGG
Os02g55250            GAVGGGGGAS-----------------DDHSAAAFDDSALLASRLRQHQID----GGG
Os09g25040            G-MHHHHHQQ-----------------QQQFGGPYDESAMLASRLRQHQISGGGGGGG
TA2544                RSSEESPDDE-----------------GLNYAPPYGGSSLLASRHRMHLGT----SPA
DV982110              --------------------------------------------------------
WO051050seqID516      SPGRSMVLQLS-------------------TGSASASQLLASMGNSPRGTAVMTQSPSTG
AT4G02590             G-------------------------------APPMMLQLGSGEEG-------
TC10015_part          GG------------------------------APPMMLQLGSGEEG-------
AT1G03040             VG------------------------------PPPMMLQLGSGNEGN------
TA13791               NATT-----------------------------AGAPMLLQLSSGGGT-------
TC60118               NATT-----------------------------AGAPMLLQLSSGGGT-------
TC60119               TAAG-----------------------------AGAPMLLQLSSGGGT-------
TC61833               TTAG-----------------------------AGAPMFLQLSSGDGA-------
TA3392                -------------------------------------AGSPMMLQLSSGDGS-------
WO051050seqID1671     VT-------------------------------AATPMMLQLGSGDGSGGHGHGH
Pt_TC63334            AAA------------------------------AQASMMLQLSSGDGS-------
TA3263                AAA------------------------------AQASMMLQLSSGDGS-------
Pt_scaff_II.416       TAS------------------------------VQAPMMLQLSSGDGS-------
TA2825               ---------------------------------TPMMLQLSSGDGS-------
TC207545            ----------------------------------MMLQLNSGDAA-------
TC229602             --------------------------------------------------------
TA46156              NDVN-------------------------------LAAPMVLQLGSGEGS-------
AK247217            --------------------------------PAMMLQLNSGDGS-------
```

FIGURE 2 (continued)

```
TA30646            -----------------------------------PAMMLQLNSGDGS-------
TC8633             -----------------------------------PAMLLQLNSGDAS-------
TC15501            TTT-------------------------------PGGSMALQLSSSGGD-------
TA1140             --IT------------------------------GCLPVNTADSTGSET-------
TA3490_part        --IA------------------------------GCLPGNTTDANASET-------
CK293938           ----------------------------------AGLPPVADVGTSSET-------
TC12771            ----------------------------------AGLPPVADVGTSSET-------
TC172581           ----------------------------------SGLP-VADVGTPSET-------
TA28621            ----------------------------------SGLP-VADVGTPSET-------
TA21665            ----------------------------------ASLP-VTDLTAGASS-------
TA46194            ---------------------------------GHDPNMVGTGSTMVL-------
DY268946           ----------------------------------------GGATSAADIRSLA
CO123623           -----------------------------------------GGG-----FR---
Pt_scaff_70.65     VGST------------------------------SLPMMGLQLGSSAANVGLMRSS
Pt_scaff_XIII.403  MGST------------------------------SLPMMGLQLGSCAGNVGLLRSN
TA1616             VGST-------------------------------MPMMGLQLGSASSGGGGLRT-
TA55042            ---------------------------------------MLQLGSTTGAFRGAS--
WO051050seqID77    VLQL------------------------------GSGRGGVEDGGGRGGGGGLRG-
TC3698             ---------------------------------ASTDATEALNYADSS-------
Os03g58330         ---------------------------------PGAAPGG-RAAGDQ-------
TA139285           ---------------------------------PGATAGGGRAPGDQ-------
TabHLH11           --------------------------------SLSAAG-RPSGDQ-------
TC104646           ---------------------------------AAGGHAGAGAGGDH-------
TA126400           ---------------------------------AAGGHAGAGVGGDH-------
Os07g08440         ---------------------------------GAGGVGGFGSGDHH-------
Os02g35660         AELLRHGDGLG------------FAAMDDDGGAAMLGALEPCAMPLTDSGGPPVICSSS
DT543504           -----------------------------------------NGGESSEFRRSFT----
Pt_scaff28.86      GLQCNGILPTLSSLNY--PNQLPIVGDLT-SYLSFSEASNAGCNGREQSEYLRSLK----
GSVIVT00016367001  GLQLNTPTSTPCSLDLGSPKQLSLIGGMSRSSRPFTELDHVGCNGNEPSDFQRSVG----
TC19278            ---------------------------------------------------------
TA14134            ------------------------------------------MDDIFDQLLSSSWE----
TC68930            ----------------------------------------MLARENRSGDTEHD----
TC140470           PGTFDLQLDTPGLLELPHALSSSIESNGSEVSAFLADVQAVSSASTLCSTFQNVPSYMEP
TC253044           --------------------------------------------MLLCSTIQVIQ-----
Os06g09370         SGTFDAKLDTPGLAELPHALSSSIDSNGSDISAFLADVHAVSSAPTLCSAFQNVSSFMEP
```

FIGURE 2 (continued)

```
AT2G24260           HDVDSRNQPIMMMPLNDG-------------------------------------------
AT4G30980           HDADSRNQ-ITMIPLSHN-------------------------------------------
AT5G58010           -------------------------------------------------------------
TA12416             NNNSAAAKFMMQQQQQQ------------IMMAAARGGIG-------LPLSLGNG-----
TA5414              NNNSAAAKFMMQQQQQQ----------IMMAAARGGIG-------LPLSLGNG-----
TC205173            ---------MLQHQLLR----------------DSGLLN-------MPLSLPG------
GSVIVT00017237001   NSSAAKSALMLQQQLLLSRGVAMGRSPSNGSGAGESGLLQ-------LPLSLSNGDSCLV
TC7102              DAVAAAKALLLQQQLLLS------------RTLAGNGLR-------SPTGASGDNGFL-
TA34455             -AAAAAKALMLQQQLLLS------------RTLAGNGLR-------SPNGASGDNGLLS
TC7103              -GGAAAKALMLQQQLLLS------------RGLLAG----------------------
TA37666             -AAAAAKALMLQQQLLLS------------RGIAGN-----------------------
TC67603             PFAAMKMMLMMQQQQQQQ------------QMMLAGRPT-------VASAAAGG-----
Os06g08500          EKP------VMLQLSELHR------QAG---GGEEDGSGA------FSPLPLFTDRTNVP
Os02g55250          DKP------IMLQLSDLHR------HHGLAAGDDSGGAAG-----FLPLSLFADRS---
Os09g25040          GGAAAVKQMVLQQLADLRQGHHMM-LQGLGGRSPAGGGGGGGDGGLLLPLTLGSGGS---
TA2544              DSS------MFLQLGGQILLHP---TAG-ASGGESGGFLR------LPLSLGSGGSGVS
DV982110            ---------MLPLPLSLG-------------QAGKSGDMN--------------------
WO051050seqID516    GSCNGSDGGMLPLPLSLG-------------QAGKSGDIN--------------------
AT4G02590           ------SHMGGLGGSGP---------------TGFHNQ--------MFPLGLSLDQGKG-
TC10015_part        ------GHMGGLGGG-----------------SGFHNQ--------MFPLGLSLEQGKG-
AT1G03040           -----HNHMGAIGGGGP---------------VGFHNQ--------MFPLGLSLDQGKG-
TA13791             ------GHIGAIGGGGG---------------GAFHGQ--------VFPLGLSLEQGK--
TC60118             ------GHIGAIGGGGG---------------GAFHGQ--------VFPLGLSLEQGK--
TC60119             ------AHIGGIGGGGG---------------GAFHGQ--------VFPLGLSLEQGK--
TC61833             ------SHLGGIGGGGG---------------GAFPGQ--------VFPLGLSLDQGKS-
TA3392              ------SHISALGGGVS---------------SGYHGQ--------VFPLGLSLEQGK--
WO051050seqID1671   GHGHGQGHLSALGGGGAGAGGGGG-----GGGGGYHGA--------VFPLGLSLEQGK--
Pt_TC63334          ------GHISDLGG-APGGGS----------AGFHG---------FPLGLSLEQGK--
TA3263              ------GHISDLGG-APGGGS----------AGFHG---------FPLGLSLEQGK--
Pt_scaff_II.416     ------GHISALGG-APGGGG----------AGFHG---------FPLGLSLEQGK--
TA2825              ------NHITALGGGGGGGGG----------AGFHG---------FPLGLSLEQGK--
TC207545            ------THLA--------------------SFHAP--------PYQLGLSLDQGE--
TC229602            -------------------------------------------------------------
TA46156             ------GHIAG--------------------GYQGT--------MFPLGLRLEQGK--
AK247217            ------SQFTGVGLGVGLG----------GGG-FHGHGG----GGSFPLGLSLEQGK--
```

FIGURE 2 (continued)

```
TA30646            ------GQFTGVGLGVGLG-----------GGG-FHGHGG----GASFPLGLSLEQGK--
TC8633             ------GQFSGMGLGVGLG----------GGGGFHHPSQ----SGSFPLGLSLEGGK--
TC15501            --------ITGSGGYHHHHQHQHH-----HQLSGGGGG--------FPLGLSLEQSGK-
TA1140             ------VSVHHQQQ-----------------QPQQQLQ----PQKFPLGLSLDNGRET
TA3490_part        ------VSVHRQQQ-----------------QQQ--------PVFPLGLSLDNGRET
CK293938           ------TSFTSAS-------------------AAGLQ----QPLFPLGLSLENGRDD
TC12771            ------TSFTSSSVA-----------------AAGLQ----QPLFPLGLSLDNGRDD
TC172581           ------TSFTSASAVSHLN------------SAAAAGLQ----QPLFPLGLSLDNGRDD
TA28621            ------TSFTSASAVSHLN------------SAAAAGLQ----QPLFPLGLSLDNGRDD
TA21665            ------ENSTSGVS------------------QLQQ----QPLFPLGLSLDNGFAD
TA46194            ------QLSSGDGSG----------------HVAGGGFQ----GPVFPLGLSLESGIPA
DY268946           MG--ISMGMMPLGLNLEHS------------FLRQHE------DHNSNHNNNNNTNA-
CO123623           -----GIGMMPLGLNLEHG------------FLR-HE------DGVVVDNNNNNASC-
Pt_scaff_70.65     AN--NNMGMMPLGLNLEHHG-----------FLRQQQ------DDGSSSLDTNNSNN-
Pt_scaff_XIII.403  AN--NNMGMMPLGLNLEHHG-----------FLRQQQ------DDSGSSLDTNNSNN-
TA1616             ----NNMGMMPLGLNLE--------------FLRQQ-------EDGSGALDNNNHTN-
TA55042            -------GLMPLGLNLEQAA-----------FLRHQVN----VDDDVVHVNVDDATIH
WO051050seqID77    -----MGVMMPLGLNLEQGG-----------LFRHED----VENSTSASSTTSAIN-
TC3698             --------TTGVTP-----------------------------MFSLGLSLDQQPK-
Os03g58330         -----------------------------------------PFSLALSLDAAAAA
TA139285           -----------------------------------------PFSLALSLD-AAAA
TabHLH11           -----------------------------------------PFSLALSLDAASDA
TC104646           -----------------------------------------PFPLALSLDAAAE-
TA126400           -----------------------------------------PFPLALSLDAAAE-
Os07g08440         -----------------------------------------PFPLALSLDAEGAG
Os02g35660         SNDSSGSEHSAAMPAGGGFLVGEQQQHVPPAAYAAGGVLPSMAAGEETPQSFGFGSLFNG
DT543504           ------------------------GLETLSPIPQLWHLQPYDSVSSLPTLVGQTIVD
Pt_scaff28.86      ------------------------NLQNLSSIPQLWPSQSYEGVSSLPPLMGQDRIE
GSVIVT00016367001  ------------------------NFQALPPIPQLWSQPSYGGGSSLSPVMGEYKMQ
TC19278            ----------------------------------------------------------
TA14134            ------------------------DINGAD-------------RSSLDSSAG-----
TC68930            ------------------------DLQGTL-------------LTSYTGPQG-----
TC140470           VSLEAFSFQGMQNAAMFNNASHSNGNLSVFDEATMASLHDSKEFLNG-SISSLGTGQQSQ
TC253044           ------------------------MGNLSVFDEATMASLHDSKEFLSG-SISSFGTAEQSQ
Os06g09370         VNLDAFGFQGAQNVAMLNKTSLPNGNPSLFDNAAIASLHDSKEFLNGGSIPSFGTVLQAL
```

```
AT2G24260          --------------------SSVHALYNGFSVAGSLPN----------FQIP
AT4G30980          --------------------HPNDALFNGFSTG-SLP--------FHLP
AT5G58010          --------------------PPQSLFHALAVDAPFPDQ------FHHP
TA12416            ----------------GDNDIVD-VSSFKS-QGGDGSVQALYNGFT-GSLHG----STQPQHFHHL
TA5414             ----------------GDNDIVD-ASSFKS-QQGGDGSVQALYNGFT-GSLHG----STQPQHFHHL
TC205173           DR--------------NDVVVDASTFESPNPSGKASVQTLYNGFA-GSLHGVGQSSNQTQHFQHP
GSVIVT00017237001  ----------------SQNDVVDGSSSFKSPNQGGDGSVQALYNGFA-GALHGSGQASNQAQNFHHP
TC7102             ----------------NMLGNGDQNDSVGNPANDSSVQALFNGFT-GSLGQ---NSSQPQHFLHP
TA34455            -L--------------PLNLSNNGDQNDGVANPANDNSVQALFNGFT-GSLGQ---TSNQPQHFHHP
TC7103             ----------------NGDQNDDVGNPENEISVQALYNGFA-GSLGQ---TSNQPQHFHHP
TA37666            ----------------GGDQNDDGLNPGNDISVQALYNGFA-GSLGQ---TSNQSQHFHHS
TC67603            ----------------GITTNHQGGEGSMQALYNVFGDGSLHG---TMQAKSFVGA
Os06g08500         P---------------REEMEGGFKSP--NAAAGGEHALFNGFGVHGGSG----GAGQ--PPF
Os02g55250         ----------------QDDIDAAFKSP--NGARG-DHALYNGFGAAGMHG----AAAMQPPPF
Os09g25040         ----------------GGDVQALLKAAAANSAGGDAGGVYGGGFAGSLH----QQQHFQPH
TA2544             TS--------------AIFGDRSRGEIDPP-FESSNPTEAEVLYNDGFTGSLP-----RAVQASLHQ
DV982110           ----------------EPGSREEIEASFKSVNNARDSNLGGLFQPFAVSPRGVR------PTGQNFHAQ
WO051050seqID516   ----------------ESRSRDEIEASFKSANHARDSNLGGLFPPFAASPRGVR------PTGQNFHTQ
AT4G02590          ----------------PGELRPEGG-HGSGKRFSDDVVDNRCSSMKP-------------
TC10015_part       ----------------QGFLRPEGGSLGTGKRFSDDV---------MKP-------
AT1G03040          ----------------HGFLKPDET----GKRFQDDVLDNRCSSMKP-------
TA13791            ----------------GGFLKPEEASG--SSKRFRNEVVDGRAFSVKN-------
TC60118            ----------------GGFLKPEEASG--SSKRFRNEVVDGRAFSVKN-------
TC60119            ----------------GGFLKPEEASG-SGKRFRNGVVDDRASSVKN-------
TC61833            ----------------GGFLKPEEGSGGGSSRRFRDEVVDGRASSVKN-------
TA3392             ----------------GGFLKPEEASG-SGKRFP------EEHAIKN-------
WO051050seqID1671  ----------------GGFLKPEEASG-SGKRYPEEVVDGRASTVKNLHSPLPNFPDV
Pt_TC63334         ----------------GGFLKPEEASG-SGKRFRDEIVDGRA---KN-------
TA3263             ----------------GGFLKPEEASG-SGKRFRDEIVDGRA---KN-------
Pt_scaff_II.416    ----------------GGFLKPEEASG-SGNRFRDDIVDGRV---RN-------
TA2825             ----------------GGFLKPEEASG-SGNRFRDDIVDGRV---RN-------
TC207545           ----------------GGFLKPEEASG-SGKRFRDDVVDGRANTVKN-------
TC229602           ----------------GPFLTPEDASG-SGKRFRDDAVDTRP---NN-------
TA46156            ----------------MKPDEASA-SGKRFRDDVVDNRAKH-------
AK247217           ----------------SSFLKPEDASG-SGKRFREEVIDGRASTVKN-------
                   ----------------GGFLKMDDV-SAPGRRFRDDVVDSRASSSVKP-------
```

**FIGURE 2 (continued)**

**FIGURE 2 (continued)**

```
TA30646             --------------------GGFLKMDDV-SAPGRRFRDDVVDSRASSSVKP----------
TC8633              -------------------SGFMKMDDVPAVPGRRFRDDVVDSRASSSVKP----------
TC15501            --------------------GGFFKPDEASG-SGKRFRDDFVDLKAVSSTNNDNNDRGDSVH
TA1140             IGGA-FAGQ-----QQQLQQQQQRERGGSMNMSGLFPQFENLQSHSLLHTVP--------
TA3490_part        IG-A-FAG----------QQQQRERGGSMNMTGLFPSFENLQSHSLLHSVP--------
CK293938          VS---DAG----------AYAVKHEREG-INIGNLYAGLEHLQSHAVRHAVPPVHHI---
TC12771           VS---DAG----------AYAVKHEREG-INIGNLYAGLEHLQSHAVRHAVPPVHHI---
TC172581          VG---DAG----------PYAVKHERDG-MNIGNLYAGLEHLQSHAVRHAVPPVHHI---
TA28621           VS---EAG----------AYAVKHERDG-MNIGNLYAGLEHLQSHAVRHSVPSVHHV---
TA21665           AN---NTG----------GFQVKTEREA-MNMGNLYPGLEHLQSHAVCLSVPQVHQV---
TA46194           TQVAPGSGERFRDDVDARGSSGRSERES-VHLDGLFPAYGHVQSLSVRPAVPQVH-----
DY268946          -------SSSSPNNSSATSGINERDSVHMPSLFPAFGQLQ--SQSAR--PPLPRPPQ---
CO123623          -------SAAS-----AVSGISERDSMHMVSLFPPFGQMQ--TQQIRASPPPPQPPPQ--
Pt_scaff_70.65    -------INNASPFSITSAGFLGRDSVHMTSLFPTFGQLQ--IHSSIRSAPPPLGPPQ--
Pt_scaff_XIII.403 -------INNTPP-SIKSARILGRDSVHMTSLFPTFGHLQ--IHSSIRPTPPPPGPPQ--
TA1616            ---------NNNNNVSSSTTSVINDRDSVHMASLFPTFGQLQ--TQT-LR--PPP--PPH--
TA55042           QHHLTLHNNNNNSSSPSSTAPITDRDSMHMRGLFSAFGQLHTPIRPTLPLPPPPRQPQLHL
WO051050seqID77   -------------------MEREAGVHHGNNMTS-CLFPAFGQFQ--THQSVQPPPPHPPPPQ--
TC3698            --------------------SDSSQSEREHPVQFACLFPQYGHNNQVHQTRPNLSPP-----
Os03g58330        EASGSGKRLGVGD--DAEGGGSKADRETVQLTGLFPPVFGGGG-VQPPNLRPT-------
TA139285          EASGSGK--------HADGG--KADREAIQLPGLFPPAFGGG--VQPPHLRAT-------
TabHLH11          SGSRGGIG-------DDAGN--AAERDGVQLPGLFPPVFGGG--LQPPHLRPSH------
TC104646          -------------------PKPDRDPVQLAGLFPPVFAGAGGVQQPHLRGP-------
TA126400          -------------------AKPDRDPVQLAGLFPPVFAGAGGVHQPHLRGP-------
Os07g08440        AARRLLDG--------GHDGGRTDRDPVQLAGLFAPVFGAAAGVQPPHLRAP-------
Os02g35660        ----DLLQEATVSKYHHHQQQQQLGVVPSSQPHHLNDDIDFNTGKLMSFASGQQHVTPSI
DT543504          DE----GNIN--------RFIEIDEILQPENLSASINSKGQQDMQNSFCSSFPADHP---
Pt_scaff28.86     GSGLRGGNLDDDMHIMGKGYMGMDEILRLDKLSASPTTEGKEDLQSCPFSSGIAEPN---
GSVIVT00016367001 GFGLQGEYVDNEMDIMRNRYVG-DEILQLDNISSAIPIKGKEEQHTHPFSPSAVGPH---
TC19278           ------------------------------------------------------------
TA14134           -------------------GPTNCLPGDSMGTLQASSRVSTMSVTPSSHMSPNLE---
TC68930           -------------------GQT-VLPR-TPGAQSHQQNLSTQTIQSGEGTSLQQY---
TC140470          LAGGGLKAEQQ-----EQNTMCNIPLPSFVSASQMAVSEAQGALIPSKTTSITHNNK---
TC253044          LAGSGLKAEQQ-----EQNAMCNIPLP-FASGSQMAVSEAQGAMIPSKISSTIHNNK---
Os06g09370        GAG-GLKAAQQ-----EQN-IRNIPLPTFTSGSHLAVTDAQGPPLPSKIPPLIHDHN---
```

EP 2 230 310 B1

FIGURE 2 (continued)

```
AT2G24260              QG-------------------SGGGLMNQQGQTQTQTQPQASASTATGGTV---------
AT4G30980              QG-------------------SGG---------QTQTQSQATASATTGGAT---------
AT5G58010              QE-------------------SGGPTMG----SQEGLQPQGTVSTTS-------------
TA12416                QGGS-----------------MPGQTFGAP--GPVMNQTQAQASGSTGGGGGGG------
TA5414                 QGGS-----------------MPGQTFGAP--GPVMNQTQAQASGSTGGGG---------
TC205173               QGSSN----------------PMQGQNFGAVPAGGGSATNQAPASGAAAGGA--------
GSVIVT00017237001      QGGS-----------------MQAQNYG-----------APAT----------------
TC7102                 QGGR-----------------MQAQSFGAPATAPAMNQTPAASG-SAGGG---T------
TA34455                QGGS-----------------MQSQSFG----APAMNQTPAASG-SAGGGGG-S------
TC7103                 QGGS-----------------MQAQSFGAPASS-TMNQTPAASGGSAGGG----------
TA37666                --------------------QAQSFGAPAASLTMNQTPAASG-SAGGA----------
TC67603                NSATE----------------MTQSQGSG-----PTAGEAVTAPE--------------
Os06g08500             GQ--------------------------------------------------------
Os02g55250             GQGG-----------------SMPAQSFGGGAAASGGGGGGGSASAAAAAGASS-G----
Os09g25040             PQTAP----------------TIPTQSFGGGGGGGGGGGTASGGGAAQPQAGAA-G----
TA2544                 QLLR-----------------QPQNYGAATLTG------QALAITATASGT-A------
DV982110               PGQ------------------VPLQGYGGMPQPQHQNPPPTGVG---------------
WO051050seqID516       SGQ------------------VPMQVYGGMPQPQHQNPSPTGVG---------------
AT4G02590              ----------------------------------VFHGQPMQQ--PPPSAPH-------
TC10015_part           ----------------------------------VFHGQPMQQQ-PAPAAPH-------
AT1G03040              ----------------------------------IFHGQPMSQ--PAPPMPH-------
TA13791                ----------------------------------VFHGQPVPA--TVAAGPH-------
TC60118                ----------------------------------VFHGQPVPA--TVAAGPH-------
TC60119                ----------------------------------VFHGQPMQA--TVSAAPH-------
TC61833                ----------------------------------VFHGSPMPA--TVAPSPH-------
TA3392                 ----------------------------------VFHGQPLPS--PVPAAPH-------
WO051050seqID1671      SQMASFRN---------------------------RDVFHGQPVPN--PVPAAPH-------
Pt_TC63334             ----------------------------------VFHGQPMPT--TVAIAPH-------
TA3263                 ----------------------------------VFHGQPMPT--TVAIAPH-------
Pt_scaff_II.416        ----------------------------------VFHGQPMPT--TVTAATH-------
TA2825                 ----------------------------------VFHGQPMPT--TMAAAPH-------
TC207545               ----------------------------------VFDGQPMPT--TVPTAPH-------
TC229602               ----------------------------------VFHGQPMPT--TMPAAPH-------
TA46156                ----------------------------------AFHGQPMQA--TVAAAPH-------
AK247217               ----------------------------------GFHGQPMPS------MPH-------
```

FIGURE 2 (continued)

EP 2 230 310 B1

```
TA30646               ----------------------------------GFHGQPMPS------MPH---------
TC8633                ----------------------------------GFHGQPMPS------MPH---------
TC15501               LNNLFPAFGHVQ--QQQQAHSVRPPSHQNQINQPFLGHPTPG--AVAVVPH---------
TA1140                -------------------------------QGFQGQPTSS--TAVTVPH---------
TA3490_part           -------------------------------QAFQGQSTTS--TAVTVPH---------
CK293938              -------------------------------QSFQGQPTTS--TTMTVPH---------
TC12771               -------------------------------QPFQGQPTTS--TTVTVPH---------
TC172581              -------------------------------QPFQGPPTTS--TTVTVPH---------
TA28621               -------------------------------QPFQGPPTTS--TTVTVPH---------
TA21665               -------------------------------QPFQGHPTSS--AIVTIPH---------
TA46194               -------------------------------QAFHGQPTPV--PITAAPH---------
DY268946              -------------------------------VQQFQSQP-------AAAPQ---------
CO123623              -----------------------------LHQPFHSQPTSGPV--AAAPH---------
Pt_scaff_70.65        -----------------------------IHQ-FNSQPTSGAV--SAVPQ---------
Pt_scaff_XIII.403     -----------------------------IHQ-INSHPNPGAV--SAVPQ---------
TA1616                -----------------------------LHQPFHGQPTPGVV--SAASQ---------
TA55042               H--------------------------------HHNQFQGQAAAAAPASMAAMPQ---------
WO051050seqID77       -----------------------------LHPAFLNQPAVG------SPN---------
TC3698                -----------------------------QVLHGQAMQS--SMAATPQ---------
Os03g58330            ----------------------------PPTQVFHPQQSKQGGAAVGP----------
TA139285              ----------------------------PPTQVFHAQQPKQGGAAVGP----------
TabHLH11              ----------------------------PPPQMFHAQQPKQGGPAGGP----------
TC104646              ----------------------------PPPQMFQAQ-PKPGEGGMAP----------
TA126400              ----------------------------PPPQMFQAQ-PKPGEGGMAP----------
Os07g08440            ----------------------------PPPQVFHAQ-PKPGEGAMAAPQP--------
Os02g35660            DSLQIDQKEFSSGLHHLNLSSLISGPLASFNATQSHRQPAEACGGKNGGAAPFVNLSEVL
DT543504              --------------------------------ITKTMIGLPSLVQGASPNLN--------
Pt_scaff28.86         --------------------------------VNMSMNQLSSMPQTTSAAPV--------
GSVIVT00016367001     --------------------------------MTMTASGLQSLPQTTVGTAS--------
TC19278               -----------------------------------------------------------
TA14134               ----------------------------GQAQNNDVQ----NSSSSVI--------
TC68930               ----------------------------GNHSAVSQL----QSGAGGG--------
TC140470              ----------------------------SEYPIPISHSADVQHKANSG--------
TC253044              ----------------------------SEYPVPISHSADAQNKANSA--------
Os06g09370            ----------------------------SEY--PINHSSDVEPQANSA--------
```

```
AT2G24260          -------------AAPPQSRTKIRARRGQATDPHSIAERLRRERIAERMKALQELVPNGN---KT
AT4G30980          -----------AQP-QTKPKVRARRGQATDPHSIAERLRRERIAERMKSLQELVPNGN---KT
AT5G58010          ----------APVVRQKPRVRARRGQATDPHSIAERLRRERIAERMKSLQELVPNTN---KT
TA12416            --------NTPAQQPKQRVRARRGQATDPHSIAERLRRERIAERMKALQELVPNAN---KT
TA5414             --------NTPAQQPKQRVRARRGQATDPHSIAERLRRERIAERMKALQELVPNAN---KT
TC205173           ---------PAQPRQRVRARRGQATDPHSIAERLRRERIAERMKALQELVPNAN---KT
GSVIVT00017237001  ----------RVRARRGQATDPHSIAERLRRERIAERMKALQELVPNAN---KT
TC7102             -------TPAAQPKQQRVRARRGQATDPHSIAERLRRERIAERMKALQELVPNAN---KT
TA34455            -------TPAAQPKQQRVRARRGQATDPHSIAERLRRERIAERMKALQELVPNAN---KT
TC7103             --------QPKQQKVRARRGQATDPHSIAERLRRERIAERMKSLQELVPNAN---KT
TA37666            --------QPKQQKVRARRGQATDPHSIAERLRRERIAERMKSLQELVPNAN---KT
TC67603            -------KPKQRVRARRGQATDPHSIAERLRRVRIAERMKALQELVPNAN---KT
Os06g08500         --------------LRRERIAERMKSLQELVPNAN---KT
Os02g55250         ------GGAAAPPRQRQRARRGQATDPHSIAERLRRERIAERMKALQELVPNAN---KT
Os09g25040         ------GGAPAPPRQRVRARRGQATDPHSIAERLRRERIAERMKALQELVPNANKLMQT
TA2544             -------GAGTAPPKPRVRARRGQATDPHSIAERLRRERIAERMKALQELVPNAN---KT
DV982110           -------AAPPVRPRVRARRGQATDPHSIAERLRRERIAERMKALQELVPNSN---KT
WO051050seqID516   ------AAPPVRPRVRARRGQATDPHSIAERLRRERIAERMKALQELVPNSN---KT
AT4G02590          ------QPTSIRPRVRARRGQATDPHSIAERLRRERIAERIRALQELVPTVN---KT
TC10015_part       ------QPTSIRPRVRARRGQATDPHSIAERLRRERIAERIRALQELVPTVN---KT
AT1G03040          ------QQSTIRPRVRARRGQATDPHSIAERLRRERIAERIRSLQELVPTVN---KT
TA13791            -------PP-AMRPRVRARRGQATDPHSIAERLRRERIAERIRALQELVPSVN---KT
TC60118            -------PP-AMHPRVRARRGQATDPHSIAERLRRERIAERIRALQELVPSVN---KT
TC60119            -------PP-TMRPRVRARRGQATDPHSIAERLRRERIAERIRALQELVPSVN---KT
TC61833            -------PP-TMRPRVRARRGQATDPHSIAERLRRERITERIRALQELVPSVN---KT
TA3392             -------PP-TMRPRVRARRGQATDPHSIAERLRRERIAERIRALQELVPSVN---KT
WO051050seqID1671  -----PP-AMRPRVRARRGQATDPHSIAERLRRERIAERIRALQELVPSVN---KT
Pt_TC63334         -------PP-AMRPRVRARRGQATDPHSIAERLRRERIAERIRQLQDLVPSVN---KT
TA3263             -------PP-AMRPRVRARRGQATDPHSIAERLRRERIAERIRALQELVPSVT---KT
Pt_scaff_II.416    -----PP-AMRPRVRARRGQATDPHSIAERLRRERIAERIRALQELVPSVN---KT
TA2825             -------PP-AMRPRVRARRGQATDPHSIAERLRRERIAERIRALQELVPSVN---KT
TC207545           -------PP-TMRPRVRARRGQATDPHSIAERLRRERIAERIRALQELVPSVN---KT
TC229602           -------PP-AVRPRVRARRGQATDPHSIAERLRRERIAERIRALQELVPSVN---KT
TA46156            -------PP-AIRPRVRAIRGQATDPHSIAERLRRERIAERIRALQELVPSVN---KT
AK247217           -------PP-AIRPRVRARRGQATDPHSIAERLRRERIAERIRALQELVPSVN---KT
```

**FIGURE 2  (continued)**

```
TA30646             ---------PP-AIRPRVRARRGQATDPHSIAERLRRERIAERIRALQELVPSVN---KT
TC8633              ---------PP-AIRPRVRARRGQATDPHSIAERLRRERIAERIRALQELVPNVN---KT
TC15501            ---------PP-AIRPRVRARRGQATDPHSIAERLRRERIAERMKALQELVPSSN---KT
TA1140             ---------PP-TIRPRVRARRGQATDPHSIAERLRRERIAERMRALQELVPSCN---KT
TA3490_part        ---------PP-SIRPRVRARRGQATDPHSIAERLRRERIAERMRALQELVPSCN---KT
CK293938           ---------SP-AIRPRVRARRGQATDPHSIAERLRRERISERIKALQELVPSCN---KT
TC12771            ---------PP-AIRPRVRARRGQATDPHSIAERLRRERISERIKALQELVPSCN---KT
TC172581           ---------PP-SIRPRVRARRGQATDPHSIAERLRRERISERIKALQELVPSCN---KT
TA28621            ---------PP-SIRPRVRARRGQATDPHSIAERLRRERISERIKALQELVPSCN---KT
TA21665            ---------QP-AIRPRVRAQRGQATDPHSIAERLRRERISERIKALQELVPSCN---KT
TA46194            ---------PP-AIRPKVRARRGQATDPHSIAERLRRERIAERMKALQELVPSSN---KT
DY268946           ---------PP-AVRPRVRARRGQATDPHSIAERLRRERIADRMRALQELVPSCN---KT
CO123623           ---------PP-AVRPRVRARRGQATDPHSIAERLRRERIAERMKALQELVPSCN---KT
Pt_scaff_70.65     ---------PP-GIRPRVRARRGQATDPHSIAERLRRVRITERVKALQELVPTCN---KT
Pt_scaff_XIII.403  ---------PP-GIRPRVRARRGQATDPHSIAERLRRVRITERVKALQELVPTCN---KT
TA1616             ---------PP-AIRPRVRARRGQATDPHSIAERLRRERIAERMKALQELVPTAN---KT
TA55042            ---------PP-GIRPRVRARRGQATDPHSIAERLRRERIAERMKALQELVPSIN---KT
WO051050seqID77    ---------QP-AIRPRARARRGQATDPHSIAERLRRERINERMKALQELVPSCN---KT
TC3698             ---------PT-GPRPRVRARRGQATDPHSIAERLRRERIAERIRALQELVPSTN---KT
Os03g58330         --------QPPAPRPKVRARRGQATDPHSIAERLRRERIAERMRALQELVPNTN---KT
TA139285           --------QPPAPRPKVRARRGQATDPHSIAERLRRERIAERMRALQELVPNTN---KT
TabHLH11           --------QPPAPRPKVRARRGQATDPHSIAERLRRERIAERMRALQELVPNTN---KT
TC104646           --------QPPAPRPKVRARRGQATDPHSIAERLRRERIAERMRALQELVPNTN---KT
TA126400           --------QPPAPRPKVRARRGQATDPHSIAERLRRERIAERMRALQELVPNTN---KT
Os07g08440         -------QQPPAPRPKVRARRGQATDPHSIAERLRRERIAERMRALQDLVPNTN---KT
Os02g35660         PKGNGSGSAGNGAPKPRVRARRGQATDPHSIAERLRREKISDRMKDLQELVPNSN---KT
DT543504           ------NGSDRTVKPRVRARRGQATDPHSIAERLRREKIAERMKNLQELVPNSN---KT
Pt_scaff28.86      -------EGCNGTGKTRVRARRGHATDPHSIAERLRREKIAERMKNLQELVPNSN---KV
GSVIVT00016367001  -------GGCNGTGKPRVRARRGQATDPHSIAERLRREKIAERMKNLQELVPNSN---KT
TC19278            ---------------------------------------------MKNLQELVPNSN---KT
TA14134            -------AGENTPYLLKELLVCSQATDPHSIAERERRERIAKNLKSLQELVPNAN---KT
TC68930            -------NAANGAARPRVRARRGQATDPHSIAERLRRERIAERMKSLQELVPNSN---KT
TC140470           ------NGNSASAKPRARARRGQATDPHSIAERLRREKISERMKNLQDLVPNSN---KA
TC253044           ------NGNSASAKPRARARRGQATDPHSIAERLRREKISERMKNLQDLVPNSN---KA
Os06g09370         ------PGNSANAKPRTRARRGQATDPHSIAERLRREKISERMKNLQVLVPNSN---KA
                                                   :: ** ***. .    :.
```

FIGURE 2 (continued)

```
AT2G24260          DKASMLDEIIDYVKFLQLQVK-----------VLSMSRLGGAASVSSQIS-----EAGGSH-
AT4G30980          DKASMLDEIIDYVKFLQLQVK-----------VLSMSRLGGAASASSQIS----EDAG---
AT5G58010          DKASMLDEIIEYVRFLQLQVK-----------VLSMSRLGGAGSVGPRLNGLSAEAGGRLN
TA12416            DKASMLDEIIDYVKFLQLQVK-----------VLSMSRLGGAAAVAPLVADMSSEG-GGGD
TA5414             DKAASMLDEIIDYVKFLQLQVK----------VLSMSRLGGAAAVAPLVADMSSEGAGGGD
TC205173           DKÄSMLDEIIDYVKFLQLQVK-----------VLSMSRLGGAAAVAPLVADMSSEG--GGD
GSVIVT00017237001  DKASMLDEIIDYVKFLQLQVK-----------VLSMSRLGGAAAVAPLVADMSSEG--GGD
TC7102             DKASMLDEIIDYVKFLQLQVK-----------VLSMSRLGGAAAVAPLVADMSSEG-RG--
TA34455            DKASMLDEIIDYVKFLQLQVK-----------VLSMSRLGGAAAVAPLVADMSSEG-RG--
TC7103             DKASMLDEIIDYVKFLQLQVK-----------VLSMSRLGGAAAVAPLVADRSSEG-GGGD
TA37666            DKASMLDEIIDYVRFLQLQVK-----------VLSMSRLGGAAAVAPLVADRSSEG-GG-D
TC67603            DKASMLDEIIDYVRFLQLQVK-----------VLSMSRLGGAAAVAPLVS----EG--GGD
Os06g08500         DKASMLDEIIDYVKFLQLQVK-----------VLSMSRLGGAGMAPLVASMSSE--GNSN
Os02g55250         DKASMLDEIIDYVKFLQLQVK-----------VLSMSRLGGASAVAPLVANMSSESNGNGN
Os09g25040         DKASMLDEIIDYVKFLQLQVKASTYTKLLIHVLSMSRLGGAAAVAPLVADMSSE--GRGG
TA2544             DKASMLDEIIDYLKFLQLQVK-----------VLSMSRLGGAAAVAPLVADMSSE------
DV982110           DKASMLDEIIDYVKFLQLQVK-----------VLSMSRLGGAGAVAPLVADIPAEG-----
W0051050seqID516   DKASMLDEIIDYVKFLQLQVK-----------VLSMSRLGGAGAVAPLVADIPAEG-----
AT4G02590          DRAAMIDEIVDYVKFLRLQVK-----------VLSMSRLGGAGAVAPLVT-DMPLSSSVED
TC10015_part       DRAAMIDEIVDYVKFLRLQVK-----------VLSMSRLGGAGAGAPLVT-DMPLSSSVED
AT1G03040          DRAAMIDEIVDYVKFLRLQVK-----------VLSMSRLGGAGAVAPLVT-EMPLSSSVED
TA13791            DRAAMLDEIVDYVKFLRLQVK-----------VLSMSRLGGAAGAVAPLVT-DLPLSS-VED
TC60118            DRAAMLDEIVDYVKFLRLQVK-----------VLSMSRLGGAAGAVAPLVT-DLPLSS-VED
TC60119            DRAVMLDEIVDYVKFLRLQVK-----------VLSMSRLGGAGAVAPLVT-DIPLSS-VEY
TC61833            DRAVMLDEIVDYVKFLRLQVK-----------VLSMSRLGGAGAVAPLVT-DIPLSS-VED
TA3392             DRAAMLDEIVDYVKFLRLQVK-----------VLSMSRVGAPGAVAPLVTTDLPLSS-VED
W0051050seqID1671  DRAAMLDEIVDYVKFLRLQVK-----------VLSMSRLGAAGAVAPLVT-DIPLSS-VEE
Pt_TC63334         DRATMLDEIVDYVKFLRLQVK-----------VLSMSRLGGAGAVAPLVT-DIPLSS-VED
TA3263             DRATMLDEIVDYVKFLRLQVK-----------VLSMSRLGGAGAVAPLVT-DIPLSS-VED
Pt_scaff_II.416    DRAAMLDEIVDYVKFLRLQVK-----------VLSMSRLGGAGAVAPLVT-DIPLSS-VED
TA2825             DRAAMLDEIVDYVKFLRLQVK-----------ILSMSRLGGAGAVAPLVT-DIPLSP-VED
TC207545           DRAAMLDEIVDYVKFLRLQVK-----------VLSMSRLGGAGAVAPLVT-DIPLSS-VED
TC229602           DRAAMLDEIVDYVKFLRLQVK-----------VLSMSRLGGAGAVAPLVT-DIPLSS-VEE
TA46156            DRAAMLDEIVDYVKFLRLQVK-----------VLSMSRLGGAGAVAPLVT-DIPLAS-VEE
AK247217           DRAVMLDEIVDYIKFLRLQVK-----------VLSMSRLGGAGAVAPLVT-DIPISS-VEE
```

**FIGURE 2 (continued)**

**FIGURE 2 (continued)**

```
TA30646               DRAVMLDEIVDYVKFLRLQVK----------VLSMSRLGGAGAVAPLVT-DIPISS-VEE
TC8633                DRAIMLDEIVDYVKFLRLQVK----------VLSMSRLGGAAAVAPLVT-EIPISS-VEE
TC15501               DRAAMLDEIVDYVKFLRLQVK----------VLSMSRLGGAGAVAQLVA-DIPLSS-AEG
TA1140                DKAAMLDEILDYVKFLRLQVK----------VLSMSRLGGAGAVAQLVS-DVPLQS-VEG
TA3490_part           DKAAMLDEILDYVKFLRLQVK----------VLSMSRLGGAGAVAQLVS-DVPLQS-VEG
CK293938              DRAAMLDEILDYVKFLRLQVK----------VLSMSRLGGASAVAQLVA-DIPLQS-MEG
TC12771               DRAAMLDEILDYVKFLRLQVK----------VLSMSRLGGASAVAQLVA-DIPLQS-VEG
TC172581              DRAAMLDEILDYVKFLRLQVK----------VLSMSRLGGASAVAQLVA-DIPLNL-WEG
TA28621               DRAAMLDEILDYVKFLRLQVK----------VLSMSRLGGASAVAQLVA-DIPLQS-VEG
TA21665               DRAAMLDEILDYVKFLRLQVK----------VLSMSRLGGTSAAAQVVA-DIPLQS-VEG
TA46194               DRAAMLDEIVDYVKFLRLQVK----------VLSMSRLGGAGAVAQLVA-DIPLPA-VEG
DY268946              DRAAMLDEIVDYVKFLRLQVK----------VLSMSRLGAAGAVAQLVA-DVPLSSALE-
CO123623              DRAAMLDEIVDYVKFLRLQVK----------VLSMSRLGAAGAVAQLVA-DVPLLS-VE-
Pt_scaff_70.65        DRAAMLDEIVDYVKFLRLQVK----------VLSMSRLGAAGAVAQLVA-DVPLSSVQ--
Pt_scaff_XIII.403     DRAAMLDEIVDYVKFLRLQIK----------VLSMSRLGAAGAVAQLVA-DVPLSSVQIK
TA1616                DRAAMIDEIVDYVKFLRLQVK----------VLSMSRLGAAGAVAQLVA-DVPLASVE--
TA55042               DRAAMLDEIVDYVKFLRLQVK----------VLSMSRLGGAGAVAQLVA-DVPLSAVEG-
WO051050seqID77       DRAAMLDEIVDYVKFLRLQVK----------VLSMSRLGGAGAVAQLVA-DVPLSSAEG-
TC3698                DRAVMLDEIVEYVKFLRLQVK----------VLSMSRLGGAGAVAQLVA-DIPMSSSVEG
Os03g58330            DRAAMLDEILDYVKFLRLQVK----------VLSMSRLGGAGAVAQLVA-DIPLSV--KG
TA139285              DRAAMLDEILDYVKFLRLQVK----------VLSMSRLGGAGAVAQLVA-DIPLSV--KG
TabHLH11              DRAVMLDEILDYVKFLRLQVK----------VLSMSRLGGAGAVAQLVS-DIPLSV--KG
TC104646              DRAAMLDEILDYVKFLRLQVK----------VLSMSRLGGAGAVAQLVA-DIPLSV--KG
TA126400              DRAAMLDEILDYVKFLRLQVK----------VLSMSRLGGAGAVAQLVA-DIPLSV--KG
Os07g08440            DRAAMLDEILDYVKFLRLQVK----------VLSMSRLGGAGAVAQLVA-DIPISV--KG
Os02g35660            NKASMLDEIIDYVKFLQLQVK----------VLSMSRLGAAEAVVPLLTETQTESPGFLL
DT543504              DKASILDEIIGYVKFLQLQVK----------VLSMSRLGAAAAVVPLITDGRAEVSN--G
Pt_scaff28.86         DKASMLDEIIEYVKFLQLQVK----------VLSMSRLGAAGAVIPLLTDGQPEGHN--S
GSVIVT00016367001     DKASMLDEIIEYVKFLQLQVK----------VLSMSRLGAAEAVVPLITDGQAEGSK--G
TC19278               DKASMLDEIIEYVRFLQLQVK----------VLSMSRLGATGAVVPLITENQPEGST--S
TA14134               DKASMLDEIIDYVKFLQLQVK----------VLSMSRLGGAGAVAPLIADVPAEGSG--S
TC68930               DKASMLDEIIEYVKFLQLQVK----------VLSMSRLGGAGAVAPLIADVPSQGSG--G
TC140470              DKSSMLDEIIDYVKFLQLQVK----------VLSMSRLGAPGAVLPLLAESQTEGRSNSP
TC253044              DKSSMLDEIIDYVKFLQLQVK----------VLSMSRLGAPGAVLPLLAESQTEGRSNSP
Os06g09370            DKASMLDEIIDYVKFLQLQVK----------VLSMSRLGAPGAVLPLLRESQTECHSNPS
                      ::: ::***: *::**:**:*          :*****:*.. .     :
```

EP 2 230 310 B1

```
AT2G24260          --GNASSAMVGGS-----------QTAGNSNDSVTMTEHQVAKLMEEDMGSAMQYLQGK
AT4G30980          -----------GS-----------HENTSSSGEAKMTEHQVAKLMEEDMGSAMQYLQGK
AT5G58010          ALTAPCNGLNGNG-----------NATGSSNESLRSTEQRVAKLMEEDMGSAMQYLQGK
TA12416            CIQANGR-NPNG------------AQTTSANDSLTVTEHQVAKLMEEDMGSAMQYLQGK
TA5414             CIQANGR-NPNG------------AQTTSANDSLTVTEHQVAKLMEEDMGSAMQYLQGK
TC205173           CIQANGKSNGGGAQASTTNTNTNQTTATTTSNDSLTMTEHQVAKLMEEDMGSAMQYLQGK
GSVIVT00017237001  CIQASGTSGPTGGRATNG-------TQTTTSNDSLTVTEHQVAKLMEEDMGSAMQYLQGK
TC7102             --EGNGGRGG-NG-----------TASSSNN-DSMTVTEHQVAKLMEEDMGSAMQYLQGK
TA34455            --EGNVGRGG-NG-----------TAASSSNKETMTVTEHQVAKLMEEDMGSAMQYLQGK
TC7103             CAQANGGGRGSNG-----------TTSLANNDS-MTMTEHQVAKLMEEDMGSAMQYLQGK
TA37666            CVQGNGGRGGSNG-----------TTSSANNDSSMTMTEHQVAKLMEEDMGSAMQYLQGK
TC67603            CIQTSANGGSHPRNSN---.-----GDQTPSANDRLTMTEHQVAKLMEEDMGSAMQYLQEK
Os06g08500         GSSNGGGGKASKGGTGGEGGGGGGGGGGGGTGGGMRVTEQQVAKMMEEDMGTAMQYLQGK
Os02g55250         ATSSSGNGEAANGSSNGDNNGGG---------TLRVTEQQVAKLMEEDMGSAMQYLQGK
Os09g25040         GAANGGAPAAAGSDS-----------------LTVTEQQVAKLMEEDMGTAMQYLQGK
TA2544             --SGGGAGTPRSNNDG----------------LTAAEQQVAKLLEEDMGSAMQYLQGK
DV982110           --ANAPQGTRTNG-----------SQNSSPDGLALTERQVAKLMEEDMGTAMQYLQGK
WO051050seqID516   --ANAPQGTRTNS-----------SQNSSPDGLALTERQVAKLMEEDMGSAMQYLQGK
AT4G02590          ETGEGGRTP---------------QPAWEKWSNDGTERQVAKLMEENVGAAMQLLQSK
TC10015_part       ESGEGGRAP---------------QPAWEKWSNDGTERQVAKLMEENVGAAMQLLSSK
AT1G03040          ET----------------------QAVWEKWSNDGTERQVAKLMEENVGAAMQLLQSK
TA13791            ESGEGGRS----------------QPAWEKWSNDGTERQVAKLMEEDVGAAMQFLQSK
TC60118            ESGEGGRS----------------QPAWEKWSNDGTERQVAKLMEEDVGAAMQFLQSK
TC60119            ESGEGGRS----------------QPAWEKWSNDGTEQQVAKLMEENVGAAMQFLQSK
TC61833            ESGDGGRN----------------QPAWEKWSNDGTERQVAKLMEENVGAAMQFLKSK
TA3392             ESGEGVRN----------------QPAWEKWSNDGTERQVAKLMEENVGAAMQFLQSK
WO051050seqID1671  EGGEGGRN----------------QPAWEKWSNDGTERQVAKLMEENVGAAMQFLQSK
Pt_TC63334         ETGEGGRN----------------QPAWEKWSNDGTERQVAKLMEENVGAAMQFLQSK
TA3263             ETGEGGRN----------------QPAWEKWSNDGTERQVAKLMEENVGAAMQFLQSK
Pt_scaff_II.416    ETGEGGRN----------------QLAWEKWSNDGTERQVAKLMEENVGAAMQFLQSK
TA2825             ETGEGGRN----------------QPAWEKWSNDGTERQVAKLMEENVGAAMQFLQSK
TC207545           EGGEG-RN----------------QPAWEKWSNDGTERQVAKLMEENVGAAMQFLQSK
TC229602           EGGEGARN----------------RPAWDKWSNDGTERQVAKLMEENVGAAMQFLQSK
TA46156            EASEGGRN----------------EPAWEKWSNDGTERQVAKLMEENVGAAMQFLQSK
AK247217           ESSEGGNNN---------------QPAWEKWSSDGTERQVAKLMEENVGAAMQFLQSK
```

FIGURE 2 (continued)

**FIGURE 2 (continued)**

```
TA30646            ESSEGGNNN----------------QPAWEKWSSDGTERQVAKLMEENVGAAMQFLQSK
TC8633             EISEGGNN-----------------QPAWEKWSSDGTERQVAKLMEENVGSAMQFLQSK
TC15501           GASEGGSN-----------------QPAWEKWSTDGTEHQVAKLMEEDVGAAMQFLQSK
TA1140            DGSENGYNNQQ-Q------------GQAAWENWSNDDTEREVAKLMEEDVGAAMQFLQSK
TA3490_part      DTSENGYN-----------------QPAWENWSNDDTEREVAKLMEEDVGAAMQFLQSK
CK293938         DSAESKSN-----------------QHIWEKWSNVDTEQEVAKLMEEDVGAAMQYLQSK
TC12771          DSAESRSN-----------------QHIWEKWSNVDTEQEVAKLMEEDVGAAMQYLQSK
TC172581         DSGESRSN-----------------QHIWDKWSNVDTEREVAKLMEEDVGAAMQYLQSK
TA28621          DSGESRSN-----------------QHIWDKWSNVDTEQEVAKLMEEDVGAAMQYLQSK
TA21665          DTCESHSN-----------------QHVWEKWSDSETEQEVAKLMEEDVGTAMQYLQSK
TA46194          ETGEGGSN-----------------QQAWDKWSNDGTEREVAKLMEEDVGAAMQFLQSK
DY268946         GESIDGG-----S------------SQPEWEKWSNDGTEQQVAKLMEEDIGAAMQFLQSK
CO123623         GDGAGGG------------------TQPPWEKWSNDGTEQQVAKLMEEDIGAAMQFLQSQ
Pt_scaff_70.65   GEGIEGG-----A------------NQQAWENWSNDGTEQEVAKLMEEDVGAAMQLLQSK
Pt_scaff_XIII.403 GEGNEGG-----A------------NQQSWENWSNDDTEQEVAKLMEEDVGAAMQFLQSK
TA1616           GESIDGAA----A------------NQQTWEKWSNDGTEQQVAKLMEEDIGAAMQFLQSK
TA55042          DQDIEGG-----A------------NEQAWDKWSNDGTEQQVAKLMEEDVGAAMQFLQSK
WO051050seqID77  -EIIEGG-----N-----------NQPAWEKWLTDGTEQQVAKLMEEDVGAAMQFLQSK
TC3698           DSSESGKTS----------------YQGWEKWSTDGTERQVAKLMEEDVGAAMQFLQSK
Os03g58330       EASDSG------G------------NQQIWEKWSTDGTERQVAKLMEEDIGAAMQFLQSK
TA139285         EASDSG------S------------KQQIWEKWSTDGTERQVAKLMEEDIGAAMQFLQSK
TabHLH11         EASDGG------S------------KQQIWEKWSTDGTEKQVAKLMDEDIGAAMQFLQSK
TC104646         EASDSG------S------------TQHIWEKWSTDGTEKQVAKLMEEDIGAAMQFLQSK
TA126400         EAGDGGGAPQQQQ------------QQHVWEKWSTDGTEKQVAKLMEEDIGAAMQFLQSK
Os07g08440       EASDSG------S------------KQQIWEKWSTDGTEKQVAKLMEEDIGAAMQFLQSK
Os02g35660       SPRSSSGERQAGAGAVTG----GLPGDQPELLDGGAMFEQEVVKLMEDNMTTAMQYLQSK
DT543504         LSLAPLAG-----------------QGVDFSPSPDQVVFEQEVVKLMESNMTMAMQYLQSK
Pt_scaff28.86    LSLSPSAG-----------------LGIDISPSADQIAFEQEVLKLLESDVTMAMQYLQSK
GSVIVT00016367001 LSLSPSAG-----------------QAEDICQSPDQIAFEQEVVKLMESNVTMAMQYLQSK
TC19278          HFLSQSAG-----------------RDVKHIESPNTLTFEQEVVKLMESNMTKAMQFLQRK
TA14134          LASAALGQ--------------AGG--SLSQDGLAFEQNVVKLMEKDMTAAMQYLQNK
TC68930          VMSTALGQ--------------ASGPLTLSQDGLAFEQEVARLMESNMTSAMQYLQNK
TC140470         LSSPTTSQ-----------------GLLDVAGPEDSLVFEQEVIKLMETSITNAMQYLQNK
TC253044         LSSPTASQ-----------------GLLDAAGPEDSLVFEQEVIKLMETSITNAMQYLQNK
Os06g09370       LSASTISQ-----------------GPPDMPDSEDSSAFEQEVVKLMETSIISAMQYLQNK
                                             *:.*  ::::  .:   *** *. :
```

```
AT2G24260          GLCLMPISLATAISTATCHSRNPLIPG--------------------------------AVADVG
AT4G30980          GLCLMPISLATTISTATCPSRSPFVK-----------------------------------DTG
AT5G58010          GLCLMPISLATAISSSTTHSRGSLFN--------------------------------PISSAV
TA12416            GLCLMPISLATAISTATCHSRNPIISTSNNNNNN-----GNPH-------HNPLLQSNGE
TA5414             GLCLMPISLATAISSATCHSRNPIISTSNNNNNN-----GNPH-------HNPLFQSNGE
TC205173           GLCLMPISLATAISTATCPTRNVVNPLINAAA------GATH-------FPTAANPAGE
GSVIVT00017237001  GLCLMPISLATAISTTTCHSRNPMVAAAAVAASNI-NNGSHT-------HPLLPNSNAD
TC7102             GLCLMPISLATAISTATCHSRNPMIPNGNGGNNPLLGGGGGL-------ATNGGGGEAG
TA34455            GLCLMPISLATAISTS---TRIPLLSP---------------------------------EAG
TC7103             GLCLMPISLATAISTATCHSMKLNNPLLHGGGSG-----GSA-------AINGGG--G
TA37666            GLSLMPISLATAISTSTCHSMKPNNPLLLGGGS----------------AINGGGETGG
TC67603            GLCLMPISLATAISSATSRSRNPMINHENPANGS---------------HLLIQSSGGD
Os06g08500         GLCLMPISLASAISSATSSA------SLLSRPSI---------------RHAGAPPQTM
Os02g55250         GLCLMPISLATAISSATSS-------SLLPRTGG---------------GAGGSLHEGG
Os09g25040         GLCLMPISLASAISSATCHLRPPVVAAAQQFPAG---------------LGAAAAAHH
TA2544             GLCLMPISLASAISSATCRP------RPPPG------------------VNLRYPAAGD
DV982110           GLCLMPISLASAINNSGSRPQAPSTPSXXLLAS----------------NVNDRQVL
WO051050seqID516   GLCLMPISLASAINNSGARPQAPSTPSLQGLLPP---------------NANDRQIL
AT4G02590          ALCMMPISLAMAIYHSQPPDTSS--------------------------------------VV
TC10015_part       ALCMMPISLAMAIYHSQPPDTT--------------------------------------
AT1G03040          ALCIMPISLAMAIYHSQPPDTSSS------------------------------------IV
TA13791            ALCVMPISLATAIYHTQSPDTSS--------------------------------------VV
TC60118            ALCVMPISLATAIYHTQSPDTSS--------------------------------------VV
TC60119            SLCIMPISLATAIYHTQVPDTSS--------------------------------------VV
TC61833            ALCIMPISLATAIYHSQPLDTSS--------------------------------------IV
TA3392             ALCIMPISLATAIYHSQPPE---------------------------------------
WO051050seqID1671  ALCIMPISLATAIYQTQPPDTSS--------------------------------------LV
Pt_TC63334         ALCIMPISLATAIYHTQPPDTTT-------------------------------------IV
TA3263             ALCIMPISLATAIYHTQPPDTTS-------------------------------------IV
Pt_scaff_II.416    ALCIMPITLATAIYHTQPPDTTT-------------------------------------IV
TA2825             ALCIMPISLATAIYHTQAPDTST-------------------------------------IV
TC207545           ALCIMPVSLASAIYQSQPSGTSS-------------------------------------IV
TC229602           ALCIMPISLASAIYQSQPPDTSS-------------------------------------IV
TA46156            ALCIMPISLASAIYHSQQPDTTP-------------------------------------LI
AK247217           ALCIMPISLASAIYHSQPPDTSS-------------------------------------LV
```

FIGURE 2   (continued)

**FIGURE 2 (continued)**

```
TA30646           ALCIMPISLASAIYHSQPPDTSS---------------------------------------LV
TC8633            ALCIMPISLASAIYHSQPPDTSS---------------------------------------LI
TC15501           ALCIMPISLASAIYHDQPPDAST---------------------------------------MI
TA1140            ALCIMPISLASLIYPNHQPDVKP---------------------------------------EP
TA3490_part       ALCIMPISLASLIYPPQTPDSNS---------------------------------------HV
CK293938          SLCIMPISLAALIYPTQQPDDMS---------------------------------------MV
TC12771           SLCIMPISLAALIYPTQQPDDPS---------------------------------------MV
TC172581          SLCIMPISLAALIYPTQQPDDQS---------------------------------------LV
TA28621           SLCIMPISLAALIYPTQQPDDQS---------------------------------------LV
TA21665           SLCIMPISLAALIYPTQQSDNQS---------------------------------------MV
TA46194           ALCIMPISLAAAIYPAHQTDTPT---------------------------------------LI
DY268946          ALCIMPISLASAIYRTRQPDAPA---------------------------------------FV
CO123623          ALCIVPISLASAIFPAHQPDAPT---------------------------------------IV
Pt_scaff_70.65    ALCIMPVSLASAIFRARPPNAPT---------------------------------------LV
Pt_scaff_XIII.403 ALCIMPISLASAIFRARPPNAST---------------------------------------LI
TA1616            ALCIMPISLASAILRTHPPDAPS---------------------------------------II
TA55042           ALCIMPISLASAIFRMPQSEAST---------------------------------------GI
WO051050seqID77   TLCIMPISLASAIFRTSQPDMPR---------------------------------------SI
TC3698            ALCIMPISLAAAICQTNPSTEISF--------------------------------------M-
Os03g58330        ALCMMPISLAMAIYDTQQTQDGQ---------------------------------------PV
TA139285          ALCMMPISLAMAIYDTQHSQDGQ---------------------------------------PV
TabHLH11          ALCMMPVSLAMAIYDTQHSQDGQ---------------------------------------PV
TC104646          ALCMMPISLAMAIYDTQHSQDGH-S-------------------------------------LM
TA126400          ALCMMPVSLAMAIYDTQHPLDGHGH-------------------------------------SL
Os07g08440        ALCMMPISLAMAIYDTQHSQDGH---------------------------------------SV
Os02g35660        GLCLMPVALASAISAQKGTSSAAVRPEKKKNGDG-----------------DGGGDEEDV
DT543504          GFCLMPVALAAAISNVK-------SSTS---------------------------------S
Pt_scaff28.86     GLCLMPIALAAAISSVK-------ASLSGTTSEERKNNGYT------------SGLVS
GSVIVT00016367001 GLCLMPIALATAISSGK-------AASSGTGSDEGK---------------------N
TC19278           GLCLMPIALAAAISGSK-------APPASLSTEGKKSILTNGFVHHNSGQSNIGPGQIS
TA14134           GLCLMPIALATAISSSTGKPLLGSGVTNSVDSTSDRQSSGNQPASLTVDSCSGALGMGSA
TC68930           GLCLMSIALATAISSSSGKPVLATVPGTGVDS-SEKQNSDMQSIVLPFSSSSTTMGLRAT
TC140470          GLCLMPIALASAISNQKG--------------------------------------------T
TC253044          GLCLMPIALASAISNQKG--------------------------------------------T
Os06g09370        GLCLMPIALASAISNQKGM-------------------------------------------A
                  :.::.::**  *
```

```
AT2G24260          GPSPPNLSGMTIQ--STSTKMG------SGNGKLNGNGVTERSSSIAVKEAVSVSKAATG
AT4G30980          VPLSPNLS------TTIVA---NGNG------SSLVTVKDAPSVSKP---
AT5G58010          AAEDSNVT------ATAVA---APEAS------STMDDVSASKA---
TA12416            GPTSPSMS------VLTVQS---ATMGNG------GADGSVKDAASVSKP---
TA5414             GPTSPSMS------VLTVQS---ATMGNG------GADGSVKDAASVSKP---
TC205173           GPSSPSMS------VLTVQS---AIAGN------DGAASVSKP---
GSVIVT00017237001  GPSSPSMS------VLTVQS---ATMGNG------LADAPVKDAASVSKP---
TC7102             GPSSPKLS------VLTVQS---ATIGNG------GIDPSVKDATSVFEA---
TA34455            RPASPTLS------ALTVQS---ATIGN------AAGKDATSLFEA---
TC7103             GPSSPSLS------ASTVQS---ATMGN------GGA---
TA37666            GPSSPTLS------ASTVQS---ATMGN------GGT---
TC67603            GPSSPSMS------VLTVQS---ATMGNG------GLEG---GAASVSKP---
Os06g08500         LDAAGPTS------PAAMS---NGDDP------RHAKADGGAGGTQ---
Os02g55250         NGTSPPLV------NGTAT---GCDDAGVFFSVKFVVELSFLLNEDCRGKEESK
Os09g25040         HQLSAAAA------AAAMRGHLPGLNADGSVPASPSMSVLTAQSAMANGGGAADGEG
TA2544             APPSPSIS------ALTVQ------SSNAGSAGADAS-
DV982110           EPS---------------------------
WO051050seqID516   EPSNSALSALTSTSMTIQSSISSPGSGITEQGDNAHKAVNGTRNPKDSREANSVTKSNGI
AT4G02590          KPENNPPQ---------------------
TC10015_part       --------------------------
AT1G03040          KPEMNPPP---------------------
TA13791            KPETNPPS---------------------
TC60118            KPETNPPS---------------------
TC60119            KPETNPPA---------------------
TC61833            KPETDPPP---------------------
TA3392             --------------------------
WO051050seqID1671  KSESNPPS---------------------
Pt_TC63334         KPETNPPS---------------------
TA3263             KPETNPQS---------------------
Pt_scaff_II.416    KPETNPPS---------------------
TA2825             KPETNPPS---------------------
TC207545           KPETNPPS---------------------
TC229602           KPETNPPS---------------------
TA46156            KPQTNPPS---------------------
AK247217           KPETNPPS---------------------
```

**FIGURE 2   (continued)**

**FIGURE 2 (continued)**

```
TA30646            KPETNPPS---------------------------------------------
TC8633             KPETNPPS---------------------------------------------
TC15501            KPEPNAPS---------------------------------------------
TA1140             SAPS-------------------------------------------------
TA3490_part        KPETV------------------------------------------------
CK293938           KPEPAAPS---------------------------------------------
TC12771            KPEPAAPS---------------------------------------------
TC172581           KPEAAAPS---------------------------------------------
TA28621            KPEAAAPS---------------------------------------------
TA21665            KPEQAAPL---------------------------------------------
TA46194            KPEPNAPS---------------------------------------------
DY268946           KPESSTPSYCLNNNWSQQLAHNDSKYLITGTYSYRILLVLLSNQIIQIPMQRSHSSFP--
CO123623           ----------------------------------------------------
Pt_scaff_70.65     KPESNPPS---------------------------------------------
Pt_scaff_XIII.403  NTESNTPS---------------------------------------------
TA1616             KPESNTPS---------------------------------------------
TA55042            KPESNSH----------------------------------------------
WO051050seqID77    KPESSAPS---------------------------------------------
TC3698             -----------------------------------------------------
Os03g58330         KHEPNTPS---------------------------------------------
TA139285           KPEPNTPS---------------------------------------------
TabHLH11           KPEPNNTA---------------------------------------------
TC104646           KPEPNTSS---------------------------------------------
TA126400           KPEPNASS---------------------------------------------
Os07g08440         KPEPNTPS---------------------------------------------
Os02g35660         KGEFDAPRRPPVGRPKEMRSRV-------------------------------
DT543504           SSSSVPASD---------------ESKKLGLH--QQYSNQQHLQQ---------------
Pt_scaff28.86      SSSSITGIDTHPMSNDNNIATGTLSSKGMIVNGCNEVVKQEVLKNT-------------
GSVIVT00016367001  AATPVAATP-------------VATAYLALG---HIIHLLMAMF--------------
TC19278            SDGETRSEENIIGIHSREDFSSNSCSGTSIKKEGTNTTNFTREMNQE-------------
TA14134            GFGSDFLLKENTVEKRGRELVPTAESNGAEFGIISSLPKLRKKE---------------
TC68930            ASGSDAPINENSINKASIEKVVAEKSNGISPGLSSDVPKVGSQSREELLKRAQ------
TC140470           SAAAIPPER--------------------------------------------
TC253044           SAAAIPPER--------------------------------------------
Os06g09370         AAAAIPPEK--------------------------------------------
```

FIGURE 2. (continued)

| | |
|---|---|
| AT2G24260 | MQFDESDARVWIWFEIRREDDIVELLWQSGQVVGTNQTHRQSYDPPPILRGSGSGRGEEN |
| AT4G30980 | ------------------------------------------------------------ |
| AT5G58010 | ------------------------------------------------------------ |
| TA12416 | ------------------------------------------------------------ |
| TA5414 | ------------------------------------------------------------ |
| TC205173 | ------------------------------------------------------------ |
| GSVIVT00017237001 | ------------------------------------------------------------ |
| TC7102 | ------------------------------------------------------------ |
| TA34455 | ------------------------------------------------------------ |
| TC7103 | ------------------------------------------------------------ |
| TA37666 | ------------------------------------------------------------ |
| TC67603 | ------------------------------------------------------------ |
| Os06g08500 | ------------------------------------------------------------ |
| Os02g55250 | LLVQKGP----------------------------------------------------- |
| Os09g25040 | SQLKDAASVSKP------------------------------------------------ |
| TA2544 | ------------------------------------------------------------ |
| DV982110 | ------------------------------------------------------------ |
| WO051050seqID516 | GGPALSKNAVKGEDEPQRRTGQ-------------------------------------- |
| AT4G02590 | ------------------------------------------------------------ |
| TC10015_part | ------------------------------------------------------------ |
| AT1G03040 | ------------------------------------------------------------ |
| TA13791 | ------------------------------------------------------------ |
| TC60118 | ------------------------------------------------------------ |
| TC60119 | ------------------------------------------------------------ |
| TC61833 | ------------------------------------------------------------ |
| TA3392 | ------------------------------------------------------------ |
| WO051050seqID1671 | ------------------------------------------------------------ |
| Pt_TC63334 | ------------------------------------------------------------ |
| TA3263 | ------------------------------------------------------------ |
| Pt_scaff_II.416 | ------------------------------------------------------------ |
| TA2825 | ------------------------------------------------------------ |
| TC207545 | ------------------------------------------------------------ |
| TC229602 | ------------------------------------------------------------ |
| TA46156 | ------------------------------------------------------------ |
| AK247217 | ------------------------------------------------------------ |

TA30646
TC8633
TC15501
TA1140
TA3490_part
CK293938
TC12771
TC172581
TA28621
TA21665
TA46194
DY268946
CO123623
Pt_scaff_70.65
Pt_scaff_XIII.403
TA1616
TA55042
WO051050seqID77
TC3698
Os03g58330
TA139285
TabHLH11
TC104646
TA126400
Os07g08440
Os02g35660
DT543504
Pt_scaff28.86
GSVIVT00016367001
TC19278
TA14134
TC68930
TC140470
TC253044
Os06g09370

FIGURE 2   (continued)

FIGURE 3

**FIGURE 4**

**SEQ ID NO: 244, TabHLH11 encoding sequence, Triticum aestivum**
ATGGCGGGGCAGCCGCCGCAGGGCCCGGAGGACGACTTCCTCGACCAGTTCTTCTCCCTCACCAAC
TCCCTCTCCGCCGCCGGCCGCCCCTCCGGCGACCAGCCCTTCTCCCTCGCCCTCAGCCTCGACGCC
GCCTCGGACGCCTCCGGCAGCAGGGGCGGCATCGGCGACGACGCCGGCAACGCCGCGGAGCGGGAC
GGCGTGCAGCTCCCCGGCCTCTTCCCGCCGGTGTTCGGCGGTGGCCTCCAGCCGCCGCACCTTCGC
CCCAGCCACCCTCCCCCACAGATGTTCCACGCGCAGCAGCCGAAGCAGGGCGGGCCGGCTGGAGGG
CCGCAGCCGCCGGCGCCGAGGCCGAAGGTGCGGGCGCGTCGCGGTCAGGCGACTGATCCCCACAGC
ATCGCGGAGAGGCTAAGAAGAGAGAGGATAGCGGAAAGGATGAGGGCCCTACAGGAATTGGTCCCC
AATACGAACAAGACAGATAGGGCAGTTATGCTAGATGAGATCCTGGATTATGTGAAGTTCCTTAGG
CTTCAAGTAAAGGTGTTAAGCATGAGCAGATTGGGTGGTGCTGGTGCTGTTGCACAGCTGGTTTCT
GACATTCCACTTTCAGTTAAGGGGGAAGCAAGCGATGGTGGGAGCAAACAGCAGATATGGGAAAAG
TGGTCAACGGATGGCACGGAAAAACAGGTTGCGAAGCTGATGGACGAGGACATCGGTGCCGCAATG
CAATTTCTCCAATCAAAGGCTCTCTGCATGATGCCAGTCTCCCTTGCCATGGCTATCTATGACACA
CAACATTCACAGGACGGCCAACCAGTGAAGCCTGAACCCAACAACACTGCCTAG

**SEQ ID NO: 245, TabHLH11, Triticum aestivum**
MAGQPPQGPEDDFLDQFFSLTNSLSAAGRPSGDQPFSLALSLDAASDASGSRGGIGDDAGNAAERD
GVQLPGLFPPVFGGGLQPPHLRPSHPPPQMFHAQQPKQGGPAGGPQPPAPRPKVRARRGQATDPHS
IAERLRRERIAERMRALQELVPNTNKTDRAVMLDEILDYVKFLRLQVKVLSMSRLGGAGAVAQLVS
DIPLSVKGEASDGGSKQQIWEKWSTDGTEKQVAKLMDEDIGAAMQFLQSKALCMMPVSLAMAIYDT
QHSQDGQPVKPEPNNTA

**SEQ ID NO: 246, Motif 1**
(E/D)(D/S/E)(F/M)(L/F)(D/E/Q/L)(Q/H/E)

**SEQ ID NO: 247, Motif 2**
RA(R/I/Q)RG(Q/H)ATDPHSIAER

**SEQ ID NO: 248, Motif 3**
(M/I/V/L)(K/R)(A/S/Q/D/N)LQ(E/D/V)LVP

**SEQ ID NO: 249, Motif 4**
(M/I)(L/I)DEI(I/V/L)(D/E/G)Y(V/L/I)(K/R)FL(Q/R)LQ(V/I)K

**SEQ ID NO: 250, Motif 5**
(V/I)LSMSR(L/V)G

**SEQ ID NO: 251, Motif 6**
V(A/V/L/I)(K/R)(L/M)(M/L)(E/D)(E/D/S/K/T)(D/N/S)(M/V/I)(G/T/I)XAMQ
(Y/L/F)L

**SEQ ID NO: 252, Motif 7**
(M/V)(P/S)(I/V)(S/T/A)LA

**SEQ ID NO: 253, Motif 8**
SIAERLRRERIAERMRALQELVPNTNKTDRAVMLDEILDYVKFLRLQVKVL

## FIGURE 5

**SEQ ID NO: 254, prm009718 (fwd):**
GGGGACAAGTTTGTACAAAAAAGCAGGCTTAAACAATGGCGGGGCANCCG

**SEQ ID NO: 255, prm009719 (rev):**
GGGGACCACTTTGTACAAGAAAGCTGGGTATGGGTGTTGCAGCTGCTGTT

**SEQ ID NO: 256, Rice Gos2 promoter**
AATCCGAAAAGTTTCTGCACCGTTTTCACCCCCTAACTAACAATATAGGGAACGTGTGCTAAATAT
AAAATGAGACCTTATATATGTAGCGCTGATAACTAGAACTATGCAAGAAAAACTCATCCACCTACT
TTAGTGGCAATCGGGCTAAATAAAAAAGAGTCGCTACACTAGTTTCGTTTTCCTTAGTAATTAAGT
GGGAAAATGAAATCATTATTGCTTAGAATATACGTTCACATCTCTGTCATGAAGTTAAATTATTCG
AGGTAGCCATAATTGTCATCAAACTCTTCTTGAATAAAAAAATCTTTCTAGCTGAACTCAATGGGT
AAAGAGAGAGATTTTTTTTAAAAAAATAGAATGAAGATATTCTGAACGTATTGGCAAAGATTTAAA
CATATAATTATATAATTTTATAGTTTGTGCATTCGTCATATCGCACATCATTAAGGACATGTCTTA
CTCCATCCCAATTTTTATTTAGTAATTAAAGACAATTGACTTATTTTTATTATTTATCTTTTTTCG
ATTAGATGCAAGGTACTTACGCACACACTTTGTGCTCATGTGCATGTGTGAGTGCACCTCCTCAAT
ACACGTTCAACTAGCAACACATCTCTAATATCACTCGCCTATTTAATACATTAGGTAGCAATATC
TGAATTCAAGCACTCCACCATCACCAGACCACTTTTAATAATATCTAAAATACAAAAAAATAATTTT
ACAGAATAGCATGAAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACA
GAACAACCCACAAAAAACGATGATCTAACGGAGGACAGCAAGTCCGCAACAACCTTTTAACAGCAG
GCTTTGCGGCCAGGAGAGAGGAGGAGAGGCAAAGAAAACCAAGCATCCTCCTTCTCCCATCTATAA
ATTCCTCCCCCCTTTTCCCCTCTCTATATAGGAGGCATCCAAGCCAAGAAGAGGGAGAGCACCAAG
GACACGCGACTAGCAGAAGCCGAGCGACCGCCTTCTCGATCCATATCTTCCGGTCGAGTTCTTGGT
CGATCTCTTCCCTCCTCCACCTCCTCCTCACAGGGTATGTGCCTCCCTTCGGTTGTTCTTGGATTT
ATTGTTCTAGGTTGTGTAGTACGGGCGTTGATGTTAGGAAAGGGGATCTGTATCTGTGATGATTCC
TGTTCTTGGATTTGGGATAGAGGGGGTTCTTGATGTTGCATGTTATCGGTTCGGTTTGATTAGTAGT
ATGGTTTTCAATCGTCTGGAGAGCTCTATGGAAATGAAATGGTTTAGGGATCGGAATCTTGCGATT
TTGTGAGTACCTTTTGTTTGAGGTAAAATCAGAGCACCGGTGATTTGCTTGGTGTAATAAAGTAC
GGTTGTTTGGTCCTCGATTCTGGTAGTGATGCTTCTCGATTTGACGAAGCTATCCTTTGTTTATTC
CCTATTGAACAAAAATAATCCAACTTTGAAGACGGTCCCGTTGATGAGATTGAATGATTGATTCTT
AAGCCTGTCCAAAATTTCGCAGCTGGCTTGTTTAGATACAGTAGTCCCCATCACGAAATTCATGGA
AACAGTTATAATCCTCAGGAACAGGGGATTCCCTGTTCTTCCGATTTGCTTTAGTCCCAGAATTTT
TTTTCCCAAATATCTTAAAAAGTCACTTTCTGGTTCAGTTCAATGAATTGATTGCTACAAATAATG
CTTTTATAGCGTTATCCTAGCTGTAGTTCAGTTAATAGGTAATACCCCTATAGTTTAGTCAGGAGA
AGAACTTATCCGATTTCTGATCTCCATTTTTAATTATATGAAATGAACTGTAGCATAAGCAGTATT
CATTTGGATTATTTTTTTTATTAGCTCTCACCCCTTCATTATTCTGAGCTGAAAGTCTGGCATGAA
CTGTCCTCAATTTTGTTTTCAAATTCACATCGATTATCTATGCATTATCCTCTTGTATCTACCTGT
AGAAGTTTCTTTTTGGTTATTCCTTGACTGCTTGATTACAGAAAGAAATTTATGAAGCTGTAATCG
GGATAGTTATACTGCTTGTTCTTATGATTCATTTCCTTTGTGCAGTTCTTGGTGTAGCTTGCCACT
TTCACCAGCAAAGTTC

**SEQ ID NO: 257, expression cassette**
AATCCGAAAAGTTTCTGCACCGTTTTCACCCCCTAACTAACAATATAGGGAACGTGTGCTAAATAT
AAAATGAGACCTTATATATGTAGCGCTGATAACTAGAACTATGCAAGAAAAACTCATCCACCTACT
TTAGTGGCAATCGGGCTAAATAAAAAAGAGTCGCTACACTAGTTTCGTTTTCCTTAGTAATTAAGT
GGGAAAATGAAATCATTATTGCTTAGAATATACGTTCACATCTCTGTCATGAAGTTAAATTATTCG

**FIGURE 5 (continued)**

```
AGGTAGCCATAATTGTCATCAAACTCTTCTTGAATAAAAAAATCTTTCTAGCTGAACTCAATGGGT
AAAGAGAGAGATTTTTTTTAAAAAAATAGAATGAAGATATTCTGAACGTATTGGCAAAGATTTAAA
CATATAATTATATAATTTTATAGTTTGTGCATTCGTCATATCGCACATCATTAAGGACATGTCTTA
CTCCATCCCAATTTTTATTTAGTAATTAAAGACAATTGACTTATTTTTATTATTTATCTTTTTTCG
ATTAGATGCAAGGTACTTACGCACACACTTTGTGCTCATGTGCATGTGTGAGTGCACCTCCTCAAT
ACACGTTCAACTAGCAACACATCTCTAATATCACTCGCCTATTTAATACATTTAGGTAGCAATATC
TGAATTCAAGCACTCCACCATCACCAGACCACTTTTAATAATATCTAAAATACAAAAAATAATTTT
ACAGAATAGCATGAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACA
GAACAACCCACAAAAAACGATGATCTAACGGAGGACAGCAAGTCCGCAACAACCTTTTAACAGCAG
GCTTTGCGGCCAGGAGAGAGGAGGAGAGGCAAAGAAAACCAAGCATCCTCCTTCTCCCATCTATAA
ATTCCTCCCCCTTTTCCCCTCTCTATATAGGAGGCATCCAAGCCAAGAAGAGGGAGAGCACCAAG
GACACGCGACTAGCAGAAGCCGAGCGACCGCCTTCTCGATCCATATCTTCCGGTCGAGTTCTTGGT
CGATCTCTTCCCTCCTCCACCTCCTCCTCACAGGGTATGTGCCTCCCTTCGGTTGTTCTTGGATTT
ATTGTTCTAGGTTGTGTAGTACGGGCGTTGATGTTAGGAAAGGGGATCTGTATCTGTGATGATTCC
TGTTCTTGGATTTGGGATAGAGGGGTTCTTGATGTTGCATGTTATCGGTTCGGTTTGATTAGTAGT
ATGGTTTTCAATCGTCTGGAGAGCTCTATGGAAATGAAATGGTTTAGGGATCGGAATCTTGCGATT
TTGTGAGTACCTTTTGTTTGAGGTAAAATCAGAGCACCGGTGATTTTGCTTGGTGTAATAAAGTAC
GGTTGTTTGGTCCTCGATTCTGGTAGTGATGCTTCTCGATTTGACGAAGCTATCCTTTGTTTATTC
CCTATTGAACAAAATAATCCAACTTTGAAGACGGTCCCGTTGATGAGATTGAATGATTGATTCTT
AAGCCTGTCCAAAATTTCGCAGCTGGCTTGTTTAGATACAGTAGTCCCCATCACGAAATTCATGGA
AACAGTTATAATCCTCAGGAACAGGGGATTCCCTGTTCTTCCGATTTGCTTTAGTCCCAGAATTTT
TTTTCCCAAATATCTTAAAAAGTCACTTTCTGGTTCAGTTCAATGAATTGATTGCTACAAATAATG
CTTTTATAGCGTTATCCTAGCTGTAGTTCAGTTAATAGGTAATACCCCTATAGTTTAGTCAGGAGA
AGAACTTATCCGATTTCTGATCTCCATTTTTAATTATATGAAATGAACTGTAGCATAAGCAGTATT
CATTTGGATTATTTTTTTATTAGCTCTCACCCCTTCATTATTCTGAGCTGAAAGTCTGGCATGAA
CTGTCCTCAATTTTGTTTTCAAATTCACATCGATTATCTATGCATTATCCTCTTGTATCTACCTGT
AGAAGTTTCTTTTTGGTTATTCCTTGACTGCTTGATTACAGAAAGAAATTTATGAAGCTGTAATCG
GGATAGTTATACTGCTTGTTCTTATGATTCATTTCCTTTGTGCAGTTCTTGGTGTAGCTTGCCACT
TTCACCAGCAAAGTTCATTTAAATCAACTAGGGATATCACAAGTTTGTACAAAAAAGCAGGCTTAA
ACAATGGCGGGGCAGCCGCCGCAGGGCCCGGAGGACGACTTCCTCGACCAGTTCTTCTCCCTCACC
AACTCCCTCTCCGCCGCCGGCCGCCCCTCCGGCGACCAGCCCTTCTCCCTCGCCCTCAGCCTCGAC
GCCGCCTCGGACGCCTCCGGCAGCAGGGGCGGCATCGGCGACGACGCCGGCAACGCCGCGGAGCGG
GACGGCGTGCAGCTCCCCGGCCTCTTCCCGCCGGTGTTCGGCGGTGGCCTCCAGCCGCCGCACCTT
CGCCCCAGCCACCCTCCCCCACAGATGTTCCACGCGCAGCAGCCGAAGCAGGGCGGGCCGGCTGGA
GGGCCGCAGCCGCCGGCGCCGAGGCCGAAGGTGCGGGCGCGTCGCGGTCAGGCGACTGATCCCCAC
AGCATCGCGGAGAGGCTAAGAAGAGAGAGGATAGCGGAAAGGATGAGGGCCCTACAGGAATTGGTC
CCCAATACGAACAAGACAGATAGGGCAGTTATGCTAGATGAGATCCTGGATTATGTGAAGTTCCTT
AGGCTTCAAGTAAAGGTGTTAAGCATGAGCAGATTGGGTGGTGCTGGTGCTGTTGCACAGCTGGTT
TCTGACATTCCACTTTCAGTTAAGGGGGAAGCAAGCGATGGTGGGAGCAAACAGCAGATATGGGAA
AAGTGGTCAACGGATGGCACGGAAAAACAGGTTGCGAAGCTGATGGACGAGGACATCGGTGCCGCA
ATGCAATTTCTCCAATCAAAGGCTCTCTGCATGATGCCAGTCTCCCTTGCCATGGCTATCTATGAC
ACACAACATTCACAGGACGGCCAACCAGTGAAGCCTGAACCCAACAACACTGCCTAG
```

SEQ ID NO: 258, A. cepa_TC3698

```
MASNNNPQPPSDGLNDDFFDQIFSMPSAFAASSASTDATEALNYADSSTTGVTPMFSLGLSLDQQP
KSDSSQSEREHPVQFACLFPQYGHNNQVHQTRPNLSPPQVLHGQAMQSSMAATPQPTGPRPRVRAR
RGQATDPHSIAERLRRERIAERIRALQELVPSTNKTDRAVMLDEIVEYVKFLRLQVKVLSMSRLGG
AGAVAQLVADIPMSSSVEGDSSESGKTSYQGWEKWSTDGTERQVAKLMEEDVGAAMQFLQSKALCI
MPISLAAAICQTNPSTEISFM
```

**FIGURE 5 (continued)**

**SEQ ID NO: 259, A. thaliana_bHLH007-11-AT1G03040**

MANNNNIPHDSISDPSPTDDFFEQILGLSNFSGSSGSGLSGIGGVGPPPMMLQLGSGNEGNHNHMG
AIGGGGPVGFHNQMFPLGLSLDQGKGHGFLKPDETGKRFQDDVLDNRCSSMKPIFHGQPMSQPAPP
MPHQQSTIRPRVRARRGQATDPHSIAERLRRERIAERIRSLQELVPTVNKTDRAAMIDEIVDYVKF
LRLQVKVLSMSRLGGAGAVAPLVTEMPLSSSVEDETQAVWEKWSNDGTERQVAKLMEENVGAAMQL
LQSKALCIMPISLAMAIYHSQPPDTSSSIVKPEMNPPP

**SEQ ID NO: 260, A. thaliana_bHLH059-11-AT4G02590**

MASNNPHDNLSDQTPSDDFFEQILGLPNFSASSAAGLSGVDGGLGGGAPPMMLQLGSGEEGSHMGG
LGGSGPTGFHNQMFPLGLSLDQGKGPGFLRPEGGHGSGKRFSDDVVDNRCSSMKPVFHGQPMQQPP
PSAPHQPTSIRPRVRARRGQATDPHSIAERLRRERIAERIRALQELVPTVNKTDRAAMIDEIVDYV
KFLRLQVKVLSMSRLGGAGAVAPLVTDMPLSSSVEDETGEGGRTPQPAWEKWSNDGTERQVAKLME
ENVGAAMQLLQSKALCMMPISLAMAIYHSQPPDTSSVVKPENNPPQ

**SEQ ID NO: 261, A. thaliana_bHLH066-11-AT2G24260**

MMNSSLLTPSSSSSSHIQTPSTTFDHEDFLDQIFSSAPWPSVVDDAHPLPSDGFHGHDVDSRNQPI
MMMPLNDGSSVHALYNGFSVAGSLPNFQIPQGSGGGLMNQQGQTQTQTQPQASASTATGGTVAAPP
QSRTKIRARRGQATDPHSIAERLRRERIAERMKALQELVPNGNKTDKASMLDEIIDYVKFLQLQVK
VLSMSRLGGAASVSSQISEAGGSHGNASSAMVGGSQTAGNSNDSVTMTEHQVAKLMEEDMGSAMQY
LQGKGLCLMPISLATAISTATCHSRNPLIPGAVADVGGPSPPNLSGMTIQSTSTKMGSGNGKLNGN
GVTERSSSIAVKEAVSVSKAATGMQFDESDARVWIWFEIRREDDIVELLWQSGQVVGTNQTHRQSY
DPPPILRGSGSGRGEENAPLSQPPPHLHQQNLFIQEGEMYSWLHHSYRQNYFCSELLNSTPATHPQ
SSISLAPRQTIATRRAENFMNFSWLRGNIFTGGRVDEAGPSFSVVRESMQVGSNTTPPSSSATESC
VIPATEGTASRVSGTLAAHDLGRKGKAVAVEAAGTPSSGVCKAETEPVQIQPATESKLKAREETHG
TEEARGSTSRKRSRTAEMHNLAERRRREKINEKMKTLQQLIPRCNKVESDSVSTLISLLKFQRWMM
LSSTSNRYRAKYKYALQNRMCFKPMVQHGKSSYVSSFVEMMSTGQGMMSPMMNAGNTQQFMPHMAM
DMNRPPPFIPFPGTSFPMPAQMAGVGPSYPAPRYPFPNIQTFDPSRVRLPSPQPNPVSNQPQFPAY
MNPYSQFAGPHQLQQPPPPPPFQVTLYHGIHCVVAGNPYV

**SEQ ID NO: 262, A. thaliana_bHLH069-11-AT4G30980**

MNSSSLLTPSSSPSPHLQSPATFDHDDFLHHIFSSTPWPSSVLDDTPPPTSDCAPVTGFHHHDADS
RNQITMIPLSHNHPNDALFNGFSTGSLPFHLPQGSGGQTQTQSQATASATTGGATAQPQTKPKVRA
RRGQATDPHSIAERLRRERIAERMKSLQELVPNGNKTDKASMLDEIIDYVKFLQLQVKVLSMSRLG
GAASASSQISEDAGGSHENTSSSGEAKMTEHQVAKLMEEDMGSAMQYLQGKGLCLMPISLATTIST
ATCPSRSPFVKDTGVPLSPNLSTTIVANGNGSSLVTVKDAPSVSKP

**SEQ ID NO: 263, A. thaliana_bHLH082-11-AT5G58010**

MENGNGEGKGEFINQNNDFFLDSMSMLSSLPPCWDPSLPPPPPPPQSLFHALAVDAPFPDQFHHPQ
ESGGPTMGSQEGLQPQGTVSTTSAPVVRQKPRVRARRGQATDPHSIAERLRRERIAERMKSLQELV
PNTNKTDKASMLDEIIEYVRFLQLQVKVLSMSRLGGAGSVGPRLNGLSAEAGGRLNALTAPCNGLN
GNGNATGSSNESLRSTEQRVAKLMEEDMGSAMQYLQGKGLCLMPISLATAISSSTTHSRGSLFNPI
SSAVAAEDSNVTATAVAAPEASSTMDDVSASKA

**SEQ ID NO: 264, A. vulgaris_TC15501**

MANNNNPTEGLTDDFLDQILSYGAHEGNLAGNDVNLAGTTTPGGSMALQLSSSGGDITGSSGGYHHH
HQHQHHHQLSGGGGGGFPLGLSLEQSGKGGFFKPDEASGSGKRFRDDFVDLKAVSSTNNDNNDRGDS
VHLNNLFPAFGHVQQQQQAHSVRPPSHQNQINQPFLGHPTPGAVAVVPHPPAIRPRVRARRGQATD
PHSIAERLRRERIAERMKALQELVPSSNKTDRAAMLDEIVDYVKFLRLQVKVLSMSRLGGAGAVAQ
LVADIPLSSAEGGASEGGSNQPAWEKWSTDGTEHQVAKLMEEDVGAAMQFLQSKALCIMPISLASA
IYHDQPPDASTMIKPEPNAPS

**FIGURE 9 (continued)**

**SEQ ID NO: 265, A. vulgaris_TC19278**
MKNLQELVPNSNKTDKASMLDEIIEYVRFLQLQVKVLSMSRLGATGAVVPLITENQPEGSTSHFLS
QSAGRDVKHIESPNTLTFEQEVVKLMESNMTKAMQFLQRKGLCLMPIALAAAISGSKAPPASLSTE
GKKSILTNGFVHHNSGQSNIGPGQISSDGETRSEENIIGIHSREDFSSNSCSGTSIKKEGTNTTNF
TREMNQE

**SEQ ID NO: 266, B. napus_TC10015_part**
HASETPSDDFFEQILGLPNFSASSSDGGLGGGGAPPMMLQLGSGEEGGHMGGLGGGSGFHNQMFPL
GLSLEQGKGQGFLRPEGGSLGTGKRFSDDVMKPVFHGQPMQQQPAPAAPHQPTSIRPRVRARRGQA
TDPHSIAERLRRERIAERIRALQELVPTVNKTDRAAMIDEIVDYVKFLRLQVKVLSMSRLGGAGAG
APLVTDMPLSSSVEDESGEGGRAPQPAWEKWSNDGTERQVAKLMEENVGAAMQLLSSKALCMMPIS
LAMAIYHSQPPDTT

**SEQ ID NO: 267, C. clementina_DY268946**
MFLQLGSGGPSPGAGGGATSAADIRSLAMGISMGMMPLGLNLEHSFLRQHEDHNSNHNNNNNTNAS
SSSPNNSSATSGINERDSVHMPSLFPAFGQLQSQSARPPLPRPPQVQQFQSQPAAAPQPPAVRPRV
RARRGQATDPHSIAERLRRERIADRMRALQELVPSCNKTDRAAMLDEIVDYVKFLRLQVKVLSMSR
LGAAGAVAQLVADVPLSSALEGESIDGGSSQPEWEKWSNDGTEQQVAKLMEEDIGAAMQFLQSKAL
CIMPISLASAIYRTRQPDAPAFVKPESSTPSYCLNNNWSQQLAHNDSKYLITGTYSYRILLVLLSN
QIIQIPMQRSHSSFP

**SEQ ID NO: 268, C. longa_TA2544**
MQPSSKETERMAQSHAALQLELQNGGGGAQNDDFFEQTMSGFSTAAWAPELGTPRSLFGARSSEES
PDDEGLNYAPPYGGSSLLASRHRMHLGTSPADSSMFLQLGGQILLHPTAGASGGESGGFLRLPLSL
GSGGGSGVSTSAIFGDRSRGEIDPPFESSNPTEAEVLYNDGFTGSLPRAVQASLHQQLLRQPQNYGA
ATLTGQALAITATASGTAGAGTAPPKPRVRARRGQATDPHSIAERLRRERIAERMKALQELVPNAN
KTDKASMLDEIIDYLKFLQLQVKVLSMSRLGGAAAVAPLVADMSSESGGGAGTPRSNNDGLTAAEQ
QVAKLLEEDMGSAMQYLQGKGLCLMPISLASAISSATCRPRPPPGVNLRYPAAGDAPPSPSISALT
VQSSNAGSAGADAS

**SEQ ID NO: 269, C. paradisi hybrid_TA3392**
MANNPNEASSTDDFLEQILGIPNFGSAESGLAASDGGLAAGSPMMLQLSSGDGSSHISALGGGVSS
GYHGQVFPLGLSLEQGKGGGFLKPEEASGSGKRFPEEHAIKNVFHGQPLPSPVPAAPHPPAMRPRVR
ARRGQATDPHSIAERLRRERIAERIRALQELVPSVNKTDRAAMLDEIVDYVKFLRLQVKVLSMSRV
GAPGAVAPLVTTDLPLSSVEDESGEGVRNQPAWEKWSNDGTERQVAKLMEENVGAAMQFLQSKALC
IMPISLATAIYHSQPPE

**SEQ ID NO: 270, C. sinesis TA12416**
MQPCSSGGEMQGISSLLSNGSHEQQSQIHQSATFDPTSQDDFLEQMLSSLPSCSWTDLKSPWGVVD
LNPNNNNNNINNKQPRDLSDETAPSTTQENNVPAGFQFDESMILASKMRQHQISGNTGSGNNNSAA
AKFMMQQQQQQIMMAAARGGIGLPLSLGNGGDNDIVDVSSFKSQQGGDGSVQALYNGFTGSLHGST
QPQHFHHLQGGSMPGQTFGAPGPVMNQTQAQASGSTGGGGGGGGNTPAQQPKQRVRARRGQATDPHS
IAERLRRERIAERMKALQELVPNANKTDKASMLDEIIDYVKFLQLQVKVLSMSRLGGAAAVAPLVA
DMSSEGGGGDCIQANGRNPNGAQTTSANDSLTVTEHQVAKLMEEDMGSAMQYLQGKGLCLMPISLA
TAISTATCHSRNPIISTSNNNNNNGNPHHNPLLQSNGEGPTSPSMSVLTVQSATMGNGGADGSVKD
AASVSKP

**FIGURE 5 (continued)**

**SEQ ID NO: 271, E. grandis WO2005001050 seqID77**
MAGSNPQEGLGDEFFEQILAVPPGAYSGGSTPMVLQLGSGRGGVEDGGGRGGGGGLRGMGVMMPLG
LNLEQGGLFRHEDVENSTSASSTTSAINMEREAGVHHGNNMTSCLFPAFGQFQTHQSVQPPPPHPP
PPQLHPAFLNQPAVGSPNQPAIRPRARARRGQATDPHSIAERLRRERINERMKALQELVPSCNKTD
RAAMLDEIVDYVKFLRLQVKVLSMSRLGGAGAVAQLVADVPLSSAEGEIIEGGNNQPAWEKWLTDG
TEQQVAKLMEEDVGAAMQFLQSKTLCIMPISLASAIFRTSQPDMPRSIKPESSAPS

**SEQ ID NO: 272, E. grandis WO2005001050 seqID1671**
MANNPSDGASDEFLEHLLGLPNFAAAAEAGLAPGDGTGLSVTAATPMMLQLGSGDGSGGHGHGHGH
GHGQGHLSALGGGGAGAGGGGGGGGGGYHGAVFPLGLSLEQGKGGFLKPEEASGSGKRYPEEVVDG
RASTVKNLHSPLPNFPDVSQMASFRNRDVFHGQPVPNPVPAAPHPPAMRPRVRARRGQATDPHSIA
ERLRRERIAERIRQLQDLVPSVNKTDRAAMLDEIVDYVKFLRLQVKVLSMSRLGAAGAVAPLVTDI
PLSSVEEEGGEGGRNQPAWEKWSNDGTERQVAKLMEENVGAAMQFLQSKALCIMPISLATAIYQTQ
PPDTSSLVKSESNPPS

**SEQ ID NO: 273, G. hirsutum CO123623**
DSGGGGFRGIGMMPLGLNLEHGFLRHEDGVVVDNNNNNASCSAASAVSGISERDSMHMVSLFPPFG
QMQTQQIRASPPPPQPPPQLHQPFHSQPTSGPVAAAPHPPAVRPRVRARRGQATDPHSIAERLRRE
RIAERMKALQELVPSCNKTDRAAMLDEIVDYVKFLRLQVKVLSMSRLGAAGAVAQLVADVPLLSVE
GDGAGGGTQPPWEKWSNDGTEQQVAKLMEEDIGAAMQFLQSQALCIVPISLASAIFPAHQPDAPTI
V

**SEQ ID NO: 274, G. hirsutum DT543504**
MISNGGESSEFRRSFTGLETLSPIPQLWHLQPYDSVSSLPTLVGQTIVDDEGNINRFIEIDEILQP
ENLSASINSKGQQDMQNSFCSSFPADHPITKTMIGLPSLVQGASPNLNNGSDRTVKPRVRARRGQA
TDPHSIAERLRREKIAERMKNLQELVPNSNKTDKASILDEIIGYVKFLQLQVKVLSMSRLGAAAAV
VPLITDGRAEVSNGLSLAPLAGQGVDFSPSPDQVVFEQEVVKLMESNMTMAMQYLQSKGFCLMPVA
LAAAISNVKSSTSSSSSSVPASDESKKLGLHQQYSNQQHLQQ

**SEQ ID NO: 275, G. hirsutum TC60118**
MANNPNESPADDFLEQILGLSHFAPTETGLAGPDGRLSGNATTAGAPMLLQLSSGGGTGHIGAIGG
GGGGAFHGQVFPLGLSLEQGKGGFLKPEEASGSSKRFRNEVVDGRAFSVKNVFHGQPVPATVAAGP
HPPAMHPRVRARRGQATDPHSIAERLRRERIAERIRALQELVPSVNKTDRAAMLDEIVDYVKFLRL
QVKVLSMSRLGAAGAVAPLVTDLPLSSVEDESGEGGRSQPAWEKWSNDGTERQVAKLMEEDVGAAM
QFLQSKALCVMPISLATAIYHTQSPDTSSVVKPETNPPS

**SEQ ID NO: 276, G. hirsutum TC60119**
MANNTNEAPAADDFLEQILGLPNFAPSETGLAGSDAGLAATAAGAGAPMFLQLSSGDGAAHIGGIG
GGGGGAFHGQVFPLGLSLEQGKGGFLKPEEASGSGKRFRNGVVDDRASSVKNVFHGQPMQATVSAA
PHPPTMRPRVRARRGQATDPHSIAERLRRERITERIRALQELVPSVNKTDRAVMLDEIVDYVKFLR
LQVKVLSMSRLGGAGAVAPLVTDIPLSSVEYESGEGGRSQPAWEKWSNDGTEQQVAKLMEENVGAA
MQFLQSKSLCIMPISLATAIYHTQVPDTSSVVKPETNPPA

**SEQ ID NO: 277, G. hirsutum TC61833**
MANNPNEASADDFLEQILGFPNFAPSETGLAGPDGGLTGTTAGAGTPMFLQLSSGDGASHLGGIGG
GGGGAFPGQVFPLGLSLDQGKSGGFLKPEEGSGGSSRRFRDEVVDGRASSVKNVFHGSPMPATVAP
SPHPPTMRPRVRARRGQATDPHSIAERLRRERIAERIRALQELVPSVNKTDRAVMLDEIVDYVKFL
RLQVKVLSMSRLGGAGAVAPLVTDIPLSSVEDESGDGGRNQPAWEKWSNDGTERQVAKLMEENVGA
AMQFLKSKALCIMPISLATAIYHSQPLDTSSIVKPETDPPP

**FIGURE 5 (continued)**

SEQ ID NO: 278, G. hirsutum TC67603
MQPCSREMQAMNSLLNPTQQIPLQDLQINGNRHHHQQIHVPSSSQFQYPTPTSTTHHDDSFLEQIL
SSTTSFPWSDETGPPNPEDNNNAGFNYDEMVLASKLRQHQINGGAAGNPFAAMKMMLMMQQQQQQQ
QMMLAGRPTVASAAAGGGITTNHQGGEGSMQALYNVFGDGSLHGTMQAKSFVGANSATEMTQSQGS
GPTAGEAVTAPEKPKQRVRARRGQATDPHSIAERLRRVRIAERMKALQELVPNANKTDKASMLDEI
IDYVKFLQLQVKVLSMSRLGGAAAVAPLVSEGGGDCIQTSANGGSHPRNSNGDQTPSANDRLTMTE
HQVAKLMEEDMGSAMQYLQEKGLCLMPISLATAISSATSRSRNPMINHENPANGSHLLIQSSGGDG
PSSPSMSVLTVQSATMGNGGLEGGAASVSKP

SEQ ID NO: 279, G. max TC229602
MKPDEASASGKRFRDDVVDNRAKHVFHGQPMPTTMPAAPHPPAIRPRVRAIRGQATDPHSIAERLR
RERIAERIRALQELVPSVNKTDRAAMLDEIVDYVKFLRLQVKVLSMSRLGGAGAVAPLVTDIPLSS
VEEEGGEGARNRPAWDKWSNDGTERQVAKLMEENVGAAMQFLQSKALCIMPISLASAIYQSQPPDT
SSIVKPETNPPS

SEQ ID NO: 280, G. max TC205173
MLQHQLLRDSGLLNMPLSLPGNDVVVDASTFESPNPSGKASVQTLYNGFAGSLHGVGQSSNQTQHF
QHPQGSSNPMQGQNFGAVPAGGGSATNQAPASGAAAGGAPAQPRQRVRARRGQATDPHSIAERLRR
ERIAERMKALQELVPNANKTDKASMLDEIIDYVKFLQLQVKVLSMSRLGGAAAVAPLVADMSSEGG
GDCIQANGKSNGGGAQASTTNTNTNQTTATTTSNDSLTMTEHQVAKLMEEDMGSAMQYLQGKGLCL
MPISLATAISTATCPTRNVNVNPLINAAAGATHFPTAANPAGEGPSSPSMSVLTVQSAIAGNDGAA
SVSKP

SEQ ID NO: 281, G. max TA55042
MAGNSGSEGLGDDFFEQILAVPEAGTVGMLQLGSTTGAFRGASGLMPLGLNLEQAAFLRHQVNVDD
DVVHVNVDDATIHQHHLTLHNNNNSSSPSSTAPITDRDSMHMRGLFSAFGQLHTPIRPTLPLPPPP
RQPQLHLHHHNQFQGGQAAAAPASMAAMPQPPGIRPRVRARRGQATDPHSIAERLRRERIAERMKAL
QELVPSINKTDRAAMLDEIVDYVKFLRLQVKVLSMSRLGGAGAVAQLVADVPLSAVEGDQDIEGGA
NEQAWDKWSNDGTEQQVAKLMEEDVGAAMQFLQSKALCIMPISLASAIFRMPQSEASTGIKPESNS
H

SEQ ID NO: 282, G. max TC207545
MMLQLNSGDAATHLASFHAPPYQLGLSLDQGEGPFLTPEDASGSGKRFRDDAVDTRPNNVFDGQPM
PTTVPTAPHPPAVRPRVRARRGQATDPHSIAERLRRERIAERIRALQELVPSVNKTDRAAMLDEIV
DYVKFLRLQVKVLSMSRLGGAGAVAPLVTDIPLSSVEEEGGEGRNQPAWEKWSNDGTERQVAKLME
ENVGAAMQFLQSKALCIMPVSLASAIYQSQPSGTSSIVKPETNPPS

SEQ ID NO: 283, G. raimondii TA13791
MANNPNESPADDFLEQILGLSHFAPTETGLAGPDGRLSGNATTAGAPMLLQLSSGGGTGHIGAIGG
GGGGAFHGQVFPLGLSLEQGKGGFLKPEEASGSSKRFRNEVVDGRAFSVKNVFHGQPVPATVAAGP
HPPAMRPRVRARRGQATDPHSIAERLRRERIAERIRALQELVPSVNKTDRAAMLDEIVDYVKFLRL
QVKVLSMSRLGAAGAVAPLVTDLPLSSVEDESGEGGRSQPAWEKWSNDGTERQVAKLMEEDVGAAM
QFLQSKALCVMPISLATAIYHTQSPDTSSVVKPETNPPS

SEQ ID NO: 284, H. petiolaris TA1140
MSTDPPEGYGDDFLEQILAIPSYNITGCLPVNTADSTGSETVSVHHQQQQPQQQLQPQKFPLGLSL
DNGRETIGGAFAGQQQQLQQQQQRERGGSMNMSGLFPQFENLQSHSLLHTVPQGFQGQPTSSTAVT
VPHPPTIRPRVRARRGQATDPHSIAERLRRERIAERMRALQELVPSCNKTDKAAMLDEILDYVKFL
RLQVKVLSMSRLGGAGAVAQLVSDVPLQSVEGDGSENGYNNQQQGQAAWENWSNDDTEREVAKLME
EDVGAAMQFLQSKALCIMPISLASLIYPNHQPDVKPEPSAPS

FIGURE 5 (continued)

**SEQ ID NO: 285, H. vulgare TC140470**
MNYSDGSFFPSWPGNSASENYSFVDGSVESYTEEGSMPTTGYFRARSNQNLTFDEHEQNPAMLANG
CLPYNTQTDLLSGEILSDDKPSNSLVELSQLQNNVCLQNNLIPPGTLQCNSTPGTFDLQLDTPGLL
ELPHALSSSIESNGSEVSAFLADVQAVSSASTLCSTFQNVPSYMEPVSLEAFSFQGMQNAAMFNNA
SHSNGNLSVFDEATMASLHDSKEFLNGSISSLGTGQQSQLAGGGLKAEQQEQNTMCNIPLPSFVSA
SQMAVSEAQGALIPSKTTSITHNNKSEYPIPISHSADVQHKANSGNGNSASAKPRARARRGQATDP
HSIAERLRREKISERMKNLQDLVPNSNKADKSSMLDEIIDYVKFLQLQVKVLSMSRLGAPGAVLPL
LAESQTEGRSNSPLSSPTTSQGLLDVAGPEDSLVFEQEVIKLMETSITNAMQYLQNKGLCLMPIAL
ASAISNQKGTSAAAIPPER

**SEQ ID NO: 286, L. perenis TA3490 part**
MATDPPEGYGDDFLEQILAIPSYNIAGCLPGNTTDANASETVSVHRQQQQQQPVFPLGLSLDNGRE
TIGAFAGQQQQRERGGSMNMTGLFPSFENLQSHSLLHSVPQAFQGQSTTSTAVTVPHPPSIRPRVR
ARRGQATDPHSIAERLRRERIAERMRALQELVPSCNKTDKAAMLDEILDYVKFLRLQVKVLSMSRL
GGAGAVAQLVSDVPLQSVEGDTSENGYNQPAWENWSNDDTEREVAKLMEEDVGAAMQFLQSKALCI
MPISLASLIYPPQTPDSNSHVKPETV

**SEQ ID NO: 287, N. benthamiana CK293938**
MATNQPEGYADDFLEQILAIPSYAGLPPVADVGTSSETTSFTSASAAGLQQPLFPLGLSLENGRDD
VSDAGAYAVKHEREGINIGNLYAGLEHLQSHAVRHAVPPVHHIQSFQGQPTTSTTMTVPHSPAIRP
RVRARRGQATDPHSIAERLRRERISERIKALQELVPSCNKTDRAAMLDEILDYVKFLRLQVKVLSM
SRLGGASAVAQLVADIPLQSMEGDSAESKSNQHIWEKWSNVDTEQEVAKLMEEDVGAAMQYLQSKS
LCIMPISLAALIYPTQQPDDMSMVKPEPAAPS

**SEQ ID NO: 288, N. benthamiana TC7102**
MQAMNQYDPTSSHDDFLDQILSSVPSSSLCWPDLSKSWDPHHHHLSPPLPPNPSSGDDHQLPPSNP
LQHFQYDDQSSSFLAAKLRQHQITGGGGGGGDAVAAAKALLLQQQLLLSRTLAGNGLRSPTGASGD
NGFLNMLGNGDQNDSVGNPANDSSVQALFNGFTGSLGQNSSQPQHFLHPQGGRMQAQSFGAPATAP
AMNQTPAASGSAGGGTTPAAQPKQQRVRARRGQATDPHSIAERLRRERIAERMKALQELVPNANKT
DKASMLDEIIDYVKFLQLQVKVLSMSRLGGAAAVAPLVADMSSEGRGEGNGGRGGNGTASSSNNDS
MTVTEHQVAKLMEEDMGSAMQYLQGKGLCLMPISLATAISTATCHSRNPMIPNGNGGNNPLLGGGG
GLATNGGGGEAGGPSSPKLSVLTVQSATIGNGGIDPSVKDATSVFEA

**SEQ ID NO: 289, N. benthamiana TC7103**
MQAMNSQMSLQEENGASSGQNMSHSHFDPTSSHDDFLQQILSSVPSSSPWPDLSSGGDGHFDDQSI
FVASKLRQNQITGGGGGGAAAKALMLQQQLLLSRGLLAGNGDQNDDVGNPENEISVQALYNGFAGS
LGQTSNQPQHFHHAQGGSMQAQSFGAPASSTMNQTPAASGGSAGGGQPKQQKVRARRGQATDPHSI
AERLRRERIAERMKSLQELVPNANKTDKASMLDEIIDYVRFLQLQVKVLSMSRLGGAAAVAPLVAD
RSSEGGGGDCAQANGGGRGSNGTTSLANNDSMTMTEHQVAKLMEEDMGSAMQYLQGKGLCLMPISL
ATAISTATCHSMKLNNPLLHGGGSGGSAAINGGGGGPSSPSLSASTVQSATMGNGGA

**SEQ ID NO: 290, N. benthamiana TC8633**
MANNPSEGPDDFFDQILGFPAYNGAETNLAGSDAGAIPPAMLLQLNSGDASGQFSGMGLGVGLGGG
GGFHHPSQSGSFPLGLSLEGGKSGFMKMDDVPAVPGRRFRDDVVDSRASSSVKPGFHGQPMPSMPH
PPAIRPRVRARRGQATDPHSIAERLRRERIAERIRALQELVPNVNKTDRAIMLDEIVDYVKFLRLQ
VKVLSMSRLGGAAAVAPLVTEIPISSVEEEISEGGNNQPAWEKWSSDGTERQVAKLMEENVGSAMQ
FLQSKALCIMPISLASAIYHSQPPDTSSLIKPETNPPS

**FIGURE 5 (continued)**

SEQ ID NO: 291, N. tabacum TC12771
MATNQPGGYADDFLEQILAIPSYAGLPPVADVGTSSETTSFTSSSVAAAGLQQPLFPLGLSLDNGR
DDVSDAGAYAVKHEREGININGNLYAGLEHLQSHAVRHAVPPVHHIQPFQGQPTTSTTVTVPHPPAI
RPRVRARRGQATDPHSIAERLRRERISERIKALQELVPSCNKTDRAAMLDEILDYVKFLRLQVKVL
SMSRLGGASAVAQLVADIPLQSVEGDSAESRSNQHIWEKWSNVDTEQEVAKLMEEDVGAAMQYLQS
KSLCIMPISLAALIYPTQQPDDPSMVKPEPAAPS


SEQ ID NO: 292, O. sativa Os02g35660
MGGFAYPFTPSPAWSRDAVFAGSPWAAGGVSSLADALVSYGAVDDEEAAFLGKTAASSPSTARLHE
QQQLLLEAELLRHGDGLGFAAMDDDGGAAMLGALEPCAMPLTDSGGPPVICSSSSNDSSGSEHSAA
MPAGGGFLVGEQQQHVPPAAYAAGGVLPSMAAGEETPQSFGFGSLFNGDLLQEATVSKYHHHQQQQ
QLGVVPSSQPHHLNDDIDFNTGKLMSFASGQQHVTPSIDSLQIDQKEFSSGLHHLNLSSLISGPLA
SFNATQSHRQPAEACGGKNGGAAPFVNLSEVLPKGNGSGSAGNGAPKPRVRARRGQATDPHSIAER
LRREKISDRMKDLQELVPNSNKTNKASMLDEIIDYVKFLQLQVKVLSMSRLGAAEAVVPLLTETQT
ESPGFLLSPRSSSGERQAGAGAVTGGLPGDQPELLDGGAMFEQEVVKLMEDNMTTAMQYLQSKGLC
LMPVALASAISAQKGTSSAAVRPEKKKNGDGDGGGDEEDVKGEFDAPRRPPVGRPKEMRSRV


SEQ ID NO: 293, O. sativa Os02g55250
MQPNARQMRGGGGGGGGGQDDFFDQMLSTLPAVWSELGSGKPAWDLTAGAVGGGGGASDDHSAAAF
DDSALLASRLRQHQIDGGGDKPIMLQLSDLHRHHGLAAGDDSGGAAGFLPLSLFADRSQDDIDAAF
KSPNGARGDHALYNGFGAAGMHGAAAMQPPPFGQGGSMPAQSFGGGAAASGGGGGGSASAAAAAGA
SSGGGAAAPPRQRQRARRGQATDPHSIAERLRRERIAERMKALQELVPNANKTDKASMLDEIIDYV
KFLQLQVKVLSMSRLGGASAVAPLVANMSSESNGNGNATSSSGNGEAANGSSNGDNNGGGTLRVTE
QQVAKLMEEDMGSAMQYLQGKGLCLMPISLATAISSATSSSLLPRTGGGAGGSLHEGGNGTSPPLV
NGTATGCDDAGVFFSVKFVVELSFLLLNEDCRGKEESKLLVQKGP


SEQ ID NO: 294, O. sativa Os03g58330
MAGQQPQQQGPPEDDFFDQFFSLTSSFPGAAPGGRAAGDQPFSLALSLDAAAAAEASGSGKRLGVG
DDAEGGGSKADRETVQLTGLFPPVFGGGGVQPPNLRPTPPTQVFHPQQSKQGGAAVGPQPPAPRPK
VRARRGQATDPHSIAERLRRERIAERMRALQELVPNTNKTDRAAMLDEILDYVKFLRLQVKVLSMS
RLGGAGAVAQLVADIPLSVKGEASDSGGNQQIWEKWSTDGTERQVAKLMEEDIGAAMQFLQSKALC
MMPISLAMAIYDTQQTQDGQPVKHEPNTPS


SEQ ID NO: 295, O. sativa Os06g08500
. MQPSSRDTVAGGGGEGTQDDFFDQMLSTLPSAWADLGGGGGGAAGKSPWEVDPAAAAAASQVFDES
ALLASRLRHHQIGGAGGGGGEKPVMLQLSELHRQAGGGEEDGSGAFSPLPLFTDRTNVPPREEMEG
GFKSPNAAAGGEHALFNGFGVHGGSGGAGQPPFGQLRRERIAERMKSLQELVPNANKTDKASMLDE
IIDYVKFLQLQVKVLSMSRLGGAAGMAPLVASMSSEGNSNGSSNGGGGKASKGGTGGEGGGGGGGG
GGGGTGGGMRVTEQQVAKMMEEDMGTAMQYLQGKGLCLMPISLASAISSATSSASLLSRPSIRHAG
APPQTMLDAAGPTSPAAMSNGDDPRHAKADGGAGGTQ


SEQ ID NO: 296, O. sativa Os06g09370
MDYSAGSYMWPGNSGSENYNFVDGSSESYAEEGSLPPSGYFMGAGSDRSLKITENERNPTMLANGC
LPYNTQAHPLSGQILPKGELPNNLLDLQQLQNSSNLRSNSIPPGVLQCNSTSGTFDAKLDTPGLAE
LPHALSSSIDSNGSDISAFLADVHAVSSAPTLCSAFQNVSSFMEPVNLDAFGFQGAQNVAMLNKTS
LPNGNPSLFDNAAIASLHDSKEFLNGGSIPSFGTVLQALGAGGLKAAQQEQNIRNIPLPTFTSGSH
LAVTDAQGPPLPSKIPPLIHDHNSEYPINHSSDVEPQANSAPGNSANAKPRTRARRGQATDPHSIA
ERLRREKISERMKNLQVLVPNSNKADKASMLDEIIDYVKFLQLQVKVLSMSRLGAPGAVLPLLRES
QTECHSNPSLSASTISQGPPDMPDSEDSSAFEQEVVKLMETSIISAMQYLQNKGLCLMPIALASAI
SNQKGMAAAAAIPPEK

**FIGURE 5 (continued)**

**SEQ ID NO: 297, O. sativa Os09g25040**
MAAAAAAGQISLDDLRNGGGVAANAGGGGGVHDDFLDQMLSSLPPSAWPDLAAGKAAEDDAEGMHH
HHHQQQQQFGGPYDESAMLASRLRQHQISGGGGGGGGGGAAAVKQMVLQQLADLRQGHHMMLQGLGG
RSPAGGGGGGGDGGLLLPLTLGSGGSGGDVQALLKAAAANSAGGGDAGGVYGGGFAGSLHQQQQHF
QPHPQTAPTIPTQSFGGGGGGGGGGGTASGGGAAQPQAGAAGGGAPAPPRQRVRARRGQATDPHSIA
ERLRRERIAERMKALQELVPNANKLMQTDKASMLDEIIDYVKFLQLQVKASTYTKLLIHVLSMSRL
GGAAAVAPLVADMSSEGRGGGAANGGAPAAAGSDSLTVTEQQVAKLMEEDMGTAMQYLQGKGLCLM
PISLASAISSATCHLRPPVVAAAQQFPAGLGAAAAAAHHHQLSAAAAAAAMRGHLPGLNADGSVPA
SPSMSVLTAQSAMANGGGGAADGEGSQLKDAASVSKP

**SEQ ID NO: 298, O. sativa Os07g08440**
MAGQQPPTGGAEDDFFDHFFSIPSAAAAGAGGVGGFGSGDHHPFPLALSLDAEGAGAARRLLDGGH
DGGRTDRDPVQLAGLFAPVFGAAAGVQPPHLRAPPPPQVFHAQPKPGEGAMAAPQPQQPPAPRPKV
RARRGQATDPHSIAERLRRERIAERMRALQDLVPNTNKTDRAAMLDEILDYVKFLRLQVKVLSMSR
LGGAGAVAQLVADIPISVKGEASDSGSKQQIWEKWSTDGTEKQVAKLMEEDIGAAMQFLQSKALCM
MPISLAMAIYDTQHSQDGHSVKPEPNTPS

**SEQ ID NO: 299, P. abies DV982110**
MLPLPLSLGQAGKSGDMNEPGSREEIEASFKSVNNARDSNLGGLFQPFAVSPRGVRPTGQNFHAQP
GQVPLQGYGGMPQPQHQNPPPTGVGAAPPVRPRVRARRGQATDPHSIAERLRRERIAERMKALQEL
VPNSNKTDKASMLDEIIDYVKFLQLQVKVLSMSRLGGAGAVAPLVADIPAEGANAPQGTRTNGSQN
SSPDGLALTERQVAKLMEEDMGTAMQYLQGKGLCLMPISLASAINNSGSRPQAPSTPSXXXLLASN
VNDRQVLEPS

**SEQ ID NO: 300, P. deltoides TA3263**
MANNPTEPPADDFLQEILGMPNFASAEAGLVGADAGLAGAAAAQASMMLQLSSGDGSGHISDLGGA
PGGGSAGFHGFPLGLSLEQGKGGFLKPEEASGSGKRFRDEIVDGRAKNVFHGQPMPTTVAIAPHPP
AMRPRVRARRGQATDPHSIAERLRRERIAERIRALQELVPSVNKTDRATMLDEIVDYVKFLRLQVK
VLSMSRLGGAGAVAPLVTDIPLSSVEDETGEGGRNQPAWEKWSNDGTERQVAKLMEENVGAAMQFL
QSKALCIMPISLATAIYHTQPPDTTSIVKPETNPQS

**SEQ ID NO: 301, P. radiata WO2005001050 seqID516**
MSLLPPGIGGQGLNSQENDPYPYDDSQFLANRLRQHQLSGNSSMNQTPNQASQGINETNTSPGRSM
VLQLSTGSASASQLLASMGNSPRGTAVMTQSPSTGGSCNGSDGGMLPLPLSLGQAGKSGDINESRS
RDEIEASFKSANHARDSNLGGLFPPFAASPRGVRPTGQNFHTQSGQVPMQVYGGMPQPQHQNPSPT
GVGAAPPVRPRVRARRGQATDPHSIAERLRRERIAERMKALQELVPNSNKTDKASMLDEIIDYVKF
LQLQVKVLSMSRLGGAGAVAPLVADIPAEGANAPQGTRTNSSQNSSPDGLALTERQVAKLMEEDMG
SAMQYLQGKGLCLMPISLASAINNSGARPQAPSTPSLQGLLPPNANDRQILEPSNSALSALTSTSM
TIQSSISSPGSGITEQGDNAHKAVNGTRNPKDSREANSVTKSNGIGGPALSKNAVKGEDEPQRRTG
Q

**SEQ ID NO: 302, P. sitchensis TA14134**
MDDIFDQLLSSSWEDINGADRSSLDSSAGGPTNCLPGDSMGTLQASSRVSTMSVTPSSHMSPNLEG
QAQNNDVQNSSSSVIAGENTPYLLKELLVCSQATDPHSIAERERRERIAKNLKSLQELVPNANKTD
KASMLDEIIDYVKFLQLQVKVLSMSRLGGAGAVAPLIADVPAEGSGSLASAALGQAGGSLSQDGLA
FEQNVVKLMEKDMTAAMQYLQNKGLCLMPIALATAISSSTGKPLLGSGVTNSVDSTSDRQSSGNQP
ASLTVDSCSGALGMGSAGFGSDFLLKENTVEKRGRELVPTAESNGAEFGIISSLPKLRKKE

**FIGURE 5 (continued)**

**SEQ ID NO: 303, P. taeda TC68930**
MLARENRSGDTEHDDLQGTLLTSYTGPQGGQTVLPRTPGAQSHQQNLSTQTIQSGEGTSLQQYGNH
SAVSQLQSGAGGGNAANGAARPRVRARRGQATDPHSIAERLRRERIAERMKSLQELVPNSNKTDKA
SMLDEIIEYVKFLQLQVKVLSMSRLGGAGAVAPLIADVPSQGSGGVMSTALGQASGPLTLSQDGLA
FEQEVARLMESNMTSAMQYLQNKGLCLMSIALATAISSSSGKPVLATVPGTGVDSSEKQNSDMQSI
VLPFSSSSTTMGLRATASGSDAPINENSINKASIEKVVAEKSNGISPGLSSDVPKVGSQSREELLK
RAQ

**SEQ ID NO: 304, P. trichocarpa scaff II. 416**
MANNPTEPPTDDFLQEILGMPNFASAEAGLVGADAGLAGTASVQAPMMLQLSSGDGSGHISALGGA
PGGGGAGFHGFPLGLSLEQGKGGFLKPEEASGSGNRFRDDIVDGRVRNVFHGQPMPTTVTAATHPP
AMRPRVRARRGQATDPHSIAERLRRERIAERIRALQELVPSVNKTDRAAMLDEIVDYVKFLRLQVK
ILSMSRLGGAGAVAPLVTDIPLSPVEDETGEGGRNQLAWEKWSNDGTERQVAKLMEENVGAAMQFL
QSKALCIMPITLATAIYHTQPPDTTTIVKPETNPPS

**SEQ ID NO: 305, P. trichocarpa scaff 70. 65**
MAGNPPPEGLGDDFFEQIILAGQPPGYGGEAVGSTSLPMMGLQLGSSAANVGLMRSSANNNMGMMPL
GLNLEHHGFLRQQQDDGSSSLDTNNSNNINNASPFSITSAGFLGRDSVHMTSLFPTFGQLQIHSSI
RSAPPPLGPPQIHQFNSQPTSGAVSAVPQPPGIRPRVRARRGQATDPHSIAERLRRVRITERVKAL
QELVPTCNKTDRAAMLDEIVDYVKFLRLQVKVLSMSRLGAAGAVAQLVADVPLSSVQGEGIEGGAN
QQAWENWSNDGTEQEVAKLMEEDVGAAMQLLQSKALCIMPVSLASAIFRARPPNAPTLVKPESNPP
S

**SEQ ID NO: 306, P. trichocarpa scaff XIII. 403**
MAGNPPPEGLGDDFLEQILAAQPPGYGGGGEVMGSTSLPMMGLQLGSCAGNVGLLRSNANNNMGMM
PLGLNLEHHGFLRQQQDDSGSSLDTNNSNNINNTPPSIKSARILGRDSVHMTSLFPTFGHLQIHSS
IRPTPPPPGPPQIHQINSHPNPGAVSAVPQPPGIRPRVRARRGQATDPHSIAERLRRVRITERVKA
LQELVPTCNKTDRAAMLDEIVDYVKFLRLQIKVLSMSRLGAAGAVAQLVADVPLSSVQIKGEGNEG
GANQQSWENWSNDDTEQEVAKLMEEDVGAAMQFLQSKALCIMPISLASAIFRARPPNASTLINTES
NTPS

**SEQ ID NO: 307, P. trichocarpa scaff28. 86**
MISSNQSVESLATQDTSSVVLGSESDYAVDKVLISEQARLQNDCQNCNGNPSPDGMARGNLKFGNT
GLQCNGILPTLSSLNYPNQLPIVGDLTSYLSFSEASNAGCNGREQSEYLRSLKNLQNLSSIPQLWP
SQSYEGVSSLPPLMGQDRIEGSGLRGGNLDDDMHIMGKGYMGMDEILRLDKLSASPTTEGKEDLQS
CPFSSGIAEPNVNMSMNQLSSMPQTTSAAPVEGCNGTGKTRVRARRGHATDPHSIAERLRREKIAE
RMKNLQELVPNSNKVDKASMLDEIIEYVKFLQLQVKVLSMSRLGAAGAVIPLLTDGQPEGHNSLSL
SPSAGLGIDISPSADQIAFEQEVLKLLESDVTMAMQYLQSKGLCLMPIALAAAISSVKASLSGTTS
EERKNNGYTSGLVSSSSSITGIDTHPMSNDNNIATGTLSSKGMIVNGCNEVVKQEVLKNT

**SEQ ID NO: 308, P. trichocarpa TC63334**
MANNPTEPPTDDFLQEILGMPNFASAEAGLVGADAGLAGAAAAQASMMLQLSSGDGSGHISDLGGA
PGGGSAGFHGFPLGLSLEQGKGGFLKPEEASGSGKRFRDEIVDGRAKNVFHGQPMPTTVAIAPHPP
AMRPRVRARRGQATDPHSIAERLRRERIAERIRALQELVPSVTKTDRATMLDEIVDYVKFLRLQVK
VLSMSRLGGAGAVAPLVTDIPLSSVEDETGEGGRNQPAWEKWSNDGTERQVAKLMEENVGAAMQFL
QSKALCIMPISLATAIYHTQPPDTTTIVKPETNPPS

**FIGURE 5 (continued)**

**SEQ ID NO: 309, P. trifoliata TA5414**
MQPCSSGGEMQGISSLLSNGSHEQQSQIHQSATFDPTSQDDFLEQMLSSLPSCSWTDLKSPWGVVD
LNPNNNNNNINNKQPRDLSDETAPSTTQENNVPAGFQFDESMILASKMRQHQISGNTGSGNNNSAA
AKFMMQQQQQQQIMMAAARGGIGLPLSLGNGGDNDIVDASSFKSQQGGDGSVQALYNGFTGSLHGS
TQPQHFHHLQGGSMPGQTFGAPGPVMNQTQAQASGSTGGGGNTPAQQPKQRVRARRGQATDPHSIA
ERLRRERIAERMKALQELVPNANKTDKASMLDEIIDYVKFLQLQVKVLSMSRLGGAAAVAPLVADM
SSEGAGGGDCIQANGRNPNGAQTTSANDSLTVTEHQVAKLMEEDMGSAMQYLQGKGLCLMPISLAT
AISSATCHSRNPIISTSNNNNNNGNPHHNPLFQSNGEGPTSPSMSVLTVQSATMGNGGADGSVKDA
ASVSKP

**SEQ ID NO: 310, R. communis TA1616**
MAGNPPPEGLGDDFFEQILAVQPGYGGGDGGGGGDVVGSTMPMMGLQLGSASSGGGGLRTNNMGMM
PLGLNLEFLRQQEDGSGALDNNNHTNNNNNNVSSSTTSVINDRDSVHMASLFPTFGQLQTQTLRPP
PPPHLHQPFHGQPTPGVVSAASQPPAIRPRVRARRGQATDPHSIAERLRRERIAERMKALQELVPT
ANKTDRAAMIDEIVDYVKFLRLQVKVLSMSRLGAAGAVAQLVADVPLASVEGESIDGAAANQQTWE
KWSNDGTEQQVAKLMEEDIGAAMQFLQSKALCIMPISLASAILRTHPPDAPSIIKPESNTPS

**SEQ ID NO: 311, R. communis TA2825**
MANNPTEPPADDFLQEILGLPNFASADAAGLVGADGALATPMMLQLSSGDGSNHITALGGGGGGGG
GAGFHGFPLGLSLEQGKGGFLKPEEASGSGKRFRDDVVDGRANTVKNVFHGQPMPTTMAAAPHPPT
MRPRVRARRGQATDPHSIAERLRRERIAERIRALQELVPSVNKTDRAAMLDEIVDYVKFLRLQVKV
LSMSRLGGAGAVAPLVTDIPLSSVEDETGEGGRNQPAWEKWSNDGTERQVAKLMEENVGAAMQFLQ
SKALCIMPISLATAIYHTQAPDTSTIVKPETNPPS

**SEQ ID NO: 312, S. bicolor TC104646**
MAGQPPPPGGSEDDFLEHFFAFPSAASAAAGGHAGAGAGGDHPFPLALSLDAAAEPKPDRDPVQLA
GLFPPVFAGAGGVQQPHLRGPPPPQMFQAQPKPGEGGMAPQPPAPRPKVRARRGQATDPHSIAERL
RRERIAERMRALQELVPNTNKTDRAAMLDEILDYVKFLRLQVKVLSMSRLGGAGAVAQLVADIPLS
VKGEASDSGSTQHIWEKWSTDGTEKQVAKLMEEDIGAAMQFLQSKALCMMPISLAMAIYDTQHSQD
GHSLMKPEPNTSS

**SEQ ID NO: 313, S. lycopersicum AK247217**
MANNPSEGPSDDFFDQILGFPAYNGAEPNLAGNDAGAIPPAMMLQLNSGDGSSQFTGVGLGVGLGG
GGFHGHGGGGSFPLGLSLEQGKGGFLKMDDVSAPGRRFRDDVVDSRASSSVKPGFHGQPMPSMPHP
PAIRPRVRARRGQATDPHSIAERLRRERIAERIRALQELVPSVNKTDRAVMLDEIVDYIKFLRLQV
KVLSMSRLGGAGAVAPLVTDIPISSVEEESSEGGNNNQPAWEKWSSDGTERQVAKLMEENVGAAMQ
FLQSKALCIMPISLASAIYHSQPPDTSSLVKPETNPPS

**SEQ ID NO: 314, S. lycopersicum TA21665**
MADNPPEVYAADDFLEQILAIPSYASLPVTDLTAGASSENSTSGVSQLQQQPLFPLGLSLDNGFAD
ANNTGGFQVKTEREAMNMGNLYPGLEHLQSHAVCLSVPQVHQVQPFQGHPTSSAIVTIPHQPAIRP
RVRAQRGQATDPHSIAERLRRERISERIKALQELVPSCNKTDRAAMLDEILDYVKFLRLQVKVLSM
SRLGGTSAAAQVVADIPLQSVEGDTCESHSNQHVWEKWSDSETEQEVAKLMEEDVGTAMQYLQSKS
LCIMPISLAALIYPTQQSDNQSMVKPEQAAPL

**FIGURE 5 (continued)**

SEQ ID NO: 315, S. lycopersicum TC172581
MAANQPEGYADDFLEQILAIPPYSGLPVADVGTPSETTSFTSASAVSHLNSAAAAGLQQPLFPLGL
SLDNGRDDVGDAGPYAVKHERDGMNIGNLYAGLEHLQSHAVRHSVPSVHHVQPFQGPPTTSTTVTV
PHPPSIRPRVRARRGQATDPHSIAERLRRERISERIKALQELVPSCNKTDRAAMLDEILDYVKFLR
LQVKVLSMSRLGGASAVAQLVADIPLNLWEGDSGESRSNQHIWDKWSNVDTEREVAKLMEEDVGAA
MQYLQSKSLCIMPISLAALIYPTQQPDDQSLVKPEAAAPS

SEQ ID NO: 316, S. tuberosum TA30646
MANNPSEGPSDDFFDQIMGFPAYNGAETNLAGNDAGAIPPAMMLQLNSGDGSGQFTGVGLGVGLGG
GGFHGHGGGASFPLGLSLEQGKGGFLKMDDVSAPGRRFRDDVVDSRASSSVKPGFHGQPMPSMPHP
PAIRPRVRARRGQATDPHSIAERLRRERIAERIRALQELVPSVNKTDRAVMLDEIVDYVKFLRLQV
KVLSMSRLGGAGAVAPLVTDIPISSVEEESSEGGNNNQPAWEKWSSDGTERQVAKLMEENVGAAMQ
FLQSKALCIMPISLASAIYHSQPPDTSSLVKPETNPPS

SEQ ID NO: 317, S. tuberosum TA28621
MAANQPEAYADDFLEQILAIPSYSGLPVADVGTPSETTSFTSASAVSHLNSAAAAGLQQPLFPLGL
SLDNGRDDVSEAGAYAVKHERDGMNIGNLYAGLEHLQSHAVRHSVPSIHHVQPFQGPPTTSTTVTV
PHPPSIRPRVRARRGQATDPHSIAERLRRERISERIKALQELVPSCNKTDRAAMLDEILDYVKFLR
LQVKVLSMSRLGGASAVAQLVADIPLQSVEGDSGESRSNQHIWDKWSNVDTEQEVAKLMEEDVGAA
MQYLQSKSLCIMPISLAALIYPTQQPDDQSLVKPEAAAPS

SEQ ID NO: 318, S. tuberosum TA34455
MQAMNSLLSQQQQSQMSLQDLQNGGNGGSTGGVGGLGQHNMGHFHFDPTSSHDDFLEQILSSVPSS
SPWPDLSKSWDPHHHLSSPPHNPSSGEDQPPSNPLHSQFHYDDQASSLLASKLRQHQITGGGAAAA
AKALMLQQQLLLSRTLAGNGLRSPNGASGDNGLLSLPLNLSNNGDQNDGVANPANDNSVQALFNGF
TGSLGQTSNQPQHFHHPQGGSMQSQSFGAPAMNQTPAASGSAGGGGGSTPAAQPKQQRVRARRGQA
TDPHSIAERLRRERIAERMKALQELVPNANKTDKASMLDEIIDYVKFLQLQVKVLSMSRLGGAAAV
APLVADMSSEGRGEGNVGRGGNGTAASSSNKETMTVTEHQVAKLMEEDMGSAMQYLQGKGLCLMPI
SLATAISTSTRIPLLSPEAGRPASPTLSALTVQSATIGNAAGKDATSLFEA

SEQ ID NO: 319, S. tuberosum TA37666
MQAMNSFQSTGENGASSGEHISHSHFDPSSSHDDFLQQILSSVPSSSPWPEISGDGHPYNFDDHQS
TLLASKLRQHQINGGSSAAAAAKALMLQQQLLLSRGIAGNGGDQNDDGLNPGNDISVQALYNGFAG
SLGQTSNQSQHFHHSQAQSFGAPAASLTMNQTPAASGSAGGAQPKQQKVRARRGQATDPHSIAERL
RRERIAERMKSLQELVPNANKTDKASMLDEIIDYVRFLQLQVKVLSMSRLGGAAAVAPLVADRSSE
GGGDCVQGNGGRGGSNGTTSSANNDSSMTMTEHQVAKLMEEDMGSAMQYLQGKGLSLMPISLATAI
STSTCHSMKPNNPLLLGGGSAINGGGETGGGPSSPTLSASTVQSATMGNGGT

SEQ ID NO: 320, T. aestivum TC253044
MLLCSTIQVIQMGNLSVFDEATMASLHDSKEFLSGSISSFGTAEQSQLAGSGLKAEQQEQNAMCNI
PLPFASGSQMAVSEAQGAMIPSKISSTIHNNKSEYPVPISHSADAQNKANSANGNSASAKPRARAR
RGQATDPHSIAERLRREKISERMKNLQDLVPNSNKADKSSMLDEIIDYVKFLQLQVKVLSMSRLGA
PGAVLPLLAESQTEGRSNSPLSSPTASQGLLDAAGPEDSLVFEQEVIKLMETSITNAMQYLQNKGL
CLMPIALASAISNQKGTSAAAIPPER

FIGURE 5 (continued)

**SEQ ID NO: 321, V. vinifera GSVIVT00016367001**
MDEYLDHLFSSTSWSDVNVKEGSSWICGEPSQTNGMLSDSIGIYEGDKKNSPVGMTNSNLMIEGLV
TQDTSSIVLGGESDHGLGKGLLLEEAPRQQDLQNTEGNSSLNGAVNGSSEVGYVGLQLNTPTSTPC
SLDLGSPKQLSLIGGMSRSSRPFTELDHVGCNGNEPSDFQRSVGNFQALPPIPQLWSQPSYGGGSS
LSPVMGEYKMQGFGLQGEYVDNEMDIMRNRYVGDEILQLDNISSAIPIKGKEEQHTHPFSPSAVGP
HMTMTASGLQSLPQTTVGTASGGCNGTGKPRVRARRGQATDPHSIAERLRREKIAERMKNLQELVP
NSNKTDKASMLDEIIEYVKFLQLQVKVLSMSRLGAAEAVVPLITDGQAEGSKGLSLSPSAGQAEDI
CQSPDQIAFEQEVVKLMESNVTMAMQYLQSKGLCLMPIALATAISSGKAASSGTGSDEGKNAATPV
AATPVATAYLALGHIIHLLMAMF

**SEQ ID NO: 322, V. vinifera GSVIVT00017237001**
MLSTLPSWSDLPANPKSPWELNASNPISMPSNKSRDLSDDTTPSNPDNVQFAFDESAMLASKLRQH
QISGNSSAAKSALMLQQQLLLSRGVAMGRSPSNGSGAGESGLLQLPLSLSNGDSCLVDRSQNDVVD
GSSSFKSPNQGGDGSVQALYNGFAGALHGSGQASNQAQNFHHPQGGSMQAQNYGAPATRVRARRGQ
ATDPHSIAERLRRERIAERMKALQELVPNANKTDKASMLDEIIDYVKFLQLQVKVLSMSRLGGAAA
VAPLVADMSSEGGGDCIQASGTSGPTGGRATNGTQTTTSNDSLTVTEHQVAKLMEEDMGSAMQYLQ
GKGLCLMPISLATAISTTTCHSRNPMVAAAAVAASNINNGSHTHPLLPNSNADGPSSPSMSVLTVQ
SATMGNGLADAPVKDAASVSKP

**SEQ ID NO: 323, V. vinifera TA46156**
MASNPSEAPADDFLEQILGIPTYPAADPNLAANDVNLAAPMVLQLGSGEGSGHIAGGYQGTMFPLG
LRLEQGKSSFLKPEDASGSGKRFREEVIDGRASTVKNAFHGQPMQATVAAAPHPPAIRPRVRARRG
QATDPHSIAERLRRERIAERIRALQELVPSVNKTDRAAMLDEIVDYVKFLRLQVKVLSMSRLGGAG
AVAPLVTDIPLASVEEEASEGGRNEPAWEKWSNDGTERQVAKLMEENVGAAMQFLQSKALCIMPIS
LASAIYHSQQPDTTPLIKPQTNPPS

**SEQ ID NO: 324, V. vinifera TA46194**
MATNPPEGYADDFLEQILAIPSYPGHDPNMVGTGSTMVLQLSSGDGSGHVAGGGFQGPVFPLGLSL
ESGIPATQVAPGSGERFRDDVDARGSSGRSERESVHLDGLFPAYGHVQSLSVRPAVPQVHQAFHGQ
PTPVPITAAPHPPAIRPKVRARRGQATDPHSIAERLRRERIAERMKALQELVPSSNKTDRAAMLDE
IVDYVKFLRLQVKVLSMSRLGGAGAVAQLVADIPLPAVEGETGEGGSNQQAWDKWSNDGTEREVAK
LMEEDVGAAMQFLQSKALCIMPISLAAAIYPAHQTDTPTLIKPEPNAPS

**SEQ ID NO: 325, Z. mays TA139285**
MAGQPPPQGPEDDFFDQFFSMTAGGSYPGATAGGGRAPGDQPFSLALSLDAAAAEASGSGKHADGG
KADREAIQLPGLFPPAFGGGVQPPHLRATPPTQVFHAQQPKQGGAAVGPQPPAPRPKVRARRGQAT
DPHSIAERLRRERIAERMRALQELVPNTNKTDRAAMLDEILDYVKFLRLQVKVLSMSRLGGAGAVA
QLVADIPLSVKGEASDSGSKQQIWEKWSTDGTERQVAKLMEEDIGAAMQFLQSKALCMMPISLAMA
IYDTQHSQDGQPVKPEPNTPS

**SEQ ID NO: 326, Z. mays TA126400**
MAGQPPPSGASEDDFLEHFFAFPSAASAGAAGGHAGAGVGGDHPFPLALSLDAAAAEAKPDRDPVQL
AGLFPPVFAGAGGVHQPHLRGPPPPQMFQAQPKPGEGGMAPQPPAPRPKVRARRGQATDPHSIAER
LRRERIAERMRALQELVPNTNKTDRAAMLDEILDYVKFLRLQVKVLSMSRLGGAGAVAQLVADIPL
SVKGEAGDGGGAPQQQQQQHVWEKWSTDGTEKQVAKLMEEDIGAAMQFLQSKALCMMPVSLAMAIY
DTQHPLDGHGHSLKPEPNASS

**FIGURE 5 (continued)**

**SEQ ID NO: 327, A. cepa TC3698**
CACTTCACTTCCCCTTTCTTCATTTCTTCAATACCTACAAACCCTAGCTTCACTTTACTATTTAAA
AATGGCGTCTAATAACAACCCGCAACCTCCTTCCGATGGACTCAACGACGACTTCTTCGACCAAAT
TTTTTCCATGCCTTCCGCCTTCGCCGCCTCCTCAGCCTCTACTGACGCCACCGAAGCTCTCAACTA
TGCTGATTCCTCCACCACTGGGGTTACCCCGATGTTCTCACTTGGCCTCAGCTTGGATCAGCAGCC
TAAGTCTGATTCGTCCCAATCGGAAAGAGAGCATCCGGTTCAGTTTGCTTGCTTGTTTCCACAGTA
TGGGCACAATAATCAAGTTCATCAAACCCGGCCTAATCTTTCTCCTCCTCAAGTTCTTCATGGACA
AGCAATGCAGAGCAGTATGGCTGCAACACCACAACCTACAGGTCCACGGCCAAGGGTTAGGGCTAG
GAGAGGCCAAGCTACCGATCCTCATAGTATAGCTGAGCGATTGAGGAGGGAAAGAATAGCAGAAAG
GATTAGAGCTTTGCAGGAATTGGTGCCCAGCACTAATAAGACCGATAGAGCTGTGATGCTGGATGA
AATTGTAGAGTATGTGAAATTCTTGAGGCTTCAAGTAAAGGTACTAAGCATGAGTAGACTAGGTGG
TGCTGGTGCTGTTGCACAGCTGGTAGCCGATATCCCTATGTCTTCTTCAGTTGAGGGTGATTCAAG
TGAAAGTGGGAAGACAAGCTACCAAGGGTGGGAAAAATGGTCAACCGACGGCACCGAGCGTCAGGT
GGCAAAACTAATGGAAGAAGATGTAGGGGCTGCAATGCAATTTCTCCAATCAAAAGCCCTCTGCAT
CATGCCCATATCTTTAGCAGCTGCAATTTGTCAAACGAATCCCTCAACCGAAATCTCTTTCATG

**SEQ ID NO: 328, A. thaliana bHLH007-11-AT1G03040**
GTCTGTGTCTCTTCGTCCTCATTAGCTTAAGCATCATCATTCTCTCACCTAACAACTCCACACAAG
ATTCTTCGCATGGAAAGTTGTTCTTCGACTTCTCTTCTCTAACTCGCTATCTTTTAACTCACCCAG
CTCCACTGAGTCGAAAATTTCAAACCTTTACTCGTTTCCTTCATGGCTAATAACAACAACATCCCA
CATGATAGCATCTCCGATCCATCTCCTACCGACGATTTCTTCGAGCAGATCCTCGGGCTTTCCAAC
TTCTCCGGTTCTTCAGGTTCTGGTCTCTCTGGAATCGGCGGCGTGGGTCCACCTCCGATGATGCTT
CAGCTTGGTTCAGGCAACGAAGGGAATCATAATCATATGGGTGCCATTGGAGGAGGTGGACCTGTA
GGGTTTCATAATCAGATGTTTCCGTTGGGATTAAGTCTCGATCAAGGGAAAGGACATGGCTTTCTT
AAACCTGATGAAACTGGTAAACGTTTCCAAGACGATGTTCTTGATAATCGATGTTCCTCTATGAAA
CCTATTTTCCATGGGCAGCCAATGTCACAGCCAGCTCCACCAATGCCGCATCAACAGTCTACTATT
CGGCCTAGAGTTAGGGCTAGGCGAGGTCAAGCTACCGATCCACATAGCATCGCTGAGAGGCTCCGA
AGGGAAAGAATAGCAGAACGGATCAGGTCGTTGCAGGAACTTGTACCTACCGTTAACAAGACAGAT
AGGGCTGCTATGATCGACGAGATTGTCGATTATGTAAAGTTTCTCAGGCTCCAAGTTAAGGTCCTG
AGCATGAGCCGTCTTGGTGGAGCCGGTGCTGTCGCACCACTAGTCACTGAAATGCCATTATCTTCA
TCAGTTGAGGATGAGACGCAGGCCGTGTGGGAGAAATGGTCAAACGATGGGACAGAGAGGCAAGTG
GCTAAGCTGATGGAAGAAACGTTGGAGCAGCGATGCAACTTTTGCAATCAAAGGCTCTTTGCATA
ATGCCGATCTCATTGGCAATGGCGATTTACCATTCTCAGCCACCAGACACATCTTCTTCAATCGTC
AAACCAGAGATGAATCCTCCACCGTAGATTTTTGTTCATCCAACGGTCCCCAGCTGATGATTGACA
TTTTGCTCTGTTTCCCACTACTAGACTTTTGTGACTCATGAAAGGTAAGTAAAAAGGCATTGGAGA
TGGAATCTAAGTAGGATTTGTGCAGTAAAGAAGTAAAACGGGATCTGTCAAAAGAAGGAAAAAGCT
CTCGCTTGCTTGGCTAGTATTTATCATTTTGATGAAAGTAACTCTTTTTTGTTCAAAGACTTTAGT
GTGATTTTCAGGACCAAGGGCTTTGAGGGTAGTGCTAGCTGTAGTAATAGTAATGAAGGTGTGGGA
TCGTGTTTCTGAATTATGTAAAAAAGGAAGAAAAAACAAATGTTGGTATTATATTATGGTTTTGCC
TCTC

**SEQ ID NO: 329, A. thaliana bHLH059-11-AT4G02590**
GCTCCTTTCTCGTCTCTGTCTTCTTCGTCCTCATTCGTTTTAAAGCATCAAAATTTCATCAACCCA
AAATAGATTAAAAAAATCTGTAGCTTTCGCATGTAAATCTCTCTTTGAAGGTTCCTAACTCGTTAA
TCGTAACTCACAGTGACTCGTTCGAGTCAAAGTCTCTGTCTTTAGCTCAAACCATGGCTAGTAACA
ACCCTCACGACAACCTTTCTGACCAAACTCCTTCTGATGATTTCTTCGAGCAAATCCTCGGCCTTC
CTAACTTCTCAGCCTCTTCTGCCGCCGGTTTATCTGGAGTTGACGGAGGATTAGGTGGTGGAGCAC
CGCCTATGATGCTGCAGTTGGGTTCCGGAGAAGAAGGAAGTCACATGGGTGGCTTAGGAGGAAGTG

**FIGURE 5. (continued)**

104

GACCAACTGGGTTTCACAATCAGATGTTTCCTTTGGGGTTAAGTCTTGATCAAGGGAAAGGACCTG
GGTTTCTTAGACCTGAAGGAGGACATGGAAGTGGGAAAAGATTCTCAGATGATGTTGTTGATAATC
GATGTTCTTCTATGAAACCTGTTTTCCACGGGCAGCCTATGCAACAGCCACCTCCATCGGCCCCAC
ATCAGCCTACTTCAATCCGTCCCAGGGTTCGAGCTAGGCGTGGTCAGGCTACTGATCCACATAGCA
TCGCTGAGCGGCTACGTAGAGAAAGAATAGCAGAACGGATCAGGGCGCTGCAGGAACTTGTACCTA
CTGTGAACAAGACCGATAGAGCTGCTATGATCGATGAGATTGTCGATTATGTAAAGTTTCTCAGGC
TCCAAGTCAAGGTTTTGAGCATGAGCCGACTTGGTGGAGCCGGTGCGGTTGCTCCACTTGTTACTG
ATATGCCTCTTTCATCATCAGTTGAGGATGAAACGGGTGAGGGTGGAAGGACTCCGCAACCAGCGT
GGGAGAAATGGTCTAACGATGGGACTGAACGTCAAGTGGCTAAACTGATGGAAGAGAACGTTGGAG
CCGCGATGCAGCTTCTTCAATCAAAGGCTCTTTGTATGATGCCAATCTCATTGGCAATGGCAATTT
ACCATTCTCAACCTCCGGATACATCTTCAGTGGTCAAGCCTGAGAACAATCCTCCACAGTAGGATT
TCTGCAATAAAGAGTTTGTACAGCTAATCCAACTGTCCAACATGGGTTTTTCTTCTGCTCTAATGA
CTCTGGTTTCTTCTCTCCTCTCTCACCCACTTGAAAGGTAAAAAAGTGAAAAAGGCTTTGTAGATG
GAATCAATGTAGGATTTGCAGTAGAGGGAAAAAAAATGTCAAAAAGCTCAATTGATCAAGTATTAT
TGTAATCATTGTACCTTTATTTTAGGTGGACTTTGATGAAAGCAACTTTTTGTTTTCAAGACTTTA
GTGGGAGGTTGAGGAAGGAGCTTGAAGGGTGTTATTTATTAGTAGTAGTAGTAGTGGGAAGTTGTG
GGACCTTGTTGAGTTGTGTTCAAATTGAAGAAAAAACAAGTATTTGTAATTTGTCACCCCTTGTAT
TATTATTTATTTTGTATGACTTTGGAGTAGTATAATTAATTATCAAATAATATTATTAGTTTGATC
TAGT

**SEQ ID NO: 330, A. thaliana bHLH066-11-AT2G24260**
TCTTCCCAAAAAAAAGTGTAGAAGCAGAAGAAACCCATCATCATCATGATGAACTCTTCTCTTCTA
ACTCCTTCTTCTTCATCTTCTTCCCATATCCAAACTCCATCAACAACTTTCGACCACGAAGACTTC
CTCGATCAAATCTTTTCCTCGGCCCCGTGGCCCTCCGTCGTCGATGATGCTCATCCTCTTCCCTCC
GATGGCTTCCACGGCCACGATGTCGACTCAAGGAATCAGCCGATCATGATGATGCCTTTGAATGAT
GGCTCCTCCGTCCACGCTCTTTATAATGGCTTCTCCGTCGCCGGATCTCTTCCTAACTTTCAAATC
CCTCAGGGATCGGGAGGAGGATTGATGAACCAACAAGGACAAACGCAAACGCAAACGCAACCTCAG
GCGAGCGCGTCTACAGCTACTGGTGGTACGGTGGCGGCTCCGCCGCAGAGTAGGACTAAAATCCGA
GCTAGGAGAGGTCAAGCAACTGATCCTCATAGTATCGCCGAAAGGTTACGAAGAGAGAGAATTGCG
GAAAGAATGAAAGCTCTTCAAGAACTCGTTCCTAACGGCAATAAGACAGACAAGGCATCGATGCTC
GATGAGATCATAGATTATGTCAAGTTTTTACAACTCCAAGTCAAGGTACTGAGCATGAGCAGATTG
GGAGGTGCTGCTTCCGTTTCTTCTCAAATCTCCGAGGCTGGTGGATCCCACGGGAACGCATCCTCC
GCCATGGTCGGCGGTAGCCAGACGGCCGGAAACTCCAACGACAGCGTTACAATGACGGAACATCAA
GTGGCCAAACTAATGGAAGAAGACATGGGCTCGGCCATGCAATATCTTCAAGGGAAAGGTCTTTGT
CTCATGCCAATCTCTTTAGCCACCGCCATCTCAACCGCCACGTGTCACTCCCGTAACCCTTTGATC
CCTGGAGCTGTTGCCGACGTCGGAGGTCCTTCCCCTCCCAATCTTTCTGGCATGACCATACAGTCG
ACGAGTACAAAAATGGGTAGCGGTAATGGGAAATTAAACGGTAACGGCGTGACCGAGAGGTCGTCT
TCTATCGCCGTTAAAGAGGCCGTATCCGTTTCGAAAGCGTGATAACGGCCGTTTACTTTTGGGGTT
AGGCAGAGAGATACCAAAAACAAAGAGAAAGTGGGGTGAAGTGGGAGATAAAGTCAAAGTCTACGA
AGAATGAGGTTGAGGTTGTTTGATGTGTTCTCTGAACGAAGCCATATTTTTCGTAGAGTTTTGGTT
CTTTTACCTACGCACTACAAAACCATTCAAGGGACATTTCTTTTTCTTTTTTAAATATATGGTTGC
AATATGTTTTATTTTTATTAAATAAAGTTGGTATCGTTGATCTGAATATTAGGCTAGAAAAAGAAA
AAAATAGTATATTGGTTTTTC

**SEQ ID NO: 331, A. thaliana bHLH069-11-AT4G30980**
AACATTCATTTTCATTCTCACTCCCCACCAAAAAAAAAAAAAAAACAGAAGCCATGAACTCCTCGTCT
CTTCTAACTCCTTCATCATCTCCTTCTCCACATCTTCAATCTCCTGCAACATTCGACCACGATGAT
TTCCTCCACCACATCTTCTCCTCCACTCCTTGGCCCTCATCCGTTCTCGACGACACTCCTCCACCA

**FIGURE 5 (continued)**

ACTTCCGATTGTGCCCCCGTCACTGGATTCCACCACCACGACGCCGATTCAAGAAACCAGATCACT
ATGATTCCTTTGTCACATAACCATCCTAATGACGCTCTCTTCAATGGCTTCTCCACCGGATCTCTC
CCTTTCCACCTCCCTCAAGGATCGGGAGGTCAAACGCAAACGCAGTCGCAGGCGACGGCGTCAGCC
ACCACCGGTGGTGCAACGGCGCAACCTCAGACAAAGCCTAAAGTCCGAGCTAGGAGAGGTCAAGCC
ACTGATCCTCACAGTATCGCCGAACGGTTACGGAGAGAGAGGATAGCGGAAAGAATGAAATCTCTT
CAAGAACTTGTCCCTAATGGTAACAAGACAGACAAAGCATCAATGCTCGATGAGATTATCGATTAT
GTCAAGTTCTTACAGCTCCAAGTCAAGGTACTAAGCATGAGTAGACTGGGCGGTGCTGCTTCTGCT
TCTTCTCAAATCTCTGAGGATGCCGGTGGATCCCACGAAAACACCTCCTCCTCCGGCGAGGCGAAG
ATGACGGAGCACCAAGTTGCAAAGCTAATGGAAGAGGACATGGGATCAGCCATGCAATATCTACAA
GGCAAAGGTCTTTGCCTCATGCCCATCTCGTTAGCCACCACCATCTCCACCGCCACGTGTCCTTCT
CGTAGCCCCTTCGTTAAAGATACCGGCGTTCCTTTGTCTCCTAACCTATCCACTACAATAGTTGCT
AACGGTAATGGCTCATCGTTGGTCACCGTTAAAGACGCTCCCTCCGTTTCCAAGCCGTGATAACGG
CCATTTGTCCATTTCATTTTCCCTTTTTTGGGTGGGAAAGAGAGAAAAAAGTTTAGAAGACAAAGA
CAAGTGGGATAGGTGGTTTTGGTCAAAGTTTAGAAAGAATAAGGTCGTGTTTTCGGATACGACACC
GTATTTGCGTACACTTTGGTTTTCTGTCTTTACCTACTACAAACCACCCATAAGCACACTCATGTT
ATCATGTTTTTTTTTTTTGGTTTATAAAGTTATATCCTTAA

## SEQ ID NO: 332, A. thaliana bHLH082-11-AT5G58010

ATGGAAAATGGAAATGGAGAAGGAAAAGGAGAATTCATAAACCAAAACAATGACTTCTTCCTTGAT
TCCATGTCAATGCTCTCCTCTCTCCCTCCTTGTTGGGATCCATCTCTTCCTCCTCCTCCTCCTCCG
CCACAATCTCTTTTCCACGCTCTAGCCGTCGACGCCCCCTTCCCCGACCAGTTCCATCATCCTCAG
GAGTCAGGAGGTCCAACAATGGGTAGCCAAGAAGGGTTACAGCCACAAGGTACAGTGTCGACCACG
AGCGCACCTGTTGTTCGTCAAAAGCCAAGAGTTCGAGCCAGGAGAGGCCAAGCCACCGATCCTCAC
AGTATCGCTGAGAGGCTTAGAAGGGAACGCATCGCAGAGCGTATGAAGTCTCTCCAAGAACTGGTT
CCCAACACCAACAAGACGGATAAGGCATCAATGTTGGACGAGATCATCGAGTATGTTAGGTTCCTT
CAGCTTCAAGTCAAAGTACTAAGCATGAGCAGATTGGGAGGTGCAGGATCAGTTGGTCCACGCCTC
AATGGTCTCTCCGCAGAGGCAGGAGGACGGCTCAATGCCCTCACTGCACCGTGCAATGGCTTAAAT
GGGAATGGAAACGCTACAGGATCGTCCAATGAGAGCCTAAGGTCAACAGAACAAAGAGTGGCGAAA
CTGATGGAGGAAGACATGGGATCAGCAATGCAATACCTTCAAGGGAAGGGGCTTTGCTTAATGCCA
ATATCTCTCGCAACCGCGATATCATCATCAACTACTCACTCTCGTGGATCCCTCTTTAACCCCATC
TCCAGTGCTGTAGCAGCAGAGGATTCAAATGTAACAGCAACTGCAGTTGCTGCACCTGAAGCCTCG
TCTACTATGGATGATGTATCTGCTTCCAAGGCCTGAACCATAGAATACTCATGTGTTTCGTTTTCT
CAGTTTAAGTCAAACGCTAATCTCCCTATCTTTTTACTATTTTCTCTGCTAAGCAACAATAATATA
AAACAGTTGCAACAAAGAACAAAACCGAC

## SEQ ID NO: 333, A. vulgaris TC15501

CACTTTCAAAACTCTCTCTCTCTTTCTCTTCCCGAAACTCAAAATTTCTCCGGTTTATCGGGTATC
GGATCTGTAACTTGTTTCGCCGACAACCTCGTTTTTCGATTTTCTCTCATAAACCCAAAACAAAAC
CCTTTGTCTATTTCTATGTTTAATGTTCTTCAAAAACCCTAACACTTCAATCAAACACGCATACAC
TCTTACTCTCTTCTTTCTCACAACATGGCTAATAATAATAATCCTACAGAAGGATTAACAGATGAT
TTCCTTGATCAGATCTTATCTTATGGTGCTCACGAAGGTAATTTGGCGGGAAATGATGTTAATTTG
GCGGGAACAACTACACCTGGAGGTTCGATGGCCTTGCAGTTGAGTTCTTCTGGAGGTGATATTACC
GGTAGTGGTGGGTATCATCATCATCATCAGCATCAACATCATCATCAGTTAAGTGGTGGTGGTGGT
GGTTTTCCGTTAGGGTTAAGTTTAGAACAAAGTGGTAAAGGTGGTTTTTTTAAACCAGATGAAGCT
TCTGGAAGTGGTAAGCGGTTTCGTGACGATTTCGTTGATTTGAAAGCTGTTTCTTCTACTAACAAC
GATAACAACGATAGAGGAGACTCTGTACATTTGAATAATTTGTTTCCAGCATTTGGACATGTGCAA
CAACAGCAACAGGCTCACTCTGTTCGACCTCCTTCACATCAAAATCAAATCAACCAGCCTTTCCTT
GGGCATCCAACACCTGGGGCTGTTGCTGTTGTACCACATCCACCTGCCATTCGGCCTAGAGTGCGA

**FIGURE 5 (continued)**

GCAAGAAGGGGGCAAGCCACTGATCCTCACAGTATTGCAGAGAGGTTACGTAGAGAAAGAATAGCT
GAAAGAATGAAGGCACTACAGGAACTGGTTCCTAGTTCCAACAAGACTGATAGGGCAGCTATGCTT
GATGAAATTGTTGATTATGTTAAATTTCTAAGGCTCCAGGTCAAGGTTTTGAGCATGAGCAGACTG
GGAGGAGCTGGTGCAGTAGCACAGCTTGTGGCTGACATTCCACTTTCATCAGCTGAGGGCGGAGCC
AGTGAAGGTGGCAGTAATCAGCCAGCATGGGAGAAATGGTCTACAGATGGCACAGAACATCAAGTA
GCAAAGCTCATGGAAGAAGATGTTGGAGCTGCCATGCAATTCCTCCAATCTAAAGCACTTTGCATC
ATGCCCATTTCACTTGCCTCTGCAATATACCACGACCAACCACCCGATGCCTCCACAATGATCAAA
CCCGAACCTAATGCACCTTCATAAAATGAAAAGGATACATGCAATTGGACGTGCCCATGAACAACT
CCAAACTTACAACTATCCCACCTTCCATTTGTTCCCCCCACGTCAGACTTAGTCAACTCCCCTCAG
CATTGCAATCAAAGTCAGATGCCATATTATTCGTTCTGCCTTTCATTTTTTTCGTCAAATAGGTTG
TAATGTGATGCCTAGAAAAATAATCATAGGTACTCCTGATGTGGTGTGGGATAGTGGTGGATAGCC
AGAACACTTTCCGGTGGGGACCCTTCTTTTGGATAGTGGAGGGTGGGGCATTTTTAAAGGGGGAAG
ATGGGTACATAGACATGATTAATGTATTAGAAGGAAACTGAAAAGGGAATTGAAATGGATATTGGA
TAGTTGTTGGTCGCTTAAAAAAGTTGTGTTGGAAGGTTGCACTACTCGGTTTTGAGTGGGTTGTGT
TTTTTGTAAAGTGCTTGATTTATTTTTCTCAAGTATCTCTGAGTCAT

**SEQ ID NO: 334, A. vulgaris TC19278**
TCAACTGCTGTTGGCAGTTGTAATGGGGCTGTGAAGCTCGTGTGAGAGCTCGCAGGGGTCAAGCAA
CGGATCCACACAGTATTGCTGAAAGGCTTCGAAGAGAGAAAATAGCAGAGAGGATGAAGAATCTAC
AAGAACTTGTTCCAAACTCTAATAAGACGGATAAAGCATCTATGCTTGATGAAATCATTGAGTATG
TCAGGTTTCTTCAGCTTCAAGTCAAGGTTCTAAGCATGAGCAGGCTGGGGGCAACAGGGGCAGTTG
TTCCCCTCATTACAGAAAATCAGCCTGAGGGATCTACTAGTCATTTTCTGTCGCAGTCAGCTGGTC
GAGATGTTAAACATATTGAATCTCCTAACACCCTTACATTTGAGCAAGAAGTAGTGAAGCTGATGG
AATCCAACATGACCAAAGCTATGCAATTTCTGCAGAGAAAAGGCCTCTGCCTAATGCCTATTGCCC
TTGCAGCTGCCATTTCCGGTAGTAAAGCACCACCCGCCTCTCTTTCTACTGAGGGAAAGAAGTCTA
TTTTGACCAATGGCTTTGTTCATCATAACAGTGGGCAATCTAACATTGGGCCAGGTCAGATATCAT
CTGATGGAGAGACTAGATCAGAGGAAAACATAATTGGGATTCACAGCAGAGAAGACTTTTCAAGCA
ACAGCTGCAGTGGGACCAGCATTAAAAAAGAGGGAACAAACACCACAAACTTCACCAGAGAAATGA
ATCAAGAGTAGACATAGTTTTGCCGGTCTCAATCTTTGTGGCTTCATACCTGGTCTCCAATTCTCA
AGTTTCAGTAACCTAGCAAGTAGATAAGTAGCAGAGTCTGGGACTGTTTTGTTTTTTTTTTCTCGC
TTCAAAGATGAGAATGTGGTATAGATTTCTAATTATACGTTATAGGTTTCATTGTACATGCACACA
TATATGC

**SEQ ID NO: 335, B. napus TC10015 part**
CCCACGCGTCCGAAACACCTTCCGATGACTTCTTCGAGCAAATCCTCGGTCTCCCCAACTTCTCAG
CCTCTTCATCCGACGGCGGGTTAGGCGGAGGAGGAGCACCGCCGATGATGCTGCAGCTGGGCTCCG
GCGAGGAAGGAGGTCACATGGGTGGGCTAGGAGGAGGAAGTGGGTTTCACAACCAGATGTTTCCTC
TAGGTTTGAGTCTTGAACAAGGCAAAGGACAAGGCTTTCTTAGACCCGAAGGTGGTAGTCTTGGAA
CTGGGAAACGTTTCTCTGATGACGTCATGAAACCCGTTTTTCATGGGCAGCCAATGCAACAACAGC
CAGCTCCAGCGGCGCCGCATCAGCCTACATCGATCCGTCCCAGGGTTCGAGCTAGGCGTGGTCAGG
CTACTGACCCACATAGCATAGCTGAAAGGCTTCGTAGGGAAAGAATAGCGGAGCGGATCAGGGCGT
TGCAAGAGCTTGTACCTACTGTTAACAAGACAGATAGAGCTGCTATGATTGATGAGATTGTGGATT
ATGTAAAGTTTCTCAGGCTCCAAGTTAAGGTTTTGAGCATGAGTCGTCTTGGTGGAGCCGGTGCAG
GTGCTCCACTTGTTACTGATATGCCTTTGTCATCATCAGTTGAGGATGAATCTGGTGAAGGTGGAA
GGGCACCACAACCTGCGTGGGAGAAATGGTCTAACGATGGCACTGAACGCCAAGTCGCTAAACTGA
TGGAAGAAACGTTGGAGCAGCGATGCAGCTTCTTTCATCTAAGGCGCTTTGTATGATGCCAATCT
CATTGGCTATGGCTATTTACCATTCTCAGCCTCCAGATACAACTTC

**FIGURE 5 (continued)**

**SEQ ID NO: 336, C. clementina DY268946**
CAACCGTGTTACACCGACCCATCTGCTGCTGCTTCAAACTCCGCTGATGACATACCCATGTTCCTA
CAGCTGGGCTCCGGCGGCCCTTCTCCTGGTGCCGGAGGAGGCGCCACCAGTGCTGCTGACATAAGA
AGTTTGGCTATGGGAATCAGCATGGGAATGATGCCTCTAGGGTTAAATTTGGAGCATAGCTTCTTA
AGGCAGCATGAAGATCATAATAGTAACCATAACAACAACAACAACACTAATGCTTCTTCTTCTTCT
CCTAATAATTCTTCTGCCACTTCTGGAATCAACGAGAGAGATTCCGTGCACATGCCAAGTTTGTTT
CCAGCGTTTGGACAGTTGCAAAGTCAATCAGCCCGGCCGCCGCTGCCACGTCCTCCTCAAGTACAG
CAGTTTCAGAGCCAACCAGCAGCCGCACCGCAGCCTCCAGCTGTCCGTCCAAGGGTGCGAGCAAGA
CGAGGGCAAGCCACGGATCCTCACAGCATTGCCGAGCGGTTGCGTAGGGAAAGAATTGCGGATAGA
ATGAGGGCTCTGCAAGAGCTGGTTCCTAGCTGCAACAAGACGGATAGGGCAGCCATGCTTGATGAA
ATCGTGGATTACGTGAAGTTTCTAAGGCTTCAGGTCAAGGTTTTGAGCATGAGTAGACTGGGTGCA
GCTGGTGCCGTGGCTCAGCTTGTAGCTGATGTTCCTCTGTCATCAGCTCTTGAGGGAGAGAGCATT
GATGGTGGAAGCAGTCAGCCAGAATGGGAGAAATGGTCAAATGACGGAACTGAACAACAAGTAGCC
AAGCTAATGGAAGAAGACATTGGAGCTGCTATGCAGTTCCTTCAATCCAAGGCACTTTGCATCATG
CCCATATCACTTGCCAGTGCAATCTATCGCACCCGTCAACCGGATGCCCCAGCATTCGTCAAGCCT
GAATCGAGCACCCCTTCATATTGTCTAAACAACAATTGGAGTCAGCAGTTGGCTCACAATGACTCA
AAATATTTAATCACGGGTACCTATTCCTATAGAATACTTTTGGTCCTCTTATCCAACCAAATAATT
CAAATTCCAATGCAACGTTCCCATTCTTCTTTCCCTTAATTAACCTAGGGCAAAAAGGGAAATGA
AAAGAAGGCAATGGGCAATGGCAAGGGGGAAAAAGGGGAATTTTTTTCCAAAGTTTTTTTTTTTA
TAAAAACTCCCCTTTTCAAAAAAAATTATAAAGGGGGGGGGGGGGTTTTTTAGGGGGGGGGGAAAA
AACCCCCAAAAAAGGTTTTCGGGGGGTGTTTTATATG

**SEQ ID NO: 337, C. longa TA2544**
GCACCCGTTGAGACCTAACCTTCCCCGGGAAACTGGCGGCTGTGCCTATGCAACCGAGCAGCAAAG
AGACGGAAAGGATGGCGCAGTCCCACGCCGCCCTCCAGCTGGAGCTCCAGAACGGCGGCGGAGGCG
CCCAGAACGACGATTTCTTCGAGCAGACGATGTCCGGCTTCTCCACCGCCGCCTGGGCACCGGAGT
TGGGGACCCCCAGGTCGCTTTTCGGGGCGCGGTCTTCGGAGGAGTCGCCGGACGACGAGGGATTGA
ACTACGCCCCACCGTACGGTGGGTCGTCTCTTCTCGCCTCGCGGCACCGGATGCACCTCGGCACCT
CGCCGGCGGATTCGTCGATGTTTCTCCAGCTCGGTGGGCAGATTCTGTTACATCCTACGGCCGGCG
CCTCCGGGGGCGAGTCCGGGGGCTTCCTCCGGTTGCCCCTCTCCCTCGGTAGCGGAGGCTCTGGGG
TCTCGACTTCTGCCATCTTCGGCGACAGATCCCGAGGAGAAATCGACCCGCCGTTCGAATCGTCCA
ATCCCACCGAGGCTGAGGTGTTGTATAACGACGGATTCACCGGATCGCTTCCACGGGCGGTGCAAG
CGTCCCTTCATCAGCAACTCCTCCGACAACCCCAGAACTACGGAGCTGCTACGCTGACCGGACAGG
CTCTTGCGATAACAGCAACGGCTTCAGGTACCGCCGGCGCAGGTACTGCGCCACCCAAGCCGAGGG
TGAGGGCCAGAAGAGGTCAAGCGACCGATCCCCATAGCATTGCCGAAAGACTTCGGAGGGAGAGAA
TCGCAGAGCGTATGAAAGCTCTGCAGGAATTGGTGCCCAACGCTAACAAGACGGACAAGGCGTCGA
TGCTGGACGAGATCATCGACTACCTTAAATTTCTCCAGCTCCAAGTCAAGGTCTTGAGCATGAGCA
GATTGGGCGGAGCAGCCGCCGTCGCCCCGCTCGTCGCTGACATGTCTTCAGAGAGCGGCGGCGGAG
CGGGAACGCCGAGGAGTAATAACGATGGCCTGACGGCGGCGGAGCAGCAGGTGGCGAAGTTGCTGG
AGGAGGACATGGGTTCAGCGATGCAGTACCTGCAGGGGAAAGGCCTCTGCCTCATGCCCATCTCCC
TCGCCTCCGCCATCTCCTCCGCCACCTGCCGTCCTCGGCCTCCCCCGGTGTCAACCTCCGCTACC
CGGCAGCCGGCGACGCGCCTCCGTCGCCGAGCATATCGGCGCTGACAGTCCAATCGTCCAACGCCG
GCAGCGCCGGCGCCGACGCCAGCTAGGATGGAGCTGTTCGTTAAGATCGCTGTCCAAAAAGTGCTT
GGAATTGGAATTTCAATCCTTAGT

## FIGURE 5 (continued)

**SEQ ID NO: 338, C. paradisi hybrid TA3392**
CGACTTTGCCGAGTCAAACTCATGGCGAACAACCCGAACGAAGCCTCCTCGACTGACGATTTCCTC
GAGCAAATCCTCGGAATTCCTAATTTCGGTTCTGCGGAGTCCGGCTTGGCGGCTAGCGATGGCGGC
TTGGCTGCAGGCTCGCCGATGATGCTGCAGCTGAGCTCCGGCGACGGCTCCAGCCACATCTCGGCT
CTCGGAGGCGGAGTAAGTAGTGGGTATCATGGGCAGGTGTTTCCGTTAGGCTTGAGCTTGGAGCAA
GGTAAAGGAGGATTTTTGAAGCCCGAGGAAGCCTCTGGAAGCGGAAAGCGGTTTCCTGAAGAACAT
GCTATAAAAAATGTTTTTCATGGCCAACCACTGCCCTCACCTGTTCCTGCAGCTCCACATCCACCA
GCAATGCGCCCCAGGGTGCGAGCTAGAAGAGGACAGGCCACTGATCCACACAGCATTGCTGAACGT
CTACGAAGAGAAAGAATAGCAGAAAGAATTAGGGCACTGCAGGAGCTGGTCCCTAGTGTCAACAAG
ACTGATCGAGCAGCCATGCTTGATGAAATTGTGGATTATGTGAAGTTTTTGAGGCTTCAAGTGAAG
GTTTTGAGTATGAGTAGAGTGGGCGCTCCTGGCGCTGTGGCTCCACTGGTAACAACCGACCTCCCA
CTATCATCAGTCGAGGATGAATCTGGTGAAGGGGTAAGAAACCAACCAGCCTGGGAGAAATGGTCA
AATGATGGCACAGAGCGACAGGTAGCTAAGCTCATGGAAGAAAATGTTGGGGCTGCCATGCAGTTC
CTTCAATCAAAGGCTCTTTGCATAATGCCGATCTCACTGGCCACGGCAATTTACCATTCGCAGCCA
CCGGAATGAAGAGGTAGGATGGATAATGGTTGGTTGTTTGAAAATGCTTAGTGTTTTCCCAGTCCA
ACATAAAATGATAGTGCTTGCTTTGTCGAGGAGGCGTGGGCTTTGTTTTCAAAAAGTAATTTGACT
TCTTTCTCATTTTCTCCTGAGTCATTCATTATTGGAGGGCGACGGGGCTGCTGACGATGGCTGATG
GCTGATGGCATTGGTATTGGTGGACTGCGTTTGGTATAAATTACTTACGTATTTGAACTTAACCTG
ATTTTCAATTCTAAAGTCATTTACTGCGGTACAAAATTTGAAAAATCTATTACTGAATTTAGTTGA
TAATAACCAACTTAGCGTGTTCCTTTCGGGGATCATCCATCCAACTC

**SEQ ID NO: 339, C. sinesis TA12416**
GGGGAAACACCAAATTCCTTCTCTCCCTCTCTCTCCCTCTCTCTCTCTCTTCTTCCTGTTCTCACC
TATTTAGTATTTAACATCCATCAGAAGAATGGTTTCCATTCTCTTGAAGAAACTCTTGACTCTTCA
AACTTAAATTTCCATATCGTAAAGACTTATTCTCAAGAAATTAAGATGCAGCCCTGCAGCAGCGGC
GGAGAAATGCAAGGAATCAGCTCGCTCTTGAGCAACGGAAGCCACGAGCAGCAGTCGCAGATCCAT
CAAAGCGCGACGTTTGATCCAACTTCGCAAGACGACTTCTTAGAGCAAATGCTATCCTCTCTCCCA
TCGTGTTCTTGGACTGACTTGAAGTCTCCCTGGGGGGTTGTTGACCTTAACCCTAATAACAATAAT
AATAATATTAACAACAAGCAGCCCAGAGACTTATCCGACGAAACGGCGCCGTCCACTACTCAAGAG
AATAATGTCCCTGCAGGGTTTCAGTTCGACGAGTCCATGATTTTAGCTTCCAAGATGAGACAGCAT
CAGATCAGCGGGAATACTGGGAGTGGGAATAATAATTCTGCTGCAGCAAAGTTTATGATGCAGCAG
CAGCAGCAACAAATCATGATGGCTGCTGCTAGAGGTGGCATCGGGTTGCCTTTATCACTTGGAAAT
GGTGGCGATAACGACATCGTTGATGTCTCGTCATTCAAGTCCCAACAGGGAGGAGATGGCTCAGTG
CAAGCACTCTACAATGGCTTCACTGGATCTTTGCATGGCTCAACCCAGCCTCAACATTTTCATCAT
CTTCAGGGAGGATCGATGCCCGGGCAGACCTTTGGGGCACCAGGGCCGGTTATGAACCAGACGCAG
GCTCAGGCAAGTGGGTCAACCGGTGGTGGTGGTGGTGGTGGAAACACACCCGCTCAGCAGCCTAAA
CAAAGGGTTAGGGCTAGGAGAGGTCAAGCTACTGACCCTCACAGCATAGCTGAAAGATTGCGCAGA
GAGAGAATTGCAGAGAGAATGAAAGCTCTTCAAGAACTTGTACCTAATGCCAACAAGACAGATAAG
GCTTCGATGCTGGATGAGATCATTGACTATGTCAAATTCCTCCAGCTGCAAGTGAAGGTCCTGAGC
ATGAGTAGATTGGGCGGTGCTGCTGCTGTCGCACCCCTTGTTGCTGATATGTCCTCAGAGGGAGGA
GGAGGTGATTGTATCCAAGCAAACGGGCGGAACCCTAACGGAGCTCAGACGACGTCAGCTAACGAC
AGCTTAACTGTGACGGAACACCAAGTGGCTAAGCTGATGGAAGAAGATATGGGGTCAGCCATGCAG
TACTTACAAGGCAAAGGGCTTTGTCTAATGCCCATCTCACTTGCAACTGCTATATCGACTGCCACG
TGTCACTCTAGGAACCCCATCATCAGCACCAGCAACAACAACAATAACAACGGCAATCCCCACCAC
AATCCTTTGCTTCAGTCCAATGGTGAGGGCCCGACCTCACCTAGCATGTCAGTGCTGACTGTCCAG
TCAGCAACTATGGGTAACGGTGGGGCTGACGGCTCCGTCAAAGATGCTGCTTCCGTTTCCAAGCCT
TGATCAACGGCGTCGACCGACTTTATGATGCGGCTCGTCCAAAGTCTACGAAAATAAGGCGTCACT
AGATGTTGGACCTTCACGACGTCGTATTTGTGTAGACTTTGTGACTGAATCTAACAAACAACAAAA

**FIGURE 5 (continued)**

AAGCAAGAAAGAAGCCAATCCCAAGAACAAAAACCTTATTTTATGTTATTATTATTATTTTAATTT
GCGGGTTAGGTTTTGTTAATTTTACTATTATTAAATTTTTAATTTCTCAGATGGGATATCTCCCTC
TTTTTCTTTTATTTAATATTCTCTGTCATGATTCTCTATTAAATAATTATTTACAAAAGCCTTTAA
TC

**SEQ ID NO: 340, E. grandis WO2005001050 seqID77**
AGCACTTTCACTTATCAGAGGCCGAAAGCAAAACCCCCTGAACACTTCAAAGAAAAGAGGTTTTTT
CAGACACCCTCTCGCCCTCTCTCTCTCTCTCTCTGAGCTGAGAGACAATACCAGCTTATAAAAC
CCGAGACCCATAAAACCTCCCATCGGAATTCCCGAATCACGTTACCGGGTCATCGGGTTTCGGATC
TGCCACCCAGTTCACCAACACCACCACCACCACCTTCCTTCCCCCCCTCCTTTTTGGGTTTCCCAA
TGAGCACATCTCACCTCCACTTCCTCCCTCCCCTTCCCTTAAACATTCTCTCCCACCAATAATTTC
CACATTGGCAACCAATTTCTTATTCATCATCCCCTCCCCGAGTCCCACCAACCTTAGGCTCTCTCT
CTCTCTCTCTCTGCCACTCAATCTTTCCACTTAGAAAGAAACCAACTTTTTGCTGGGTTTCGCA
TAGAGCAGAAAACCCATCGTAGACTTTGATCTCCAGTTGACACTTCTATAGAGAAAGGGAGATAGA
GTGGAGAGAGAGAGAGAGTTTATAATCATCGGCTTATGGCAGGCTCAAATCCGCAGGAAGGACT
GGGGGATGAGTTCTTTGAGCAGATCTTGGCCGTGCCGCCCGGAGCTTACTCCGGTGGCTCCACGCC
CATGGTCTTGCAACTCGGCTCCGGCCGCGGCGGCGTGGAAGATGGCGGCGGCAGAGGAGGCGGCGG
GGGGCTGAGGGGCATGGGGGTGATGATGCCCCTGGGGCTGAATTTGGAGCAAGGTGGATTGTTTAG
GCACGAGGATGTTGAGAACAGTACTAGTGCATCTTCCACCACTTCTGCCATCAATATGGAGAGAGA
AGCAGGAGTCCACCACGGCAACAACATGACAAGCTGCTTGTTTCCAGCATTTGGACAGTTCCAGAC
TCATCAGTCAGTCCAGCCACCTCCTCCCCATCCACCCCCTCCTCAACTTCACCCGGCCTTCTTGAA
TCAGCCCGCAGTGGGATCGCCGAACCAGCCGGCGATACGCCCAAGGGCACGAGCTAGACGAGGGCA
AGCCACGGATCCTCACAGCATCGCTGAGCGGTTGCGCCGAGAAAGAATTAATGAAAGAATGAAGGC
GTTGCAGGAGTTGGTTCCCAGTTGCAACAAGACGGATAGAGCAGCCATGCTTGATGAAATCGTGGA
TTACGTGAAGTTCTTGAGACTCCAAGTCAAGGTCCTAAGCATGAGTAGATTGGGCGGAGCTGGTGC
TGTGGCTCAACTTGTAGCTGATGTACCCCTCTCATCAGCTGAGGGCGAGATCATCGAAGGCGGGAA
CAATCAACCGGCGTGGGAGAAGTGGTTGACAGACGGCACAGAGCAGCAAGTGGCTAAGCTGATGGA
AGAAGATGTTGGGGCTGCAATGCAGTTCCTCCAGTCCAAGACGCTCTGCATCATGCCCATTTCGCT
CGCCTCCGCAATCTTCCGCACAAGCCAACCAGACATGCCCCGGTCCATCAAGCCCGAATCCAGCGC
CCCTTCCTGAAGCACCCTGAACAAGTTCCTGGCGACATCATAGTTGCAACAATGCCTAGGTTTCTC
AGGTCTGACCGGTTCGAGGTTGTCCCCAAGTCGACGTAACTCCTTTGCCAACCAAGTTGCTTAGTG
CCCTCTGTTCATAGTTCACTGTCGCTGTCTTAGAGTGGGTGTGAACTTGTGGTTCTCATGCCTTAA
TTACAACCTAAAAAAATGCATCAGTTTCATGTTTTTTCCAATTCATCCAAGGAAAAGTCAAAAGCA
ACTTTATTCCAAATTCAGCTTATTAAAACCTATCTTCTGATGAAAAAAAAAAAA

**SEQ ID NO: 341, E. grandis WO2005001050 seqID1671**
.CCTCCTCGCGTCCCTCTGCGTTCCGCCAGCTAACCGAAATTCCGGCCACCCCCGCCGGAGCTCCGC
CGTGAAACCCTCTCTCCGTCTCCGCCTCCGCCTCCGCCTCCGTCTCCGTCGCTTCCTTCCGCCGTC
GCGACCGTAGATAGATGCCCTTCCCGCCCGCTCATGGCGAACAACCCGAGTGACGGCGCCAGCGAC
GAGTTCCTGGAGCACCTCCTCGGCCTCCCCAACTTCGCTGCCGCGGCGGAGGCCGGCCTCGCCCCG
GGCGACGGGACCGGCCTGTCCGTCACCGCGGCCACCCCGATGATGCTCCAGCTCGGCTCCGGCGAC
GGCTCCGGCGGCCACGGCCACGGCCACGGCCACGGCCATGGCCAAGGCCACTTGTCGGCCCTCGGC
GGCGGGGGAGCGGGGGCGGGGGGCGGCGGTGGCGGCGGTGGCGGGGGGTACCATGGGGCGGTGTTC
CCGCTGGGGCTGAGCTTGGAGCAGGGGAAGGGAGGGTTCCTGAAGCCCGAGGAGGCGTCCGGGAGC
GGCAAGCGGTACCCCGAGGAGGTCGTCGACGGCAGAGCTTCGACTGTGAAAAACCTGCACTCCCCG
TTGCCCAACTTCCCAGATGTTTCGCAAATGGCTTCTTTTAGGAACCGAGATGTGTTTCATGGACAA
CCAGTGCCCAACCCTGTTCCTGCAGCACCACATCCACCAGCAATGCGCCCGAGAGTAAGGGCTAGA
CGAGGACAAGCTACAGATCCCCACAGCATCGCTGAACGATTGCGGAGAGAGAGAATAGCAGAAAGG

**FIGURE 5 (continued)**

ATTCGACAATTGCAAGATCTGGTTCCAAGTGTTAACAAGACAGATAGAGCTGCCATGCTTGATGAA
ATTGTGGACTATGTGAAGTTCCTGAGGCTCCAAGTTAAGGTTTTAAGCATGAGCAGATTGGGCGCA
GCTGGTGCAGTGGCCCCACTTGTGACCGACATCCCACTATCATCAGTCGAGGAAGAAGGCGGTGAA
GGTGGAAGAAACCAGCCAGCGTGGGAAAAGTGGTCGAATGACGGCACGGAGCGGCAAGTTGCTAAG
CTCATGGAGGAGAATGTTGGCGCCGCAATGCAGTTCCTTCAATCCAAGGCTCTCTGCATCATGCCA
ATCTCTCTAGCCACCGCGATCTACCAAACTCAACCGCCCGACACCTCCTCCCTTGTCAAGTCTGAA
AGCAATCCACCATCCTAGACCAGCCACAACACAGGCACCTCCCCTTTTTCCAGGTCCCACCAAAGG
CTCAAGACTTAAATGCTCCTTGGTCCCCGCTTTAGTTTGTCCCGTTTCTACACCTTCAAAAACCAG
TTGATGGCCCTTCTTCCTTCCTCAAATTGCAATCAAAGTCAGATGCCATAGCCCTTGCTTTTCTGC
CCTCTCTTTAAGCTTTTCGCCCGGACAGGTTCAATGTCAATGCCTAATAAAATACTAGCAGGCCCT
TCATGTCTTGCCAAGTTCAACTAAAGGAGAGATATTCAAACCCTCACTTGATATGCTCTCTCTCTC
TCTCTCTCTCTCTCTCTCTCTCTCTCTCTCTCTCTCTCTCTCTCTCTACCAATAGAATAT
GCTTAGTGGAGAATGATGTGAGCGACTTTATAGTGGGTGGGGGCTCTTGTTTTTCTTGGTAGTGGG
TCGTGGGACCTTGTAAAAGGGATTGGTTGTGTAAAGTACCATTTGTATAATGTAATGCGTAATGTG
TTCGATGAGGGGGAATGGATTGCTTGGTAGAAAATTGAGAAAAAAAAAA

**SEQ ID NO: 342, G. hirsutum CO123623**
CGATAGTGGTGGTGGTGGGTTTAGAGGAATAGGCATGATGCCTTTAGGGTTGAATTTGGAACATGG
ATTCTTAAGACATGAAGATGGAGTTGTAGTTGACAACAACAACAACAATGCTTCTTGTTCAGCTGC
TTCTGCTGTTTCTGGAATCAGTGAGAGAGATTCTATGCATATGGTAAGCTTGTTTCCACCTTTTGG
ACAAATGCAAACTCAGCAAATCCGCGCTTCACCGCCACCTCCTCAGCCTCCACCGCAGCTCCACCA
GCCATTTCACAGCCAGCCAACATCAGGTCCAGTTGCTGCTGCACCGCACCCACCTGCCGTCCGTCC
AAGGGTACGGGCTAGGCGAGGACAGGCAACGGATCCCCACAGTATTGCTGAGCGGTTGCGTAGAGA
AAGAATAGCTGAAAGAATGAAGGCTTTGCAAGAGTTGGTTCCTAGCTGCAATAAGACGGATAGGGC
CGCTATGCTTGATGAAATTGTGGATTATGTGAAGTTTCTTAGACTTCAAGTTAAGGTTCTGAGCAT
GAGCAGACTCGGTGCAGCAGGTGCGGTGGCTCAGCTTGTTGCCGATGTACCCCTACTATCCGTTGA
GGGTGATGGTGCAGGAGGTGGAACCCAGCCTCCTTGGGAGAAATGGTCAAACGATGGCACTGAACA
GCAAGTCGCTAAGCTGATGGAAGAAGACATCGGAGCTGCCATGCAGTTTCTTCAGTCCCAGGCACT
TTGCATCGTGCCGATATCACTGGCATCTGCGATCTTCCCTGCACATCAACCTGATGCACCAACAAT
CGTC

**SEQ ID NO: 343, G. hirsutum DT543504**
ATGATAAGCAATGGAGGTGAATCTTCTGAGTTTCGGAGATCATTTACAGGCTTGGAAACTCTTTCT
CCAATTCCACAATTATGGCATCTACAACCTTATGACTCTGTTTCTTCCCTTCCCACTTTGGTGGGA
CAAACCATAGTAGATGATGAAGGCAATATTAACAGATTTATTGAGATTGATGAAATTCTGCAACCT
GAGAACTTATCTGCTTCCATCAATTCAAAGGGACAACAGGACATGCAAAATTCCTTTTGCTCTTCT
TTTCCCGCTGACCACCCTATAACAAAGACCATGATTGGGTTGCCATCTCTGGTGCAGGGTGCATCG
CCTAACCTGAACAATGGTTCCGACCGAACTGTGAAGCCTCGAGTAAGGGCACGTCGAGGTCAGGCT
ACTGATCCACACAGCATTGCAGAGAGGCTTCGAAGAGAGAAGATTGCTGAAAGAATGAAAAACCTG
CAAGAACTTGTACCTAATTCCAATAAGACAGACAAAGCCTCTATACTTGATGAAATCATAGGGTAT
GTCAAGTTTCTTCAACTCCAAGTCAAGGTACTAAGCATGAGCAGGCTTGGGGCAGCAGCTGCAGTT
GTTCCTCTCATCACTGATGGTCGAGCTGAGGTATCTAATGGTTTATCACTTGCACCATTAGCGGGT
CAAGGGGTTGATTTTTCACCATCTCCTGATCAAGTTGTTTTCGAACAAGAAGTGGTGAAGCTCATG
GAATCCAATATGACAATGGCCATGCAATATCTACAAAGTAAAGGTTTCTGTCTTATGCCTGTTGCA
CTTGCTGCTGCCATATCCAATGTTAAGTCATCCACATCCTCATCGTCATCATCAGTTCCTGCTTCT
GATGAGAGTAAGAAACTTGGGCTTCACCAACAGTACTCTAATCAACAACACCTGCAGCAG .

**FIGURE 5 (continued)**

**SEQ ID NO: 344, G. hirsutum TC60118**
AAAAACCCAACACTCTTTTTCATTCCTCTGTTTTTCTCTCTCCCACTTTCCGTTTCTCTTTCAGAA
AAAGGAAAGTTCAAGCTAAGCTAAAACTCTGAACTTTCCCCTTCTCAGTCTCCTCAGGGACAGAGA
AAGATTAAACGAAAGGAAAGAAAAAGACTTCTTCTTTTTTTCCCTAAAAGAAAAGTTGATCTTTT
TTTGTTTGGTTCGATTCTCTTTAGCCATTTCCTTTTTGCATGTAGATGCTCTTTGGTAGCCTTAAC
TCGCCACTTCGACTCACTTCAATCCATGGCTAATAACCCCAACGAGTCCCCCGCTGATGATTTCCT
CGAGCAAATCCTCGGACTCTCCCACTTTGCGCCTACTGAGACTGGCTTAGCGGGGCCCGACGGTAG
ATTGTCTGGAAACGCCACGACGGCTGGAGCGCCAATGCTTCTGCAGCTCAGCTCCGGCGGTGGCAC
CGGACACATCGGCGCTATAGGCGGCGGTGGAGGCGGTGCGTTTCACGGACAGGTTTTTCCATTGGG
ATTGAGCTTGGAGCAAGGAAAAGGTGGGTTTTTGAAGCCCGAGGAAGCATCCGGTAGCAGCAAGCG
CTTCCGCAACGAGGTCGTTGATGGCAGAGCTTTTTCTGTTAAAAACGTTTTCCATGGACAACCAGT
GCCAGCTACTGTTGCTGCTGGACCACATCCACCAGCAATGCATCCGAGGGTGAGGGCTAGACGAGG
ACAGGCCACAGATCCACATAGCATTGCTGAGCGGTTGCGCAGGGAGAGAATTGCCGAAAGAATCCG
GGCATTGCAGGAACTTGTTCCTAGTGTTAACAAGACTGATAGAGCAGCCATGCTTGATGAAATTGT
AGATTATGTGAAGTTCCTGAGGCTCCAAGTTAAGGTCTTGAGCATGAGTAGGTTGGGCGCAGCTGG
TGCAGTGGCACCACTCGTAACGGACCTCCCACTATCGTCAGTTGAGGATGAAAGTGGTGAGGGAGG
AAGAAGCCAACCAGCCTGGGAGAAATGGTCAAATGATGGCACAGAGAGACAAGTAGCTAAGCTGAT
GGAAGAAGATGTCGGAGCTGCCATGCAATTCCTTCAATCAAAGGCTCTTTGCGTCATGCCAATTTC
ACTGGCCACCGCAATTTACCATACGCAATCTCCGGATACCTCCTCTGTTGTTAAGCCGGAAACAAA
TCCTCCTTCATAGACACCTCACATGAGAAAAAGAGGAAAAGAAAACGGTGTATCATATGGTGGGGG
AGACTGTGGACAAAGACGGAAGCACTTTTCTTGTGTAGTGATGGGGCATGGATTTTTGGGTTTGGT
GGGATGAGGGACCCGCAAAAGGAGATGGATGAGATGAGTATATTTACATATTATAACAATAAAATA
TTACAATATTGATAGTGTTAGTGTAAGTAACAGTGTAATTTCACAGGAAAGGGCTAAAGTAGAAGA
AGGGATAATGGTTGGCTGATTTTATAAAATGCTGATTCAATAAAATAGATGGATTGGTTCACGTTT
CAAAAAAAACCT

**SEQ ID NO: 345, G. hirsutum TC60119**
AAAAAAAGAAGAGGAAAGCCAAAACTCCAAACTTTCTATTTTGCTTTTATATTATATTTTTTTTTG
GTTCAATTCAGTTTCGCCATTTCCTTTGCATGTAGATGCTCTTTGGTTAGTCCCTAACGCCGCTAC
TTGGACTCACTTTCCGCTGCTATCTTTTTTCCGGCTTTCTCATGGCTAATAACACCAACGAGGCCC
CCGCCGCCGATGATTTCCTCGAGCAAATCCTCGGCCTCCCTAACTTTGCGCCCTCCGAAACGGGCT
TAGCTGGATCCGACGCTGGATTGGCTGCAACCGCTGCTGGAGCTGGAGCTCCAATGTTTTTGCAGC
TCAGCTCCGGTGATGGCGCCGCTCATATCGGTGGAATCGGCGGCGGTGGAGGCGGTGCGTTTCATG
GGCAGGTTTTCCCCTTGGGCTTGAGCTTGGAGCAAGGGAAAGGCGGGTTTTTGAAACCCGAGGAAG
CTTCCGGTAGTGGGAAGCGGTTTCGGAACGGGGTCGTTGATGACCGAGCTTCTTCTGTAAAAAATG
TTTTCCATGGCCAACCCATGCAAGCAACTGTTTCTGCAGCACCACATCCACCGACAATGCGTCCAA
GGGTGCGCGCTAGACGAGGACAGGCCACGGATCCTCACAGCATTGCTGAACGGTTGCGCAGGGAGA
GAATTACTGAAAGAATCAGGGCATTGCAGGAACTTGTTCCTAGTGTTAACAAGACTGATAGGGCAG
TCATGCTTGATGAAATTGTAGATTATGTCAAGTTCCTGAGGCTTCAAGTTAAGGTTTTGAGCATGA
GTAGGTTGGGCGGAGCTGGTGCAGTGGCACCGCTTGTAACAGACATTCCACTATCATCAGTAGAGT
ATGAAAGCGGTGAGGGTGGAAGAAGCCAGCCAGCATGGGAGAAGTGGTCAAATGATGGCACAGAGC
AACAGGTAGCTAAGCTGATGGAAGAAACGTTGGAGCTGCCATGCAATTCCTCCAATCAAAGTCTC
TTTGCATTATGCCTATCTCACTAGCCACTGCAATTTACCACACACAGGTACCAGATACCTCCTCTG
TTGTTAAGCCAGAAACAAATCCTCCTGCATAGACCTGAGGGGAAAAAAGGGTAGGCATCCCACAG
TCCATCCTTCATCCCGCCTTTTATTTGTCCTATTCTAACACACCCACTCCCTCCCTCTCTGGCTTG
AAAATGCCATCAAAGTCAGATGCCATAGCTCTTGCTTTTTGCCTTCCTTTCCAGCTCTTTTTCGAT
TTTTCAAAACAGGGTTTGATGTCAATGCCTTAAAAAAATCAGAGACAGGCAATTGATGTCTCGACA

**FIGURE 5 (continued)**

AGTTCGAGTAAAGGGGAAGAATTGTCAAATTTCACTTGATATGCTCTTCTTTTTTGTATCAAAATG
GGATAGTGGACAATGATGGGAGCACTTTTGTAGTGGGGCTCTGATTTTGTGGTTAGTGGATTGTGG
GACCTGCAAAAAAAAAAAAAAGGATAGAGATGATCCAGGGTATAATATTGTTATTGTAACAATGTA
TTTTGAAGATAAAATCGAATGCAAAAGTAGAAGAATTGATATT

SEQ ID NO: 346, G. hirsutum TC61833
AAAAAATCCCTTTGAAAAAAAAAATTCCCTCTTTTCCTTTCTGTTACTTTCACAGAGCTGAAAGTA
CAGAACAGAAAAGAAACCCCAAACCTCAAAATTTTCTCTTTCTCACGTTGATAGAAGGATTTAAGC
TAGAACGGCAAAGATATGAAATAATATCGTGTTTTTTGTCTATAAATATTTTGTTGGGTTTTCAAT
TCTTTTTTCGCCATTTCTTTTGCATGTTGATGATCTTTGGTGGCTTTAACTCGGTACTTGGACTCA
CTTCCGCTACTACTTCTTCGGGTTTCACATGGCTAATAACCCCAACGAGGCTTCCGCTGATGATTT
CCTCGAGCAAATCCTCGGATTCCCTAACTTCGCGCCTTCTGAGACGGGTTTGGCGGGACCCGACGG
TGGACTGACAGGAACAACTGCTGGTGCCGGAACGCCAATGTTTCTACAGCTCAGCTCTGGTGATGG
AGCCAGTCATCTCGGCGGAATCGGCGGCGGTGGAGGCGGCGCGTTTCCCGGACAGGTTTTTCCGTT
GGGGTTGAGCTTAGACCAAGGGAAAAGCGGCGGGTTCTTGAAGCCGGAGGAAGGTTCTGGTGGTAG
CAGCAGGCGGTTTCGCGATGAAGTCGTTGATGGCAGGGCTTCCTCAGTTAAAAACGTTTTCCATGG
TTCACCAATGCCGGCTACCGTTGCTCCATCACCGCATCCACCAACAATGCGTCCAAGGGTGCGGGC
TAGACGAGGACAGGCCACGGACCCGCATAGCATTGCTGAACGGTTGCGGAGGGAAAGAATTGCCGA
AAGAATCCGAGCATTACAGGAACTTGTTCCTAGTGTTAACAAGACTGATAGAGCGGTCATGCTTGA
TGAAATTGTAGATTATGTCAAGTTCCTGAGGCTCCAAGTTAAGGTTTTGAGTATGAGTAGGTTAGG
CGGGGCGGGGGCAGTGGCACCGCTTGTTACAGACATCCCATTATCATCGGTCGAGGACGAAAGCGG
CGACGGTGGAAGGAACCAACCGGCATGGGAGAAATGGTCAAACGACGGCACCGAGCGACAAGTAGC
CAAGCTCATGGAAGAAACGTAGGAGCTGCCATGCAATTCCTTAAATCAAAGCTCTTTGCATTAT
GCCAATCTCACTAGCCACCGCCATCTACCACTCGCAACCACTGGATACCTCTTCTATCGTCAAGCC
CGAAACAGATCCCCCACCATAACTCTAACAAACAAAAAATAAGGGTATGCATCCCACACAGCCCAT
CCTATATGTCCAACACTATGAGGAAAAAAAAAGGAAGGGGGGGCCTTCATGTCCGGATTTGTCAAT
GGTGATCTCTTTTGTAGTCAATGGTGGGGGTAGGGGATAATGATGGAAGAAGAGGGGCACTCTCAT
AGTGGGGGCTTTAAATTTTTTTGTT

SEQ ID NO: 347, G. hirsutum TC67603
CAAAAAGAAAAAAAGAGAAACTCGTCCCTTCATCTTCAATCTCATTCATCATCAAAGCAAAAAAAA
AACCCTAATTATGCAGCCTTGTAGTCGTGAAATGCAAGCAATGAACTCTCTCTTAAACCCGACCCA
ACAAATCCCTCTCCAAGACCTTCAAATCAACGGTAACAGACACCACCATCAACAGATCCACGTCCC
ATCATCATCGCAGTTCCAGTATCCCACACCCACTTCAACAACCCACCATGACGACAGCTTCTTAGA
ACAGATACTCTCCTCCACCACGTCCTTCCCGTGGTCCGACGAAACGGGTCCTCCCAACCCCGAGGA
TAACAATAACGCCGGCTTCAACTACGATGAGATGGTTCTAGCTTCCAAGCTTCGACAGCATCAAAT
CAACGGCGGAGCTGCTGGTAACCCTTTTGCCGCTATGAAGATGATGTTGATGATGCAACAACAACA
ACAGCAACAGCAGATGATGTTAGCGGGAAGACCCACGGTTGCCTCCGCCGCCGCCGGAGGCGGAAT
CACCACCAATCATCAGGGTGGAGAGGGTTCCATGCAAGCTTTGTATAATGTTTTTGGCGATGGATC
TTTGCATGGAACTATGCAGGCGAAAAGCTTTGTGGGGGCTAATTCAGCAACAGAGATGACCCAAAG
TCAGGGAAGTGGTCCGACGGCCGGAGAAGCGGTGACGGCGCCGGAAAAGCCTAAACAAAGAGTTAG
GGCAAGGAGGGGTCAAGCTACTGACCCCCACAGTATCGCGGAAAGACTACGTAGAGTTAGAATTGC
AGAGAGAATGAAAGCTCTTCAAGAACTGGTTCCCAACGCCAACAAGACAGATAAAGCTTCAATGCT
TGATGAGATCATCGACTATGTCAAATTCCTCCAGCTCCAAGTGAAGGTTCTGAGTATGAGCAGATT
GGGTGGTGCTGCTGCTGTTGCTCCCCTTGTTTCTGAGGGAGGTGGCGATTGTATTCAAACAAGTGC
CAATGGTGGGTCCCATCCACGAAATTCCAACGGCGACCAAACGCCGTCGGCCAACGACAGACTGAC
GATGACGGAGCACCAAGTGGCCAAGCTTATGGAAGAGGACATGGGCTCCGCCATGCAGTATCTGCA
AGAGAAGGGTCTGTGCCTCATGCCGATCTCTCTCGCCACCGCCATCTCAAGCGCCACGTCTCGTTC

FIGURE 5 (continued)

AAGGAACCCCATGATCAACCATGAAAACCCGGCCAACGGCAGCCACCTTTTGATTCAATCAAGCGG
CGGCGATGGACCTTCCTCGCCTAGCATGTCCGTGTTGACAGTCCAGTCAGCCACCATGGGTAACGG
TGGCCTTGAAGGTGGCGCAGCCTCGGTTTCCAAGCCCTGATAACGGCGGTGACTGACATACGTAGA
GAGGAATTGAAGGGAAACAGCTTTTAGTCTATGAGAATAAGGCGGTCTTCTTCCCAAGTATTTTCT
TAAAAGTACGAAACGACGCCGTATTTGACGTAGACTTATAAGCTAACCCCTAAGACTGACCTCATC
CAATCAAC

**SEQ ID NO: 348, G. max TC229602**
GGATTCATGAAGCCCGACGAAGCCTCCGCTAGCGGAAAGCGCTTTCGCGACGACGTCGTTGATAAT
AGAGCTAAGCATGTTTTTCATGGGCAACCCATGCCTACTACTATGCCTGCTGCTCCTCATCCTCCA
GCAATACGTCCTAGGGTGCGGGCCATAAGAGGACAGGCTACAGATCCACACAGCATAGCTGAACGA
TTGCGCAGAGAAAGAATAGCAGAAAGAATCAGGGCATTGCAAGAACTGGTTCCAAGTGTCAACAAG
ACAGATAGAGCTGCCATGTTAGATGAAATTGTGGATTATGTCAAGTTCTTAAGGCTTCAAGTGAAG
GTTTTGAGCATGAGTAGACTGGGTGGAGCCGGTGCAGTGGCACCACTGGTAACTGACATCCCATTA
TCATCAGTCGAGGAAGAAGGTGGTGAAGGTGCGCGAAACCGGCCAGCATGGGACAAGTGGTCAAAT
GATGGCACAGAAAGACAGGTAGCTAAGCTTATGGAAGAAACGTTGGGGCTGCCATGCAGTTTCTT
CAATCAAAGGCTCTCTGCATCATGCCAATCTCACTGGCATCGGCAATATACCAGTCACAACCACCG
GACACTTCTAGCATAGTCAAGCCTGAAACTAATCCTCCTTCATAGACCTAATTCACATGTTGCACA
ATCTATTATTATTGGTCCCATCCCAGGCTCACCTAATACTTGCTGGGACCCACACCTAACGCGTTA
TTTGTCTGTTCCAAATCCCCCCCCATAAAATAAGTCATTGGCAAGCATCAAGTTGCAATCAAAGTC
AGATGCCATAATTCTTCCTTTTTTTGCCTTTCTTTCAGCTTTTGTCAATACAGGGTTCGATATCAA
TGCCTTGGAAAAGACAAGCAAACCCCTATATCATGTCTTGCCGTATTTCAAGTAGAGAGGAAGACA
TTGTCAAAAGTTCAATTGATATGCGCTTCTTTTCTAGTGTCAAATGGGTGGGGGTAGTGGACCATG
ATGTAGGCGCTTTTATAGTGGGGCTTAGTTTTTGTAGTGGGCTGTGGGACCTTCTAAAAGGT

**SEQ ID NO: 349, G. max TC205173**
GTCCAACAACAACAACGACGCTACTACTAATGTCGCATCTTTTTCCTCCTTCGATGAGCACTCCAC
CTTAGCCTCCAAGTTCCGCAACCACCAGATCAGCTCCAATAACAACGCGCCCAAAAACGCCGCGGC
GGCGGCTCTCATGCTCCAACACCAACTCCTCAGAGACTCTGGTCTTCTCAACATGCCTCTTTCCTT
GCCCGGAAACGACGTCGTGGTTGACGCTTCTACCTTCGAATCCCCCAATCCGAGTGGTAAAGCTTC
AGTCCAAACTCTCTACAACGGGTTCGCCGGATCTCTGCATGGCGTAGGCCAATCCTCGAACCAGAC
TCAACATTTTCAACACCCTCAGGGAAGTTCGAATCCGATGCAAGGGCAAAATTTTGGGGCGGTGCC
AGCTGGTGGTGGTAGCGCGACGAATCAGGCTCCAGCGAGTGGCGCTGCCGCGGGTGGCGCGCCGGC
TCAGCCGAGGCAGAGGGTTCGAGCGAGGAGAGGTCAAGCCACGGACCCACACAGCATCGCTGAAAG
GTTACGTAGGGAGAGAATCGCCGAGAGAATGAAGGCCCTACAAGAACTGGTTCCCAATGCCAACAA
GACAGACAAGGCATCCATGCTAGATGAGATCATCGATTACGTGAAGTTCCTGCAGCTCCAAGTCAA
GGTTCTGAGCATGAGCAGATTGGGCGGTGCTGCTGCTGTCGCTCCCCTTGTTGCTGATATGTCCTC
TGAGGGAGGCGGAGACTGCATTCAAGCCAACGGCAAGAGTAACGGCGGTGGGGCCCAGGCTTCAAC
CACCAACACCAACACCAACCAAACGACAGCGACAACAACCTCAAACGATAGCCTAACGATGACGGA
GCACCAGGTCGCGAAACTAATGGAAGAAGACATGGGTTCGGCTATGCAGTATCTTCAAGGAAAAGG
TCTTTGCCTCATGCCAATTTCACTTGCCACTGCAATCTCCACTGCCACGTGTCCCACCAGGAACGT
TAACGTTAACCCCTTGATCAACGCCGCCGCCGGAGCCACCCATTTTCCGACCGCAGCCAACCCCGC
CGGCGAAGGGCCGTCGTCTCCGAGCATGTCCGTACTGACGGTGCAGTCCGCCATCGCAGGCAACGA
CGGCGCCGCCTCCGTTTCGAAGCCGTGATGACGGTGTTTGAGTGACTTTATAGGAAAAAGAAGAAA
AAAACAAAGTGAACAAAGGACAGAAAATGACGTGTGTTTTTTTTTCTTAAAAAAAAAAGGAGCCGTT
AAAGTCTACGAAAATAAGGCGTCAGGGATACGGAGGACTCGACGACGCCGTATTTACCTAGACTTT
GTTTGGCTGATGCTAACTTTCAACCAACCAATTACAAACTCCACCATGCCAATTACCTACTTCTTC
GTCTTCTTTTTCTTTATTATTATTGTTAATTGTTAACTGTCGCTTTGGGTTTCTTCTGTTCATTGT
TTTTAAAAATCTGGAGTGGAATTTCTCTTTTTGGTGTT

**FIGURE 5 (continued)**

SEQ ID NO: 350, G. max TA55042

TTTTCTTCTTCCTTTTCGTGCTCTCTCTCAAATTCAACACGAGAGACACAAAACAAAACATTCTCG
GGAAGCGTAACCGCCCAGAATTCCGACCATTTTCACAGACCTTGTTTTCTGCTCTTGATGTTTTTC
ACCCCCCAAATGTGGCAATAAAAAACGGGTGGCTTAATTTGACACAATCGGTGGTTATTGCAAAGT
TGAGATGGCAGGTAATAGTGGTTCAGAGGGGTTGGGCGATGATTTCTTCGAACAGATCTTAGCAGT
GCCTGAAGCCGGCACAGTGGGAATGTTGCAATTGGGGTCCACAACTGGTGCTTTCAGAGGAGCTTC
TGGGTTAATGCCTCTGGGCTTGAATTTGGAACAAGCTGCCTTCCTAAGACACCAAGTTAACGTTGA
CGACGACGTTGTTCATGTTAATGTTGATGATGCCACCATCCACCAACATCACCTCACTCTCCATAA
CAACAACAACTCCTCATCACCCTCTTCCACTGCACCAATCACCGATAGGGATTCTATGCATATGAG
GGGTTTATTTTCCGCGTTTGGACAACTGCATACTCCTATCCGCCCCACGCTGCCATTGCCTCCACC
ACCTCGTCAGCCACAACTCCACCTCCACCATCATAACCAATTTCAGGGCCAGGCAGCTGCAGCTCC
AGCGTCAATGGCTGCTATGCCACAACCACCTGGGATCCGCCCTCGTGTGAGAGCAAGAAGAGGGCA
AGCTACAGATCCTCACAGTATTGCTGAGCGGTTGCGTCGTGAAAGAATCGCTGAAAGAATGAAGGC
ATTGCAGGAATTAGTTCCCAGCATCAATAAGACGGACAGAGCGGCGATGCTTGATGAAATTGTGGA
CTATGTGAAGTTCCTTAGGCTTCAGGTTAAGGTCCTGAGCATGAGTAGACTGGGTGGAGCTGGTGC
TGTTGCTCAGCTTGTGGCTGATGTTCCCCTTTCTGCAGTGGAGGGGGATCAGGACATAGAAGGTGG
AGCCAATGAGCAAGCCTGGGACAAGTGGTCCAATGATGGCACAGAACAACAGGTGGCTAAGCTTAT
GGAAGAAGATGTGGGAGCAGCTATGCAATTTCTTCAGTCCAAGGCACTTTGTATCATGCCCATATC
TCTAGCCTCAGCCATTTTTCGTATGCCTCAATCAGAAGCATCAACAGGCATCAAGCCTGAATCTAA
CAGTCACTGATATTAGCGATAGAACCGAAACAAAAAGTATCATGACAGACACTAGATAATTAAATT
TCTTCATCCTTAATTTATGACAAGGGTAATATTCTACTTTCTTGTATAATTTAATTTTCCTGATTG
GATGGAGAAGGATCTAGTCGGTGCAGCAGCTGGTTCACTCTTCTCAAGTTTTGTCAAAGAAGGAGT
AATTGTTATTCCTATTAAGTAAAATCGGAGGAGCCAAGCAAATGTCCTTTTCATGGTGAAGATTAA
TATTTTCAATGGCCTTATTCAAGTTTTTCTTGCAAATATCTGTGGTGGGGTTGGTGGTGGATGACC
AAAATATTTGTAGTGGAACCTCTTTTCTTGGTTTAGTATTTTTAGTCAGTGGGGAGTGGGACCATT
TAAAAGGAAAGGATGGTTACGAATGATGTATCGGGAGAAAACTTTAATACAAGTTGAATTATAATA
TGGTTAGTTACCTTGTAAAGATGTTTTGGAAGCATCTGACCATTGGAAAAGCTAAAGATATTTTAA
AATAATCACACATTTTGAGTCTTGTTTGCTTCTAAAACAAGTGATTTCCAGCACAAAATTAGGTGA
GAGTACTGAACCGACTGCTTGCTGAGAAAGTGGAAAATGACAGTTTGTTAATTTACTGAACAAAAA
AA

SEQ ID NO: 351, G. max TC207545

CGCGTCCGCTCCGCCGACGCCTCTCCGATGATGCTTCAGCTCAACTCCGGCGACGCCGCCACCCAC
CTAGCCTCCTTCCACGCGCCGCCCTACCAGCTCGGCCTCAGCCTGGACCAAGGTGAGGGACCCTTC
CTCACGCCTGAGGACGCTTCTGGAAGCGGCAAGCGCTTCCGCGACGACGCCGTTGATACCAGACCT
AACAACGTTTTTGATGGGCAACCCATGCCTACAACTGTTCCTACTGCTCCACATCCACCAGCAGTG
CGACCTAGAGTGCGGGCTAGAAGAGGACAGGCTACAGATCCACATAGTATAGCTGAAAGGTTGCGC
AGAGAGAGAATAGCAGAAAGAATCAGGGCTTTGCAAGAACTGGTCCCTAGTGTCAACAAGACAGAT
AGAGCTGCCATGCTGGACGAAATTGTGGATTATGTCAAGTTCTTGAGGCTTCAAGTGAAGGTTTTG
AGCATGAGTAGATTGGGTGGAGCTGGTGCAGTGGCACCACTGGTAACTGATATCCCATTATCGTCA
GTCGAGGAAGAAGGCGGTGAGGGAAGAAACCAGCCAGCCTGGGAGAAGTGGTCAAATGATGGCACA
GAAAGACAGGTAGCCAAGCTTATGGAAGAAAATGTGGGTGCTGCCATGCAGTTTCTTCAATCAAAA
GCACTCTGCATCATGCCCGTCTCACTAGCTTCAGCAATATACCAGTCACAACCCTCAGGCACTTCT
AGTATAGTTAAGCCTGAAACTAATCCTCCTTCATAATCATAGTCAGAACTCTGGCACAGATGCACC
ATCCAGTGGTCCCATCAAGGGCTCACCTAACACTGCTAGGACCCACGTGTAATTTGCCTGTTTCAA
ACCCCCTTAACCTTAGTCATTGGTTCGCATCAAGTTGCATTCAAGGTTGGATGCCATAGTCTTTCC
TTTTTGTCGTTCTTTCAACTTTTTTGTCTAGACAGGGTTTGATGTCAATGCCCTTCAAAACACAAG
CAAACCCCATCTTGATAAATTTCATGTAAAGGGAAAGAAATTGTCGAAGTTCACTTGATATGCTCT

**FIGURE 5 (continued)**

TCCTTTTTGTATCAATTAGTGGGGGTTAGTGGAACGTGATGGAAGCGCTTTTATAGTGGGGCTTGG
TTTTTTGTAGTGGGCAGTGGGACCTTCTAAAAGGTTAGGGTGGTGTGTATATTTTATTAGTGATGT
AACTCTTAGGGAATTTGAATTGCAGAGCTGAAATGGATAATGATTGCTTTAACAATGCTGGGTGCT
GCTAGTCCACCAGAAAATGATATTGCTTTTTTTTTTTTTATTTCAGGTGTGCTCTAGTTAGTTATT
TAAAAGTACTTTCGACCCCTTTTTTCTCATGCAGGGAGAGATGGAAGGAGAGTATGGTGCTATTTT
AAACCA

**SEQ ID NO: 352, G. raimondii TA13791**
TTCCTTTGTTTTTCTTTCTCTCACTTCCCGTTTCTCTTTCAGAAAAAGGAAAGTTCAAGCTAAGCT
AAAACTCTGAACTTTCCCCTTCTCAGTCTCCTCAGGGACAGAGAAAGATTAAACGAAAGGAAAAGA
AAAAGACTTCTTCTTTTTTTCCCTAAAAGAAAAGTTGATCTTTTTTTGTTTGGTTCGATTCTCTTT.
AGCCATTTCCTTTTTGCATGTAGATGCTCTTTGGTAGCCTTAACTCGCCACTTCGACTCACTTCAA
TCCATGGCTAATAACCCCAACGAGTCCCCGCTGACGATTTCCTCGAGCAAATCCTCGGACTCTCC
CACTTTGCGCCTACTGAGACTGGCTTAGCGGGGCCCGACGGTAGATTGTCTGGAAACGCCACGACG
GCTGGAGCGCCAATGCTTCTGCAGCTCAGCTCCGGCGGTGGCACCGGACACATCGGCGCTATAGGC
GGCGGTGGAGGCGGTGCGTTTCACGGACAGGTTTTTCCATTGGGATTGAGCTTGGAGCAAGGAAAA
GGTGGGTTTTTGAAGCCCGAGGAAGCATCCGGTAGCAGCAAGCGCTTCCGCAACGAGGTCGTTGAT
GGCAGAGCTTTTTCTGTTAAAAACGTTTTCCATGGACAACCAGTGCCAGCTACTGTTGCTGCTGGA
CCACATCCACCAGCAATGCGTCCGAGGGTGAGGGCTAGACGAGGACAGGCCACAGATCCACATAGC
ATTGCTGAGCGGTTGCGCAGGGAGAGAATTGCCGAAAGAATCCGGGCATTGCAGGAACTTGTTCCT
AGTGTTAACAAGACTGATAGAGCAGCAATGCTTGATGAAATTGTAGATTATGTGAAGTTCCTGAGG
CTCCAAGTTAAGGTCTTGAGCATGAGTAGGTTGGGCGCAGCTGGTGCAGTGGCACCACTCGTAACG
GACCTCCCACTATCGTCAGTTGAGGATGAAAGTGGCGAGGGAGGAAGAAGCCAACCAGCCTGGGAG
AAATGGTCAAATGATGGCACAGAGAGACAAGTAGCTAAGCTGATGGAAGAAGATGTCGGAGCTGCC
ATGCAATTCCTTCAATCAAAGGCTCTTTGCGTCATGCCAATTTCACTGGCCACTGCAATTTACCAC
ACGCAATCTCCGGATACCTCCTCTGTTGTTAAGCCGGAAACAAATCCTCCTTCATAGACACCTCAC
ATGAGAAAAAGAGGAAAAGAAAACGGTGTATCATATGGTGGGGGAGACTGTGGACAAAGACGGAAG
CACTTTTCTTGTGTAGTGATGGGGCATGGATTTTTGGGTTTGGTGGGATGTGGGACCCGCAAAAGG
AGATGGATGAGATGAGTATATTTACATATTATAACAATAAAATATTACAATATTGATAGTGTTAGT
GTAAGTAACAGTGTAATTTCACAGGGAAGGGCTAAAGTAGAAGAAGGGATAATGGTTGGCTGATTT
TATAAAATGCTGATTGAATAAAATAGATGGATTGGTTCACGTTTC

**SEQ ID NO: 353, H. petiolaris TA1140**
CGGGGATCTCATCTTTTCACTCTTCTTCCCTCTCTCTCTCTCTCTTCTTTCTCTCTCTAGGTTTTA
GGATTCGCCGGCGATACGCTTCTCCGGCAGGCATGTCGACTGACCCGCCGGAGGGCTACGGTGACG
ACTTTCTCGAACAAATTCTCGCGATTCCGTCGTACAACATCACCGGATGCTTACCTGTTAATACCG
CTGATTCTACTGGCTCTGAAACAGTTTCCGTTCATCATCAACAACAACAACCGCAACAACAACTGC
AACCGCAAAAGTTTCCGTTAGGTTTGAGTTTAGATAACGGCCGTGAAACAATCGGAGGAGCGTTTG
CAGGACAGCAACAGCAACTGCAGCAGCAGCAGCGTGAGAGAGGAGGGAGCATGAATATGAGTG
GTTTGTTTCCTCAGTTTGAGAATTTGCAGTCACACTCGCTGTTACATACTGTTCCACAGGGTTTTC
AAGGGCAGCCAACTAGCAGTACAGCAGTGACTGTGCCTCATCCGCCTACGATTCGCCCTAGGGTAC
GAGCTCGACGAGGACAAGCTACAGATCCTCATAGCATAGCTGAGCGTCTACGCCGAGAAAGAATTG
CAGAAAGAATGAGGGCTTTGCAGGAGCTTGTTCCCAGCTGCAACAAGACTGATAAGGCTGCTATGC
TTGACGAGATTCTGGATTATGTGAAGTTCTTACGACTCCAGGTCAAGGTTCTTAGCATGAGTAGGC
TGGGTGGAGCTGGTGCAGTGGCACAGCTCGTATCTGATGTCCCGTTGCAATCTGTTGAGGGGGACG
GGAGTGAAAATGGATATAATAATCAACAGCAGGGCCAGGCAGCGTGGGAGAATTGGTCAAACGACG
ACACAGAACGTGAAGTAGCAAAGCTGATGGAAGAGGATGTTGGGGCGGCCATGCAATTCCTGCAGT
CAAAAGCATTATGCATCATGCCCATTTCACTTGCTTCACTAATCTACCCTAATCACCAACCTGATG

**FIGURE 5 (continued)**

TTAAACCCGAGCCATCTGCTCCTTCTTAGAGAGCATCTTACATGCTTCTCTCTTATAACAACTACT
ACTACTACTACCTTTTTCTGTCATTTGGTTTTGTGCCCCAACCCCAACCCGATGTCTTCTCTAATT
GATGGACATTTTGTATGAGGAAAGTAGGAAGGAAACAGGGGATGTGTATGGCCATCTCATTGTGTC
CTGGGGATTTTATTTATTATCTAGATTAAAATGTATTCTAAGAAAATCTCTTCACATGGACAACCC
TACCCAACTTAGCTGTGTTTATTATTGTTAATATCTAAATGATTTGG


**SEQ ID NO: 354, H. vulgare TC140470**
CGGGCAGGCGACCGATCCCCACAGCATCGCAGAGAGGCTAAGAAGAGAGAGGATAGCGGAAAGGAT
GAGGGCCCTACAGGAATTGGTCCCCAATACGAACAAGACTGATAGGGCAGTTATGCTAGATGAGAT
CCTGGATTATGTGAAATTCCTTAGGCTTCAAGTAAAGGTTTTAAGCATGAGCAGATTGGGTGGTGC
TGGTGCTGTTGCACAGCTTGTTTCTGACATTCCACTTTCAGTTAAGGGGGAAGCAAGCGATAGTGG
GGGCAAACAGCAGATATGGGAAAAGTGGTCAACGGATGGCACGGAGAAACAGGTTGCGAAGCTGAT
GGACGAGGACATCGGTGCTGCAATGCAATTTCTCCAATCCAAGGCGCTCTGCATGATGCCAGTCTC
TCTTGCCATGGCTATCTATGACACACAACATTCACAGGACGGCCAACCAGTGAAGCCTGAACCCAA
CAACACTGCCTAGTATAGTAACAGCAGCTGCAAGCCGCATCCCTGCAGCTGCTAGTAGGCATGCAT
GAACCTACCATCAAAGTGTTAAGATGAAAACTGCTGGTTTTGATCCACTAACAACAAGGAGGCAGG
AAAAGACCTAAAAGATCCTCTCCACTGGAAGTTGTATCGACGGTATATGATGTCACCTACTACCCC
TTTTCCATCTTTTGTTCAAAGGTGCTTTAATTTTCAAGGATATGGTAAGCAAT


**SEQ ID NO: 355, L. perenis TA3490 part**
TTTCACCCTCCTCATACGCCCACTACGCTTCTTCCTTCTAAACCAGACTACGATTCTCCGGCATGG
CGACAGACCCGCCGGAGGGCTACGGAGACGACTTCCTTGAACAAATTCTCGCGATTCCTTCTTACA
ACATCGCCGGATGCTTGCCTGGTAATACCACCGATGCTAATGCTTCTGAAACGGTGTCTGTTCATC
GTCAACAGCAACAGCAGCAACCGGTTTTTCCGTTAGGTTTGAGTCTTGATAATGGCCGTGAAACAA
TCGGAGCGTTTGCAGGGCAGCAGCAGCAGAGGGAGAGAGGAGGGAGTATGAACATGACTGGATTGT
TTCCTTCATTTGAAAATTTGCAGTCACATTCGCTGCTTCATTCTGTTCCTCAGGCTTTTCAAGGGC
AATCAACTACCAGTACAGCTGTCACTGTTCCCCATCCACCTTCTATTCGCCCTAGGGTCCGGGCCC
GCAGAGGACAAGCCACAGATCCTCATAGCATAGCTGAGCGTCTACGCCGAGAAAGAATTGCAGAAA
GAATGCGGGCTTTGCAGGAACTTGTTCCTAGCTGCAACAAGACGGATAAGGCTGCAATGCTTGATG
AAATTCTTGATTATGTTAAGTTCTTACGGCTTCAGGTCAAGGTTCTTAGCATGAGTAGGCTGGGTG
GAGCTGGTGCAGTGGCACAACTCGTATCTGACGTCCCCTTACAATCCGTGGAGGGGGACACGAGTG
AAAACGGATATAATCAACCCGCATGGGAAAACTGGTCAAACGATGACACAGAACGTGAAGTAGCAA
AGCTCATGGAAGAAAGATGTGGGGGCCGCTATGCAATTTCTCCAATCAAAAGCTCTATGCATCATG
CCCATATCACTTGCTTCACTAATTTACCCGCCCCAAACACCCGACTCCAATTCCCATGTTAAGCCC
GAAACTGTCG


**SEQ ID NO: 356, N. benthamiana CK293938**
TAGCCGGAGATAATCTCCGGCATGGCGACAAACCAACCGGAGGGCTATGCCGACGATTTCCTCGAG
CAAATTCTCGCGATTCCTTCCTACGCTGGCTTGCCGCCGGTGGCTGACGTGGGAACTTCATCAGAA
ACGACGTCGTTCACTTCGGCATCTGCTGCTGGTCTACAGCAACCGTTGTTCCCGCTGGGACTAAGC
TTGGAAAACGGCCGTGATGACGTCAGCGATGCTGGTGCTTATGCAGTGAAGCATGAAAGAGAAGGA
ATAAATATCGGGAATTTATATGCGGGTCTAGAACATTTGCAATCTCATGCGGTTCGTCACGCTGTG
CCTCCTGTTCACCATATCCAGTCTTTCCAAGGCCAACCAACAACAAGCACAACGATGACTGTACCG
CACTCACCAGCAATTCGTCCTAGGGTTCGAGCTCGGAGGGGACAAGCCACAGATCCACATAGCATA
GCTGAGAGGTTGAGAAGAGAGAGGATATCAGAAAGAATAAAGGCTTTGCAAGAACTTGTCCCCAGC
TGCAATAAGACAGATAGGGCTGCTATGCTTGATGAGATTCTGGACTATGTGAAGTTCCTAAGGCTT
CAAGTTAAGGTATTGAGCATGAGTAGGCTGGGAGGAGCCAGTGCAGTGGCACAACTTGTTGCTGAC
ATTCCGTTGCAATCTATGGAGGGGGACAGTGCTGAAAGTAAATCCAACCAGCATATCTGGGAAAAG


**FIGURE 5 (continued)**

TGGTCCAATGTTGACACAGAACAAGAGGTTGCTAAGCTCATGGAGGAAGATGTTGGTGCAGCCATG
CAATACCTTCAGTCCAAATCACTCTGCATCATGCCCATATCACTTGCTGCACTTATCTATCCTACT
CAGCAACCGGATGACATGTCCATGGTCAAGCCGGAACCGGCAGCCCCGTCATAGACCTCCAATTTA
TTGCACGTGCCTCTGAGAACTCCT

**SEQ ID NO: 357, N. benthamiana TC7102**
CTTCTTTATTTCTCTCTTCTCCACAAAATCATCTATTTCTAAATTGAGTCATATACTTGCACAGAA
GTTACTAAAGATATAATGCAAGCCATGAACCAGTACGATCCGACGTCGTCTCACGACGATTTCCTC
GACCAAATTCTCTCTTCCGTTCCTTCTTCTTCACTTTGTTGGCCTGACCTTTCCAAATCCTGGGAC
CCACACCACCACCACCTCTCTCCGCCGCTGCCTCCTAACCCTAGCTCCGGCGATGACCACCAGCTT
CCTCCTTCTAATCCTCTTCAGCATTTCCAATATGACGACCAGTCTTCTTCTTTCCTTGCTGCCAAG
CTCCGCCAGCACCAGATCACTGGTGGTGGTGGTGGCGGCGGCGATGCTGTAGCAGCTGCTAAAGCA
CTTTTGCTCCAGCAGCAACTTTTGCTTTCTAGAACACTAGCCGGAAACGGCCTTAGGTCTCCGACT
GGAGCCTCCGGCGATAACGGCTTCCTGAACATGCTCGGTAATGGTGACCAAAACGACAGCGTCGGA
AATCCGGCTAATGACAGTTCCGTTCAAGCTCTTTTCAACGGATTCACTGGATCTCTTGGTCAAAAC
TCAAGTCAACCTCAACATTTTCTCCATCCTCAGGGAGGAAGGATGCAAGCGCAGAGTTTCGGAGCT
CCGGCAACGGCGCCGGCGATGAATCAAACTCCGGCAGCAAGTGGTTCAGCTGGTGGAGGTACAACG
CCGGCGGCACAGCCAAAGCAACAGCGAGTGAGAGCTCGTAGAGGACAAGCAACTGATCCTCACAGT
ATTGCTGAACGATTACGTAGAGAGAGAATTGCAGAGAGAATGAAGGCTTTGCAGGAGCTGGTACCC
AATGCCAATAAGACGGACAAGGCTTCAATGCTGGATGAGATCATCGACTATGTCAAATTCCTACAG
CTCCAAGTCAAAGTTCTGAGTATGAGCAGATTGGGTGGTGCTGCAGCTGTTGCTCCCCTAGTTGCT
GATATGTCCTCTGAGGGAAGAGGAGAAGGAAATGGAGGAAGGGGAGGAAACGGAACGGCGTCGTCT
TCAAACAATGACAGTATGACGGTAACGGAGCACCAGGTGGCTAAACTAATGGAGGAGGATATGGGT
TCAGCAATGCAATATCTACAAGGGAAAGGCTTATGCCTAATGCCAATTTCCTTAGCTACAGCTATT
TCAACTGCCACGTGTCACTCCAGGAATCCCATGATCCCTAACGGCAACGGTGGCAACAACCCACTA
CTAGGCGGAGGAGGAGGCCTCGCCACCAACGGCGGTGGTGGCGAGGCTGGTGGACCATCCTCTCCT
AAGTTGTCGGTTTTGACTGTCCAGTCAGCCACGATTGGTAACGGTGGAATTGATCCCTCCGTTAAA
GACGCTACTTCTGTTTTTGAAGCTTAAGAAGTTTGTTTGGAAGTTTTTTAACGGCGTTAACTGTTT
CACTGACCCCCTTTGCCACGGAGAAAAAAGAAAATGTGAAAAATAGTGCGTGGCCGTCAAAGTTAA
GTCTACCAAAATAAGGGTCTTTTAGTCTTTATTTTTATCTTTTTTTCCAGTTTCATATGAAAAAAAA
AAAAGAAGAAGAGAGAACCAAGAACGACGCCGTATTTAGGTAGACTTGGCTGAAGTAAGCTTAACT
TAAGCTAACAGCAGGAAATGCCAATGGAAATGAAGTACTTATGTTTCTTTTAATCTTTTCTCCATA
ATGATGATCCTTTGATTTCCCTGTTCTACTTAAGTTTTTCCTTAATGGAGTATTGTTTGGAAAGGA
TATATATA

**SEQ ID NO: 358, N. benthamiana TC7103**
GATCTCTCATTCATTAACGGGGAAAAGAAATGCAAGCTATGAACTCACAAATGTCACTACAAGAAG
AAAATGGAGCTTCGTCGGGCCAAAATATGAGTCACTCTCATTTTGACCCGACGTCGTCTCACGATG
ACTTTCTTCAACAGATTCTCTCTTCCGTTCCTTCTTCCTCTCCCTGGCCGGACCTCTCCAGCGGCG
GTGACGGTCATTTTGACGACCAGTCAATTTTCGTAGCTTCCAAGCTCCGGCAGAATCAGATCACCG
GCGGTGGTGGCGGTGGCGCAGCTGCTAAAGCGTTGATGCTTCAACAGCAGCTTTTGCTTTCTAGAG
GATTACTCGCCGGTAATGGTGACCAAAATGATGACGTTGGAAATCCGGAAAATGAGATTTCAGTTC
AAGCTCTTTACAATGGATTCGCTGGATCTCTTGGTCAAACCTCTAATCAACCTCAGCATTTTCACC
ATGCTCAGGGAGGATCCATGCAAGCGCAGAGTTTCGGAGCACCGGCGTCGTCGACAATGAATCAAA
CGCCGGCGGCGAGTGGTGGTTCAGCTGGTGGAGGGCAGCCAAAGCAACAGAAAGTTAGGGCTCGGA
GAGGACAAGCAACTGACCCTCACAGCATTGCTGAAAGATTACGGAGAGAAAGAATTGCTGAGAGAA
TGAAGTCGTTGCAGGAGTTGGTACCCAATGCTAATAAGACAGACAAGGCTTCAATGTTAGATGAGA
TCATCGACTATGTCAGATTCCTCCAGCTTCAAGTCAAAGTTCTGAGTATGAGTAGATTGGGTGGTG

**FIGURE 5 (continued)**

CTGCAGCTGTTGCTCCCCTAGTTGCTGATCGGTCCTCTGAGGGGGGAGGAGGTGATTGTGCACAAG
CAAATGGAGGAGGGGAGGGGCAGTAATGGAACGACGTCGTTAGCTAACAATGACAGCATGACGATGA
CGGAACACCAGGTGGCAAAGCTAATGGAGGAGGATATGGGATCAGCCATGCAATACTTACAAGGAA
AAGGCTTATGTCTTATGCCAATTTCTTTAGCCACAGCTATTTCCACCGCCACGTGTCACTCCATGA
AACTCAACAACCCACTACTACACGGCGGCGGAAGCGGAGGCTCTGCCGCCATCAACGGCGGTGGTG
GTGGACCATCCTCTCCTAGCTTGTCGGCTTCCACTGTCCAGTCAGCCACGATGGGTAACGGGGGCG
CTTGAGTTTGTTGAAATATACTTCGTAATTTACGACGTTGACTGTTTCAATTATCTTTTCTTAGAA
AAATACCGGAAAAATGTGAAAAAAGTGGTGAAAATTTCGTTATTTTAGAGCTGAAATTTCAAGGCC
TTTTCACATGGGACAAAAAAGAAAAGAAAAGAAAAAAGGGGTAATTGAAATGTTTAGCTCTAAAT
ATTATTGGTAATTTCTGATTTTAGTTCGTGTGATATTTTATTAATTGTATTTAACATTGAATTAAT
GATAATATGGGTGTTTTTGTTTATTTACATAT

SEQ ID NO: 359, *N. benthamiana* TC8633
GAAATATTTAAACAATTGCTTTTATTTGGTAACCCTATTGGACAATATATTTATTAATCAAAGCTT
TTGGTGACACCATTTGCTTTTTATTTTCTTCATCGCTCTCACTGTTCTATAGAAAGTAGCAGCAAC
CAAATAATAAACACATAGAAACACTCAAAACACACACTTCCATTTCTTTTCCTATATATATACACA
AGTACACTCGTGAGTAGTACTGAGTACTAGTTTGTACATCTAATATTACACATGTTAAAAAAACAC
TTACTAGTAATTGCTTTTCTGAGAAATTAAGGTGGTCGAGAAAAAAAATCTGTTACAGCTATGGCT
AACAACCCATCAGAAGGTCCTGATGATTTCTTTGACCAAATCTTGGGATTCCCAGCTTATAACGGA
GCAGAAACCAATTTGGCGGGAAGTGATGCCGGAGCAATTCCGCCGGCGATGCTGCTGCAGCTAAAC
TCCGGCGATGCGTCGGGTCAGTTTTCCGGAATGGGTCTTGGTGTTGGACTAGGTGGTGGTGGTGGG
TTCCATCATCCATCTCAGTCCGGTTCTTTTCCATTGGGGTTGAGTTTAGAGGGAGGGAAAAGTGGA
TTCATGAAGATGGATGATGTTCCGGCCGTACCCGGAAGGAGATTTAGAGATGATGTTGTTGATAGC
AGAGCTTCTTCTTCTGTTAAACCTGGTTTTCATGGCCAACCGATGCCTTCAATGCCACATCCCCCA
GCAATACGTCCAAGGGTAAGAGCAAGGCGAGGACAAGCTACTGATCCACACAGCATTGCAGAGAGG
TTACGTAGAGAGAGGATAGCAGAAAGAATTAGAGCATTGCAAGAGTTGGTTCCCAATGTCAATAAG
ACCGATAGAGCCATAATGCTTGATGAAATTGTAGATTATGTCAAGTTCCTACGCCTGCAAGTGAAG
GTGTTGAGTATGAGTAGGTTGGGAGGAGCTGCTGCAGTGGCACCACTTGTTACAGAAATTCCAATA
·TCATCAGTAGAGGAAGAGATCAGTGAAGGTGGAAATAATCAACCAGCCTGGGAAAAGTGGTCAAGT
GATGGTACAGAAAGGCAAGTGGCCAAACTTATGGAAGAAATGTTGGTTCTGCAATGCAATTTCTT
CAGTCTAAGGCACTCTGTATTATGCCTATTTCTCTTGCATCAGCAATTTACCACTCTCAACCACCA
GATACATCAAGTCTTATTAAGCCAGAAACTAATCCTCCTTCTTAGAGATCCTTATTAGAAAGCTGG
CTCGGACATCACGTTTAATTTTTTTTAAAAAGAAAAAGATCCTTATTAAAAAGGTGACAACATGCA
AATGACCCTGAGAATTTTCTTTGGGAGTGGATGGTCCCACAGCTATTTTTACTTATTAAGTAGTAC
TATTTAGCTTTGTTGAAAGACACGGTTCAATGTTATGTATAAAGTAACCTTTTTTTTTGTGATTGA
TATGATATATAATTCAAGCAGAAGAAGAAAAATTGTCAAAGTTCTGCC

SEQ ID NO: 360, *N. tabacum* TC12771
AAAACAGAGTCATTCCATTCCATTTCCGTTCCTCAAAACCCTTTATTAACTTCTTCTCGCCGGGAA
AAACCCTTTTCCTCGCCGGAGATAATCTCCGGCATGGCGACAAACCAACCGGGGGGCTATGCCGAC
GATTCCTCGAGCAAATTCTCGCGATTCCTTCCTACGCTGGCTTGCCGCCAGTAGCTGACGTCGGA
ACTTCATCAGAAACGACGTCGTTCACTTCGTCATCTGTTGCTGCTGCTGGTCTACAGCAACCGTTG
TTCCCGTTGGGACTAAGCTTGGATAACGGCCGTGATGACGTCAGCGATGCTGGTGCTTATGCTGTG
AAGCATGAAAGAGAAGGAATAAATATCGGGAATTTATATGCAGGTCTAGAACATTTGCAATCTCAT
GCAGTTCGTCACGCTGTGCCTCCTGTTCACCATATCCAGCCTTTCCAAGGCCAACCAACAACAAGC
ACAACAGTAACTGTACCGCACCCACCAGCAATTCGTCCTAGGGTTCGAGCTCGGAGGGGACAAGCC
ACAGATCCACATAGCATAGCTGAGAGGTTGAGAAGAGAGAGGATATCAGAAAGAATAAAGGCTTTG
CAAGAACTTGTCCCCAGCTGCAATAAGACCGATAGGGCCGCAATGCTTGATGAGATTCTGGACTAT

**FIGURE 5 (continued)**

GTGAAGTTCTTAAGGCTTCAAGTTAAGGTATTGAGCATGAGTAGGCTGGGAGGAGCCAGTGCAGTG
GCACAACTGGTTGCTGACATTCCGTTGCAATCTGTGGAGGGGGACAGTGCCGAAAGTAGATCCAAC
CAGCATATCTGGGAAAAGTGGTCCAATGTCGACACAGAACAAGAGGTCGCTAAGCTCATGGAGGAA
GATGTTGGAGCAGCCATGCAATACCTTCAGTCCAAATCACTCTGCATCATGCCCATATCACTTGCT
GCACTTATCTATCCTACTCAGCAACCGGATGACCCGTCAATGGTCAAGCCGGAACCGGCAGCCCCG
TCATAGACCTCCAATTTGTTGCACGTGCCTCTGAGAACTCCTAATAGCCTTGTGTCTTACCCTCCT
TTGTATCCTTACCTACTATCACTTGTATTTTCCTCTGCAATTGGTGGCAATGCAATCAATGTCAGA
TGCCACAATTTTTGCTTTTAGCCCAACCATTGGCCCCTTTTATTACCTTTCCGGAGTTTTCGTCAA
ACAAGGCATGATGTCATACCTTGGGCAAAGGTAAACGTCTGAAAGCACCCAATCCATTATCATCAT
TTTGGATGAGGTGAGCAGCTTTTTCAAAGTTATCTATGATTAGCTGCTCTTCTGCCATTTTGTTTG
TGGGATAGTGGGGACTCCTTTATCTTTTGCATAGTGGATTGGTGGGGCGATGTAAAAGTAATCAAC
TGGTTGTTGTAAAGTGTAATGTAATTTAAGTATATTATTGCAATGGATAGTTGATGTGGCTCTCC

**SEQ ID NO: 361, O. sativa Os02g35660**
GACGCGAGTCGAGAGACGGGAGAGCAGTGCAGTGCAGTGCAGTGCCGCCTCGTCTTCCTTCCCTCC
CTCCTCATACTCTCTCTCCTCCCGGTCTTCTCCTCCCCCGCCCAAAGCGGAGCGCGGCGGTTATAT
ATATATATATATACAGCACTTCCTCCTCCTTCCTCGCCGCACGTCGCCCTCCGACTTCGCCTCG
TCAGCCGTCGTCACCGCCCCCACCCGTGGATTGCACCGGAGATCGGAATGGGCGGCTTCGCCTACC
CCTTCACGCCGTCGCCGGCGTGGTCGCGGGACGCCGTGTTCGCTGGCTCGCCGTGGGCCGCAGGCG
GCGTCTCCTCCCTCGCCGACGCTTTGGTGAGCTACGGTGCCGTCGACGACGAGGAGGCCGCGTTCC
TCGGCAAGACCGCCGCGTCGTCTCCCAGTACGGCGAGGCTGCACGAGCAGCAGCAGCTGCTGCTGG
AGGCTGAGCTGCTGCGCCACGGCGACGGCCTCGGCTTCGCCGCGATGGACGACGACGGCGGCGCCG
CGATGCTCGGCGCGCTGGAGCCGTGCGCCATGCCGCTCACCGACAGCGGCGGCCCCCCGGTCATCT
GCAGCAGCAGCTCCAACGACAGCAGCGGGAGCGAGCACTCCGCCGCCATGCCTGCAGGAGGCGGCT
TCCTCGTCGGAGAGCAGCAGCAGCACGTGCCGCCGGCGGCCTACGCCGCGGGAGGTGTACTCCCGT
CCATGGCCGCGGGAGAAGAAACGCCGCAGAGCTTCGGCTTTGGCAGCCTCTTCAATGGCGATCTCC
TGCAGGAGGCGACCGTGAGCAAGTATCATCATCATCAGCAGCAGCAGCAGCTGGGCGTTGTGCCTT
CGTCGCAGCCGCATCATCTGAATGATGACATCGATTTCAACACTGGAAAATTGATGTCTTTTGCTT
CTGGACAGCAGCACGTTACCCCCAGCATTGACAGCCTGCAGATCGACCAGAAGGAATTCAGCAGTG
GCCTGCACCACCTCAATCTCTCCTCGCTGATTTCTGGACCACTCGCATCATTCAATGCAACACAGA
GCCATAGACAGCCTGCTGAAGCTTGCGGTGGCAAAAATGGCGGCGCCGCTCCATTTGTGAACCTAT
CTGAGGTGCTACCAAAGGGCAATGGGAGTGGTAGTGCTGGGAATGGAGCACCCAAGCCTCGTGTCA
GGGCTCGCCGAGGCCAGGCCACCGATCCCCACAGCATCGCAGAGAGGCTCCGGAGGGAGAAGATCT
CTGACAGGATGAAGGATTTACAGGAGCTTGTCCCAAACTCAAACAAGACTAACAAGGCATCCATGC
TCGATGAGATCATCGACTATGTGAAATTTCTCCAATTACAAGTCAAGGTCCTTAGCATGAGCAGGC
TTGGAGCGGCCGAAGCCGTTGTCCCTCTTCTGACAGAAACACAAACCGAGAGCCCCGGGTTTCTCC
TGTCCCCAAGATCATCATCAGGAGAAAGGCAGGCAGGAGCAGGAGCAGTAACAGGAGGGCTCCCCG
GCGATCAGCCGGAGCTCCTGGACGGCGGCGCCATGTTCGAGCAGGAGGTGGTGAAGCTGATGGAGG
ACAACATGACGACGGCGATGCAGTACCTGCAGAGCAAGGGGCTCTGCCTCATGCCGGTGGCGCTGG
CGTCGGCGATATCGGCACAGAAGGGGACGTCCTCGGCCGCCGTCCGACCGGAGAAGAAGAAGAACG
GTGATGGCGATGGCGGCGGCGACGAGGAGGACGTGAAGGGAGAGTTTGACGCACCGAGGAGGCCTC
CTGTCGGCCGGCCGAAGGAGATGAGGTCCAGAGTCTAAGCTAGAACCTCTGCTTTGTTCAGGAGAG
ATGGAGCTTAATTTAGATCCTAACACTTTGCTACTTATATGTTTATTATTATGAAATTTTAATTGC
AAGTTGCAACTGGTGATCTCGAACTACATTTTGCCGGTGATTAGAAAATTGTTATATGGAGGTTAG
TTATTAAATCATATATAATTATATTTTGTATTGTTGCGGTCCTTGCGGACAATGCCATTCAGTACC
TTGATATTTG

**FIGURE 5 (continued)**

**SEQ ID NO: 362, O. sativa Os02g55250**
ATGCAACCCAACGCCCGCCAAATGCGCGGCGGCGGCGGCGGCGGCGGCGGGCAGGACGACTTC
TTCGACCAGATGCTGTCCACGCTGCCGGCGGTGTGGTCCGAGCTCGGCTCCGGCAAGCCCGCCTGG
GACCTCACGGCCGGCGCCGTAGGAGGAGGAGGAGGAGCCTCCGATGACCACTCCGCCGCGGCGTTC
GACGACTCCGCGCTCCTCGCCTCCCGCCTCCGCCAGCACCAGATCGACGGTGGAGGCGACAAGCCC
ATCATGCTCCAACTCAGCGACCTCCACCGTCATCACGGCCTCGCCGCCGGCGATGACAGCGGCGGC
GCTGCCGGGTTCCTTCCCCTGTCGCTGTTCGCTGACCGGTCGCAGGACGACATCGACGCCGCCTTC
AAGTCCCCCAATGGCGCTAGAGGTGACCATGCGCTGTACAATGGGTTCGGGGCCGCCGGAATGCAT
GGCGCCGCCGCCATGCAGCCGCCGCCGTTCGGGCAGGGAGGATCAATGCCGGCGCAGAGCTTCGGT
GGCGGAGCGGCCGCGAGCGGCGGCGGCGGCGGCGGGTCGGCGTCGGCGGCAGCGGCGGCTGGGGCA
TCGTCGGGCGGAGGGGCGGCGGCGCCGCCGCGGCAGCGGCAGCGGGCGAGGAGAGGGCAAGCCACT
GACCCACACAGCATCGCCGAACGTCTCCGGAGGGAGAGGATAGCTGAGAGGATGAAGGCGTTGCAG
GAGCTGGTCCCAAATGCCAACAAGACCGACAAGGCATCGATGCTCGACGAGATCATTGATTATGTG
AAGTTCCTCCAGCTCCAAGTCAAGGTTCTCAGCATGAGCAGATTGGGGGGAGCTTCTGCAGTCGCG
CCTCTAGTGGCTAACATGTCATCAGAGAGTAATGGGAATGGCAATGCCACCAGCAGCAGCGGGAAC
GGCGAGGCGGCCAATGGCAGCAGCAACGGCGACAACAATGGTGGCGGCACACTGCGGGTGACGGAG
CAGCAGGTGGCGAAGCTGATGGAGGAGGACATGGGCTCCGCCATGCAGTACCTGCAGGGGAAGGGG
CTATGCCTGATGCCGATCTCTCTCGCAACGGCCATCTCCTCCGCCACCTCCTCGTCGCTCCTCCCC
CGCACTGGGGGAGGCGCTGGAGGGTCCCTACATGAAGGTGGTAATGGTACATCACCACCATTGGTG
AATGGCACCGCCACCGGATGTGACGACGCCGGAGTATTCTTTTCTGTTAAATTTGTCGTGGAGCTG
TCGTTTCTCCTGCTGAATGAAGACTGTAGAGGTAAGGAGGAATCGAAATTATTGGTCCAGAAGGGA
CCCTGA

**SEQ ID NO: 363, O. sativa Os03g58330**
ACCTCGCTTCACACTAGCCGAGGAGACGCGAGCGCGAGCTGCTCTCCTCTCCTCCCGCCCCGCGTC
GCCGACGTCGAGGCGGCGGTCGACGTCGACGTCGCCGGGGATGGCCGGGCAGCAGCCGCAGCAGCA
GGGCCCACCGGAGGACGACTTTTTCGACCAGTTCTTCTCCCTGACCAGCTCCTTCCCTGGCGCCGC
GCCGGGCGGCCGCGCCGCCGGTGACCAGCCCTTCTCCCTCGCGCTCAGCCTCGACGCCGCCGCGGC
GGCCGAGGCGTCCGGGAGCGGGAAGAGGCTCGGGGTCGGCGATGACGCCGAGGGTGGCGGCAGCAA
GGCGGATCGGGAGACCGTGCAGCTCACCGGACTCTTCCCGCCGGTGTTCGGCGGCGGCGGCGTGCA
GCCGCCGAACCTCCGCCCCACCCCGCCTACCCAGGTGTTCCACCCGCAGCAGTCGAAGCAGGGCGG
AGCAGCCGTCGGGCCGCAGCCGCCGGCGCCGAGGCCGAAGGTGCGAGCGCGGCGTGGGCAGGCGAC
CGACCCCCACAGCATCGCGGAGAGGCTAAGAAGAGAGAGAATAGCAGAAAGGATGAGGGCCCTACA
GGAATTGGTCCCCAATACAAACAAGACAGATAGGGCAGCTATGCTAGATGAGATCCTTGATTATGT
GAAATTCCTGAGGCTGCAAGTAAAGGTTTTAAGCATGAGCAGGCTGGGTGGCGCGGGTGCTGTTGC
ACAGCTGGTTGCTGATATTCCACTTTCAGTTAAGGGGGAAGCAAGCGATAGTGGGGGCAACCAACA
GATTTGGGAAAAGTGGTCAACGGATGGCACAGAAAGACAGGTAGCGAAGCTGATGGAAGAAGACAT
CGGGGCAGCGATGCAATTTCTCCAATCCAAAGCACTCTGCATGATGCCAATCTCGCTCGCCATGGC
AATCTATGACACACAACAAACACAGGATGGACAACCAGTGAAGCACGAACCCAACACTCCTTCCTA
GTATAGTAATAGCAACTGTAAGTCTGTAACATGAATCGTTACAACCCCTAGTAGACATCAACCTAT
CAAAGTGTAAGATAAAACAGCTCGTTTTGATTCACTAACAAGGAGGCAGGAAAAGACCTAAGAGAT
CCTCTCCACTTGAAGTTGTACCAATGGTATATGATGTCACCTTTCGTTATCATCTTTTGTTCAAAG
GTGCTTTGATTGCAAGGATATGGTAAGCAATAGCCTGCTCATCCTCCTACACCTTTCAATGCCCCT
ATGAGCGCGGGTGATTGAGAGAGCTACTAAAGAGTCTGGCATGCTCTCACATTGTCTCTTTTCGAG
TCTGTAGTGCACCAACCATACACTTTGTGGTGGGGACTGCTTTTTTGGTAGTGGGTGTTTGGACTA
ATCAAAAGGGATTGATGTATGCCTGTGTCTGCAGCCTGCACAATGGTTGACGGAATTTGAACACAA
GATGAAATGGAAGATGATTTTGATGGTGATGTTCATTTATCTCCATTTTTCCAAAGTGGAGGCGGA
GGCTTTGGTCAATGGAGGCTGAGCGTGCGCTAAGCTGATCGCTAGTGGGTCTGCTATCTACATCTA

**FIGURE 5 (continued)**

CATGCACTACTGTTGTATCTATTGTGACATACTCTTGATAAGAAACTGCGGTATAAAGTTTAATCT
TTCTATTGGCGATTAATCCATAGAATTACTTTGTATTTTCTGCCATGAGTTCTCAACTACCCACAT
TATTTGTGAAGAACAGCTGTTCTTGTACTGTTCACTGCCCTCTACCACAGGGTTTCCTGAGTTTGT
GGACAAATTGTGAGAATCTACAGCAAGGACTATTGAGTTTTTTTTTGTC

**SEQ ID NO: 364, O. sativa Os06g08500**
ATGCAGCCGAGCAGCCGCGACACGGTCGCCGGCGGCGGCGGCGAGGGGACGCAGGATGACTTCTTC
GACCAGATGCTCTCCACGCTGCCGTCGGCGTGGGCCGACCTGGGGGGCGGCGGCGGCGGCGCGGCG
GGGAAGTCGCCGTGGGAGGTCGATCCGGCAGCGGCGGCGGCGGCGTCGCAGGTGTTCGACGAGTCG
GCGCTGCTCGCGTCCCGCCTCCGGCATCACCAGATCGGTGGGGCTGGCGGCGGCGGGGGGAGAGAAG
CCGGTGATGCTGCAGCTGAGCGAGCTGCACCGGCAGGCCGGCGGCGGCGAGGAGGACGGGAGCGGC
GCGTTCTCGCCGCTGCCGCTGTTCACGGACCGGACGAACGTGCCGCCGCGGGAGGAGATGGAGGGC
GGCTTCAAGTCGCCCAATGCCGCCGCCGGAGGCGAGCACGCGCTGTTCAACGGGTTCGGCGTGCAC
GGCGGCAGCGGCGGCGCCGGGCAGCCGCCGTTCGGCCAGCTTCGGAGGGAGAGGATAGCGGAGCGG
ATGAAATCGCTGCAGGAGCTAGTCCCAAACGCCAACAAGACGGACAAGGCGTCGATGCTGGACGAG
ATCATCGACTACGTCAAGTTCCTGCAGCTCCAAGTCAAGGTTCTCAGCATGAGCCGGCTCGGCGGC
GCCGCCGGCATGGCGCCGCTGGTGGCGAGCATGTCGTCGGAGGGGAACAGCAACGGGAGCAGCAAT
GGCGGCGGTGGCAAGGCGAGCAAGGGCGGCACCGGCGGCGAGGGCGGCGGCGGCGGCGGAGGAGGA
GGAGGAGGTGGCACCGGTGGTGGGATGCGGGTGACGGAGCAGCAGGTGGCGAAGATGATGGAGGAG
GACATGGGCACGGCGATGCAGTACCTGCAGGGGAAGGGGCTCTGTCTGATGCCGATCTCCCTCGCC
TCCGCCATCTCGTCGGCGACCTCATCGGCGTCGCTCCTCTCCCGCCCGTCCATCCGCCACGCCGGC
GCGCCGCCGCAGACGATGCTCGATGCCGCCGGCCCGACCTCGCCGGCGGCGATGTCTAACGGCGAT
GACCCACGGCACGCAAAGGCGGACGGCGGCGCCGGCGGGACGCAATGA

**SEQ ID NO: 365, O. sativa Os06g09370**
AGAAGGGGTGTACTAGTAAACAAAACAAAAAGAATAGGTTGAGACTTTCCTGCTATCGTCAGCATT
TTCAGCCCCCCTCCCCTGGGCTGGCTCTGCATCATCATTCATCATCAGCAGCAGTCAGTCCATCCA
ACGCGTCTTCAGTCCTCACCCGCAGCAAGAAAGCCACACTTTTCCTCCTCCTCCTCCTCCTCCTCC
TCCGCCTCTTCCTCTTCCTCCCCCTTCGTTCTCCACACCCACTCTCGGGCTGCGGCTTCTCCGGCT
TCCGCGCCTCTTCCTCTGTCGGGTTAACAAAGTTACTTTCAGCGTCAGAGGGTATTTCGTACAAAA
AGGAACTGCTGTTGGTACAGGATTATCAGCTGTGAACACTTTCTGTTTCTAGAGGCCATTCTCTGT
AACACTTTGGAGCATCAATTTTGTAGCGTGCTATCATGGACTACTCTGCTGGTTCCTACATGTGGC
CTGGCAATTCAGGTTCTGAGAACTATAATTTTGTTGACGGTTCGTCGGAATCATATGCAGAAGAAG
GAAGCCTACCACCTTCAGGCTATTTCATGGGAGCTGGATCAGATCGCAGTTTAAAGATCACGGAGA
ATGAAAGGAACCCCACTATGCTTGCAAATGGATGCTTGCCATACAACACCCAGGCTCATCCATTAT
CTGGCCAGATTCTACCTAAGGGTGAGCTCCCTAACAATCTTCTGGATCTTCAACAGCTACAGAACA
GCAGCAATCTGCGAAGCAATTCAATTCCCCCAGGGGTTCTTCAGTGCAATTCAACATCTGGAACAT
TTGATGCGAAATTGGATACACCTGGTCTTGCAGAACTGCCTCATGCTTTGTCCAGTTCAATTGATA
GCAATGGTAGTGACATTTCTGCTTTTCTTGCTGATGTGCATGCCGTTTCTTCAGCCCCGACGTTGT
GTTCAGCATTCCAAAACGTTTCCTCCTTCATGGAACCAGTAAATCTAGATGCTTTCGGTTTCCAAG
GGGCACAAAATGTTGCTATGTTGAACAAAACAAGTCTTCCAAATGGGAATCCCTCGCTGTTTGATA
ATGCTGCCATAGCATCACTACATGATAGCAAAGAGTTTCTCAATGGTGGTTCCATCCCTTCGTTTG
GTACTGTCCTGCAGGCACTAGGAGCGGGTGGTTTGAAGGCTGCTCAACAGGAGCAAAATATCCGGA
ATATACCTCTCCCTACATTCACATCTGGTAGTCATTTGGCAGTTACTGATGCACAAGGGCCACCAC
TTCCTTCAAAGATACCACCATTGATCCATGACCATAATAGTGAGTACCCTATTAACCATTCTTCTG
ATGTGGAACCCCAAGCAAATTCAGCTCCTGGAAATAGTGCCAATGCGAAGCCACGTACAAGGGCTC
GCCGTGGACAGGCAACTGATCCTCACAGTATTGCTGAACGGCTTCGCCGAGAGAAAATTTCAGAAA
GGATGAAAAATCTCCAAGTCCTTGTCCCAAACTCCAATAAGGCAGATAAGGCATCAATGCTTGATG

FIGURE 5 (continued)

AAATAATTGATTATGTGAAATTTCTTCAGCTTCAGGTCAAGGTATTGAGCATGAGCAGGCTAGGAG
CTCCTGGAGCAGTTCTTCCCCTTCTTAGGGAATCTCAAACCGAGTGCCATAGTAATCCATCTCTAT
CAGCTTCAACCATTTCACAAGGGCCACCAGACATGCCAGACTCAGAAGACAGCTCTGCCTTTGAGC
AAGAGGTAGTGAAGCTGATGGAAACGAGCATCATCAGCGCGATGCAGTATCTTCAGAACAAGGGTC
TCTGCCTGATGCCCATTGCTCTCGCTTCGGCCATATCCAACCAGAAAGGCATGGCTGCTGCTGCTG
CAATCCCTCCTGAAAAATGAAAAAAAAAATGAAATCGGAAAAATATGGGGGGAACAACTGCAGAAG
ACATAATAACTCAACGGCTGCAGAAATGTGCTGGACACTGCAGGTTTTGTGAATGAAGTGAAATCC
AATCCAGGGGGTCATAAAACATAGGAAGTTATCTGACTAATTCAGGCTTATGACACAATCTACTCT
ATAGTCTATAGCTATTGTGTGCCTGTTGCACATCAGTTGATGTGTATTCTTTTGTTTTTTTTGTAT
TGCATCCGGATGCTAATTAGGTAGTAGTGTTTCCTTTGATGTACATGAGTATTATCTATGCGGTTT
TATCTAATGTGCATATAAAGTAAAATCTGGTATGTTGCTGCTA

### SEQ ID NO: 366, O. sativa Os09g25040
ATGGCTGCGGCGGCGGCGGGCCAGATCTCCCTGGACGACCTCCGCAACGGCGGCGGGGTCGCC
GCCAATGCCGGCGGCGGCGGCGGCGTCCACGACGACTTCCTCGACCAGATGCTCAGCAGCCTGCCG
CCCTCGGCGTGGCCCGACCTCGCCGCCGGGAAGGCGGCCGAGGACGACGCCGAGGGGATGCATCAC
CACCACCACCAGCAGCAGCAGCAGTTTGGTGGGCCGTACGACGAGTCGGCGATGCTGGCGTCGCGG
CTCCGGCAGCACCAGATCAGCGGCGGCGGAGGAGGAGGCGGCGGTGGCGCGGCCGCCGTGAAGCAG
ATGGTGCTGCAGCAGCTCGCCGATCTGAGGCAGGGGCACCACATGATGCTCCAGGGCTTGGGGGGA
CGGTCGCCGGCGGGGGGCGGCGGCGGCGGCGACGGGGGCCTTCTCCTCCCGCTCACCCTCGGC
AGCGGCGGATCGGGCGGCGACGTGCAGGCGTTGCTCAAGGCCGCCGCGGCCAATTCCGCCGGAGGA
GGAGACGCCGGCGGCGTCTACGGCGGCGGATTCGCCGGCTCGCTCCATCAACAGCAGCAACATTTC
CAGCCACATCCACAGACGGCGCCGACGATTCCGACGCAGAGCTTCGGCGGCGGCGGCGGCGGAGGA
GGAGGAGGAACCGCGTCAGGCGGCGGGGCGGCGCAGCCTCAGGCCGGCGCGGCTGGAGGAGGCGCG
CCGGCGCCGCCGCGGCAGCGGGTGCGGGCACGGCGAGGCCAGGCCACCGACCCCCACAGCATCGCC
GAGCGGCTTCGGAGGGAGAGGATCGCCGAGAGGATGAAGGCACTGCAAGAACTGGTTCCCAACGCC
AACAAGTTGATGCAGACGGACAAGGCTTCCATGCTGGACGAGATCATCGACTACGTCAAGTTCCTA
CAACTCCAAGTCAAGGCAAGCACGTACACTAAATTACTAATACATGTTTTGAGCATGAGCCGGTTA
GGTGGCGCCGCAGCCGTCGCGCCGCTCGTGGCCGACATGTCCTCGGAGGGGCGAGGCGGCGGCGCG
GCGAACGGCGGAGCACCGGCGGCGGCGGGGAGCGATAGCCTGACGGTGACGGAGCAGCAGGTGGCC
AAGCTGATGGAGGAGGACATGGGCACCGCCATGCAGTACCTGCAGGGGAAGGGCCTCTGCCTCATG
CCGATCTCCCTCGCGTCCGCCATCTCCTCGGCGACGTGCCATCTCCGGCCGCCGGTGGTCGCCGCC
GCGCAGCAGTTCCCGGCCGGGCTCGGCGCCGCCGCTGCCGCCGCGCACCACCACCAACTGAGCGCG
GCGGCGGCGGCGGCCATGCGCGGACACCTGCCCGGCCTGAACGCCGACGGCTCCGTGCCGGCC
TCGCCGAGCATGTCGGTGCTCACGGCGCAGTCGGCCATGGCCAACGGCGGCGGGGGCGCCGCCGAC
GGCGAGGGGTCGCAGCTCAAGGACGCCGCCTCCGTCTCCAAGCCGTGA

### SEQ ID NO: 367, O. sativa Os07g08440
ATGAGGGCCCTGCAGGACTTGGTCCCCAACACAAACAAGACAGACAGGGCAGCTATGCTAGACGAA
ATCCTTGATTATGTGAAGTTTCTTCGGCTTCAAGTAAAGGTTCTGAGTATGAGCAGGCTGGGTGGT
GCTGGTGCAGTTGCGCAGCTGGTTGCTGATATTCCAATCTCAGTTAAGGGGGAGGCAAGTGACAGT
GGGAGCAAACAACAGATTTGGGAAAAGTGGTCAACCGATGGCACAGAAAAGCAGGTAGCGAAGCTG
ATGGAAGAAGACATCGGAGCAGCAATGCAATTCCTCCAATCCAAAGCACTATGCATGATGCCAATC
TCTCTTGCGATGGCGATCTATGACACACAGCATTCGCAGGACGGACACTCAGTGAAGCCTGAACCC
AACACTCCTTCATAG

## FIGURE 5 (continued)

**SEQ ID NO: 368, P. abies DV982110**
CACAGGAGGCAGCTGTAACGGCAGCGACGGAGGAATGCTTCCTCTTCCTCTCAGTCTTGGTCAGGC
TGGAAAGTCCGGCGACATGAACGAGCCCGGATCGCGGGAAGAAATTGAAGCTTCTTTCAAGTCGGT
AAATAATGCCCGAGATTCGAACCTCGGAGGGCTATTCCAGCCGTTCGCCGTATCACCGCGGGGCGT
TCGACCGACCGGTCAAAACTTCCATGCGCAGCCCGGACAAGTGCCATTGCAAGGGTATGGTGGAAT
GCCCCAACCTCAGCATCAGAATCCACCTCCGACCGGAGTTGGTGCTGCACCACCTGTTCGACCTAG
AGTAAGGGCACGCCGTGGACAGGCTACAGATCCCCACAGTATTGCAGAACGATTGCGTAGGGAGAG
GATTGCAGAGAGAATGAAGGCTCTGCAAGAACTGGTTCCTAATTCCAATAAGACGGATAAGGCTTC
TATGCTGGATGAAATAATTGATTACGTCAAGTTTCTTCAGCTGCAAGTGAAGGTTCTAAGCATGAG
TCGACTGGGAGGTGCAGGAGCAGTTGCTCCCCTTGTGGCTGATATACCTGCTGAGGGTGCCAACGC
CCCACAAGGCACAAGAACCAACGGTAGCCAGAACTCTTCTCCGGACGGTCTAGCATTAACAGAACG
CCAAGTAGCAAAGCTAATGGAAGAAGACATGGGAACGGCCATGCAGTATCTTCAGGGAAAAGGTCT
CTGTCTGATGCCTATATCTCTGGCCTCTGCCATTAATAACTCCGGTTCGAGACCCCAGGCTCCTTC
CACCCCTTCTCNNNNNNNGACTATTGGCATCTAATGTTAACGACAGGCAGGTACTCGAACCCTCCTN
NNNNTCGGCACTATCTNNNNNNNNNCTCNNNNNNNNNATGACATACAGCCATCCATC

**SEQ ID NO: 369, P. deltoides TA3263**
ATTACGGCCGGGGGGTACTCCTCACTAGTCACTGGCTACCTGTGCTCGTATTAGAAAGCAAACCAA
AGCAAAGCGAAAAGCTGAAACCTTGAAAGCCGAAGTACTTAGCTATAAAGAAAAAGAGAAAAAGAA
AGAAAGAAGAAGGTAACCCAACAGTCCTTAACTCTCTCAATTAAAACGATTTTGCATGTAAATTGA
TGCTATTTTGTATCCCCAACTCTCTATCTCAACTCACTTCGACTCCTCCGAGTCCAACTCCAAATG
GCCAATAATCCAACCGAACCTCCAGCCGACGATTTCCTCCAGGAAATCCTCGGGATGCCTAACTTT
GCTTCGGCTGAAGCCGGGTTGGTTGGAGCCGATGCTGGCCTGGCTGGCGCCGCTGCTGCTCAAGCT
TCTATGATGCTTCAGCTTAGCTCTGGTGATGGTTCCGGCCACATCTCCGACCTTGGCGGTGCTCCT
GGAGGAGGAAGCGCCGGGTTTCACGGCTTTCCCTTGGGGCTTAGCTTGGAGCAAGGGAAGGGAGGG
TTTCTGAAGCCCGAGGAGGCGTCTGGGAGTGGAAAAAGGTTCCGTGATGAGATTGTTGATGGTAGA
GCGAAAAATGTTTTTCATGGTCAACCAATGCCGACAACGGTTGCTATAGCACCGCATCCACCAGCA
ATGCGCCCAAGGGTACGGGCTAGACGAGGTCAAGCCACAGATCCACACAGTATTGCTGAACGGTTG
CGCAGAGAAAGAATAGCCGAAAGAATCAGGGCATTGCAGGAGCTGGTTCCTAGTGTCAACAAGACG
GATCGAGCCACCATGCTTGATGAAATTGTGGATTATGTGAAGTTTTTAAGGCTTCAAGTAAAGGTA
TTAAGCATGAGTAGACTGGGCGGAGCTGGTGCTGTCGCACCACTTGTAACAGACATCCCACTATCA
TCAGTTGAGGATGAAACTGGCGAAGGAGGAAGAAACCAGCCAGCTTGGGAGAAGTGGTCGAATGAT
GGCACTGAACGGCAGGTAGCTAAGCTAATGGAGGAAATGTTGGCGCTGCCATGCAATTTCTTCAA
TCGAAGGCTCTTTGCATCATGCCCATCTCACTAGCGACGGCCATTTACCATACACAACCACCAGAT
ACCACTAGTATTGTCAAGCCAGAAACAAATCCCCAATCATAGAACCGTTGAAAGGTGGGGGAGCAT
CCCATGGTCCTGTCTAAAAGAGTAAAGTCCAAATGCTTAGGGTCCCACGTCTTATTTGTCACGTTT
CACCCCCACAAT

**SEQ ID NO: 370, P. radiata WO2005001050 seqID516**
GTTGCAAAGCAATACCCAGAAATTATTCACAATGTCATTGCTGCCTCCCGGGATAGGAGGCCAGGG
CTTGAACTCTCAGGAGAACGACCCGTATCCGTACGACGATTCCCAGTTCCTAGCTAACAGGCTAAG
GCAGCATCAACTGAGCGGTAATTCTTCGATGAATCAGACGCCAAACCAGGCCTCGCAAGGGATTAA
TGAAACCAATACGTCTCCGGGCCGATCCATGGTTTTGCAGCTAAGCACAGGAAGTGCAAGCGCTTC
ACAGCTGCTGGCCTCCATGGGAAATTCGCCTCGCGGCACCGCGGTAATGACGCAGTCGCCCAGTAC
AGGAGGCAGCTGTAATGGCAGCGACGGAGGAATGCTTCCTCTTCCTCTCAGTCTCGGCCAGGCGGG
TAAATCAGGCGACATCAACGAGTCCCGCTCGCGGGATGAAATTGAAGCTTCCTTCAAGTCGGCAAA
TCATGCCCGAGATTCGAACCTCGGAGGGCTATTTCCGCCGTTCGCCGCATCACCACGGGGCGTTCG
ACCGACAGGCCAAAACTTCCATACGCAGTCCGGACAAGTGCCAATGCAAGTGTATGGTGGAATGCC

FIGURE 5 (continued)

CCAACCTCAGCATCAGAATCCATCTCCGACCGGAGTTGGTGCTGCACCACCTGTTCGACCTCGAGT
AAGGGCACGCCGTGGACAGGCTACAGATCCCCACAGTATTGCAGAAAGATTGCGTCGGGAGAGGAT
TGCAGAGAGAATGAAGGCTCTGCAAGAACTGGTTCCTAATTCCAATAAGACGGATAAGGCTTCGAT
GCTGGATGAGATAATTGATTACGTCAAGTTTCTTCAGCTGCAAGTTAAGGTTCTAAGCATGAGTCG
ACTGGGAGGTGCAGGAGCAGTTGCTCCCCTTGTGGCTGATATACCTGCTGAGGGTGCCAACGCCCC
ACAAGGTACAAGAACCAACAGTAGCCAGAACTCTTCTCCAGACGGTCTAGCATTAACAGAACGCCA
AGTAGCAAAGCTAATGGAGGAAGACATGGGATCAGCCATGCAGTATCTTCAAGGCAAAGGTCTCTG
TCTGATGCCCATATCTCTGGCCTCAGCCATTAATAACTCCGGTGCGAGACCCCAGGCACCATCCAC
CCCTTCTCTTCAAGGGCTATTGCCACCTAATGCCAACGACAGGCAGATACTGGAACCCTCGAACTC
GGCACTCTCTGCCCTCACGTCCACCTCCATGACCATACAGTCTTCCATCAGCTCGCCTGGGAGCGG
AATAACGGAGCAAGGTGATAATGCACACAAGGCAGTGAACGGAACAAGGAATCCAAAGGATTCCAG
AGAAGCCAATTCCGTTACGAAGTCCAACGGGATAGGAGGCCCAGCTCTTTCCAAGAACGCCGTTAA
AGGAGAGGACGAACCTCAAAGGAGGACGGGCCAGTAAACAGCACCACCTTGCTAACGGAAACTGTA
CCATACTTCTATTAACGCCAGATTGCATGAAATTGTTCAAGCATACGAAATTTACTTTTCACATGC
TTCAACAATTTCTAAATAGTCGTAGTCGCAGTCGCAGTCGCTCTATGGGAGAGCATTCATTCAAAA
ATCCCAAAAACACACTTCAAATTTTCCTTTCTCCTGAAGTTTGGTGTTTATTTTTATTGAATGGGT
AAAAGAATCGAGCAAGTCTATCAAAATAAGGCACGCGCCCCACTTTGATGATGCCTTATTTAAATA
GACTTGCATAAATATGACATTATGTATGGTTGACCCAAGCGCTTTGTTAATCACTACGTAAGTTAT
ATCTTAAGTTGGATAATAATTATTGCTCATGTCAGCTACACTACGAAATTCTTGTTTATTTAAAAA .
AAAAA

**SEQ ID NO: 371, P. sitchensis TA14134**
CAGGTAAAAAGAAACTTTCTTTTTGATTCGGAATAAAATTCACACAATGTGTGAAAATTCCAAAAC
TCTCACGGTAAACGTTTTAGAATGTAACATTGACTTGCATGGGAAGGATGTGAATAATTAAAAATC
CTTCCCACACTAAAACCCACTTGATCTTTATATGCTTAATACTAATCCCGGAAGCTCTTAAACCAG
AAGATTGTACAATTGAATCTTCATACTCGAATAAAAACAACTATTTCTGGTTAAACAAAAATCGAG
AAAAGACGGACGGCCATGTTACAACCACGGCAGTTTCTCTCCTCTGGTTCTTGTTAGAAAGTGGTC
AATTATTATGCTATTTGCAGAGAACTAAGATTGCCAACAAACAGCTAGTTCATTGTAGCTGGGAGA
TTTCAGTTTAAGATGACAGGCAAAATCAACATTGCAAAAAACTCCATAAGAGATGGCTCCTAACCC
ACGTCTTTTCGTATGTGAGCCACCAGTATACTTTCCATATATTCGAGAATCCTTGAAGTTAGAAGC
AACCTTTTTTGGCCTCGGATATCTTCCCTATTCCTTTTTCCTAAGTTTCGGCAAGCTAGATATTAT
CCCAAATTCTGCTCCATTTGACTCAGCGGTGGGAACTAATTCTCTTCCTCTCTTTTCAACTGTGTT
TTCCTTTAAAAGGAAATCACTGCCAAATCCAGCAGATCCCATTCCAAGAGCACCTGAGCAACTATC
GACAGTTAATGAAGCAGGCTGATTGCCTGAGCTTTGCCTGTCACTAGTACTGTCAACAGAATTTGT
TACCCCAGAACCCAACAGTGGCTTTCCAGTTGAACTCGATATAGCAGTTGCCAGTGCAATTGGCAT
CAAACAAAGCCCTTTATTTTGCAAATACTGCATGGCTGCAGTCATATCCTTCTCCATGAGCTTCAC
AACATTTTGCTCGAAGGCTAGACCATCCTGCGATAGTGAGCCGCCAGCTTGACCCAGAGCCGCTGA
TGCCAGGCTGCCAGATCCCTCAGCTGGAACATCAGCAATTAGAGGCGCAACTGCGCCTGCACCACC
AAGCCTACTCATACTCAAAACCTTTACTTGCAGTTGTAGAAATTTGACATAGTCAATGATTTCATC
CAGCATGGACGCTTTGTCTGTCTTATTAGCGTTTGGAACAAGCTCCTGCAAGGATTTCAAATTTTT
TGCAATCCTTTCCCTGCGTTCCCGCTCAGCTATACTGTGTGGATCAGTAGCTTGACTACAAACTAA
TAGCTCCTTCAAAAGATACGGTGTGTTTTCGCCAGCAATGACACTAGAAGAACTATTCTGAACATC
ATTATTTTGAGCCTGGCCTTCTAAGTTCGGACTCATATGACTTGACGGTGTTACTGACATTGTTGA
GACTCTTGAGCTCGCTTGAAGTGTTCCCATAGAATCACCAGGCAAGCAATTTGTTGGTCCTCCAGC
ACTACTATCCAAAGATGATCTGTCGGCACCATTTATGTCTTCCCATGAAGATGATAGTAATTGATC
AAAAATGTCATCCATTACAGAACTCAAGAGCTCCACTCACTTGCAAAACAATTGCTATGAAATTGC
CCTTTGTCCAGCAATAAACTAGCTGAAACAGCCTTAGCTGATAGATTTCATTTCTTGTTGCTCTCA

**FIGURE 5 (continued)**

AAAATCCAAAATCAGGCTTTCCCCTCAGCACCTACATTAACCGCTAGCAGCAGCCATGTCCGCCTA
TAAAATAGCAGAATTTAACCAAAGAACGATTTCTCCGTTTCCAATTTAGCCTTCTTTAATGTCGCG
GCTTGGCAAAACTGCAACCAACATGTGCCGGCATTGAACGTTAACAGGAGAGCAGAGCAGAGCAGC
CACATTAGCATAACGTTATCAGAATTTCA

**SEQ ID NO: 372, P. taeda TC68930**
TTTTGGGCAAAGGTCTCACTCTGAAGACAAAATGCTAGCCAGAGAAAATAGATCTGGAGATACTGA
GCATGATGATCTTCAAGGGACCCTTTTGACATCTTATACAGGACCACAAGGAGGTCAGACAGTGCT
ACCTAGAACTCCAGGGGCACAATCCCATCAACAGAATCTTAGCACACAAACTATACAATCAGGTGA
AGGAACTTCTCTACAGCAGTATGGAAACCATTCAGCTGTTTCTCAGCTGCAGTCTGGGGCTGGTGG
TGGCAATGCTGCAAATGGAGCTGCAAGGCCACGTGTCAGGGCACGTCGAGGTCAAGCTACCGATCC
ACACAGTATTGCTGAGCGGCTTCGCAGGGAGAGGATTGCAGAAAGGATGAAATCTTTACAAGAACT
TGTTCCAAACTCTAACAAGACAGACAAAGCATCCATGCTTGATGAGATCATAGAGTATGTCAAGTT
TTTGCAGCTGCAAGTGAAGGTTTTGAGCATGAGTAGGCTTGGGGGTGCTGGTGCAGTTGCACCTCT
GATTGCTGATGTTCCATCTCAGGGATCAGGTGGTGTCATGTCAACAGCCTTGGGCCAAGCAAGTGG
GCCCTTGACTCTGTCACAGGATGGTCTTGCTTTTGAACAGGAAGTTGCAAGACTCATGGAATCGAA
CATGACCTCAGCTATGCAATATTTACAAAATAAAGGACTTTGTTTGATGTCCATTGCACTTGCAAC
AGCTATATCAAGTTCAAGTGGAAAGCCTGTACTGGCTACTGTGCCAGGAACAGGTGTTGATAGTAG
TGAGAAGCAAAATTCTGACATGCAATCCATTGTCTTACCTTTTAGTAGCAGTTCAACAACCATGGG
ACTCAGAGCTACTGCATCTGGCAGTGACGCCCCTATAAATGAGAATTCAATAAATAAAGCTAGCAT
AGAAAAAGTTGTGGCAGAAAAATCAAATGGCATTTCACCTGGGCTATCATCTGATGTGCCCAAGGT
TGGATCACAGAGCAGGGAAGAACTCTTGAAGCGAGCACAATGATTTCTATCTGCAAGTGTGTCTGA
TTTCGGTATTCAGGAGTGAGCCTCAATGCTAAGGCGGTTGGTGAGATTTTCTAGTACGCAATTTTT
TAACAGATAGGTTATATACCACTGATTATGGTTCACACATCTGGGATCGTGTGAGATTTCAATAGG
AGCAAAATGTGTAATGTATAAGTGAACCCCTGTTTTAATGAGAAATATTGGTGGAACTCTGTTGGT
GCAAGGTTGCCAACCTTTTTATTTCCACTTTCCTTTTAGGCAAATTTAAGGTTACCAAGTATTTAT
TCTAACTTGAACATGACTGGTGCTGACCATACTAGGGTCCTGCTACTGAGATACCAAGATGGCTCA
GGCATCCATTTTCCTGTTTCCCTTAGTTTTGATTTGTGGATAAGTGGATATTTATCCAAACTTTAA
ACAATTGGATGATCTTATTTCAATTTGCTTCAACAAATGTTAAATGCAATGTTCTCAATACCAAGG
TAAGTATTACCACTGGGTTGTTTTCTGTTT

**SEQ ID NO: 373, P. trichocarpa scaff II. 416**
TCACTTTGACTCCTCCCAGACCTACTCCAAATGGCCAATAACCCAACCGAACCTCCAACCGATGAT
TTCCTGCAGGAAATTCTTGGGATGCCTAATTTTGCTTCAGCTGAAGCTGGCTTGGTTGGAGCTGAC
GCTGGCTTGGCTGGCACTGCTTCTGTTCAAGCTCCTATGATGCTTCAGCTTAGCTCCGGTGATGGT
TCCGGCCACATCTCCGCACTCGGCGGTGCTCCTGGAGGCGGAGGCGCGGGATTTCACGGGTTTCCG
CTGGGGCTTAGCTTGGAGCAGGGGAAGGGAGGGTTTCTGAAGCCCGAGGAGGCGTCCGGGAGTGGG
AATCGGTTCCGTGATGATATTGTTGATGGTAGAGTTAGAAATGTTTTTCATGGTCAACCAATGCCC
ACAACAGTCACTGCAGCTACACATCCACCAGCAATGCGCCCAAGGGTACGGGCTAGGCGAGGCCAG
GCCACAGATCCTCACAGTATTGCTGAACGGTTGCGCAGAGAAAGAATAGCCGAAAGAATCAGGGCA
TTGCAGGAGCTGGTTCCTAGCGTGAACAAGACGGATCGAGCCGCCATGCTTGATGAAATTGTGGAT
TATGTGAAGTTTTTAAGGCTTCAAGTAAAGATATTAAGCATGAGTAGACTGGGCGGAGCTGGTGCT
GTTGCGCCACTTGTAACGGACATCCCACTATCACCAGTTGAGGATGAAACTGGCGAAGGCGGAAGA
AACCAACTGGCGTGGGAGAAGTGGTCAAATGATGGCACTGAAAGACAGGTAGCTAAGCTAATGGAG
GAAAATGTTGGTGCTGCCATGCAGTTTCTCCAATCAAAGGCTCTTTGCATCATGCCCATCACACTA
GCCACTGCAATTTACCACACACAACCACCAGATACCACTACTATTGTGAAACCAGAAACTAACCCC
CCGTCATAGAACCGTGGAAAGGGGGGAGCATCCCGCGGTCCCGTTTAAAAGTCACCAAATGCTTAG
GGTCCCACTCCTTATTTGTCCCG

**FIGURE 5 (continued)**

**SEQ ID NO: 374, P. trichocarpa scaff 70. 65**
AGTAGTATTTTGTGTCATATATTTGCATGCATGGCAGGAAATCCCCCACCTGAAGGGGTTAGGAGAT
GATTTCTTTGAGCAGATCTTGGCAGGGCAGCCACCTGGTTATGGTGGTGAGGCTGTGGGGTCCACC
AGCCTGCCCATGATGGGGTTGCAGTTAGGGTCTAGTGCTGCTAATGTTGGATTAATGAGGAGTAGT
GCTAATAATAATATGGGAATGATGCCTTTAGGGTTAAACTTGGAGCATCATGGGTTTTTAAGGCAA
CAACAAGATGATGGCAGTAGTTCTTTAGATACCAACAACAGCAATAATATCAATÄATGCTTCTCCT
TTTTCTATCACTTCTGCTGGATTCTTGGGGAGAGATTCTGTACACATGACTAGCTTGTTCCCAACG
TTTGGACAATTGCAGATTCATTCATCAATCCGGTCTGCACCACCACCACTTGGACCACCTCAGATT
CACCAGTTTAATAGCCAGCCAACATCAGGAGCAGTTTCAGCTGTACCACAACCACCTGGCATTCGA
CCAAGGGTGCGAGCAAGACGGGGCCAAGCTACAGATCCGCACAGTATTGCTGAGAGGTTGCGAAGA
GTAAGAATCACGGAGAGGGTGAAGGCGTTGCAGGAATTGGTTCCCACTTGCAACAAGACAGATAGG
GCAGCCATGCTGGATGAAATTGTGGATTATGTGAAGTTTCTTAGACTCCAAGTCAAGGTTTTGAGT
ATGAGCAGACTCGGTGCGGCGGGCGCTGTGGCTCAGCTTGTAGCTGATGTACCACTGTCATCAGTT
CAGGGAGAAGGCATTGAAGGAGGGGCCAATCAGCAAGCTTGGGAGAATTGGTCAAACGATGGCACT
GAACAGGAAGTAGCTAAGTTGATGGAAGAAGATGTTGGAGCTGCCATGCAGCTCCTTCAGTCCAAG
GCACTCTGCATCATGCCTGTATCACTTGCTTCAGCAATCTTCCGAGCACGCCCACCGAATGCTCCA
ACACTGGTCAAGCCCGAATCGAACCCCCCCTCATAAACCTAAGCAAGAACGTGCAAGCTTTGCATA
CACATCGATCATTCCAGGGGGCATCTGTTTCCCAAATTAAAAACTGCTCT

**SEQ ID NO: 375, P. trichocarpa scaff XIII. 403**
GTAGTATTTTGGTGTCATATATTTGCATGCATGGCAGGAAATCCTCCACCTGAAGGGGTTGGGAGAT
GATTTCCTCGAGCAGATCTTGGCAGCGCAGCCACCTGGTTACGGTGGTGGTGGGGAGGTTATGGGG
TCCACTAGCCTGCCCATGATGGGGCTGCAGTTAGGGTCTTGTGCTGGTAATGTTGGATTACTGAGG
AGTAATGCTAATAATAACATGGGAATGATGCCTTTAGGGCTGAACTTGGAGCATCATGGGTTTTTA
AGGCAACAGCAAGATGATAGTGGTAGTTCCTTAGATACCAACAACAGCAATAATATCAATAACACT
CCTCCTTCTATAAAATCTGCTCGAATCTTGGGGAGAGATTCGGTGCACATGACAAGCTTGTTTCCA
ACATTTGGACATCTGCAGATTCATTCATCAATCCGGCCTACACCACCTCCTCCTGGACCACCTCAA
ATTCACCAGATTAATAGCCATCCAAACCCAGGAGCAGTTTCAGCTGTACCACAACCGCCTGGCATA
CGGCCAAGGGTGCGAGCAAGACGGGGCCAAGCCACGGATCCACACAGTATTGCTGAGAGGCTGCGA
AGAGTAAGAATCACGGAGAGGGTGAAGGCGTTGCAAGAATTGGTTCCCACTTGCAACAAGACAGAT
AGGGCAGCCATGCTTGATGAAATTGTGGACTATGTGAAGTTTCTAAGACTCCAAATCAAGGTTCTG
AGCATGAGCAGACTAGGTGCAGCAGGCGCTGTGGCTCAGCTTGTAGCCGATGTACCATTGTCATCA
GTTCAGATTAAGGGAGAGGGCAATGAAGGAGGAGCCAATCAGCAATCTTGGGAAAATTGGTCAAAT
GATGACACTGAACAAGAAGTAGCTAAGTTAATGGAAGAAGACGTTGGAGCTGCCATGCAATTCCTT
CAGTCGAAGGCACTCTGCATCATGCCCATATCACTTGCTTCAGCAATCTTCCGAGCACGCCCACCT
AACGCATCAACACTCATCAACACCGAATCGAACACCCCTTCATAAACCTAAGCAAGAAAGTGCATA
CTTCGTGTGCTCGTCGATCATTCCAGCAGGCATCTGGGTCCCAAATCTTGTTCTTGTCC

**SEQ ID NO: 376, P. trichocarpa scaf2f8. 86**
CAAGACGACAAAAAAAATTCACCTGTAAGCATGATAAGTTCAAATCAAAGTGTGGAGTCCTTGGCC
ACTCAAGACACTTCATCTGTAGTTCTTGGCAGTGAGTCAGATTATGCTGTTGATAAAGTCTTAATT
TCTGAACAAGCTCGATTGCAAAATGATTGCCAGAATTGCAATGGAAACCCCTCCCCTGATGGAATG
GCACGTGGAAACTTAAAATTTGGAAACACAGGCCTGCAATGCAATGGAATTCTTCCTACCCTGAGT
TCTCTGAACTATCCAAATCAGCTTCCAATAGTTGGTGATTTGACATCGTATCTCTCTTTTTCTGAG
GCAAGCAATGCTGGATGCAATGGAAGGGAACAATCTGAGTACCTGAGATCCTTAAAAAATCTGCAA
AACTTATCATCAATCCCACAATTGTGGCCTTCACAATCTTATGAGGGTGTTTCTTCTCTCCCTCCT
TTGATGGGACAAGACAGAATAGAGGGATCTGGTCTTCGAGGAGGGAACTTGGATGATGATATGCAT
ATTATGGGGAAGGGATACATGGGCATGGATGAAATTCTCCGACTTGATAAGTTATCTGCATCACCT

**FIGURE 5 (continued)**

ACTACAGAGGGGAAAGAAGATTTGCAAAGTTGTCCTTTCTCATCTGGCATAGCTGAGCCCAATGTA
AACATGTCAATGAATCAATTATCATCTATGCCTCAGACTACATCAGCTGCTCCAGTAGAAGGCTGC
AATGGAACTGGAAAAACTCGTGTAAGAGCACGACGTGGCCATGCCACTGACCCACATAGTATAGCT
GAAAGGCTTCGAAGAGAGAAAATTGCTGAAAGGATGAAGAATCTGCAAGAACTTGTACCTAATTCC
AATAAGGTTGACAAGGCATCTATGCTTGATGAGATCATAGAGTATGTCAAATTTCTTCAGCTCCAA
GTCAAGGTGCTAAGCATGAGCAGGTTAGGGGCAGCCGGAGCAGTCATTCCTCTCCTCACAGATGGT
CAACCTGAGGGTCATAATAGTTTGTCGCTCTCTCCATCAGCTGGTCTAGGAATTGATATTTCTCCA
TCTGCTGATCAGATTGCCTTTGAGCAAGAAGTACTTAAGCTACTGGAATCCGATGTGACCATGGCC
ATGCAATATCTTCAAAGCAAAGGTCTCTGCCTCATGCCCATTGCTCTTGCTGCTGCCATATCCAGT
GTTAAGGCATCGTTATCAGGTACAACTTCTGAGGAGAGGAAGAACAATGGCTACACCAGTGGTCTT
GTTAGCAGCAGCAGCAGCATAACTGGCATTGACACCCATCCAATGTCCAATGATAACAATATTGCA
ACCGGTACACTTAGCAGCAAAGGCATGATTGTCAATGGCTGCAATGAGGTTGTCAAGCAAGAAGTG
CTGAAGAACACTTGATGCATTTCTAGAGCACGGAAAGTAAAGATGTAATTTCATTATTTTAGATGG
GGTTTCATTTTTCAAGCTGAGGCAGATAA

**SEQ ID NO: 377, P. trichocarpa TC63334**
GGGTCTCTCCGCTCTCAGTACTCCTCACTAGTCACTGGCTACCTGTGCTAGTATTAGAAAGCAAAC
CAAAGCAAAGCGAAAAGCTGAAACCTTGAAAGCCGAAGTACTTAGCTATAAAGAAAAAGAGGAAAA
AGAAAGAAAGAAGAAGGTAACCAAACAGTCCTTAACTCCCTCAATTAAAACGATTTTGCATGTAAA
TTGATGCTATTTTGTATCCCCAACTCGCTATCTCAACTCACTTCGACTCCTCCGAGTCCAACTCCA
AATGGCCAATAATCCAACCGAACCTCCAACCGACGATTTCCTCCAGGAAATCCTCGGGATGCCTAA
CTTTGCTTCGGCTGAAGCCGGTTTGGTTGGAGCCGATGCTGGCCTGGCTGGCGCCGCTGCTGCTCA
AGCTTCTATGATGCTGCAGCTTAGCTCCGGTGATGGTTCCGGCCACATCTCCGACCTTGGCGGTGC
TCCTGGAGGAGGAAGCGCCGGGTTTCACGGCTTTCCCTTGGGGCTTAGCTTGGAGCAAGGGAAGGG
AGGGTTTCTGAAGCCCGAGGAGGCGTCTGGGAGTGGAAAAAGGTTCCGTGATGAGATTGTTGATGG
CAGAGCGAAAAATGTTTTTCATGGTCAACCAATGCCCACAACCGTTGCTATAGCACCGCATCCACC
AGCAATGCGCCCAAGGGTACGGGCTAGACGAGGTCAAGCCACAGATCCACACAGTATTGCTGAACG
GTTGCGCAGAGAAAGAATAGCCGAAAGAATCAGGGCATTGCAGGAGCTGGTTCCTAGTGTCACCAA
GACGGATCGAGCCACCATGCTTGATGAAATCGTGGATTATGTGAAGTTTTTAAGGCTTCAAGTAAA
GGTATTAAGCATGAGTAGACTGGGCGGAGCTGGTGCTGTCGCACCACTTGTAACAGACATCCCACT
ATCATCAGTTGAGGATGAAACTGGCGAAGGAGGAAGAAACCAACCAGCTTGGGAGAAGTGGTCGAA
TGATGGCACTGAACGGCAGGTAGCTAAGCTAATGGAGGAAAATGTTGGCGCTGCCATGCAATTTCT
TCAATCGAAGGCTCTTTGCATCATGCCCATCTCACTAGCCACGGCCATTTACCATACACAACCACC
AGATACCACTACTATTGTCAAGCCAGAAACAAATCCCCCATCATAGAACCGTTGAAAGGGGGGGGA
GCATCCCATGGTCCTGTCTAAAAGAGTAAAGTCACCAAATGCTTAGGGTCCCACGTCTTATTTGTC
ACGTTTCAACCCCCACAATCAAATGTTCCCCTAGTTTCAAATTGCAATCAAAGTCAGATGCCATAG
TTCTTGCTTTTGCCTTTTCTTTCCAGCCTTTTTTTTTTCAAACGGGGTTCGATGTCAATGCCTAAA
AAAATGCAGGCAGGCCCTTGGTGTCCTGCCAAGTCCAAGTGAGAGGAGAATTTTCAAAGCTTACTC
GATATGCTCTTCTTTTGGAATCAGTGGTGGGGAAGTGGAAAATGATGGAAGCGCTTTTGTAATGGG
GCTCTAATTTTTCGTAGTGGGTTGTGGGACCTTGTAAAAGAGGCAAGACTGACGGGTTATATAAAT
ATATCATTATTGTAATAGTGTATTCAGCGTAAATGGAATGCAAAGGTAGAATTGAATAATGGCTGG
TTGTTTCAATATGCTGGGTGTTTCAAGTCCAACAGAAAATGATAGCAGTGCTACTTTGTTC

**SEQ ID NO: 378, P. trifoliata TA5414**
CTCTCTCTCTTCTTCCTGTTCTCACCTATTTAGTATTTAACATCCATCAGAAGAATGGTTTCCATT
CTCTTGAAGAAATTCTTGACTCTTCAAATTTAAATTTCCATATATCATAAAGACTTGTTCTCAAGA
AATTAAGATGCAGCCTTGCAGCAGCGGCGGTGAAATGCAAGGAATCAGCTCGCTCTTGAGCAACGG
AAGCCACGAGCAGCAGTCGCAGATCCATCAAAGCGCGACGTTTGATCCAACTTCGCAAGACGACTT

**FIGURE 5. (continued)**

CTTAGAGCAAATGCTATCCTCTCTCCCATCGTGTTCTTGGACTGACTTGAAGTCTCCCTGGGGGGT
TGTTGACCTTAACCCTAATAACAATAATAATAATATTAACAACAAGCAGCCCAGAGACTTATCTGA
CGAAACGGCGCCGTCCACTACTCAAGAGAATAATGTCCCTGCAGGGTTTCAGTTCGATGAGTCCAT
GATCTTAGCTTCCAAGATGAGACAGCATCAGATCAGCGGGAATACTGGGAGTGGGAATAATAATTC
TGCTGCAGCAAAGTTTATGATGCAGCAGCAGCAGCAACAACAAATCATGATGGCTGCTGCTAGAGG
TGGCATCGGGTTGCCTTTATCACTTGGAAATGGTGGCGATAACGACATCGTTGATGCCTCATCATT
CAAGTCCCAACAGGGAGGAGATGGCTCAGTGCAAGCACTCTACAATGGCTTCACTGGATCTTTGCA
TGGCTCAACCCAGCCTCAACATTTTCATCATCTTCAGGGAGGATCGATGCCCGGGCAGACCTTTGG
GGCACCAGGGCCGGTGATGAACCAGACGCAGGCTCAGGCAAGTGGGTCAACCGGTGGTGGTGGAAA
CACACCCGCTCAGCAGCCTAAACAAAGGGTTAGGGCTAGGAGAGGTCAAGCTACTGACCCTCACAG
CATAGCTGAAAGATTGCGCAGAGAGAGAATTGCAGAGAGAATGAAAGCTCTTCAAGAACTTGTTCC
CAATGCCAACAAGACAGATAAGGCTTCGATGCTGGATGAGATCATTGACTATGTCAAATTCCTCCA
GCTGCAAGTGAAGGTCCTGAGCATGAGTAGATTGGGCGGTGCTGCTGCTGTCGCACCCCTTGTTGC
TGATATGTCCTCAGAGGGGGCAGGAGGAGGTGATTGTATCCAAGCAAACGGGCGGAACCCTAACGG
AGCTCAGACGACGTCAGCTAACGACAGCTTAACTGTGACGGAACACCAAGTGGCTAAGCTGATGGA
AGAAGATATGGGGTCAGCCATGCAGTACTTACAAGGCAAAGGGCTTTGTCTAATGCCCATCTCACT
TGCAACTGCTATATCGTCTGCCACGTGTCACTCTAGGAACCCCATCATCAGCACCAGCAACAACAA
CAATAACAACGGCAATCCCCACCACAATCCTTTGTTTCAGTCCAATGGGGAAGGCCCGACCTCACC
TAGCATGTCAGTGCTGACTGTCCAGTCAGCAACTATGGGTAACGGTGGGGCTGACGGCTCCGTCAA
AGATGCTGCTTCCGTTTCCAAGCCCTGATCAACGGACGTCGACTGACTTTATGATGAGGCTCGTCC
AAAGTCTACGAAAATAAGGCGTCACTAGATGTTGGACCTTCACGACGTCGTATTTGTGTAGACTTT
GTGACTGAATCTAACAAACAACAAGAAAGCAAGAAAGAAGCCAATCCCAAGAACAAAAACCTTATT
TTATGTTGTTATTATTATTTTAATTTGCGGGTTAGGTTTTGTTAATTTTACTATTATTAAAATTTT
AATTTCTCTGATGGGATATCTCCCTCTTTTTCTTTTATTTAATATTCTCTGTCATGATTCTCTATT
AAATAATTATTTATAAAAGCCTTTAATAATTAGATTATAATT

**SEQ ID NO: 379, R. communis TA1616**
CCCAACGCTGCATTTTCAGGATTTCTCTCTCTACACTACAAGAACTTGAAGCCAGTACCCATAAAA
CCATATACCATCTTGGATTCCACAACCCCTTTCCTGGTTATTGGGCTGTGGATCTGCCACCCAATT
CACCAACACTTTCTTTTTCTCGGGTTTCGCATACTTCAATTCCCCTCTTCTCTCGTCTCTTCTATT
TGTCTCTCTAAAAACATACTCTCTCCCTCTCTCTCTCTCTCTCTCTCCTCAGCTAATTGAAA
CATCAAAAAGGAAAAAGCATTACCATATTTTTTACACTCTAAAACATCAAACTTTATCACTAATAC
TACGACTGCTACTGCTACTCTAAAACTACTTTTGCAATATTATTGCATGGCGGGAAATCCCCCACC
TGAGGGATTAGGAGATGATTTCTTTGAGCAGATCTTGGCAGTGCAGCCAGGGTATGGAGGCGGTGA
TGGTGGTGGCGGTGGAGACGTGGTGGGGTCCACCATGCCTATGATGGGTCTACAGTTGGGGTCTGC
CAGTAGTGGTGGTGGTGGATTGAGAACTAATAATATGGGCATGATGCCTTTAGGGTTAAACTTAGA
GTTCTTGAGGCAACAAGAAGATGGTAGTGGTGCTTTAGATAACAACAACCATACCAATAACAACAA
TAATAATGTTTCATCTTCTACAACTTCTGTTATCAATGATAGAGATTCTGTTCACATGGCGAGTTT
GTTCCCAACGTTTGGACAGTTGCAAACCCAGACGCTCCGGCCTCCACCACCACCTCACCTCCACCA
GCCATTTCATGGTCAGCCAACACCAGGAGTGGTTTCTGCTGCATCACAGCCACCTGCCATTCGCCC
AAGGGTGCGAGCAAGACGAGGACAAGCCACTGATCCTCACAGCATTGCTGAACGGCTGCGTAGAGA
GAGGATTGCAGAGAGAATGAAGGCTTTGCAGGAGTTGGTTCCTACAGCCAACAAGACGGACAGGGC
AGCCATGATTGATGAGATTGTGGACTATGTTAAGTTTCTAAGGCTTCAAGTGAAGGTATTGAGCAT
GAGTAGACTGGGAGCAGCAGGTGCAGTGGCTCAGCTTGTAGCTGATGTACCGCTAGCATCAGTTGA
GGGAGAGAGCATTGACGGAGCAGCAGCAAATCAGCAGACTTGGGAGAAGTGGTCAAATGATGGTAC
TGAGCAACAAGTAGCTAAGTTGATGGAAGAAGACATAGGAGCTGCTATGCAATTCCTCCAGTCCAA
GGCACTTTGCATCATGCCTATATCACTTGCTTCAGCAATCCTTCGAACACACCCCCCTGATGCACC
ATCAATTATCAAGCCTGAATCAAACACCCCATCATAAACCTAACCAAGAAATTAATCTCCTTTCAC

**FIGURE 5 (continued)**

GTGCAGATGAATTGCTACCAGCAGACATCTGGGCCCCAAGTGCTGGTATAGTCCCATATAACTCAT
TCTAACTCCTCATTATAAATATATATATATATATAATAAAGAAACAATATCCAGTTGGTGCAGCTG
TTCATTTTCTTTAGGAGGAGTCATTTTCATGTCAAGTAAGAAGAATCACGGGGCACTTGATGTTTA
TAATGCTATGGTCAAGGCAAATCCAAAATATTCAAAAAGCTCCATTGATGTGCTTCTATTTCTTGT
ATCTGTGGTGGGGTTAGTGGAGAATGACCAAAAAATTAATAGATAGTGCTAAGAAACTCTTCTGTT
TCATTTCTCTTTTGGATAGTGGAAGTGTAGGGCCATCAAAAAGGGGGTTGATAGGCAGTGTGCATG
ATGTATTTGAAAAATTTGTGTGGAAGTTGAATCGGATAAATGTAGGTTTGTCGTGTAAATCCATGT
TGGAAACTTCATGCTTGACAAT

**SEQ ID NO: 380, R. communis TA2825**
CTCCTCTCAGGCCGACTCCTGCAAATGGCAAACAATCCCACAGAACCTCCAGCCGACGATTTCCTC
CAAGAAATACTCGGCCTACCTAACTTCGCTTCGGCTGACGCAGCCGGCTTGGTCGGAGCCGACGGT
GCCTTGGCTACTCCGATGATGCTGCAGCTTAGCTCTGGAGACGGCTCCAACCACATCACTGCCCTA
GGTGGAGGGGGAGGAGGAGGAGGAGGCGCTGGATTTCACGGGTTTCCGTTGGGGCTAAGCTTGGAG
CAAGGAAAAGGAGGGTTTTTAAAGCCTGAGGAAGCTTCGGGAAGTGGAAAGAGGTTTCGTGATGAC
GTTGTCGATGGCAGAGCTAATACTGTTAAGAACGTTTTTCATGGTCAACCAATGCCCACAACTATG
GCTGCAGCCCCACATCCTCCCACAATGCGCCCCAGGGTGCGGGCTAGACGAGGACAAGCCACAGAT
CCACACAGCATTGCTGAGCGGTTGCGCAGAGAAAGAATAGCTGAAAGAATTAGGGCATTGCAGGAG
CTGGTTCCTAGTGTCAACAAGACAGATAGAGCTGCCATGCTTGATGAAATTGTGGATTATGTGAAG
TTTCTAAGGCTCCAAGTGAAGGTATTAAGCATGAGTAGATTGGGCGGAGCTGGTGCAGTCGCACCA
CTTGTAACGGACATCCCACTATCATCAGTTGAGGATGAAACTGGGGAAGGTGGGAGAAACCAACCA
GCATGGGAGAAGTGGTCGAACGATGGCACAGAACGACAAGTGGCTAAACTGATGGAAGAAAATGTG
GGTGCTGCCATGCAGTTTCTGCAATCAAAGGCTCTTTGCATCATGCCCATCTCACTGGCCACAGCA
ATTTACCATACACAAGCACCGGATACCTCAACTATTGTAAAACCAGAAACAAATCCCCCATCATAG
ACCGTACAAACGGGTAAGCATCCCATGGTCCCATCTAAAAAGGTCGCCTAAGTGCTTGGGGTCCCA
CATTTTGTTTGTCCCGTT

**SEQ ID NO: 381, S. bicolor TC104646**
TACTGGACTTCCCCTTCCCCAGCAGAGCAGAGCAGACCAACCGCTTGTCGCGAGCCGCCCGAAGCT
TCGGGCCCTGACGCGTGGGCCCCGACCGCGCCCGCCCCCGCCCCCGCGTCGCCGGGCATGGCGGGG
CAGCCGCCGCCGCCCGGGGGCTCCGAGGACGACTTCCTCGAGCACTTCTTCGCCTTCCCCTCCGCG
GCCTCCGCCGCCGCCGGGGGACACGCGGGCGCCGGTGCCGGCGGGGACCACCCCTTCCCCCTCGCC
CTCAGCCTCGACGCCGCCGCCGAGCCCAAGCCGGACCGTGACCCCGTGCAGCTCGCCGGCCTCTTC
CCGCCGGTGTTCGCTGGCGCCGGCGGCGTGCAGCAACCGCACCTCCGCGGCCCACCGCCTCCGCAG
ATGTTCCAGGCGCAGCCGAAGCCGGGCGAGGGAGGCATGGCGCCGCAGCCGCCAGCCCCACGGCCC
AAGGTGCGCGCGCGGCGGGGGCAGGCCACCGATCCCCACAGTATCGCGGAGAGGCTAAGGAGAGAG
AGAATTGCAGAAAGGATGAGGGCATTGCAGGAATTGGTCCCCAACACAAACAAGACAGATAGGGCA
GCTATGCTAGATGAGATCCTTGATTACGTGAAGTTTCTTAGGCTTCAAGTAAAGGTTTTGAGTATG
AGCAGACTTGGTGGTGCTGGTGCAGTTGCACAGCTGGTTGCTGATATTCCACTCTCAGTTAAGGGT
GAGGCAAGCGACAGTGGGAGCACACAGCACATATGGGAAAAGTGGTCAACTGATGGCACAGAAAAG
CAGGTAGCGAAGCTGATGGAAGAAGACATCGGGGCAGCGATGCAGTTCCTTCAATCCAAAGCGCTA
TGCATGATGCCGATCTCGCTTGCAATGGCAATCTACGACACCCAACATTCCCAGGATGGCCACTCA
CTGATGAAGCCTGAGCCCAACACATCCTCGTAGTATAGTAACATCAACCCTAGCGTGCATTTCAAC
CCCCTAATACTATTAGGCACCGATATATCCAAAAAAAAAAAGTGTATGATATACGGAGCGTTCCAA
TGTGCTAACCGTGAGATAGGAAAGGACCTTAAATTGGTATATGATGTAGCCTCCCCTTTCCCTATT
TTATTTCAAATCAGAGCAG

**FIGURE 5 (continued)**

SEQ ID NO: 382, S. lycopersicum AK247217
AGCAAAGCTTTAGGTGACTCCCTTTCCTTTATTTTCTTCATGGCTCTCACTGCTCTTTAGAAAGTA
GCAAAACACACACACAAACAACCCAAAACACACACTTCCATTTCCATTTTTTGCCTATATATATAT
ATATATGTAACAAGTACTTGTTAGAGTACTAATTTGTACACCCTCTTATCAATTCCGTTGTAAA
GAACACTTTTTTAACTAGTATTTGTTCAATTGAATGCTTTTCTGAGGAATTAAGGTGGTCGAAAAC
AGCTCCGATCCAAGAAAGATTGAATCTTTATAGTTTCTGCTATGGCTAACAACCCTTCTGAGGGAC
CTTCTGATGATTTCTTTGACCAAATCTTGGGATTTCCTGCTTACAATGGAGCAGAACCTAATTTGG
CGGGAAATGATGCCGGAGCTATACCGCCGGCGATGATGCTCCAGCTTAACTCTGGTGATGGGTCAA
GTCAGTTTACTGGAGTGGGTCTTGGGGTTGGTTTAGGTGGTGGGGGGTTCCATGGTCATGGTGGGG
GTGGTTCTTTTCCTTTAGGGTTGAGTTTAGAACAAGGGAAAGGTGGGTTCTTGAAGATGGATGATG
TTTCAGCACCTGGAAGGAGGTTTAGAGATGATGTTGTTGATAGCAGAGCTTCTTCTTCTGTCAAAC
CTGGTTTTCATGGACAACCGATGCCTTCAATGCCGCATCCCCCTGCAATACGTCCAAGGGTGAGAG
CTAGGCGAGGACAAGCTACTGATCCACATAGCATAGCGGAGAGGTTACGTAGAGAGAGGATAGCAG
AAAGAATTAGAGCATTGCAAGAGTTGGTTCCCAGTGTCAATAAGACTGATAGAGCTGTAATGCTTG
ATGAAATTGTAGATTATATCAAATTCCTACGGCTGCAAGTGAAGGTGTTGAGCATGAGTAGGTTAG
GAGGAGCTGGTGCAGTAGCACCACTTGTTACAGACATTCCAATATCATCAGTAGAGGAAGAGAGCA
GTGAAGGTGGAAATAATAACCAACCAGCTTGGGAAAAGTGGTCAAGTGATGGCACAGAAAGGCAAG
TGGCTAAACTCATGGAAGAAAATGTTGGTGCTGCAATGCAATTTCTTCAGTCTAAAGCACTATGTA
TAATGCCTATTTCTCTTGCATCAGCAATTTATCACTCTCAGCCACCAGATACATCAAGTCTTGTTA
AGCCAGAAACAAATCCTCCTTCATAGATGAACACCCTTATAGAAAAAGGTGACATAACATGCTAAT
GACCTTAAAGAATTTTCTTGCAGTGGATGGTTCCTACTTTTATCTATTCAAGTAATATTTAGCTTT
TTGTTGAAAAAACAGGGTTCTTTTTTAAAAATATTTATGTATAAAGTAAACCATTTTCTATCATTT
ATATATTTGAGTAGTTTGTTCT

SEQ ID NO: 383, S. lycopersicum TA21665
GCTCATTTTCCGTTCCTCTTAAAGCTCAATAAACCTATACGCCGGCAAACTCCGGCATGGCGGACA
ACCCACCGGAGGTATATGCTGCCGATGATTTTCTCGAGCAAATTCTGGCGATTCCCTCTTATGCTA
GCCTGCCGGTAACTGATTTAACCGCCGGTGCCTCATCGGAGAATTCAACATCTGGTGTCTCTCAGC
TCCAGCAGCAGCCGTTGTTCCCATTGGGGTTAAGTTTGGATAATGGCTTTGCTGACGCCAACAACA
CTGGAGGTTTTCAAGTGAAGACTGAAAGAGAAGCAATGAACATGGGGAATTTGTATCCAGGTCTAG
AACATTTGCAATCTCATGCTGTTTGCCTAAGTGTTCCTCAAGTTCACCAAGTCCAGCCTTTTCAAG
GCCACCCTACATCAAGCGCAATAGTAACAATACCACACCAACCTGCAATTCGTCCTAGGGTTCGGG
CACAAAGAGGACAGGCCACTGATCCACATAGTATTGCTGAGCGGTTAAGGAGAGAAAGGATATCAG
AACGAATAAAGGCTTTACAGGAACTTGTCCCCAGCTGCAATAAGACCGACAGGGCAGCAATGCTTG
ATGAGATTCTGGACTATGTGAAGTTCTTAAGGCTTCAAGTTAAGGTACTGAGCATGAGTAGGTTGG
GAGGAACTAGCGCGGCGGCACAAGTTGTTGCTGATATTCCATTGCAGTCTGTTGAGGGAGACACCT
GTGAAAGTCATTCCAACCAGCACGTCTGGGAGAAGTGGTCTGATTCTGAAACAGAGCAAGAGGTAG
CAAAGCTCATGGAGGAAGATGTTGGAACAGCCATGCAGTACCTTCAATCCAAATCACTCTGCATCA
TGCCTATTTCACTTGCTGCACTTATCTATCCGACTCAGCAAAGTGACAACCAATCAATGGTCAAGC
CAGAACAAGCGGCCCCATTGTAGACTTCTAAATTACTGCAGGTGCAATCAAGAACTCCTAATTTCT
TTTAGGCCCATCCGTCTATATATCCTTACCTATTTTCTCTTGAATCACCCTCTGAAATCCTTGGTA
ATGCAATCAAACTCAGATGACACAATTTTTGCTTTTAGCTCAATCATTGGCCCCTTTATCAACTTT
CTTGAGTTCAGTCACACCTTGAACAATGGCAACACTTAAGAAAGCAACCAATCCATCTATCTATAT
CATTGTAGGGAGGAGTGTATATTTTTTTTTCCAAACTCTCTGTTATTAGCTGCTTGTTTCCCCCTC
ACTCTTGTGGGTTGGTTGTGACTCCTTGTATCTTTTTTATAGTGGATAGCTAGCGATGTATTATAA
AAGAAAATTGTCCCAGCTTGCATTTGGTGGACTGTGCGGGGAGAAAGAAACCAAGTTTGTTTGAAG
ACAAAGCCATTAGTATACAGAAACTAAAGATGGAATGTTTAGGGCTTTTCTTTTTTTGGTGTAACC
ATAGCTTAATGGATGATTTAGAATCAACTTATGATGTACTAGATTCCTTGTGAAGATTAAGGAT
TAGGAGCTTCTTCATTTCTTTATTTCTGTTCTTTTGCTCATCTGGATGTATATATGGGGGATTACA
CACCCTTTGTGTAGTTTGTTATTAATACACAAAAATGTTACCTGTTCC

FIGURE 5 (continued)

SEQ ID NO: 384, S. lycopersicum TC172581
TTCCATTTCAGTTCCTCAAAACCCTTAACTTCTTCTCGCCGGGAAAAAAATTAATTTCGCCGGATA
AATCTCCGGCATGGCAGCAAACCAACCGGAAGGCTATGCCGACGATTTCCTCGAGCAAATTCTCGC
TATTCCTCCCTACTCTGGCTTGCCGGTTGCTGATGTTGGCACTCCATCAGAGACGACGTCGTTTAC
CTCGGCGTCTGCCGTATCTCATCTTAACTCTGCCGCTGCTGCTGGCCTACAGCAACCTTTGTTTCC
GCTGGGATTAAGCTTGGATAACGGCCGTGATGACGTCGGCGATGCAGGTCCTTATGCAGTGAAGCA
TGAAAGAGATGGAATGAATATCGGAAATCTATATGCAGGTCTAGAACACTTGCAATCTCATGCAGT
TCGTCACTCTGTGCCTTCTGTTCACCATGTCCAGCCTTTTCAAGGCCCACCAACAACGAGCACAAC
AGTAACTGTGCCACACCCACCTTCAATTCGTCCTAGGGTTCGAGCTCGGAGGGGACAAGCCACAGA
TCCACATAGCATTGCTGAGAGGCTGAGAAGAGAGAGGATATCAGAAAGAATAAAGGCTTTGCAGGA
ACTGGTACCCAGCTGCAATAAGACAGATAGGGCCGCAATGCTTGATGAGATTCTGGACTATGTGAA
GTTCTTAAGGCTTCAAGTTAAGGTACTGAGCATGAGTAGGCTGGGAGGAGCCAGTGCAGTGGCACA
ACTTGTTGCTGACATTCCATTACAATCTGTGGGAGGGGGACAGTGGTGAAAGTAGATCCAACCAGC
ATATATGGGATAAGTGGTCCAATGTTGACACAGAACGAGAGGTTGCTAAGCTCATGGAGGAAGACG
TCGGTGCAGCCATGCAATACCTTCAGTCTAAATCACTCTGCATCATGCCCATATCACTTGCTGCAC
TCATCTATCCTACTCAACAACCGGATGACCAGTCCCTGGTCAAGCCTGAAGCAGCAGCCCCATCAT
AGACCTTCAATTCGTTGCACGTGCCTCTAAGAACTCCCAATAGCCTTGTGTCCCCCCTCCTTTGTA
TCCTTACCTACCATCCATTTGTATTTTCCTGTGTAATTGGTGGCAATGCAATCAATGTCAGATGCC
ACAACTTTTGCTT

SEQ ID NO: 385, S. tuberosum TA30646
TTAGAAAGTAGCAACACACACAAACACCCAAAACACACACTTCCATTTCCATTTTTTTGCCTATAT
ATATATATATGTAACAAGTACTTGTGAGAGTACTAATTTGTACACCATCTTATCATTTTACTTG
TAAAGAACACTTTTTTAAGTAGTATTTGTTCAATTGAATGCTTTTCTGAGAAATTAAGGTGGTCGA
AAACAGCTCCGATCCAAGAAAGATTGAATCTTTATAGTTCTGCTATGGCTAACAACCCTTCTGAGG
GACCTTCTGATGATTTCTTTGACCAAATCATGGGATTTCCTGCTTACAATGGAGCAGAAACTAATT
TGGCGGGAAATGATGCCGGAGCTATACCGCCGGCGATGATGCTCCAGCTTAACTCCGGTGATGGGT
CGGGTCAATTTACTGGAGTGGGTCTTGGGGTTGGGTTAGGTGGTGGGGGGTTCCATGGTCATGGTG
GGGGTGCTTCTTTTCCTTTAGGGTTGAGTTTAGAACAAGGGAAAGGTGGGTTCTTGAAGATGGATG
ATGTTTCAGCACCTGGAAGGAGGTTTAGAGATGATGTTGTTGATAGCAGAGCTTCTTCTTCTGTCA
AACCTGGTTTTCATGGACAACCCATGCCTTCAATGCCGCATCCCCCTGCAATACGTCCAAGGGTGA
GAGCTAGGCGAGGACAAGCTACTGATCCACACAGCATTGCGGAGAGGTTACGTAGAGAGAGGATAG
CAGAAAGAATTAGAGCATTGCAAGAGTTGGTTCCCAGTGTCAATAAGACTGATAGAGCTGTAATGC
TCGATGAAATTGTAGATTATGTCAAATTCCTACGGCTGCAAGTGAAGGTGTTGAGCATGAGTAGGT
TAGGAGGAGCTGGTGCAGTAGCACCACTTGTTACAGACATTCCAATATCATCTGTAGAGGAAGAGA
GCAGTGAAGGTGGAAATAATAACCAACCAGCTTGGGAAAAGTGGTCAAGTGATGGCACAGAAAGGC
AAGTGGCTAAACTTATGGAAGAAAATGTTGGTGCTGCAATGCAATTTCTTCAGTCTAAGGCACTAT
GTATAATGCCTATTTCTCTTGCATCAGCAATTTATCACTCTCAACCACCAGATACATCAAGTCTTG
TTAAGCCAGAAACAAATCCTCCTTCATAGATGACACCCTTATAGAAAAAGGTGACATAACATGCTA
ATGACCTTAAGAATTTTCTTGAAGTGGATGGTTCCTACTTTTATCTATTTAAGTAATTTTTAGCTT
TTTGTTGAAAGAAACAGGGTTTTTTTTTTTTTATGTTTATGTATAAATTAAACCATTTTCTATCATT
GATATATTCAAGTAGTTTTGTTTTCAGC

SEQ ID NO: 386, S. tuberosum TA28621
GATTTCAGTTCCTCAAAACCCTTAACTTCTTCTCGCCGGAAATTTTTTTTAATTTCGCCGGGTAAA
TCTCCGGCATGGCAGCAAACCAACCGGAAGCCTATGCCGATGATTTCCTCGAGCAAATTCTCGCTA
TTCCTTCCTACTCTGGCTTGCCGGTAGCTGATGTTGGCACTCCATCAGAGACGACGTCGTTTACCT
CGGCGTCTGCCGTATCTCATCTTAACTCTGCCGCTGCTGCTGGCCTACAGCAACCTTTGTTCCCGT

FIGURE 5 (continued)

TGGGATTGAGCTTGGATAACGGCCGTGATGACGTCAGCGAAGCTGGTGCTTATGCAGTGAAGCATG
AAAGAGATGGAATGAATATCGGAAATTTATATGCAGGTCTAGAACACTTGCAATCTCATGCAGTTC
GTCACTCTGTGCCTTCTATTCACCATGTCCAGCCTTTTCAAGGCCCACCAACAACGAGCACAACAG
TAACTGTACCTCACCCACCTTCAATTCGTCCTAGGGTTCGAGCTCGGAGGGGACAAGCCACAGATC
CACATAGCATTGCTGAGAGGCTGAGAAGAGAGAGGATATCTGAAAGAATAAAGGCTTTGCAGGAAC
TGGTACCCAGCTGCAATAAGACAGATAGGGCAGCAATGCTTGATGAGATTCTGGACTATGTGAAGT
TCTTAAGGCTTCAAGTTAAGGTACTGAGCATGAGTAGGCTGGGAGGAGCCAGTGCAGTGGCACAAC
TTGTTGCTGACATTCCATTGCAATCTGTGGAGGGGGACAGTGGTGAAAGTAGATCCAACCAGCATA
TATGGGATAAATGGTCCAATGTAGACACAGAACAAGAGGTTGCTAAGCTCATGGAGGAAGACGTCG
GTGCAGCCATGCAATACCTTCAGTCTAAATCACTCTGCATCATGCCCATATCACTTGCTGCACTCA
TCTATCCTACTCAGCAACCGGATGACCAGTCACTGGTCAAGCCTGAAGCGGCAGCCCCATCATAGA
CCTTCAATTCGTTGCACGTGCCTCTGAGAACTCCTAATAGCCTTGTGTCCCCCCTCCTTTGTATCC
TTACCTACCATCCATTTGTATTTTCCTGTGTAATTGGTGGCAATGCAATCAATGTCAGATGCCACA
ACTTTTGCTTTTATCCCAACCATTGGCCCTTTTTTTTTAATTACCTTTCTGGAGTTTCGTCAAACA
AGGCATGATGTCATCATACCTTGGAGAAAGGTAAACGTATGAAAGCACCCAATCAATTAGCATCAT
TTTCGGATGAGGTGAGCTTCTTTTTCAAAGTTATCTATGATTAGCTGCTCTTCTTCCACCATTTTG
TTGTGGGATTGTGGGGACTCCCTTTATCCTTTGCATAGTGGATGGTGGGGCTTTGTAAAAGTAACC
AACTGGTTGTTGGAAAGTATATTGTAATTTTAGTATATTATTGTAATGGGTAGTTGAAGTTGCTCT
CCCTCCAAAAAGTTTTG

**SEQ ID NO: 387, S. tuberosum TA34455**
CTTCTATCTTTCTCTCTTCTCCTTTTAGAACCAAACTTTCACACACAAAGCTACTGAAGAAAAAAT
TTCTCTCTGCAAAATCTCAGAGAGGAAAAAAAAAATGCAAGCCATGAATTCACTTCTAAGTCAACA
ACAGCAGTCACAGATGTCACTGCAAGACCTTCAAAATGGCGGAAATGGCGGTTCTACCGGTGGTGT
TGGTGGTTTAGGTCAACACAATATGGGTCACTTTCACTTTGATCCGACGTCGTCTCACGACGATTT
TCTCGAACAGATTCTCTCTTCTGTTCCTTCTTCTTCTCCTTGGCCTGACCTTTCCAAATCCTGGGA
CCCACATCACCATCTCTCATCGCCGCCGCATAACCCTAGCTCCGGCGAAGATCAACCTCCTTCTAA
TCCACTTCACTCACAGTTCCATTACGACGACCAGGCTTCTTCTCTACTAGCCTCAAAGCTCCGTCA
GCATCAGATCACTGGCGGCGGCGCGGCTGCAGCTGCTAAAGCACTTATGCTACAACAGCAGCTTTT
GCTTTCTAGAACACTCGCCGGAAACGGACTCAGGTCTCCTAATGGAGCTTCCGGCGATAACGGCCT
CCTTTCCCTACCTCTAAACCTCAGTAATAATGGTGACCAAAACGATGGCGTCGCTAACCCGGCTAA
TGACAATTCCGTTCAAGCTCTTTTCAATGGATTCACCGGATCTCTTGGTCAAACCTCCAATCAACC
TCAACATTTTCATCATCCTCAGGGAGGATCGATGCAATCGCAGAGT

**SEQ ID NO: 388, S. tuberosum TA37666**
GAAAGAAAAAAACAATGCAAGCTATGAACTCATTTCAAAGCACCGGCGAAAATGGTGCTTCGTCCG
GCGAACACATCAGTCATTCCCATTTCGATCCGTCGTCGTCACACGACGACTTCCTCCAACAGATTC
TCTCTTCCGTTCCTTCTTCTTCTCCCTGGCCTGAAATCTCCGGCGACGGTCACCCCTACAATTTCG
ACGACCATCAGTCAACTCTCTTGGCTTCCAAGCTCCGGCAGCATCAGATCAACGGCGGCAGCTCTG
CTGCCGCAGCTGCTAAAGCGTTGATGCTTCAGCAGCAGCTTCTGCTTTCTAGAGGAATCGCCGGTA
ATGGCGGCGATCAAAACGACGACGGTTTAAATCCGGGGAATGATATTTCAGTTCAAGCTCTTTACA
ATGGATTCGCTGGATCTCTTGGTCAAACCTCAAATCAATCTCAGCATTTTCACCATTCTCAGGCGC
AGAGTTTCGGAGCTCCGGCGGCGTCGTTGACGATGAATCAAACGCCGGCGGCGAGTGGTTCAGCTG
GTGGAGCGCAGCCAAAGCAACAGAAAGTTAGGGCTCGAAGAGGACAAGCAACTGATCCTCACAGCA
TTGCTGAAAGATTACGGAGAGAGAGAATTGCAGAGAGAATGAAGTCTTTGCAGGAGCTGGTACCTA
ATGCCAATAAGACAGACAAGGCTTCAATGTTAGATGAGATCATCGACTATGTCAGGTTCCTCCAGC
TCCAAGTCAAAGTTCTGAGTATGAGCAGATTGGGTGGTGCTGCAGCTGTTGCTCCCCTAGTTGCTG
ATAGATCCTCTGAGGGAGGAGGTGATTGTGTACAAGGAAATGGAGGTCGGGGTGGCAGTAATGGAA

**FIGURE 5. (continued)**

CGACGTCGTCTGCAAACAATGACAGCAGCATGACGATGACGGAGCACCAGGTAGCTAAGCTAATGG
AAGAAGATATGGGATCAGCAATGCAGTATCTTCAAGGAAAAGGCTTATCCCTTATGCCAATTTCCT
TAGCCACTGCTATTTCCACCTCCACGTGTCACTCCATGAAACCCAACAACCCACTACTACTCGGCG
GAGGCTCCGCTATCAACGGCGGTGGTGAGACCGGCGGTGGACCGTCTTCTCCTACCTTGTCAGCTT
CCACTGTTCAGTCAGCTACGATGGGCAATGGCGGGACTTGAGTTAGTCCTCCTATCCCAATGTGAC
TCACTGTTTTTATTTAGAATCAATTTGATTTTATATTTCTCGTTTTATACTATTATGGAATTATTT
ATAGTCATATTAATATTGATGACTTGTTTTTAGAAA

**SEQ ID NO: 389, T. aestivum TC253044**
CACGAGGGCAAGCCAAAGCCTCCGCCTCTCTCCTCCTTTTCCGCCCAAACAAACCGCCCCCTCCCC
TCCCGTCGCCCTCACCTCCGCCCTCGCCGGCAGTAGTATACGCCCGCCACCAGCTCCGACGACCCG
CGCCTTCCCCCCCGCTCGCCCGCCGTCGTCTCCCCTGCCCGCTCCCCCGTCCCTCCGCCGCGGGTT
TGTTCTAGAGGGTGTATCTGAAGTTCTCTCAGCATCCGAGTGCGCAGCTTTGTACAAAAGGAACCA
TTCAGGCCATCTCAGATTGTCTTCATGGATGAATGATAGGATCACCAGTTGCTGTCACTTTAAGTA
TCTACAAGTCACTCTTTTCATAATATTTCTGAGCAGAGGTTGGGGAGTCAAGTTGTAAGGGTGTAA
AGATGGACTACTCTAATGGTTCTTTCTTTCCTTCATGGCCTGGCAATTCCGCTTCCGAGAATTATA
GCTTTGTTGATGGTTCAGTGGAATCATATGCAGAAGAAGGAAGTATGCCACCTACAGGCTATTTCA
GAGCTAGATCAAATCAGAATTTAACATTTGATGAGCATGAACAGAACCCTGCTATGCTTGCAAATG
GGTGCTTGCCGTACAACACCCAGACTGATCTATTATCTGGTGAGATTCTGTCAGAGGACAAACATT
CCAACAGCCTTATGGAGCTTTCCACAACTTCAGAACAATGGCAGTCTGCAAAGTAATTTAATCCCA
CCAGGGACTCTTCAGTGCACTTCAACACCTGGAACATTTGACCCGCAGTTGGATACCCCTGGCCTT
CTAGAACTTCCTCATGCCTTGTCCAGTTCAATTGAAAGCAATGGTAGTGAAGTTTCAGCTTTTCTT
GCTGATGTACATGCTGTTTCTTCAGCCTCAACTCTCTGTTCGACATTCCAAAATGTTCCTTCCTAC
ATGGAGCCAGTAAGCCTAGAAGCTTTCAGTTTTCAAGGGATACAAAATGCTCCTATGTTCAACAAT
ACAAGTCATTCAAATGGGAAACCTGTCAGTATTTGATGAGGCAACCATGGCATCACTACATGATAG
CAAAGAATTTCTCAGTGGTAGCATCTCATCTTTTGGTACGGCCGAGCAGTCACAACTAGCTGGTAG
TGGTTTGAAGGCTGAACAACAGGAACAAAATGCGATGTGCAATATTCCACTCCCTTTCGCTTCTGG
TAGTCAGATGGCAGTGAGTGAAGCACAAGGGGCAATGATTCCTTCAAAGATAAGCTCAACGATCCA
TAACAATAAAAGTGAGTACCCTGTCCCTATCAGCCATTCTGCTGATGCGCAGAACAAGGCAAATTC
AGCTAATGGAAACAGTGCCAGTGCTAAGCCACGAGCAAGGGCTCGTCGTGGACAGGCAACTGACCC
TCATAGTATTGCTGAACGGCTTCGCAGAGAGAAGATCTCAGAGAGGATGAAAAATCTCCAAGACCT
TGTACCAAACTCAAATAAGGCAGATAAATCATCAATGCTCGATGAAATAATTGATTATGTGAAATT
TCTTCAGCTTCAGGTGAAGGTCTTAAGCATGAGTAGGCTAGGAGCTCCCGGGGCAGTTCTTCCCCT
CCTTGCAGAATCTCAAACTGAGGGCCGTAGCAATTCACCTCTATCATCTCCAACCGCTTCACAAGG
GCTTCTGGATGCAGCAGGCCCAGAAGACAGCTTGGTCTTTGAGCAAGAAGTTATAAAGCTGATGGA
AACAAGCATCACAAATGCAATGCAGTACCTTCAGAACAAGGGCCTCTGCCTGATGCCTATCGCTCT
TGCTTCAGCCATATCCAACCAGAAAGGCACTTCTGCAGCTGCAATCCCTCCTGAAAGGTGAAAACA
GAAAGCACATCATGCTCCTCCCATCGGCCCGCGGAAACAACTGTCGAAATGTGCTAGAGTTCATAG
AAGTTGTGAGAAAACAAAAATCCGAGGCTGAAGGATAGGAAGTTATGCGACTAATAGTACAAGCTT
ATGGTATAATCTAGTAGCTTACTCCAAGAAACCTATACTTTTTCTCTTCTCTGTTGCACATCGGTT
GGTGTGTATTCTTTTGTTTTTTGGACGGCATTCTAACTGGGTACAAGTGTCTGGCACCTTGGGTTG
TACATGGATCTATGAGGGTTATTACCGACTGTTCATGATGTTATATGATGATCAAAGCAGACCTAG
GAAAACTCGGTTGCTTTGGGTTTCACTTGGTT

**SEQ ID NO: 390, V. vinifera GSVIVT00016367001**
ATGGATGAGTATCTGGATCACTTGTTTTCATCCACATCATGGTCAGATGTGAATGTGAAGGAGGGA
TCATCTTGGATTTGTGGTGAGCCTAGCCAAACAAATGGGATGCTTTCAGATTCAATTGGAATATAT
GAAGGTGATAAAAAAAACTCACCTGTTGGCATGACTAATTCAAACCTTATGATCGAGGGCTTGGTT

**FIGURE 5 (continued)**

ACTCAAGATACTTCATCTATTGTTCTTGGTGGAGAGTCTGATCATGGTCTTGGCAAGGGCTTACTT
TTGGAAGAAGCTCCACGGCAGCAAGACCTTCAAAACACTGAAGGCAATTCCTCCTTGAATGGAGCA
GTGAATGGCAGCTCAGAAGTTGGATACGTGGGCCTGCAACTAAATACTCCTACTTCAACCCCATGC
TCACTGGATCTTGGCTCTCCCAAGCAGCTTTCGCTGATTGGTGGCATGTCTAGGTCATCTCGTCCT
TTCACTGAGCTGGATCATGTTGGGTGCAATGGTAATGAGCCATCTGATTTTCAGAGATCAGTTGGA
AATTTTCAAGCATTGCCTCCAATTCCACAGTTGTGGTCTCAACCATCTTACGGGGGTGGTTCCTCC
TTGTCTCCTGTTATGGGAGAATACAAGATGCAGGGCTTTGGTCTGCAAGGAGAATATGTGGATAAT
GAAATGGATATCATGAGGAACAGATACGTTGGGGATGAAATTCTGCAACTTGATAACATTTCTTCA
GCAATCCCCATAAAGGGGAAAGAAGAGCAGCACACTCATCCTTTCTCTCCTTCTGCGGTTGGGCCC
CACATGACAATGACAGCATCTGGATTACAGTCTTTGCCACAGACTACGGTAGGTACTGCTTCTGGT
GGCTGTAATGGAACTGGAAAGCCGCGTGTAAGGGCTCGTCGAGGCCAGGCCACTGATCCTCACAGT
ATTGCTGAAAGGCTTCGGAGAGAGAAAATTGCTGAAAGGATGAAGAACCTGCAAGAACTTGTTCCC
AATTCCAATAAGACGGACAAAGCATCTATGCTCGACGAAATCATTGAGTACGTCAAATTTCTTCAG
CTCCAGGTCAAGGTACTAAGCATGAGTAGGCTGGGGGCAGCAGAGGCAGTTGTTCCTCTCATCACA
GATGGCCAAGCTGAGGGCTCTAAGGGTCTGTCACTTTCACCCTCGGCTGGTCAAGCTGAAGATATT
TGTCAATCTCCTGATCAAATTGCCTTTGAGCAAGAAGTAGTGAAGTTGATGGAGTCCAACGTGACC
ATGGCCATGCAATATCTTCAAAGCAAAGGTCTCTGCCTTATGCCAATTGCTCTTGCTACAGCCATA
TCCAGTGGAAAGGCAGCATCATCAGGTACTGGGTCCGATGAGGGAAAGAACGCAGCAACACCAGTA
GCAGCAACACCAGTAGCAACAGCTTACCTGGCATTGGGACACATCATACATCTTCTGATGGCAATG
TTCTGA

**SEQ ID NO: 391, V. vinifera GSVIVT00017237001**
ATGCTCTCTACTCTCCCTTCTTGGTCTGACCTCCCCGCAAACCCTAAATCTCCCTGGGAACTCAAT
GCTTCCAACCCTATCTCCATGCCTTCCAACAAGTCTAGGGATTTGTCCGACGATACCACGCCGTCT
AATCCGGACAACGTTCAGTTCGCTTTCGACGAGTCCGCCATGTTGGCCTCCAAGCTCCGCCAGCAC
CAGATCAGCGGGAACTCGTCGGCGGCCAAGTCGGCGTTGATGCTTCAGCAGCAGTTGCTTTTGTCA
CGAGGCGTCGCCATGGGTAGGTCCCCGTCGAATGGGTCCGGCGCCGGTGAGTCCGGCCTTCTTCAA
CTGCCTTTGTCACTTAGCAACGGGGATTCTTGCCTCGTTGACCGATCACAAAACGACGTCGTTGAT
GGGTCCTCCTCCTTCAAATCCCCCAATCAGGGAGGAGACGGTTCAGTTCAAGCTCTCTACAATGGC
TTCGCCGGAGCTCTTCACGGCTCCGGTCAAGCCTCCAACCAAGCTCAGAATTTCCACCATCCTCAG
GGTGGATCAATGCAGGCACAGAACTACGGAGCTCCGGCGACTAGGGTGAGGGCAAGAAGGGGTCAG
GCAACTGACCCACACAGCATCGCTGAGAGATTACGTAGAGAGAGAATTGCAGAGAGAATGAAGGCA
CTGCAGGAACTGGTTCCCAATGCCAACAAGACGGACAAGGCTTCAATGCTGGATGAGATCATTGAC
TATGTCAAATTCCTGCAGCTCCAAGTGAAGGTACTCAGCATGAGCAGGTTGGGCGGTGCCGCAGCT
GTTGCTCCCCTCGTTGCTGATATGTCCTCTGAGGGAGGTGGTGACTGTATACAGGCTAGTGGGACA
AGCGGCCCTACAGGAGGAAGGGCAACGAACGGAACACAAACAACCACATCAAACGACAGCCTCACA
GTGACGGAGCACCAGGTGGCCAAACTCATGGAGGAGGACATGGGTTCCGCCATGCAGTATCTTCAA
GGCAAAGGCTTATGCCTTATGCCCATCTCCCTCGCCACTGCCATCTCCACCACTACCTGCCACTCC
AGGAACCCCATGGTGGCTGCTGCCGCCGTCGCCGCCTCCAACATCAACAACGGCAGCCACACTCAC
CCACTCCTACCTAATTCCAACGCCGATGGCCCCTCCTCCCCCAGCATGTCCGTCCTGACCGTGCAG
TCAGCCACCATGGGGAACGGCCTGGCCGACGCGCCCGTAAAAGACGCTGCCTCCGTTTCCAAGCCC
TGA

**SEQ ID NO: 392, V. vinifera TA46156**
TCTTCCTTCTCACGGAAGCTGAGAGGCCGAGAGCTGAGACTTCACTCCAATCAAGGCCATCTCTTC
CATCTCCGAAACCCTAACCCACCTTCCGTACGGTTCCGACTCGTCCGAGTTTGAGTGATGGCGAGC
AATCCCTCGGAAGCTCCAGCGGACGATTTTCTCGAGCAAATCCTCGGAATTCCCACCTACCCCGCA
GCTGACCCTAATTTGGCTGCTAATGACGTGAATTTGGCCGCTCCTATGGTGCTTCAGCTCGGCTCC

**FIGURE 5 (continued)**

GGCGAAGGCTCTGGCCACATCGCCGGCGGGTACCAGGGAACCATGTTTCCGTTAGGGTTGAGGTTG
GAGCAGGGAAAGAGCAGCTTTCTGAAGCCCGAAGACGCGTCTGGCAGTGGCAAGCGCTTTCGTGAG
GAGGTTATTGATGGTAGAGCTTCTACCGTGAAAAATGCTTTCCATGGTCAACCAATGCAGGCTACC
GTTGCTGCAGCACCACATCCTCCTGCAATTCGCCCAAGGGTGCGGGCGAGACGTGGACAGGCCACA
GATCCACACAGCATTGCTGAGCGGTTACGCAGAGAAAGAATAGCAGAGAGAATTAGAGCACTGCAG
GAACTAGTTCCTAGTGTCAACAAGACGGATAGAGCTGCAATGCTTGATGAAATTGTGGATTATGTG
AAATTCCTAAGGCTCCAAGTAAAGGTCTTGAGCATGAGTAGATTGGGCGGAGCTGGTGCAGTTGCA
CCACTTGTAACAGACATTCCATTAGCATCAGTCGAGGAAGAAGCAAGTGAAGGGGGAAGAAATGAA
CCGGCATGGGAGAAATGGTCAAATGACGGTACAGAACGACAAGTTGCTAAGCTCATGGAAGAGAAT
GTTGGGGCTGCAATGCAGTTCCTTCAGTCCAAGGCACTCTGCATCATGCCCATCTCTCTCGCATCA
GCCATTTACCACTCTCAGCAGCCGGACACAACTCCACTGATCAAGCCCCAGACAAATCCCCCATCG
TAAAACCTCTCCCCACTCCC

**SEQ ID NO: 393, V. vinifera TA46194**
GGGAGCAAGGAAACCGAAGCAGGGCATTTCCCAAACATCTCTTACCGATTTATCGGACGACCTCTC
TGTGACCTCTTTCTCCAACAATTTGACCTTTTTGCTAAACCCGAAAACAATCCTCATCTCTTCTTT
GCTTCTCGCTGGAAAATCTATTTTCCGTTCCCGTCCATTCTCTAGCCATGGCCACCAACCCGCCCG
AAGGTTACGCCGATGATTTCCTGGAACAAATCCTCGCAATTCCCTCCTATCCTGGACACGACCCTA
ATATGGTGGGAACGGGTTCTACCATGGTGCTGCAGCTGAGTTCAGGCGACGGCTCAGGCCACGTCG
CCGGAGGTGGCTTCCAGGGGCCGGTCTTTCCGTTAGGTCTGAGCTTGGAGAGCGGGATTCCGGCGA
CACAGGTGGCGCCAGGAAGTGGCGAGCGGTTTCGAGATGACGTTGATGCCAGAGGTTCTTCAGGGA
GGAGCGAGAGAGAGTCGGTGCATTTGGATGGTTTGTTTCCGGCGTATGGACATGTACAGTCTCTCT
CCGTCCGACCCGCCGTTCCTCAAGTTCACCAGGCTTTTCATGGCCAGCCAACCCCTGTCCCAATTA
CTGCTGCGCCACACCCGCCAGCTATCCGCCCAAAGGTGCGTGCTCGGCGCGGACAAGCCACTGATC
CTCACAGCATTGCTGAGCGGCTACGTAGAGAGAGGATAGCAGAAAGAATGAAGGCTTTGCAAGAAC
TTGTCCCTAGCTCCAACAAGACGGATAGGGCTGCAATGCTTGATGAAATTGTGGACTATGTGAAGT
TCCTAAGGCTCCAAGTCAAGGTTTTGAGCATGAGTAGACTGGGAGGTGCTGGTGCAGTGGCACAAC
TCGTGGCTGACATCCCATTGCCAGCAGTTGAGGGAGAAACGGGTGAAGGTGGAAGCAACCAGCAAG
CCTGGGATAAATGGTCAAATGATGGCACAGAACGAGAAGTAGCAAAGCTCATGGAAGAAGATGTTG
GAGCTGCTATGCAGTTCCTCCAATCCAAAGCACTGTGCATCATGCCCATATCACTTGCCGCAGCAA
TATACCCTGCACACCAAACTGATACCCCCACGCTCATCAAGCCTGAGCCAAATGCTCCATCCTAGA
CCCCTCGAAAATGCACGTGCCTGTTGACAACTCCCATTGACCCCTAGTTGGCTAATCCCAAATTCC
TTAGTATCCTGAATCCATATCCTAAAGTGTTCCTCACTATCCCTTTTGGCAATGCAATCATAGTCA
GAAGCCATAGTCTTGCTTTTGCCTCTTTTTATTCTGAGTTTTGTCAAAGAAAGAGTGAATCATGCT
TTGGGAAAAAAGAAAAGGCACTTTCATCACTTCTTGCCATGCTCGAGTGAAGGGGGAAAATTTTCA
AAGCTATATTGATATGCTACTCCTTTTCATTTGTGCTGCTTGCTGGATGGCCAGAACACTTGTGGT
GGGGACTCTCTTTGGTTGATGCTGTGGTCTTGTAAAGCGGATGGGATGACTACGATTCCATGTAAT
TAAAATGAATTTGAAGAAAGCTGAAATGGATAATGGAAAGTCAATTTCAATGTTATATTGCATTAT
TTCCAGTCTTGGAGG

**SEQ ID NO: 394, Z. mays TA139285**
GTCGGCGGCAGCTGGAGAGCGAGGCCGAGCGACCAGTTACAGTTGCCGGCGGGCAAAGTGGGCCAC
TTGGTGCCAGGACATGGCCGGGCAACCGCCACCGCAGGGCCCCGAAGACGATTTCTTCGACCAGTT
CTTCTCCATGACGGCCGGCGGCTCCTACCCCGGCGCAACCGCGGGCGGCGGCCGCGCACCGGGTGA
CCAGCCGTTTTCCCTTGCGCTCAGCCTCGACGCCGCGGCCGCTGAGGCTTCCGGAAGCGGGAAGCA
CGCCGACGGTGGCAAGGCGGACCGGGAGGCTATACAGCTCCCTGGGCTCTTCCCGCCGGCGTTCGG
CGGCGGTGTGCAGCCACCCCACCTCCGCGCCACCCCGCCTACACAGGTGTTCCACGCGCAGCAGCC
GAAGCAAGGCGGTGCGGCAGTCGGGCCACAACCGCCGGCACCGAGGCCAAAGGTGCGGGCGCGGCG

**FIGURE 5** (continued)

TGGGCAGGCGACCGACCCCCACAGCATCGCGGAGAGGCTAAGAAGAGAGAGAATAGCAGAAAGGAT
GAGGGCCCTACAGGAATTGGTGCCCAATACAAACAAGACAGATAGAGCAGCTATGCTAGATGAGAT
CCTAGATTATGTGAAGTTTCTGAGGCTTCAAGTCAAGGTTCTAAGCATGAGCAGGCTGGGTGGTGC
TGGTGCTGTGGCACAGCTGGTTGCTGATATCCCACTTTCAGTTAAGGGGGAAGCAAGTGATAGTGG
GAGCAAACAGCAGATTTGGGAGAAGTGGTCAACGGACGGCACAGAAAGACAGGTCGCAAAGCTGAT
GGAAGAAGACATTGGGGCAGCGATGCAATTCCTCCAGTCCAAAGCACTCTGCATGATGCCCATCTC
GCTCGCCATGGCAATCTATGACACGCAACATTCGCAGGACGGACAACCAGTAAAACCAGAACCCAA
CACTCCTTCCTAGTATAGTGGCAGCAAGCGTAACTTGCATCCTTTCGGGGCTGTTAGCAGGCGTTA
ACCTACCAATCAAAGCGTAAGATAGAAATGCCGGCTTCGATTCACTAACAAGGAGGAGGCAGGGAA
AAAAGACCTAAAAGATCCTCTCCACTTGAAGTTGTACCAAATGGTATATGATATCGCCTTTCCCTT
TCCGATCTTTTGTTCAAAGGTGCTTTAATTGCAAGGATATGGTAAGCAATAACCTGCTCATGCTCC
TCCATCTTTCAATGCCCCCTATGAGTGTGAGTGATGGAGAGAGGGAGCTGCTAAAAGAGTCTGGCA
TGCTCTCATAATGTTTCTTTCCGGGTCTGTAGTGCACTAGCTAGCCATACACTTTGTGGTGGGGGG
GCTGCTGCTTTTCTGGTAGTGGGTGTATATGGACTAATCAAAAGGGACTGATGTATGAGTGTGTCT
GCGGCCTGCGCAAATGGTTGACAAAGTTTAGATACAGGATGAAATGGAAGATGATGTTGATAGCGA
TGTTCTTCCTTCTCCATTTTTCCAAAGTGGAGGTCCGAGGCAGGCTTTGGTCAATGGAGGCTGAGC
GTGCGTTTCAGCGTTGCTAGTGGATCTGCTATCTGCGTTGAATACTTTATTTATATGGATCCAC
ACTCTTGATAAGAACATTCG

**SEQ ID NO: 395, Z. mays TA126400**
GCTTCACACTTCCCCAGCAGAGCAGGCAAACCGCTCCCAGTCCCAGTCTCTCACCTGAGGTGACTG
GCGTCTCTTGTCGCAAGCCGCCAGAAGCTTCGGCCCCTGACGCGTGGGGGCCCCGTCCGCCCCCGC
CCCCGTCCCCACGTTGCCGGCCATGGCGGGGCAGCCACCGCCGTCCGGGGCCTCCGAGGACGACTT
CCTCGAGCACTTCTTCGCCTTCCCCTCCGCGGCCTCCGCCGGCGCTGCCGGGGGCCACGCGGGCGC
CGGTGTTGGCGGGGACCACCCCTTCCCCCTCGCCCTCAGCCTCGACGCCGCCGCCGAGGCCAAGCC
GGACCGTGATCCCGTGCAGCTCGCCGGCCTCTTCCCGCCGGTGTTCGCTGGCGCCGGCGGCGTGCA
CCAGCCGCACCTCCGCGGCCCACCGCCTCCGCAGATGTTCCAGGCGCAGCCGAAGCCGGGCGAGGG
AGGCATGGCGCCGCAGCCGCCAGCCCCGCGGCCCAAGGTGCGCGCGCGGCGGGGGCAGGCCACCGA
TCCCCACAGTATTGCGGAGAGGCTAAGGAGAGAGAGAATTGCAGAAAGGATGAGGGCATTGCAGGA
ATTAGTCCCCAACACAAACAAGACAGATAGGGCAGCCATGCTAGATGAGATCCTTGATTATGTGAA
GTTTCTTAGGCTTCAAGTAAAGGTTCTGAGTATGAGCAGACTGGGCGGTGCTGGCGCGGTTGCGCA
GCTGGTTGCTGATATTCCACTCTCAGTTAAGGGCGAGGCAGGCGACGGCGGGGGGGCGCCGCAGCA
GCAGCAGCAGCAGCACGTGTGGGAGAAGTGGTCGACGGACGGCACGGAGAAGCAGGTGGCGAAGCT
GATGGAGGAGGACATCGGGGCGGCAATGCAGTTCCTCCAGTCCAAGGCGCTGTGCATGATGCCGGT
CTCGCTCGCGATGGCGATTTACGACACCCAGCACCCCTGGACGGCCACGGCCACTCGCTGAAGCC
CGAGCCCAACGCGTCATCCTAGTACAGTAACGTCGTCCTTAGCGTGCCTTCCAATTCCAACACCCA
CCCCCCTTCTCCCTAAAAAGTACTGTTAGGCGCCGATATTATCCGGAAGAAAAAAAGAAGTGTATG
ATATACCGAGCGTGCGTTTCCAGTGTGCTGACCCGTGGGATAGGAAAGGACCGAAAATTGTTGGTA
TATGATGATTATGTAGCCTCCTGTTTGCCTGTTCTGTTTCAAATCAAAGCAATGCTCATGCATCCA
GGTTTC

**FIGURE 5 (continued)**

## REFERENCES CITED IN THE DESCRIPTION

**Patent documents cited in the description**

- US 5811238 A
- US 6395547 A
- WO 2004070039 A
- WO 2004065596 A
- US 4962028 A
- WO 0114572 A
- WO 9514098 A
- WO 9412015 A
- US 5401836 A
- US 20050044585 A
- EP 99106056 A
- US 5565350 A, Kmiec
- WO 9322443 A, Zarling
- WO 9853083 A, Grierson
- WO 9953050 A, Waterhouse
- US 4987071 A, Cech
- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- WO 0015815 A
- EP 1198985 A1
- US 5164310 A

**Non-patent literature cited in the description**

- **WANG et al.** *Planta,* 2003, vol. 218, 1-14
- **TOLEDO-ORTIZ et al.** *Plant Cell,* 2003, vol. 15, 1749-1770
- **BAILEY et al.** *Plant Cell,* 2003, vol. 15, 2497-2501
- **LI et al.** *Plant Physiol.,* 2006, vol. 141, 1167-1184
- **YI et al.** *Plant Physiol.,* vol. 138, 2087-2096
- **TERPE.** *Appl. Microbiol. Biotechnol.,* 2003, vol. 60, 523-533
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- *Plant Mol Biol.,* January 1995, vol. 27 (2), 237-48
- **MUDGE et al.** *Plant J.,* 2002, vol. 31, 341
- **NITZ et al.** *Plant Sci,* 2001, vol. 161 (2), 337-346
- **TINGEY et al.** *EMBO J.,* 1987, vol. 6, 1
- **VAN DER ZAAL et al.** *Plant Mol. Biol.,* 1991, vol. 16, 983
- **OPPENHEIMER et al.** *Gene,* 1988, vol. 63, 87
- **CONKLING et al.** *Plant Physiol.,* 1990, vol. 93, 1203
- **SUZUKI et al.** *Plant Mol. Biol.,* 1993, vol. 21, 109-119

- **BAUMBERGER et al.** *Genes & Dev.,* 2001, vol. 15, 1128
- **LAUTER et al.** *PNAS,* 1996, vol. 3, 8139
- **LIU et al.** *Plant Mol. Biol.,* 1991, vol. 153, 386-395
- **DOWNEY et al.** *J. Biol. Chem.,* 2000, vol. 275, 39420
- **W SONG.** *PhD Thesis,* 1997
- **WANG et al.** *Plant Sci.,* 2002, vol. 163, 273
- **DIENER et al.** *Plant Cell,* 2001, vol. 13, 1625
- **QUESADA et al.** *Plant Mol. Biol.,* 1997, vol. 34, 265
- **QING ; QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* 1986, vol. 208, 15-22
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **WAGNER ; KOHOM.** *Plant Cell,* 2001, vol. 13 (2), 303-318
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62

- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **TRIBBLE et al.** *J. Biol. Chem.,* 2000, vol. 275, 22255-22267
- **VELMURUGAN et al.** *J. Cell Biol.,* 2000, vol. 149, 553-566
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- **B. JENES et al.** Techniques for Gene Transfer, in: Transgenic Plants, Vol. 1, Engineering and Utilization. Academic Press, 1993, vol. 1, 128-143
- *Potrykus Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- **F.F. WHITE.** Vectors for Gene Transfer in Higher Plants; in Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- **FELDMANN K.** Methods in Arabidopsis Research. Word Scientific, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **TERADA.** *Curr Opin Biotech,* 2004, vol. 15 (2), 132-8
- **MILLER et al.** *Nature Biotechnol.,* 2007, vol. 25, 778-785
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* 2003, vol. 31, 315-318
- A generalized profile syntax for biomolecular sequences motifs and its function in automatic sequence interpretation. **BUCHER ; BAIROCH.** Proceedings 2nd International Conference on Intelligent Systems for Molecular Biology. AAAI Press, 1994, 53-61
- **HULO et al.** *Nucl. Acids. Res.,* 2004, vol. 32, D134-D137
- **BATEMAN et al.** *Nucleic Acids Research,* 2002, vol. 30 (1), 276-280
- **GASTEIGER et al.** ExPASy: the proteomics server for in-depth protein knowledge and analysis. *Nucleic Acids Res.,* 2003, vol. 31, 3784-3788
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** *BMC Bioinformatics,* 10 July 2003, vol. 4, 29
- **SMITH TF ; WATERMAN MS.** *J. Mol. Biol,* 1981, vol. 147 (1), 195-7
- Current Protocols in Molecular Biology
- **RABBANI et al.** *Plant Physiol,* 2003, vol. 133, 1755-1767

- **S.D. KUNG ; R. WU.** *Potrykus or Höfgen and Willmitzer*
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BEMATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* 1989, vol. 17, 6795-6807
- **HUSON et al.** *BMC Bioinformatics,* 2007
- **SAMBROOK.** Molecular Cloning: a laboratory manual. Cold Spring Harbor Laboratory Press, 2001
- **AUSUBEL et al.** Current Protocols in Molecular Biology, Current Protocols. 1994, vol. 1, 2
- **R.D.D. CROY.** Plant Molecular Biology Labfax. BIOS Scientific Publications Ltd (UK, 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **THOMPSON et al.** *Nucleic Acids Res,* 1997, vol. 25, 4876-4882
- **CHENNA et al.** *Nucleic Acids Res,* 2003, vol. 31, 3497-3500
- *BMC Bioinformatics,* 2003, vol. 4, 29
- **PARK ; KANEHISA.** *Bioinformatics,* 2003, vol. 19, 1656-1663
- **XUE.** *Plant J.,* 2005, vol. 41, 638-649
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659